# EUROPEAN PATENT APPLICATION

(11) **EP 4 725 508 A1**
(43) Date of publication of application: **15.04.2026**
(21) Application number: 25819212.9
(22) Date of filing: 04.06.2025
(51) Int. Cl.: A61K 47/68, C07K 16/44, A61K 39/395, A61P 35/00

(54) **LIGAND-DRUG CONJUGATE HAVING FREE PAYLOAD RECOVERY UNIT**

(30) Priority: 05.06.2024 CN 202410722924; 06.06.2024 CN 202410729765
(71) Applicant: RemeGen Co., Ltd., Shandong 264006 (CN)
(72) Inventor: FANG, Jianmin, Shandong 264006 (CN); WANG, Lili, Shandong 264006 (CN); JIANG, Jing, Shandong 264006 (CN); LI, Dong, Shandong 264006 (CN); ZHANG, Wei, Shandong 264006 (CN); LI, Yuanhao, Shandong 264006 (CN); WANG, Zhulun, Shandong 264006 (CN); MIN, Xiaoshan, Shandong 264006 (CN); WANG, Ling, Shandong 264006 (CN); XIN, Yinghao, Shandong 264006 (CN); HE, Chenglin, Shandong 264006 (CN); XU, Menglong, Shandong 264006 (CN)
(74) Representative: Maiwald GmbH
(86) International application number: PCT/CN2025/098941
(87) International publication number: WO 2025/252100

(57) **Abstract**

A ligand-drug conjugate having a free payload recovery unit, and a use thereof in preventing or treating diseases. The ligand-drug conjugate can remove a free payload by means of a connected recovery unit capable of binding to the free payload, significantly improving the safety of medication. Moreover, by means of recovering the free payload, the payload carried by the ligand-drug conjugate as a whole can be increased, thereby compensating for activity loss caused by payload shedding. Therefore, by means of introducing the recovery unit, free payload is recovered, which can significantly increase the safety and effectiveness of the ligand-drug conjugate, and expand a treatment window.

## Description

### FIELD

The present application relates to the field of biomedicine, and in particular to a ligand-drug conjugate with a free payload recovery unit and uses thereof.

### BACKGROUND

Antibody-Drug Conjugates (ADCs) are a new type of biologic drug produced by conjugating a monoclonal antibody with a cytotoxic drug (also referred to as payload) through a linker (Hafeez U, Parakh S, Gan HK, Scott AM. Antibody-Drug Conjugates for Cancer Therapy. Molecules. 2020 Oct 16;25(20):4764.). By targeting specific antigens, ADCs can effectively penetrate tumor tissues and be internalized by tumor cells to enter lysosomes and release effector molecules. They fully combine the characteristics of strong targeting selectivity of monoclonal antibodies and high activity of cytotoxic drugs, and not only can specifically target cytotoxic drugs to tumor cells but also possess effects such as sustained release and improved antitumor activity of drugs, and are one of the key development directions of tumor therapy.

The development history of ADC technology is roughly divided into 4 stages: The first generation of ADCs belongs to the early exploration stage. In this stage, understanding of key issues such as payload activity, release mode, and linker properties was basically blank. It adopted a strategy of murine antibody + high activity payload + unstable linker + random conjugation, resulting in high immunogenicity, a mixture of components with different DAR values, unstable metabolism, a narrow safety window, and high challenges in drugability; a representative variety is Mylotarg. The second generation of ADCs enriched the types of linkers, adopting a strategy of humanized antibody + high activity payload + stable linker + random conjugation, resulting in low immunogenicity, significantly improved metabolic stability, and an improved therapeutic index; representative varieties are Kadcyla and Adcetris. Compared with the first generation of ADCs, the second generation of ADCs showed good clinical efficacy and safety. The third generation learned from the experience of early ADCs: heterogeneity and instability are important factors affecting the efficacy and toxicity of ADCs in vivo, and components with high DAR values have poor stability in vivo, affecting the therapeutic window of ADCs. Therefore, site-specific conjugation and low DAR value technologies were adopted, but problems such as insufficient payload release and high drug resistance still existed. The fourth generation of ADC technology adopts a strategy of high DAR values (4 to 8) and medium-to-low activity payloads. The payloads have a high bystander effect, and the most important feature is that they have good efficacy on both tumors with low expression of surface antigens and tumors with high expression of antigens; representative varieties are Enhertu and Trodelvy. Since the first ADC drug, gemtuzumab ozogamicin (Mylotarg), was approved for marketing by the US FDA in 2009, as of May 2024, a total of 15 ADC drugs have been approved for marketing globally (see Table 1 for details), and a large number of ADC drugs are in the clinical research stage or early research stage.

**Table 1. Brief information of globally approved ADC drugs**

| No. | Trade name | ADC name | R&D | Target | Linker | Payload |
|---|---|---|---|---|---|---|
| 1 | Mylotarg | Gemtuzumab ozogamicin (GO) | Pfizer | CD33 | Cleavable hydrazone bond | Calicheamicin |
| 2 | Adcetris | Brentuximab vedotin (SGN-35) | Seagen/Takeda Pharmaceuticals | CD30 | Cleavable Dipeptide | MMAE |
| 3 | Kadcyla | Trastuzumab emtansine (T-DM1) | Roche/ ImmunoGen | HER2 | Non-cleavable thioether | DM1 |
| 4 | Besponsa | Inotuzumab ozogamicin | Pfizer/Celltech | CD22 | Cleavable hydrazone bond | Calicheamicin |
| 5 | Lumoxiti | Moxetumomab pasudotox | AstraZeneca | CD22 | Cleavable mc-VC-PABC | PE38 |
| 6 | Polivy | Polatuzumab vedotin | Roche | CD79B | Cleavable Dipeptide | MMAE |
| 7 | Padcev | Enfortumab vedotin | Seagen/Astellas Pharma | NECTIN-4 | Cleavable mc-VC-PABC | MMAE |
| 8 | Enhertu | Fam-trastuzumab deruxtecan (DS-8201) | Daiichi Sankyo/AstraZeneca | HER2 | Cleavable GGFG Tetrapeptide | Dxd |
| 9 | Trodelvy | Sacituzumab govitecan | Immunomedics | TROP-2 | Cleavable CL2A | SN38 |
| 10 | Blenrep | Belantamab mafodotin | GSK | BCMA | Non-cleavable maleimide | MMAF |
| 11 | Akalux | Cetuximab saratolacan | RakutenMedical | EGFR | Cleavable structure | IRDye700DX |
| 12 | Zynlonta | Loncastuximab tesirine-lpyl | ADC Therapeutics | CD19 | Cleavable Dipeptide | PBD(SG3199) |
| 13 | Aidixi | Disitamab vedotin | RemeGen | HER2 | Cleavable mc-VC-PABC | MMAE |
| 14 | Tivdak | Tisotumab vedotin-tftv | Genmab/ Seagen | TF | Cleavable mc-VC-PABC | MMAE |
| 15 | Elahere | Mirvetuximab soravtansine-gyxn | Immunogen/ Huadong Medicine | FRA | Cleavable SPDB | DM4 |

The activity and physicochemical properties of the payload will directly affect the antitumor efficacy of the ADC drug. There are mainly two classes of payloads in existing approved ADCs: tubulin inhibitors and DNA inhibitors, wherein DNA inhibitors are further divided into DNA damaging agents and topoisomerase inhibitors. As shown in Table 1, tubulin inhibitors are currently the most widely applied class of payloads in ADC drug development; the payloads of 8 out of 15 marketed ADC drugs are tubulin inhibitors (auristatins (MMAE), maytansinoids (e.g., DM1, DM4)). In addition, DNA damaging agents including calicheamicins, PBDs, and camptothecin derivatives (SN38, DXD) are also widely used as payloads in the construction of ADC drugs. Representative examples among currently marketed ADC drugs include Mylotarg, Besponsa, Enhertu, Trodelvy, and Zynlonta (Wang Z, Li H, Gou L, Li W, Wang Y. Antibody-drug conjugates: Recent advances in payloads. Acta Pharm Sin B. 2023 Oct;13(10):4025-4059).

Generally speaking, the presence of lysine and cysteine on an antibody provides reaction sites for payload conjugation. Early ADC drugs were basically randomly conjugated through lysine or cysteine residues. However, since approximately 40 amino acid residues of an antibody can usually react, small molecule payloads with varying Drug-to-Antibody Ratios (DARs, 0 to 8) may attach to the antibody, resulting in a wide distribution of DAR values. Since IgG1 antibodies have both interchain disulfide bonds and intrachain disulfide bonds, the interchain disulfide bonds are exposed on the exterior of the antibody and are easily reduced to expose free cysteine residues, thereby providing available sites for the conjugation of linker-payloads to the antibody. In addition, due to the limited number of binding sites and the unique reactivity of thiol groups, using cysteine as a connection site helps to reduce the heterogeneity of ADCs, therefore the most commonly used conjugation mode to date is reaction based on cysteine. However, whether it is conjugation via lysine or cysteine residues, due to insufficient stability, premature release of the payload may occur, thereby leading to off-target toxicity (Fu Z, Li S, Han S, Shi C, Zhang Y. Antibody drug conjugate: the "biological missile" for targeted cancer therapy. Signal Transduct Target Ther. 2022 Mar 22;7(1):93).

Although the development of new ADC drugs has achieved unprecedented success, there are still many challenges in the process of developing ADCs, including pharmacokinetic complexity, insufficient payload release, drug resistance, and inevitable side effects during treatment. The most common severe side effects (Grade 3 or higher) are hematological toxicities, including neutropenia, thrombocytopenia, leukopenia, and anemia. Hematological toxicities, as well as hepatotoxicity and gastrointestinal reactions, may be related to the premature release of cytotoxic payloads into blood circulation. This is consistent with conventional chemotherapy drugs that primarily affect those healthy cells which proliferate rapidly.

In addition, in the drug marketing stage, although some ADCs have demonstrated sufficient efficacy and safety, almost all ADCs exhibit certain toxicities during clinical application. As the clinical application of ADC drugs deepens, the clinical application dosages of some drugs have been reduced or medication has been stopped because they exhibited certain toxic side effects. It is estimated that after an ADC drug enters the human body, only about 0.1% of the drug amount is delivered to the targeted lesion cell population, and the vast majority of the drug decomposes "off-site" within non-targeted healthy cells, which may lead to unnecessary toxicity. Toxicity of ADCs not reaching the target lesion or target tissue can be classified as "on-target" or "off-target", and off-target toxicity is mainly caused by free toxins shed from the ADC. Intact ADCs may enter normal cells via non-specific endocytosis, or internalization by binding to target antigens or Fc/C-type lectin receptors. Payloads dissociated from ADC molecules in extracellular fluid or payloads released by other targeted/non-targeted apoptotic cells can enter normal cells through passive diffusion across the cell membrane or non-specific endocytosis. Off-target payload delivery is a key driver of ADC tolerability and ultimately determines the recommended dose for patients (Nguyen TD, Bordeau BM, Balthasar JP. Mechanisms of ADC Toxicity and Strategies to Increase ADC Tolerability. Cancers (Basel). 2023 Jan 24;15(3):713.).

To improve the tolerability of ADCs, Balthasar et al. designed neutralizing antibody fragments (Fabs or sdAbs) to bind non-conjugated circulating payload molecules, aiming to reduce the amount of cytotoxin reaching normal tissues or cells, thereby reducing the degree of off-target toxicity in subjects receiving ADCs (Bordeau BM, Nguyen TD, Polli JR, Chen P, Balthasar JP. Payload-Binding Fab Fragments Increase the Therapeutic Index of MMAE Antibody-Drug Conjugates. Mol Cancer Ther. 2023 Apr 3;22(4):459-470., Nguyen TD, Bordeau BM, Balthasar JP. Use of Payload Binding Selectivity Enhancers to Improve Therapeutic Index of Maytansinoid-Antibody-Drug Conjugates. Mol Cancer Ther. 2023 Nov 1;22(11):1332-1342.). In this method, the ADC and the neutralizing antibody fragment need to be co-administered to the subject, resulting in high production and medication costs; in addition, the dosing ratio and administration mode of the ADC and the neutralizing antibody fragment also need to be explored, increasing the complexity of dosing regimen development. Furthermore, WO2021113740A1 and WO2024044709A2 also disclose the above methods and disclose a variety of antibodies targeting small molecule toxins, including single domain antibodies and oligopeptides, such as murine antibody MA24E2 which binds only free MMAE but not MMAE on MMAE-ADC, humanized antibodies ABC3315 and ABC3320 (Heavy chain: SEQ ID NO: 163; Light chain: SEQ ID NO: 164); anti-MMAE single domain antibody MC7; anti-camptothecin toxin murine antibody 8C2, humanized 8C2 Fab, humanized 8C2 VHH; anti-camptothecin toxin single domain antibody TF7 and single domain antibody TA2, anti-calicheamicin oligopeptides C2 and C9, anti-calicheamicin single domain antibody CG4, and anti-maytansinoid single domain antibodies DF3A2, DF3A4, DF4B12, DMFH1, and DMOH9. However, it has been found that although the use of some antibodies can reduce free small molecule toxins, it may also increase the cytotoxicity of antibody-drug conjugates for unknown reasons, such as 8C2 antibody, MC7 antibody, etc.

The present disclosure creatively connects a recovery unit capable of recovering a free payload released from the ligand-drug conjugate directly in the ligand-drug conjugate, which can clear free payloads in the blood and reduce off-target toxicity; in addition, by recovering the free payload, the payload carried by the ligand-drug conjugate can usually be increased overall, compensating for the loss of activity caused by payload shedding. Therefore, by introducing the recovery unit to achieve recovery of the free payload, the safety of the ligand-drug conjugate can be significantly increased. Furthermore, the ADC dosage can be further increased, thereby correspondingly improving the effectiveness of ADC treatment and expanding the therapeutic window.

In addition, the risk of increased cytotoxicity of the antibody-drug conjugate with unknown causes appearing in the above-mentioned combination therapy (WO2021113740A1) is also effectively avoided. On the other hand, relative to combination therapy using two macromolecular products, the present disclosure can significantly reduce production costs and clinical development costs, simplify medication, reduce drug risks, and improve medication convenience for subjects.

The recovery unit can be obtained by using existing technologies to screen for antibodies, peptides, or aptamers, etc., targeting the free payload. The development of related screening technologies is becoming increasingly mature, mainly screening for antibodies or antigen-binding fragments and aptamers, etc., with high affinity and high specificity binding to small molecule targets through specific methods for treatment, diagnosis, or research. Main technologies include hybridoma technology, nanobody screening technology, computer-aided design (Al/structural biology), phage display technology, yeast display technology, single B cell cloning technology, microfluidic technology, surface plasmon resonance assisted screening, and Systematic Evolution of Ligands by Exponential Enrichment (SELEX), etc. Among them, hybridoma technology is the most traditional screening method, the main principle of which is fusing B cells of an immunized animal (such as a mouse) with myeloma cells to form hybridoma cells and screening for clones secreting the target antibody. The advantage of phage display technology is that no animal immunization is required, and screening can be performed against toxic small molecules or targets that are difficult to immunize. The present disclosure utilized the above-mentioned various technologies to screen for antibodies, peptides, or aptamers targeting small molecule drugs.

All references cited herein, including patent applications, patent publications, and UniProtKB/Swiss-Prot accession numbers, are incorporated herein by reference in their entirety, meaning that each individual reference is expressly and individually indicated to be incorporated herein by reference.

### SUMMARY

The present disclosure provides a ligand-drug conjugate with a free payload recovery unit and use thereof in preventing or treating a tumor.

In a specific aspect, the present disclosure provides a ligand-drug conjugate comprising a ligand unit targeting a tumor antigen or a non-tumor antigen, a conjugated payload unit, and a recovery unit capable of binding to a free payload released from the ligand-drug conjugate.

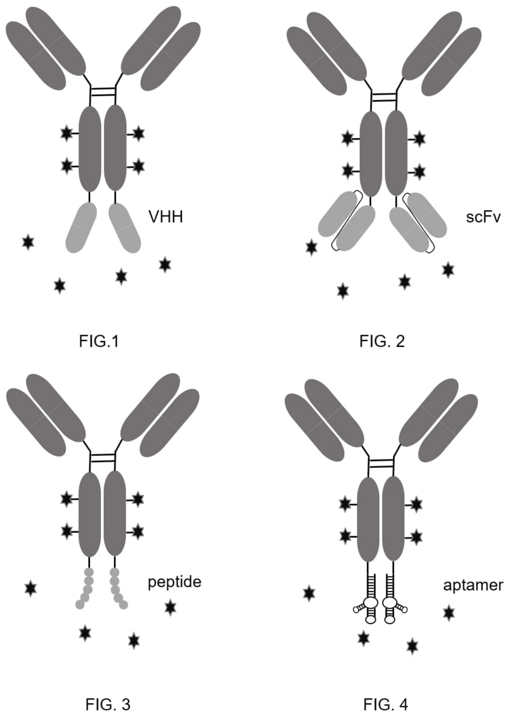

In some embodiments, the ligand-drug conjugate comprises the ligand unit in an IgG form and the recovery unit in a VHH form, wherein the recovery unit is connected directly or indirectly to the C-terminus of the ligand unit; preferably, a structure of the ligand unit-recovery unit from N-terminus to C-terminus is as follows: (1) VL-CL; (2) VH-CH1-CH2-CH3-VHH; (3) VH-CH1-CH2-CH3-VHH; and (4) VL-CL, wherein a hexagram symbol represents a conjugated or free payload, and a schematic diagram is shown in Figure 1.

In some embodiments, the ligand-drug conjugate comprises the ligand unit in an IgG form and the recovery unit in an scFv form, wherein the recovery unit is connected directly or indirectly to the C-terminus of the ligand unit; preferably, a structure of the ligand unit-recovery unit from N-terminus to C-terminus is as follows: (1) VL-CL; (2) VH-CH1-CH2-CH3-scFv; (3) VH-CH1-CH2-CH3-scFv; and (4) VL-CL, wherein the hexagram symbol represents the conjugated or free payload, and a schematic diagram is shown in Figure 2.

In some embodiments, the ligand-drug conjugate comprises the ligand unit in an IgG form and the recovery unit in a peptide form, wherein the recovery unit is connected directly or indirectly to the C-terminus of the ligand unit; preferably, a structure of the ligand unit-recovery unit from N-terminus to C-terminus is as follows: (1) VL-CL; (2) VH-CH1-CH2-CH3-peptide; (3) VH-CH1-CH2-CH3-peptide; and (4) VL-CL, wherein the hexagram symbol represents the conjugated or free payload, and a schematic diagram is shown in Figure 3.

In some embodiments, the ligand-drug conjugate comprises the ligand unit in an IgG form and the recovery unit in an aptamer form, wherein the recovery unit is connected directly or indirectly to the C-terminus of the ligand unit; preferably, a structure of the ligand unit-recovery unit from N-terminus to C-terminus is as follows: (1) VL-CL; (2) VH-CH1-CH2-CH3-aptamer; (3) VH-CH1-CH2-CH3-aptamer; and (4) VL-CL, wherein the hexagram symbol represents the conjugated or free payload, and a schematic diagram is shown in Figure 4.

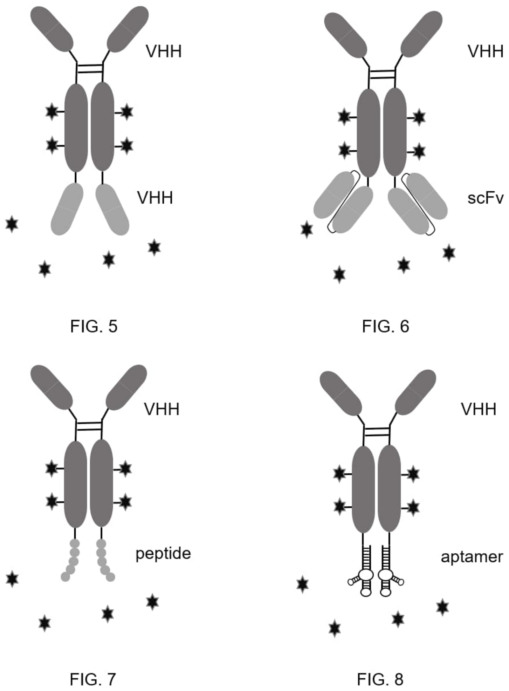

In some embodiments, the ligand-drug conjugate comprises the ligand unit in a VHH+Fc form and the recovery unit in a VHH form, wherein the recovery unit is connected directly or indirectly to the C-terminus of the ligand unit; preferably, a structure of the ligand unit-recovery unit from N-terminus to C-terminus is as follows: (1) VHH ligand-CH2-CH3-VHH recovery; and (2) VHH ligand-CH2-CH3-VHH recovery; wherein the hexagram symbol represents the conjugated or free payload, and a schematic diagram is shown in Figure 5.

In some embodiments, the ligand-drug conjugate comprises the ligand unit in a VHH+Fc form and the recovery unit in an scFv form, wherein the recovery unit is connected directly or indirectly to the C-terminus of the ligand unit; preferably, a structure of the ligand unit-recovery unit from N-terminus to C-terminus is as follows: (1) VHH-CH2-CH3-scFv; and (2) VHH-CH2-CH3-scFv; wherein the hexagram symbol represents the conjugated or free payload, and a schematic diagram is shown in Figure 6.

In some embodiments, the ligand-drug conjugate comprises the ligand unit in a VHH+Fc form and the recovery unit in a peptide form, wherein the recovery unit is connected directly or indirectly to the C-terminus of the ligand unit; preferably, a structure of the ligand unit-recovery unit from N-terminus to C-terminus is as follows: (1) VHH-CH2-CH3-peptide; and (2) VHH-CH2-CH3-peptide; wherein the hexagram symbol represents the conjugated or free payload, and a schematic diagram is shown in Figure 7.

In some embodiments, the ligand-drug conjugate comprises the ligand unit in a VHH+Fc form and the recovery unit in an aptamer form, wherein the recovery unit is connected directly or indirectly to the C-terminus of the ligand unit; preferably, a structure of the ligand unit-recovery unit from N-terminus to C-terminus is as follows: (1) VHH-CH2-CH3-aptamer; and (2) VHH-CH2-CH3-aptamer; wherein the hexagram symbol represents the conjugated or free payload, and a schematic diagram is shown in Figure 8.

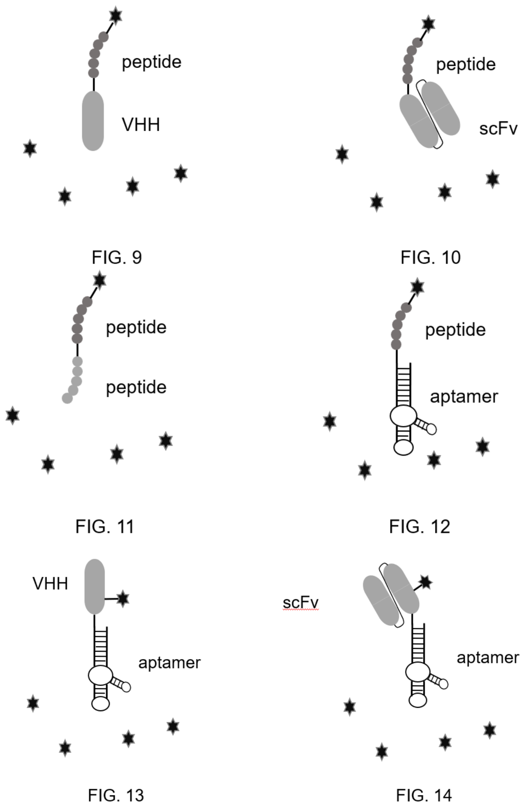

In some embodiments, the ligand-drug conjugate comprises the ligand unit in a peptide form and the recovery unit in a VHH form, wherein the recovery unit is connected directly or indirectly to the C-terminus of the ligand unit; preferably, a structure of the ligand unit-recovery unit from N-terminus to C-terminus is as follows: peptide-VHH; wherein the hexagram symbol represents the conjugated or free payload, and a schematic diagram is shown in Figure 9.

In some embodiments, the ligand-drug conjugate comprises the ligand unit in a peptide form and the recovery unit in an scFv form, wherein the recovery unit is connected directly or indirectly to the C-terminus of the ligand unit; preferably, a structure of the ligand unit-recovery unit from N-terminus to C-terminus is as follows: peptide-scFv; wherein the hexagram symbol represents the conjugated or free payload, and a schematic diagram is shown in Figure 10.

In some embodiments, the ligand-drug conjugate comprises the ligand unit in a peptide form and the recovery unit in a peptide form, wherein the recovery unit is connected directly or indirectly to the C-terminus of the ligand unit; preferably, a structure of the ligand unit-recovery unit from N-terminus to C-terminus is as follows: peptide ligand-peptide recovery; wherein the hexagram symbol represents the conjugated or free payload, and a schematic diagram is shown in Figure 11.

In some embodiments, the ligand-drug conjugate comprises the ligand unit in a peptide form and the recovery unit in an aptamer form, wherein the recovery unit is connected directly or indirectly to the C-terminus of the ligand unit; preferably, a structure of the ligand unit-recovery unit from N-terminus to C-terminus is as follows: peptide-aptamer; wherein the hexagram symbol represents the conjugated or free payload, and a schematic diagram is shown in Figure 12.

In some embodiments, the ligand-drug conjugate comprises the ligand unit in a VHH form and the recovery unit in an aptamer form, wherein the recovery unit is connected directly or indirectly to the C-terminus of the ligand unit; preferably, a structure of the ligand unit-recovery unit from N-terminus to C-terminus is as follows: VHH-aptamer; wherein the hexagram symbol represents the conjugated or free payload, and a schematic diagram is shown in Figure 13.

In some embodiments, the ligand-drug conjugate comprises the ligand unit in an scFv form and the recovery unit in an aptamer form, wherein the recovery unit is connected directly or indirectly to the C-terminus of the ligand unit; preferably, a structure of the ligand unit-recovery unit from N-terminus to C-terminus is as follows: scFv-aptamer; wherein the hexagram symbol represents the conjugated or free payload, and a schematic diagram is shown in Figure 14.

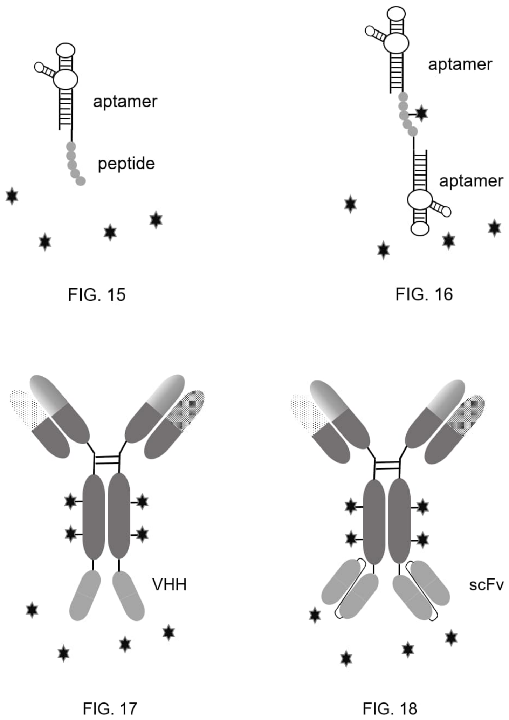

In some embodiments, the ligand-drug conjugate comprises the ligand unit in an aptamer form and the recovery unit in a peptide form, wherein the recovery unit is connected directly or indirectly to the C-terminus of the ligand unit; preferably, a structure of the ligand unit-recovery unit from N-terminus to C-terminus is as follows: aptamer-peptide; wherein the hexagram symbol represents the conjugated or free payload, and a schematic diagram is shown in Figure 15.

In some embodiments, the ligand-drug conjugate comprises the ligand unit in an aptamer form and the recovery unit also in an aptamer form, wherein the recovery unit is connected directly or indirectly to the C-terminus of the ligand unit; preferably, a structure of the ligand unit-recovery unit from N-terminus to C-terminus is as follows: aptamer_{ligand}-aptamer_{recovery}; wherein the hexagram symbol represents the conjugated or free payload, and a schematic diagram is shown in Figure 16.

In some embodiments, the ligand-drug conjugate comprises the ligand unit in a bispecific antibody form and the recovery unit in a VHH form, wherein the recovery unit is connected directly or indirectly to the C-terminus of the ligand unit; preferably, a structure of the ligand unit-recovery unit from N-terminus to C-terminus is as follows: (1) VL-CL; (2) VH-CH1-CH2-CH3-VHH; (3) VH-CH1-CH2-CH3-VHH; and (4) VL-CL, wherein a VH and a VL of (1) and (2) form a binding site specifically binding to a first antigen or a first antigen epitope, a VH and a VL of (3) and (4) form a binding site specifically binding to a second antigen or a second antigen epitope, the hexagram symbol represents the conjugated or free payload, and a schematic diagram is shown in Figure 17.

In some embodiments, the ligand-drug conjugate comprises the ligand unit in a bispecific antibody form and the recovery unit in an scFv form, wherein the recovery unit is connected directly or indirectly to the C-terminus of the ligand unit; preferably, a structure of the ligand unit-recovery unit from N-terminus to C-terminus is as follows: (1) VL-CL; (2) VH-CH1-CH2-CH3-scFv; (3) VH-CH1-CH2-CH3-scFv; and (4) VL-CL, wherein a VH and a VL of (1) and (2) form a binding site specifically binding to a first antigen or a first antigen epitope, a VH and a VL of (3) and (4) form a binding site specifically binding to a second antigen or a second antigen epitope, the hexagram symbol represents the conjugated or free payload, and a schematic diagram is shown in Figure 18.

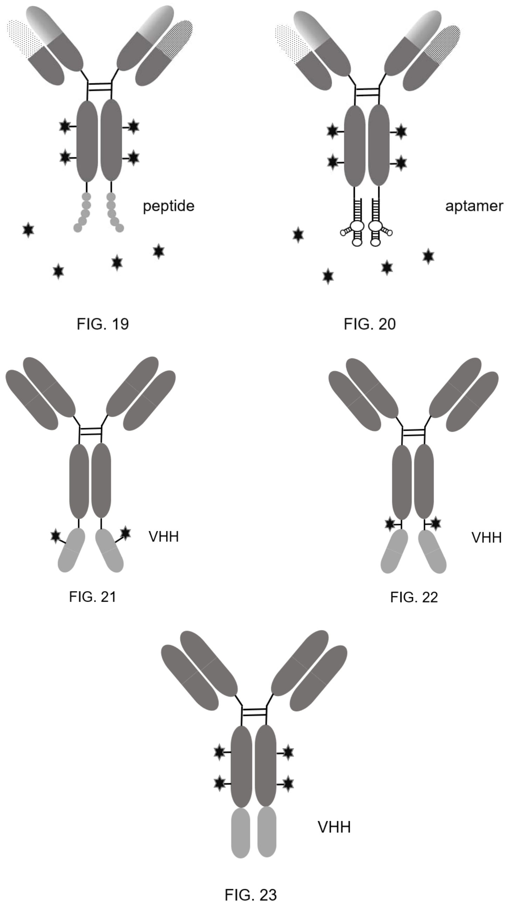

In some embodiments, the ligand-drug conjugate comprises the ligand unit in a bispecific antibody form and the recovery unit in a peptide form, wherein the recovery unit is connected directly or indirectly to the C-terminus of the ligand unit; preferably, a structure of the ligand unit-recovery unit from N-terminus to C-terminus is as follows: (1) VL-CL; (2) VH-CH1-CH2-CH3-peptide; (3) VH-CH1-CH2-CH3-peptide; and (4) VL-CL, wherein a VH and a VL of (1) and (2) form a binding site specifically binding to a first antigen or a first antigen epitope, a VH and a VL of (3) and (4) form a binding site specifically binding to a second antigen or a second antigen epitope, the hexagram symbol represents the conjugated or free payload, and a schematic diagram is shown in Figure 19.

In some embodiments, the ligand-drug conjugate comprises the ligand unit in a bispecific antibody form and the recovery unit in an aptamer form, wherein the recovery unit is connected directly or indirectly to the C-terminus of the ligand unit; preferably, a structure of the ligand unit-recovery unit from N-terminus to C-terminus is as follows: (1) VL-CL; (2) VH-CH1-CH2-CH3-aptamer; (3) VH-CH1-CH2-CH3-aptamer; and (4) VL-CL, wherein a VH and a VL of (1) and (2) form a binding site specifically binding to a first antigen or a first antigen epitope, a VH and a VL of (3) and (4) form a binding site specifically binding to a second antigen or a second antigen epitope, the hexagram symbol represents the conjugated or free payload, and a schematic diagram is shown in Figure 20.

In some embodiments, the ligand-drug conjugate comprises the ligand unit in an IgG form and the recovery unit in a VHH form, wherein the recovery unit is connected directly or indirectly to the C-terminus of the ligand unit; preferably, the payload is conjugated to the VHH, a structure of the ligand unit-recovery unit from N-terminus to C-terminus is as follows: (1) VL-CL; (2) VH-CH1-CH2-CH3-VHH; (3) VH-CH1-CH2-CH3-VHH; and (4) VL-CL, wherein the hexagram symbol represents the conjugated or free payload, and a schematic diagram is shown in Figure 21.

In some embodiments, the ligand-drug conjugate comprises the ligand unit in an IgG form and the recovery unit in a VHH form, wherein the recovery unit is connected to the C-terminus of the ligand unit via a linker unit; preferably, the payload is conjugated to the linker unit between the IgG and the VHH, and a structure of the ligand unit-recovery unit from N-terminus to C-terminus is as follows: (1) VL-CL; (2) VH-CH1-CH2-CH3-VHH; (3) VH-CH1-CH2-CH3-VHH; and (4) VL-CL, wherein the hexagram symbol represents the conjugated or free payload, and a schematic diagram is shown in Figure 22.

In some embodiments, the ligand-drug conjugate comprises the ligand unit in an IgG form and the recovery unit in a VHH form, wherein the recovery unit is connected directly to the C-terminus of the ligand unit; preferably, the IgG and the VHH are directly connected, that is, there is no linker unit between the ligand unit and the recovery unit, and a structure of the ligand unit-recovery unit from N-terminus to C-terminus is as follows: (1) VL-CL; (2) VH-CH1-CH2-CH3-VHH; (3) VH-CH1-CH2-CH3-VHH; and (4) VL-CL, wherein the hexagram symbol represents the conjugated or free payload, and a schematic diagram is shown in Figure 23.

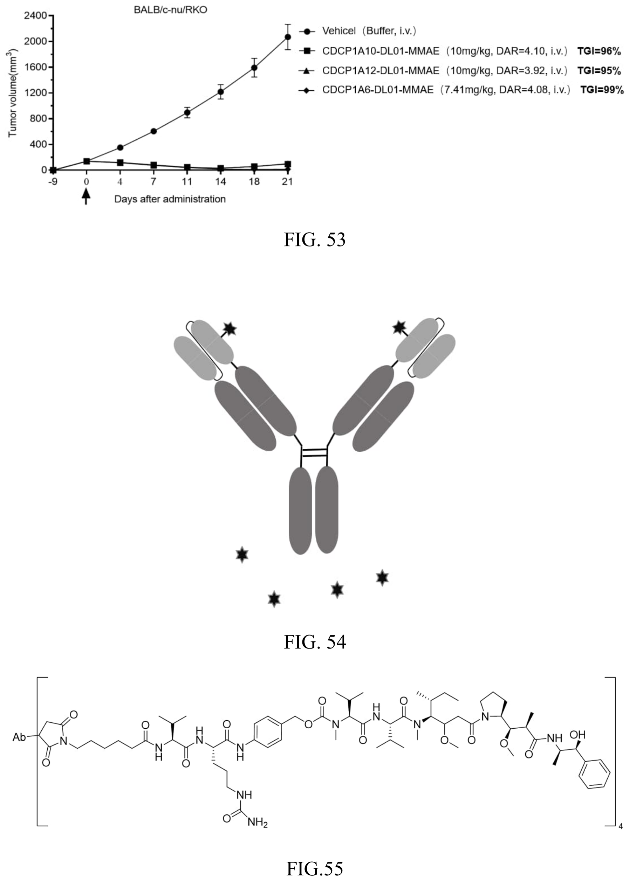

In some embodiments, the ligand-drug conjugate comprises the ligand unit in an scFv form and the recovery unit in an IgG form, wherein the recovery unit is connected directly or indirectly to the C-terminus of the ligand unit; preferably, a structure of the ligand unit-recovery unit from N-terminus to C-terminus is as follows: (1) VL-CL; (2) scFv-VH-CH1-CH2-CH3; (3) scFv-VH-CH1-CH2-CH3; and (4) VL-CL, wherein the hexagram symbol represents the conjugated or free payload, and a schematic diagram is shown in Figure 54.

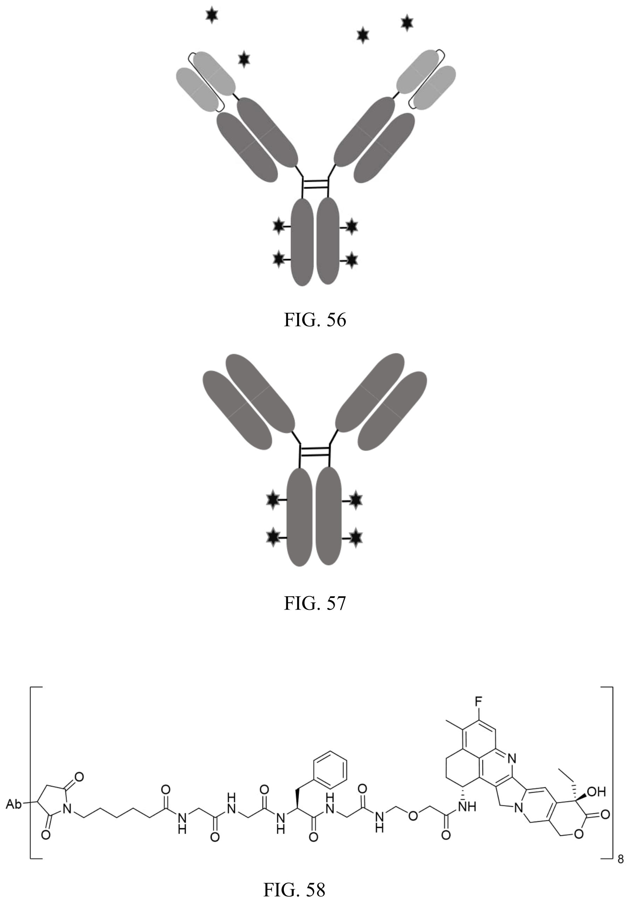

In some embodiments, the ligand-drug conjugate comprises: the recovery unit is in an scFv form, and the ligand unit is in an IgG form, wherein the recovery unit is connected directly or indirectly to the N-terminus of the ligand unit; preferably, a structure of the recovery unit-ligand unit from N-terminus to C-terminus is as follows: (1) VL-CL; (2) scFv-VH-CH1-CH2-CH3; (3) scFv-VH-CH1-CH2-CH3; and (4) VL-CL, wherein the hexagram symbol represents the conjugated or free payload, and a schematic diagram is shown in Figure 56.

In some embodiments, the ligand unit and the recovery unit are directly connected or not directly connected to each other.

In some embodiments, a linker unit is contained between the ligand unit and the recovery unit.

In some embodiments, the ligand-drug conjugate further comprises an additional functional unit.

In some embodiments, the payload is conjugated to the ligand unit and/or the recovery unit and/or the linker unit and/or the additional functional unit via the linker.

In some embodiments, the ligand unit comprises an antibody or an antigen-binding fragment, a receptor, a peptide, an oligopeptide, a peptidomimetic, a fusion protein, or an aptamer that specifically binds to a tumor antigen.

Further, the antibody includes, but is not limited to, a monoclonal antibody, a bispecific antibody, a multispecific antibody, a nanobody (heavy chain antibody, HcAb), and an immunoglobulin new antigen receptor (IgNAR); the antigen-binding fragment includes, but is not limited to, an scFv, an Fab, an Fab', an F(ab')₂, an Fd, an Fv, a dAb, an Fd, an sdAb, a VHH, and a Diabody; and the receptor includes, but is not limited to, a T cell receptor.

Further, the antibody is selected from a murine antibody, a chimeric antibody, a humanized antibody, a fully human antibody, and an artificially engineered antibody.

Further, the antibody further comprises a constant region of an immunoglobulin.

Further, the immunoglobulin is selected from human IgG1, IgG2, IgG3, and IgG4.

Further, the tumor antigen or non-tumor antigen includes, but is not limited to, CD9, CD19, CD20, CD22, CD24, CD25, CD29, CD30, CD33, CD37, CD44, CD45, CD46, CD47, CD49b, CD51, CD52, CD56, CD73(NT5E), CD79b, CD123, CD133, CD138, CD157, CD166, DLL-3, CDH6, Tissue factor, EpCAM, PSMA, MUC1, MUC16, FOLR1, GPC3, ROR1, ROR2, PD-L1, ENPP3, TDGF1, MSLN, TIM-1, LRRC15, LIV-1(ZIP6), Claudin6, Claudin9, Claudin 18.2, Mesothelin, HER2(ErbB2), HER3(ErbB3), EGFR, c-MET, SLITRK6, KIT(CD117), STEAP1, NaPi2B(SLC34A2), SLC44A4, GPNMB, AXL, CD166, B7-H3(CD276), B7-H4(VTCN1), PTK7(CCK4), EFNA4, 5T4, NOTCH3, Nectin-4, TROP-2, GPR20, EphA2, LYPD3, FGFR2, FGFR3, FRα, CEACAMs, CAIX, P-cadherin(CDH3), CDH17, GD3, Cadherin 6, LAMP1, FLT3, BCMA, SLTRK6, Lewis Y, ASCT2, CA-IX, Cripto, DPEP3, Globo H, Ly6E, RNF43, DR5, CDCP1, CEACAM6, FUT3, GD2, CD26E, CD70, CD74, CD163, PRLR, TNFα, CXCR4, LXR, IL-6, CEACAM5, GPRC5D, HLA-DR, CTLA4(CD152), FAPα and IL2R.

Further, the antibody that specifically binds to the tumor antigen or the non-tumor antigen includes, but is not limited to, Alemtuzumab, Altumomab pentetate, Amivantamab, Atezolizumab, Inetetamab, Anetumab, Avelumab, Bectumomab, Bermekimab, Bevacizumab, Blinatumomab, Brentuximab vedotin, Catumaxomab, Cemiplimab, Cetuximab, Clivatuzumab, Daclizumab, Daratumumab, Denosumab, Dinutuximab beta, Dostarlimab, Durvalumab, Edrecolomab, Enfortumab vedotin, Epcoritamab, Epratuzumab, Etaracizumab, Genmab, Glofitamab, Girentuximab, Ibritumomab, Inebilizumab, Inotuzumab ozogamicin, Ipilimumab, Isatuximab, Labetuzumab, Loncastuximab, Mogamulizumab, Loncastuximab tesirine, Mosunetuzumab, Nimotuzumab, Naratuximab, Naxitamab, Nivolumab, Ocrelizumab, Ofatumumab, Olaratumab, Oregovomab, Panitumumab, Pembrolizumab, Pertuzumab, Polatuzumab vedotin, Racotumomab, Ramucirumab, Ramucirumab, Rituximab, Sacituzumab govitecan, Teprotumumab, Tocilizumab, Tositumomab, Trastuzumab, Votumumab, Zalutumumab, Tocilizumab, Zanolimumab, and antigen-binding fragments and derivatives thereof; the peptide, oligopeptide, fusion protein that specifically binds to a specific antigen includes, but is not limited to, a bicyclic peptide (Bicycle), angiopep-2, a linear peptide, a pH-sensitive peptide, SOR-C27, A6 peptide, SOR13, and variants and derivatives thereof; the aptamer that specifically binds to a specific antigen includes AS1411, sgc8, sgc4f, sgd5a, TC01, TD05, A9, A10, APT, Min.2, AIR-3A, E3, E07, Waz, P19, Zy1, and variants and derivatives thereof. The antibody, the antigen-binding fragment, or the receptor fragment that specifically binds the tumor antigen or the non-tumor antigen, or the peptide, the oligopeptide, the peptidomimetic, the fusion protein, or the aptamer targeting specific antigens, can be obtained through existing screening technologies, artificial design, computer-aided design, or AI-assisted development based on the targeted tumor antigen or non-tumor antigen and binding requirements.

Further, the ligand-drug conjugate is capable of specifically binding to the free payload, and molecular aggregation does not occur between the ligand-drug conjugates.

Further, the recovery unit specifically binds to the free payload.

Further, the recovery unit comprises an antibody, an antigen-binding fragment, a receptor, a fusion protein, a peptide, an oligopeptide, a peptidomimetic, or an aptamer that specifically binds to the free payload.

Further, the antibody that specifically binds to the free payload includes, but is not limited to, a monoclonal antibody, a bispecific antibody, a multispecific antibody, a nanobody (heavy chain antibody, HcAb), an immunoglobulin new antigen receptor (IgNAR); the antigen-binding fragment includes, but is not limited to, an scFv, an Fab, an Fab', an F(ab')₂, an Fd, an Fv, a dAb, an Fd, an sdAb, a VHH, and a Diabody; and the receptor includes, but is not limited to, a T cell receptor.

Further, the antibody specifically binding to the free payload is selected from a murine antibody, a chimeric antibody, a humanized antibody, a fully human antibody, and an artificially engineered antibody.

Further, the recovery unit is capable of specifically binding to the free payload means that the equilibrium dissociation constant K_{D}(M) of the recovery unit to the free payload is less than 10⁻⁴, or further less than 10⁻⁵, or further less than 10⁻⁶, or further less than 10⁻⁷, or further less than 10⁻⁸, or further less than 10⁻⁹, or further less than 10⁻¹⁰, or further less than 10⁻¹¹, or further less than 10⁻¹², or further less than 10⁻¹³.

Further, the recovery unit does not bind to, or has a low affinity to, a payload in a conjugated state in the ligand-drug conjugate, and the low affinity also prevents the ligand-drug conjugate molecules from aggregating.

Further, the recovery unit specifically targets a non-exposed epitope on the conjugated payload unit.

Further, a payload epitope masking unit capable of preventing the recovery unit from binding to the payload that remains conjugated to the ligand-drug conjugate is linked to the linker or the payload.

Further, the payload epitope masking unit does not interfere with the normal release of the payload after the ligand-drug conjugate enters a target cell.

Further, the ligand-drug conjugate comprises one, two, or more recovery units.

Further, the recovery unit comprises:
(1) a VHH domain with CDR1-3 selected from amino acid sequences defined by:
   CDR1-3 as set forth in SEQ ID NOs: 103, 104, and 105;
   or (2) an amino acid sequence selected from the amino acid sequence as set forth in SEQ ID NO: 102;
   or (3) an amino acid sequence selected from combinations of VH and VL defined as follows:
      a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 7 respectively;
      a VH comprising HCDR1-3 with amino acid sequences as set forth in SEQ ID NOs: 9, 10, and 11 respectively, and a VL comprising LCDR1-3 with amino acid sequences as set forth in SEQ ID NO: 6, FAS, and SEQ ID NO: 13 respectively;
      a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 16 respectively;
      a VH comprising HCDR1-3 with amino acid sequences as set forth in SEQ ID NOs: 2, 3, and 20 respectively, and a VL comprising LCDR1-3 with amino acid sequences as set forth in SEQ ID NO: 6, YAS, and SEQ ID NO: 7 respectively;
      a VH comprising HCDR1-3 with amino acid sequences as set forth in SEQ ID NOs: 22, 23, and 24 respectively, and a VL comprising LCDR1-3 with amino acid sequences as set forth in SEQ ID NO: 26, WAS, and SEQ ID NO: 27 respectively;
      a VH comprising HCDR1-3 with amino acid sequences as set forth in SEQ ID NOs: 29, 30, and 31 respectively, and a VL comprising LCDR1-3 with amino acid sequences as set forth in SEQ ID NO: 33, KVS, and SEQ ID NO: 34 respectively;
      a VH comprising HCDR1-3 with amino acid sequences as set forth in SEQ ID NOs: 29, 36, and 37 respectively, and a VL comprising LCDR1-3 with amino acid sequences as set forth in SEQ ID NO: 33, KLS, and SEQ ID NO: 34 respectively;
      a VH comprising HCDR1-3 with amino acid sequences as set forth in SEQ ID NOs: 40, 41, and 42 respectively, and a VL comprising LCDR1-3 with amino acid sequences as set forth in SEQ ID NO: 44, KVS, and SEQ ID NO: 45 respectively;
      a VH comprising HCDR1-3 with amino acid sequences as set forth in SEQ ID NOs: 47, 48, and 42 respectively, and a VL comprising LCDR1-3 with amino acid sequences as set forth in SEQ ID NO: 50, KVS, and SEQ ID NO: 51 respectively;
      a VH comprising HCDR1-3 with amino acid sequences as set forth in SEQ ID NOs: 53, 54, and 55 respectively, and a VL comprising LCDR1-3 with amino acid sequences as set forth in SEQ ID NO: 57, DTS, and SEQ ID NO: 58 respectively;
      a VH comprising HCDR1-3 with amino acid sequences as set forth in SEQ ID NOs: 62, 63, and 64 respectively, and a VL comprising LCDR1-3 with amino acid sequences as set forth in SEQ ID NO: 66, SAS, and SEQ ID NO: 67 respectively;
      a VH comprising HCDR1-3 with amino acid sequences as set forth in SEQ ID NOs: 69, 70, and 71 respectively, and a VL comprising LCDR1-3 with amino acid sequences as set forth in SEQ ID NO: 73, WAS, and SEQ ID NO: 74 respectively;
      a VH comprising HCDR1-3 with amino acid sequences as set forth in SEQ ID NOs: 76, 77, and 78 respectively, and a VL comprising LCDR1-3 with amino acid sequences as set forth in SEQ ID NO: 80, GAS, and SEQ ID NO: 81 respectively;
      a VH comprising HCDR1-3 with amino acid sequences as set forth in SEQ ID NOs: 83, 84, and 85 respectively, and a VL comprising LCDR1-3 with amino acid sequences as set forth in SEQ ID NO: 87, SAS, and SEQ ID NO: 67 respectively;
      a VH comprising HCDR1-3 with amino acid sequences as set forth in SEQ ID NOs: 89, 90, and 91 respectively, and a VL comprising LCDR1-3 with amino acid sequences as set forth in SEQ ID NO: 93, HGT, and SEQ ID NO: 94 respectively;
      a VH comprising HCDR1-3 with amino acid sequences as set forth in SEQ ID NOs: 76, 77, and 78 respectively, and a VL comprising LCDR1-3 with amino acid sequences as set forth in SEQ ID NOs: 99, 77, and 100 respectively;
      a VH comprising an amino acid sequence as set forth in SEQ ID NO: 1, and a VL comprising an amino acid sequence as set forth in SEQ ID NO: 5;
      a VH comprising an amino acid sequence as set forth in SEQ ID NO: 8, and a VL comprising an amino acid sequence as set forth in SEQ ID NO: 12;
      a VH comprising an amino acid sequence as set forth in SEQ ID NO: 14, and a VL comprising an amino acid sequence as set forth in SEQ ID NO: 15;
      a VH comprising an amino acid sequence as set forth in SEQ ID NO: 17, and a VL comprising an amino acid sequence as set forth in SEQ ID NO: 18;
      a VH comprising an amino acid sequence as set forth in SEQ ID NO: 19, and a VL comprising an amino acid sequence as set forth in SEQ ID NO: 18;
      a VH comprising an amino acid sequence as set forth in SEQ ID NO: 21, and a VL comprising an amino acid sequence as set forth in SEQ ID NO: 25;
      a VH comprising an amino acid sequence as set forth in SEQ ID NO: 28, and a VL comprising an amino acid sequence as set forth in SEQ ID NO: 32;
      a VH comprising an amino acid sequence as set forth in SEQ ID NO: 35, and a VL comprising an amino acid sequence as set forth in SEQ ID NO: 38;
      a VH comprising an amino acid sequence as set forth in SEQ ID NO: 39, and a VL comprising an amino acid sequence as set forth in SEQ ID NO: 43;
      a VH comprising an amino acid sequence as set forth in SEQ ID NO: 46, and a VL comprising an amino acid sequence as set forth in SEQ ID NO: 49;
      a VH comprising an amino acid sequence as set forth in SEQ ID NO: 52, and a VL comprising an amino acid sequence as set forth in SEQ ID NO: 56;
      a VH comprising an amino acid sequence as set forth in SEQ ID NO: 59, and a VL comprising an amino acid sequence as set forth in SEQ ID NO: 60;
      a VH comprising an amino acid sequence as set forth in SEQ ID NO: 61, and a VL comprising an amino acid sequence as set forth in SEQ ID NO: 65;
      a VH comprising an amino acid sequence as set forth in SEQ ID NO: 68, and a VL comprising an amino acid sequence as set forth in SEQ ID NO: 72;
      a VH comprising an amino acid sequence as set forth in SEQ ID NO: 75, and a VL comprising an amino acid sequence as set forth in SEQ ID NO: 79;
      a VH comprising an amino acid sequence as set forth in SEQ ID NO: 82, and a VL comprising an amino acid sequence as set forth in SEQ ID NO: 86;
      a VH comprising an amino acid sequence as set forth in SEQ ID NO: 88, and a VL comprising an amino acid sequence as set forth in SEQ ID NO: 92;
      a VH comprising an amino acid sequence as set forth in SEQ ID NO: 95, and a VL comprising an amino acid sequence as set forth in SEQ ID NO: 96; and
      a VH comprising an amino acid sequence as set forth in SEQ ID NO: 97, and a VL comprising an amino acid sequence as set forth in SEQ ID NO: 98;
         wherein the CDR1, CDR2 and CDR3 sequences, the HCDR1, HCDR2 and HCDR3 sequences, and the LCDR1, LCDR2 and LCDR3 sequences are obtained based on an IMGT definition scheme;
   or (4) a peptide comprising an amino acid sequence defined by SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, or SEQ ID NO: 129;
   or (5) an aptamer comprising a nucleotide sequence defined by SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, or SEQ ID NO: 162.

Further, the recovery unit is obtained by immunizing a non-human animal with a conjugate of a small molecule drug and a carrier protein or a biotin-small molecule drug conjugate, or obtained by screening with phage display technology, or obtained by screening with virtual technology, or obtained by screening with SELEX technology, or obtained by screening with hybridoma technology, or obtained by screening with nanobody technology, or obtained by yeast display technology, or obtained by single B cell cloning technology, or obtained by microfluidic technology, or obtained by surface plasmon resonance assisted screening technology.

Further, the small molecule drug includes, but is not limited to, MMAE, MMAD, DM1, DM2, DM4, eribulin, Exatecan, SN38, DXD, calicheamicin, pyrrolobenzodiazepine (PBD), indolinobenzodiazepine (IBD), duocarmycin, amanitin, Maaa-1181a, taxol, daunomycin, vinblastine, Doxorubicin, methotrexate, MMAF, ML022-Dxd, ML022-D633-004, ML026-Dxd, ML031-D633-004, ML037-D633-004, ML042-Dxd, ML047-DX8951, and MPA-Dxd;

| | | |
|---|---|---|
| 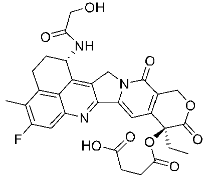 ML022-Dxd | 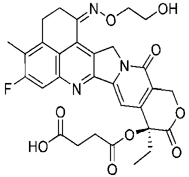 ML022-D633-004 | 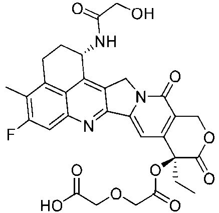 ML026-Dxd |
| 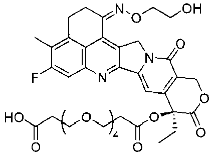 ML031-D633-004 | 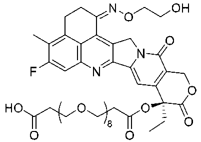 ML037-D633-004 | 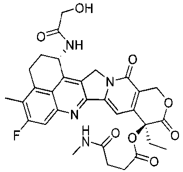 ML042-Dxd |
| 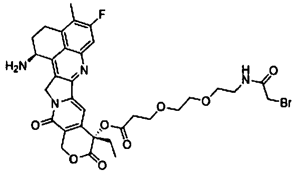 ML047-DX8951 | 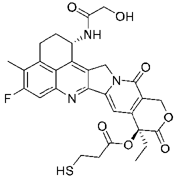 MPA-Dxd | |

preferably, the carrier protein includes, but is not limited to, KLH, BSA, and OVA.

Further, the biotin-small molecule drug conjugate includes, but is not limited to, Biotin-PEG12-Dxd, Biotin-PEG10-Dxd, Biotin-PEG6-Dxd, Biotin-PEG6-Triazole-Dxd and Biotin-PEG10-Triazole-Dxd:

| | | |
|---|---|---|
| 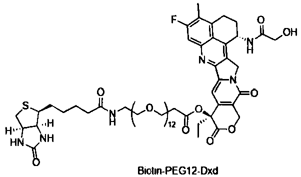 | 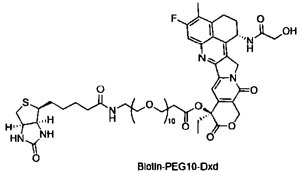 | 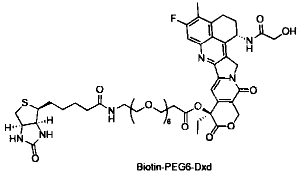 |
| 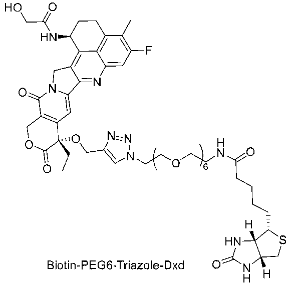 | 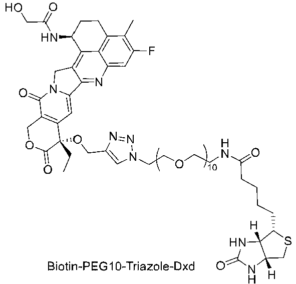 | |

Further, the conjugated payload includes a cytotoxic molecule, a nucleic acid, an immunostimulant, and a modulator.

Further, the number of payloads is one, two or more.

Further, the number of payloads is 1, 2, 3, 4, 5, 6, 7, 8 or more.

Further, when two or more payloads are conjugated, the payloads may be the same or different.

Further, the payload is conjugated to the ligand unit and/or the recovery unit and/or the linker unit and/or the additional functional unit via site-specific or random conjugation.

Still further, the cytotoxic molecule includes, but is not limited to, a tubulin inhibitor, a DNA inhibitor, or other toxin molecules; the DNA inhibitor further includes a DNA damaging agent or a topoisomerase I inhibitor.

Still further, the tubulin inhibitor further includes, but is not limited to, auristatins, maytansinoid derivatives, tubulysin, halichondrins, cryptophycins or EG5 inhibitors.

Still further, the auristatins further include, but are not limited to, auristatin F (AF), monomethyl auristatin D (MMAD), monomethyl auristatin E (MMAE), and monomethyl auristatin F (MMAF); the maytansinoid derivative further includes, but is not limited to, DM1, DM2, and DM4; the halichondrin further includes, but is not limited to, eribulin.

Still further, the DNA damaging agent further includes, but is not limited to, calicheamicins, pyrrolobenzodiazepines (PBDs), indolinobenzodiazepines (IBDs), and duocarmycins; and the topoisomerase I inhibitor further includes, but is not limited to, camptothecin derivatives.

Still further, the camptothecin derivative further includes, but is not limited to, Exatecan, SN38, and DXD.

Still further, the other toxin molecule includes, but is not limited to, amanitin, Maaa-1181a, taxol, daunomycin, vinblastine, Doxorubicin, methotrexate, or a radioisotope and/or a pharmaceutically acceptable salt thereof.

Further, the nucleic acid used as the conjugated payload includes, but is not limited to, an antisense oligonucleotide (ASO), a small interfering RNA (siRNA), a microRNA (miRNA), a small activating RNA (saRNA), a messenger RNA (mRNA), and an oligonucleotide (AOC).

Still further, the nucleic acid used as the conjugated payload includes but is not limited to DMPK siRNA, Exon-44-skipping PMO, DUX4 siRNA, Exon-51-skipping PMO, DMPK ASO, DUX4 ASO, and CpG.

Still further, the immunostimulant used as the conjugated payload includes but is not limited to a TLR7 agonist, a TLR8 agonist, a TLR9 agonist, a TGF-β inhibitor, a TNIK inhibitor, and a STING agonist.

Still further, the modulator used for the conjugated payload includes, but is not limited to, a glucocorticoid receptor modulator (such as dexamethasone, budesonide, fluticasone propionate, or glucocorticoids), an antibiotic (such as rifalogue), a kinase inhibitor (such as dasatinib), a liver X receptor (LXR) agonist, a phosphodiesterase (PDE4) inhibitor, and a bisphosphonate (alendronate).

Further, the free payload has a structure selected from the group consisting of the following structures, and an isomer or a deuterated form thereof:

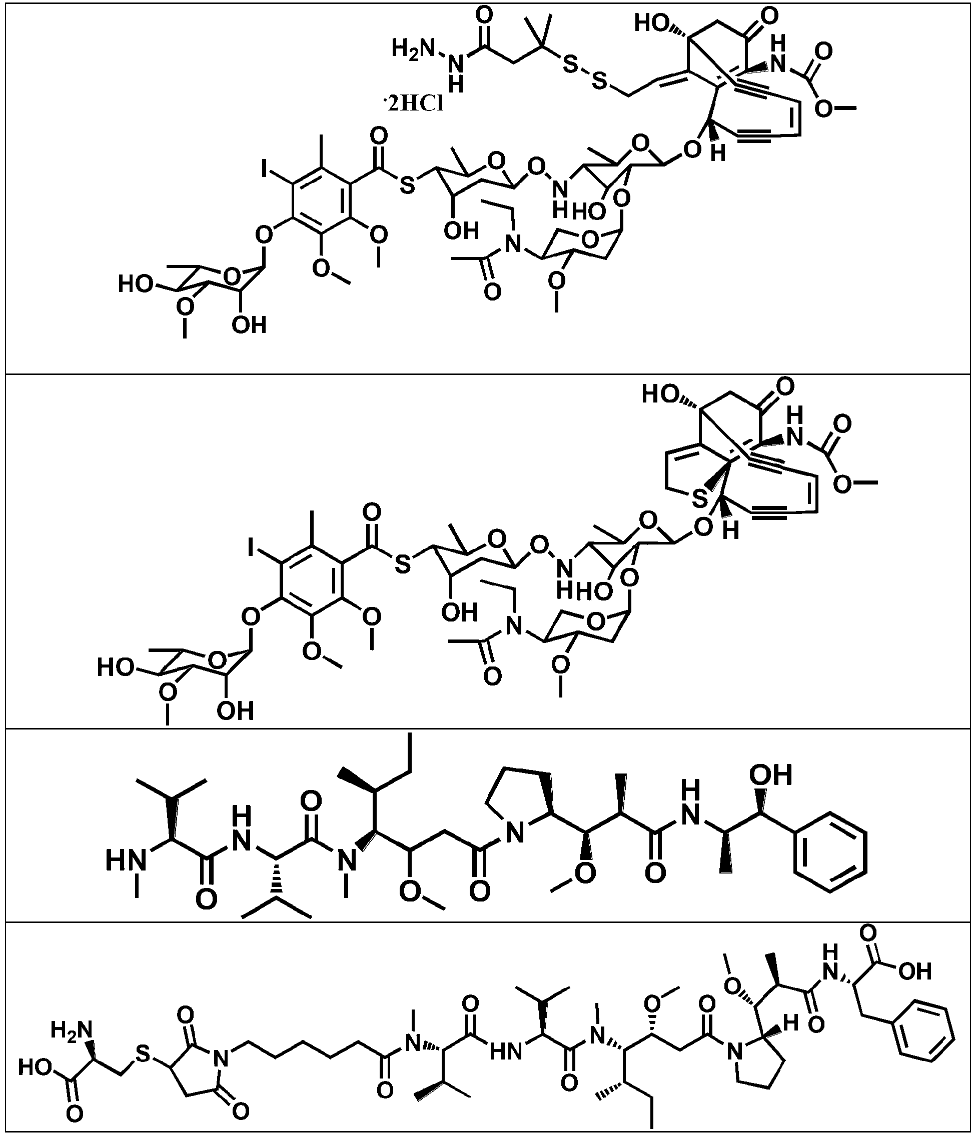

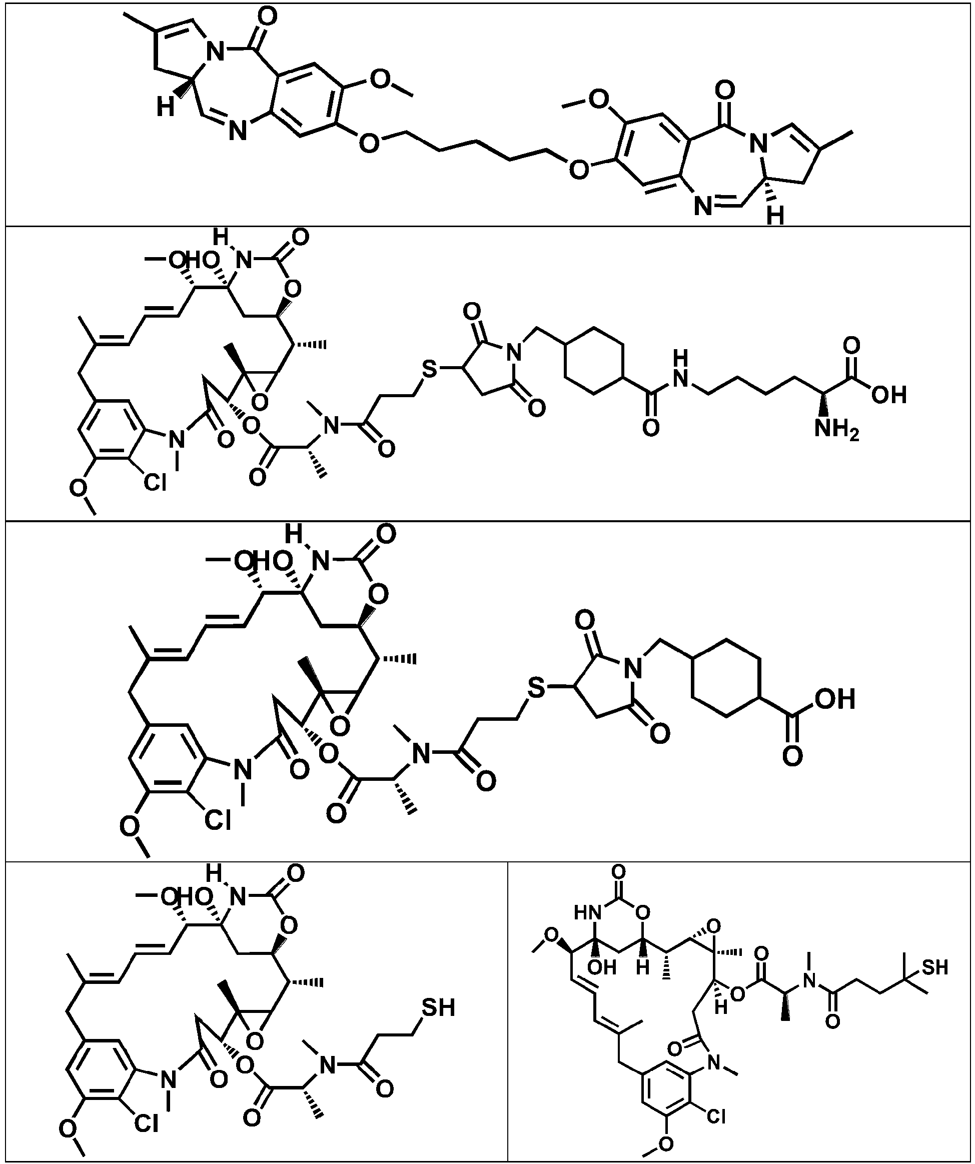

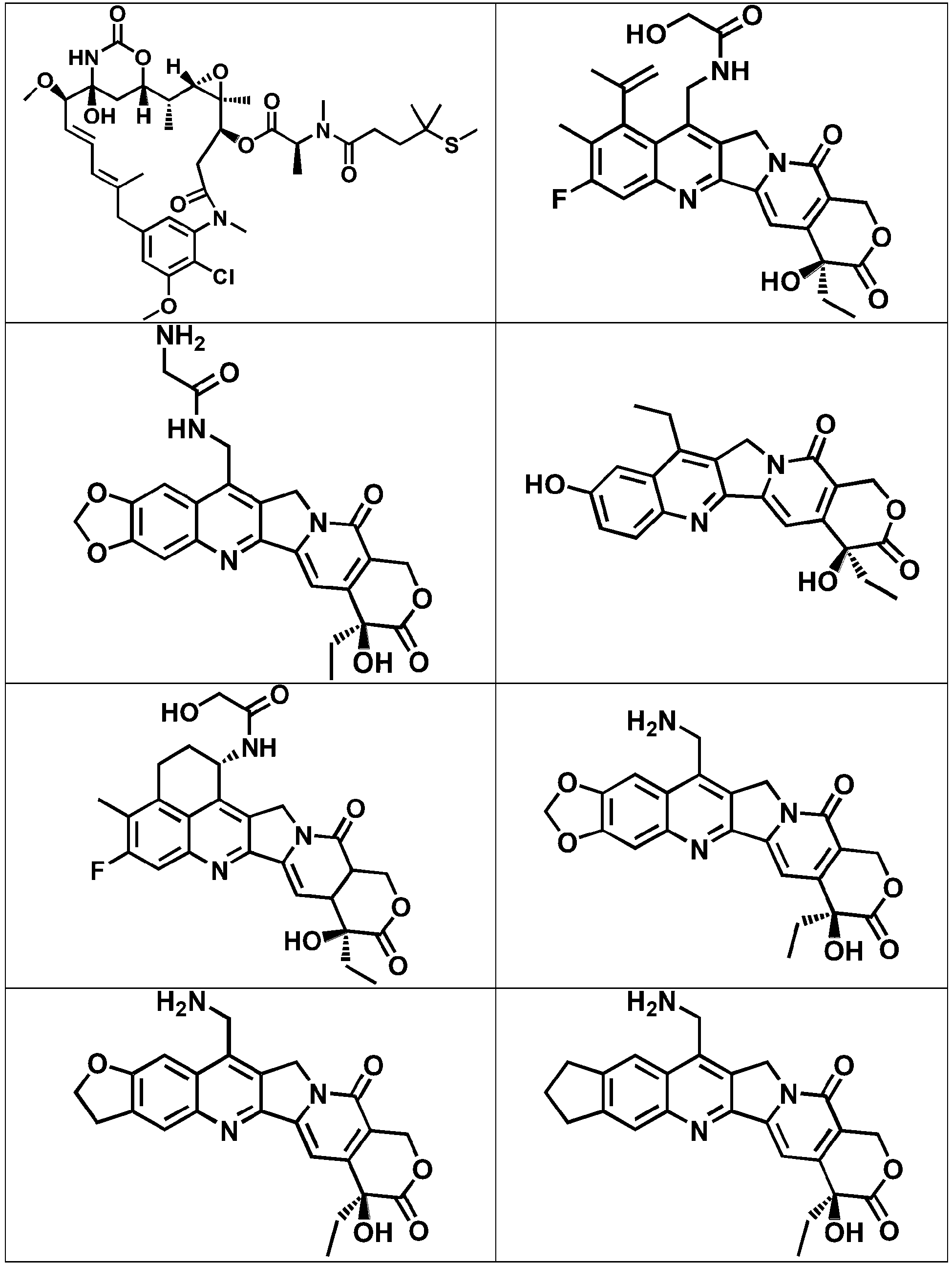

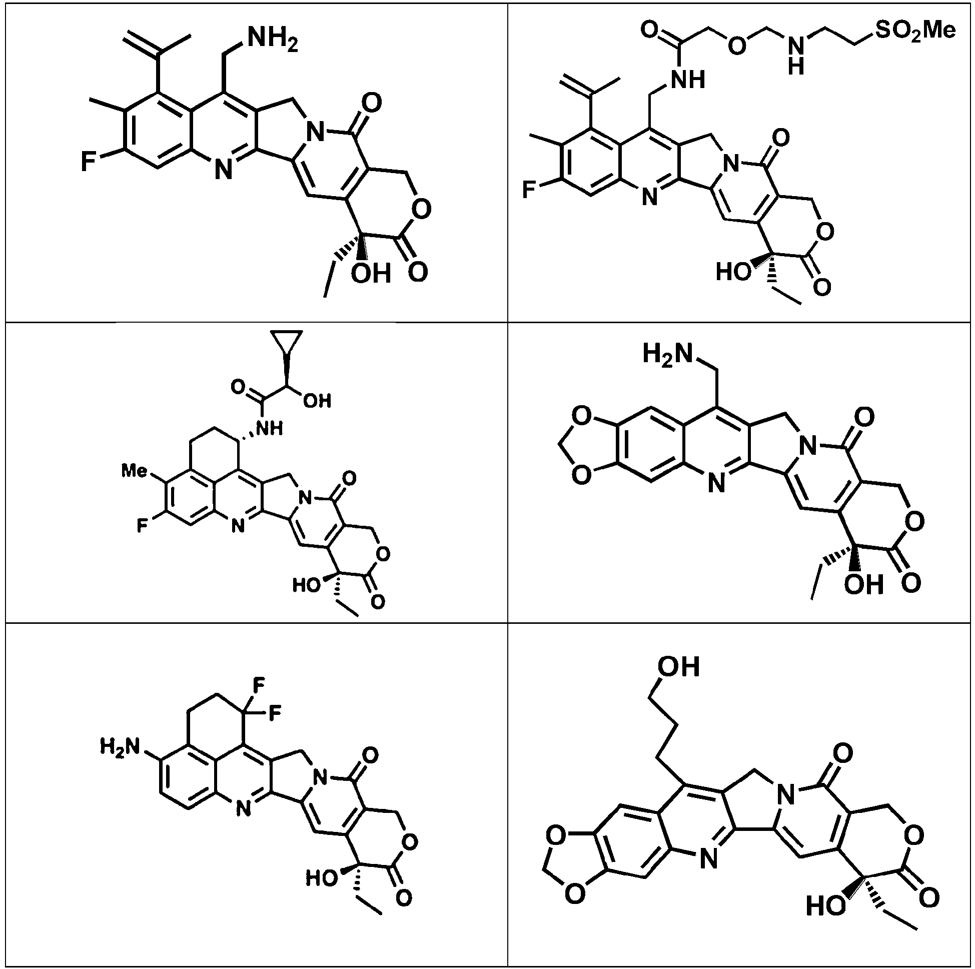

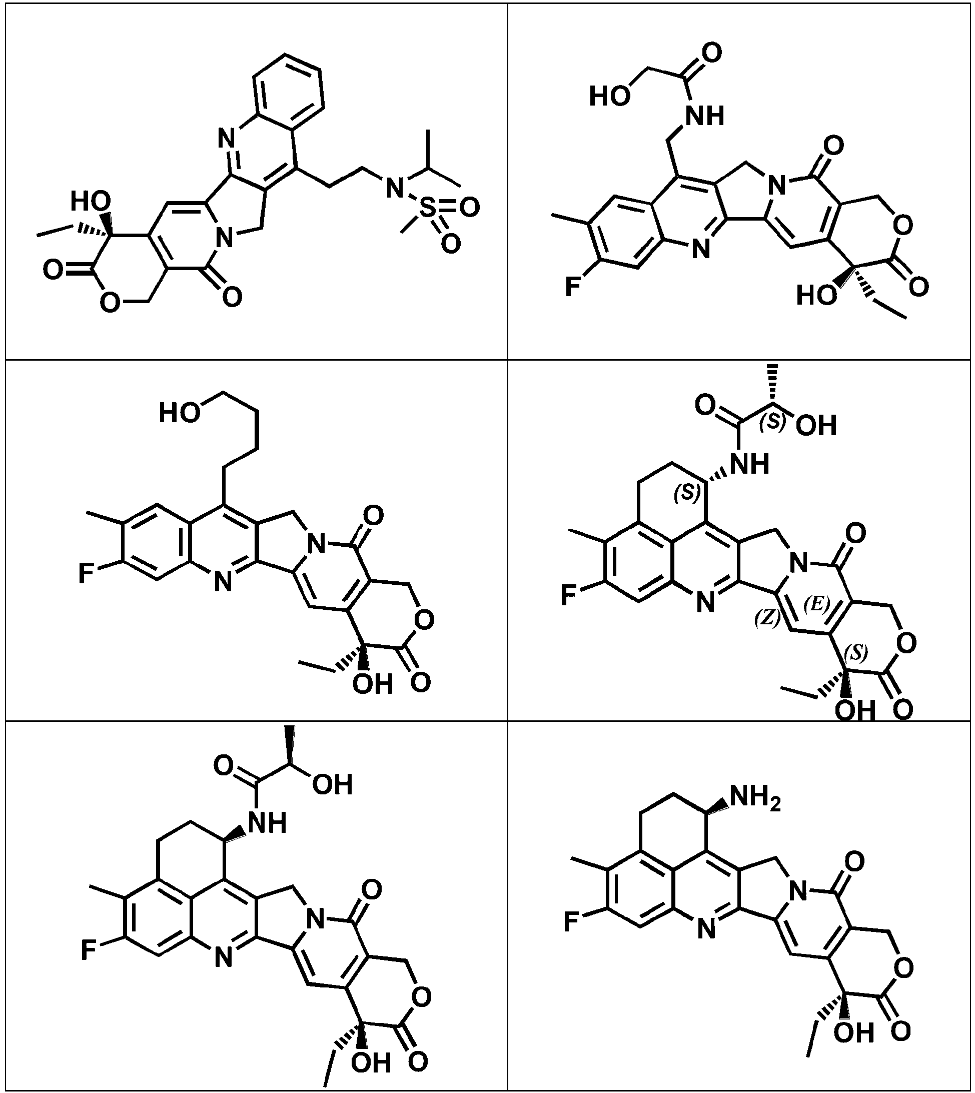

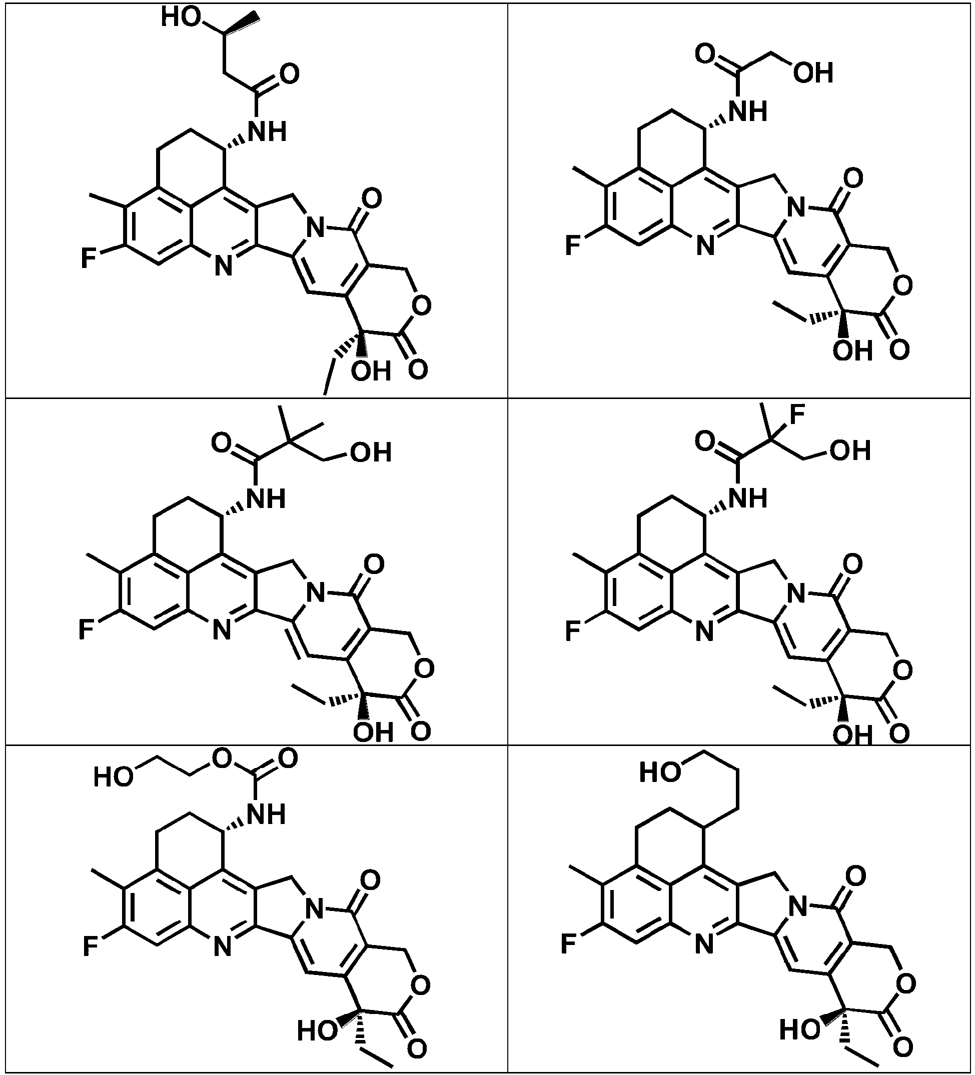

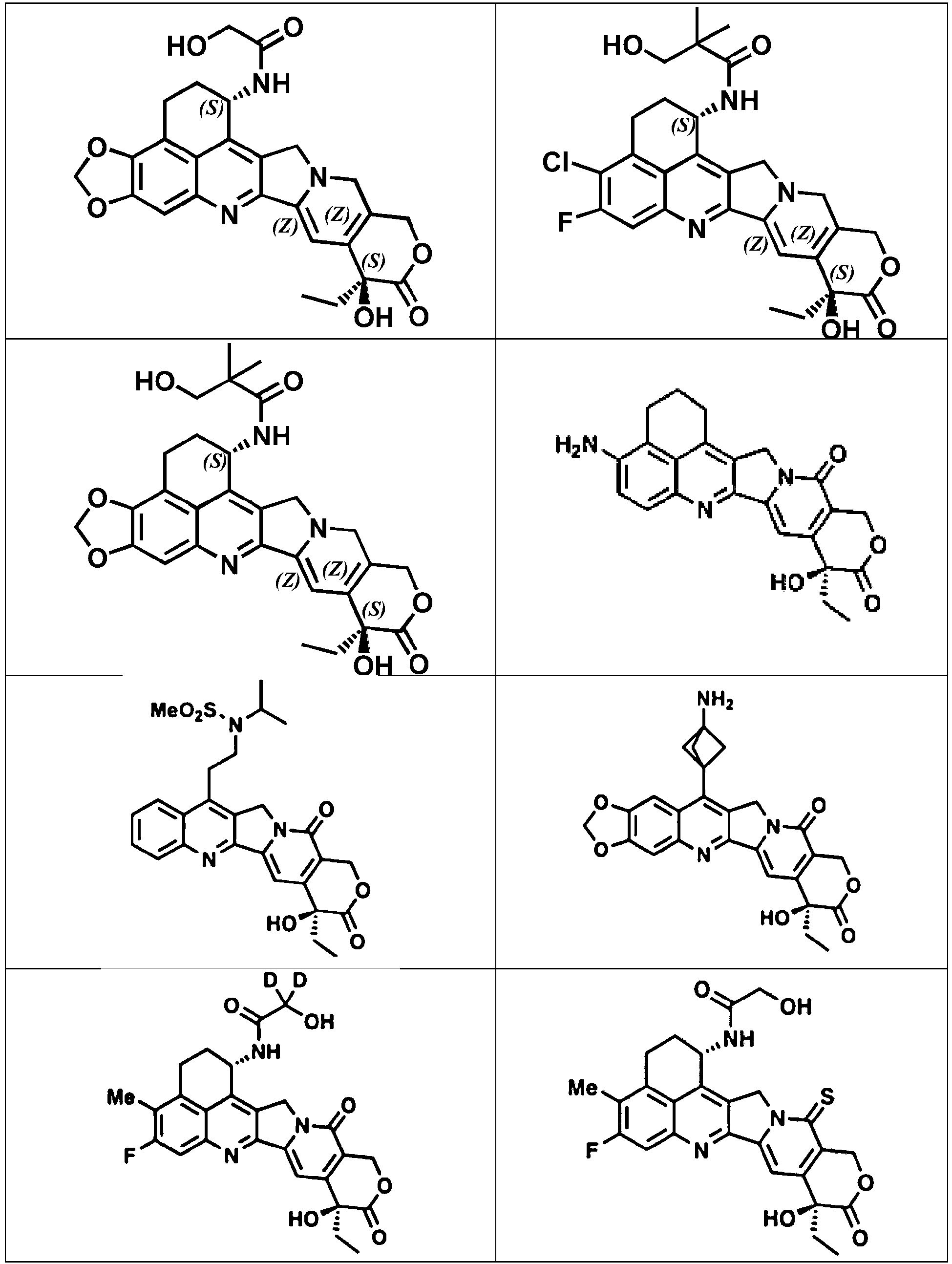

Further, the linker unit includes, but is not limited to, a peptide linker, an Fc domain or a fragment thereof; preferably, the peptide linker is a flexible peptide linker; further preferably, the peptide linker comprises one or more amino acids; further preferably, the peptide linker comprises at least 5 amino acids; or the amino acid sequence of the peptide linker comprises (GGGGS)n, (VPGXG)n, (SG)n, (EAAAK)n, (G)n, (R)n, A(EAAAK)n ALEA(EAAAK)nA, A(EAAAK)nA, (AP)n, VSQTSKLTRAETVFPDV, PLGLWA, RVLAEA, EDVVCCSMSY, GGIEGRGS, TRHRQPRGWE, AGNRVRRSVG, GFLG, or LE, wherein n is equal to 1, 2, 3 or 4.

In another aspect, the present disclosure provides a pharmaceutically acceptable salt, a deuterated form, or a solvate of the ligand-drug conjugate.

In another aspect, the present disclosure provides a pharmaceutical composition comprising the ligand-drug conjugate provided by the present disclosure or a pharmaceutically acceptable salt, deuterated form, or solvate thereof, and a pharmaceutically acceptable excipient.

In another aspect, the present disclosure provides use of a recovery unit capable of binding to a free payload released from a ligand-drug conjugate in the preparation of a ligand-drug conjugate comprising a free payload recovery unit.

Further, the recovery unit comprises an antibody or an antigen-binding fragment, a receptor, a fusion protein, a peptide, an oligopeptide, a peptidomimetic, or an aptamer that specifically binds to the free payload.

Further, the antibody that specifically binds to the free payload includes, but is not limited to, a monoclonal antibody, a bispecific antibody, a multispecific antibody, a nanobody (heavy chain antibody, HcAb), and an immunoglobulin new antigen receptor (IgNAR); the antigen-binding fragment includes, but is not limited to, an scFv, an Fab, an Fab', an F(ab')₂, an Fd, an Fv, a dAb, a Fd, a sdAb, a VHH, and a Diabody; and the receptor includes, but is not limited to, a T cell receptor.

Further, the antibody that specifically binds to the free payload is selected from a murine antibody, a chimeric antibody, a humanized antibody, a fully human antibody, and an artificially engineered antibody.

Further, the recovery unit is capable of specifically binding to the free payload means that the equilibrium dissociation constant K_{D}(M) of the recovery unit to the free payload is less than 10⁻⁴, or further less than 10⁻⁵, or further less than 10⁻⁶, or further less than 10⁻⁷, or further less than 10⁻⁸, or further less than 10⁻⁹, or further less than 10⁻¹⁰, or further less than 10⁻¹¹, or further less than 10⁻¹², or further less than 10⁻¹³.

Further, the recovery unit does not bind to, or has a low affinity to, a payload in a conjugated state in the ligand-drug conjugate, and the low affinity also prevents the ligand-drug conjugate molecules from aggregating.

Further, the recovery unit comprises:
(1) a VHH domain with CDR1-3 selected from amino acid sequences defined by:
   CDR1-3 as set forth in SEQ ID NOs: 103, 104, and 105;
   or (2) an amino acid sequence selected from the amino acid sequence as set forth in SEQ ID NO: 102;
   or (3) an amino acid sequence selected from combinations of VH and VL defined as follows:
      a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 7 respectively;
      a VH comprising HCDR1-3 with amino acid sequences as set forth in SEQ ID NOs: 9, 10, and 11 respectively, and a VL comprising LCDR1-3 with amino acid sequences as set forth in SEQ ID NO: 6, FAS, and SEQ ID NO: 13 respectively;
      a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 16 respectively;
      a VH comprising HCDR1-3 with amino acid sequences as set forth in SEQ ID NOs: 2, 3, and 20 respectively, and a VL comprising LCDR1-3 with amino acid sequences as set forth in SEQ ID NO: 6, YAS, and SEQ ID NO: 7 respectively;
      a VH comprising HCDR1-3 with amino acid sequences as set forth in SEQ ID NOs: 22, 23, and 24 respectively, and a VL comprising LCDR1-3 with amino acid sequences as set forth in SEQ ID NO: 26, WAS, and SEQ ID NO: 27 respectively;
      a VH comprising HCDR1-3 with amino acid sequences as set forth in SEQ ID NOs: 29, 30, and 31 respectively, and a VL comprising LCDR1-3 with amino acid sequences as set forth in SEQ ID NO: 33, KVS, and SEQ ID NO: 34 respectively;
      a VH comprising HCDR1-3 with amino acid sequences as set forth in SEQ ID NOs: 29, 36, and 37 respectively, and a VL comprising LCDR1-3 with amino acid sequences as set forth in SEQ ID NO: 33, KLS, and SEQ ID NO: 34 respectively;
      a VH comprising HCDR1-3 with amino acid sequences as set forth in SEQ ID NOs: 40, 41, and 42 respectively, and a VL comprising LCDR1-3 with amino acid sequences as set forth in SEQ ID NO: 44, KVS, and SEQ ID NO: 45 respectively;
      a VH comprising HCDR1-3 with amino acid sequences as set forth in SEQ ID NOs: 47, 48, and 42 respectively, and a VL comprising LCDR1-3 with amino acid sequences as set forth in SEQ ID NO: 50, KVS, and SEQ ID NO: 51 respectively;
      a VH comprising HCDR1-3 with amino acid sequences as set forth in SEQ ID NOs: 53, 54, and 55 respectively, and a VL comprising LCDR1-3 with amino acid sequences as set forth in SEQ ID NO: 57, DTS, and SEQ ID NO: 58 respectively;
      a VH comprising HCDR1-3 with amino acid sequences as set forth in SEQ ID NOs: 62, 63, and 64 respectively, and a VL comprising LCDR1-3 with amino acid sequences as set forth in SEQ ID NO: 66, SAS, and SEQ ID NO: 67 respectively;
      a VH comprising HCDR1-3 with amino acid sequences as set forth in SEQ ID NOs: 69, 70, and 71 respectively, and a VL comprising LCDR1-3 with amino acid sequences as set forth in SEQ ID NO: 73, WAS, and SEQ ID NO: 74 respectively;
      a VH comprising HCDR1-3 with amino acid sequences as set forth in SEQ ID NOs: 76, 77, and 78 respectively, and a VL comprising LCDR1-3 with amino acid sequences as set forth in SEQ ID NO: 80, GAS, and SEQ ID NO: 81 respectively;
      a VH comprising HCDR1-3 with amino acid sequences as set forth in SEQ ID NOs: 83, 84, and 85 respectively, and a VL comprising LCDR1-3 with amino acid sequences as set forth in SEQ ID NO: 87, SAS, and SEQ ID NO: 67 respectively;
      a VH comprising HCDR1-3 with amino acid sequences as set forth in SEQ ID NOs: 89, 90, and 91 respectively, and a VL comprising LCDR1-3 with amino acid sequences as set forth in SEQ ID NO: 93, HGT, and SEQ ID NO: 94 respectively;
      a VH comprising HCDR1-3 with amino acid sequences as set forth in SEQ ID NOs: 76, 77, and 78 respectively, and a VL comprising LCDR1-3 with amino acid sequences as set forth in SEQ ID NOs: 99, 77, and 100 respectively;
      a VH comprising an amino acid sequence as set forth in SEQ ID NO: 1, and a VL comprising an amino acid sequence as set forth in SEQ ID NO: 5;
      a VH comprising an amino acid sequence as set forth in SEQ ID NO: 8, and a VL comprising an amino acid sequence as set forth in SEQ ID NO: 12;
      a VH comprising an amino acid sequence as set forth in SEQ ID NO: 14, and a VL comprising an amino acid sequence as set forth in SEQ ID NO: 15;
      a VH comprising an amino acid sequence as set forth in SEQ ID NO: 17, and a VL comprising an amino acid sequence as set forth in SEQ ID NO: 18;
      a VH comprising an amino acid sequence as set forth in SEQ ID NO: 19, and a VL comprising an amino acid sequence as set forth in SEQ ID NO: 18;
      a VH comprising an amino acid sequence as set forth in SEQ ID NO: 21, and a VL comprising an amino acid sequence as set forth in SEQ ID NO: 25;
      a VH comprising an amino acid sequence as set forth in SEQ ID NO: 28, and a VL comprising an amino acid sequence as set forth in SEQ ID NO: 32;
      a VH comprising an amino acid sequence as set forth in SEQ ID NO: 35, and a VL comprising an amino acid sequence as set forth in SEQ ID NO: 38;
      a VH comprising an amino acid sequence as set forth in SEQ ID NO: 39, and a VL comprising an amino acid sequence as set forth in SEQ ID NO: 43;
      a VH comprising an amino acid sequence as set forth in SEQ ID NO: 46, and a VL comprising an amino acid sequence as set forth in SEQ ID NO: 49;
      a VH comprising an amino acid sequence as set forth in SEQ ID NO: 52, and a VL comprising an amino acid sequence as set forth in SEQ ID NO: 56;
      a VH comprising an amino acid sequence as set forth in SEQ ID NO: 59, and a VL comprising an amino acid sequence as set forth in SEQ ID NO: 60;
      a VH comprising an amino acid sequence as set forth in SEQ ID NO: 61, and a VL comprising an amino acid sequence as set forth in SEQ ID NO: 65;
      a VH comprising an amino acid sequence as set forth in SEQ ID NO: 68, and a VL comprising an amino acid sequence as set forth in SEQ ID NO: 72;
      a VH comprising an amino acid sequence as set forth in SEQ ID NO: 75, and a VL comprising an amino acid sequence as set forth in SEQ ID NO: 79;
      a VH comprising an amino acid sequence as set forth in SEQ ID NO: 82, and a VL comprising an amino acid sequence as set forth in SEQ ID NO: 86;
      a VH comprising an amino acid sequence as set forth in SEQ ID NO: 88, and a VL comprising an amino acid sequence as set forth in SEQ ID NO: 92;
      a VH comprising an amino acid sequence as set forth in SEQ ID NO: 95, and a VL comprising an amino acid sequence as set forth in SEQ ID NO: 96; and
      a VH comprising an amino acid sequence as set forth in SEQ ID NO: 97, and a VL comprising an amino acid sequence as set forth in SEQ ID NO: 98;
      wherein the CDR1, CDR2 and CDR3 sequences, the HCDR1, HCDR2 and HCDR3 sequences, and the LCDR1, LCDR2 and LCDR3 sequences are obtained based on an IMGT definition scheme;
      or (4) a peptide comprising an amino acid sequence defined by SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, or SEQ ID NO: 129;
   or (5) an aptamer comprising a nucleotide sequence defined by SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, or SEQ ID NO: 162.

Further, the recovery unit is obtained by immunizing a non-human animal with a conjugate of a small molecule drug and a carrier protein or a biotin-small molecule drug conjugate, or obtained by screening with phage display technology, or obtained by screening with virtual technology, or obtained by screening with SELEX technology, or obtained by screening with hybridoma technology, or obtained by screening with nanobody technology, or obtained by yeast display technology, or obtained by single B cell cloning technology, or obtained by microfluidic technology, or obtained by surface plasmon resonance assisted screening technology.

Further, the small molecule drug includes, but is not limited to, MMAE, MMAD, DM1, DM2, DM4, eribulin, Exatecan, SN38, DXD, calicheamicin, pyrrolobenzodiazepine (PBD), indolinobenzodiazepine (IBD), duocarmycin, amanitin, Maaa-1181a, taxol, daunomycin, vinblastine, Doxorubicin, methotrexate, MMAF, ML022-Dxd, ML022-D633-004, ML026-Dxd, ML031-D633-004, ML037-D633-004, ML042-Dxd, ML047-DX8951, and MPA-Dxd;

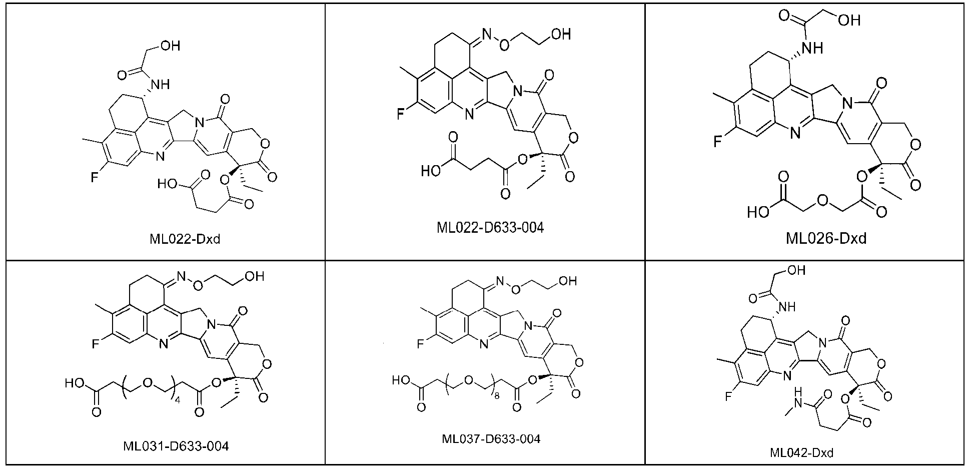

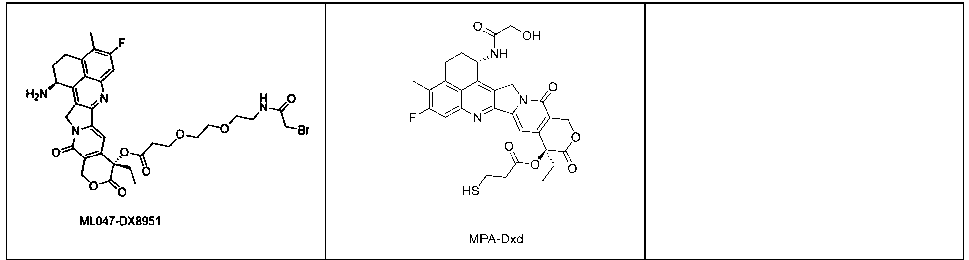

preferably, the carrier protein includes, but is not limited to, KLH, BSA, and OVA.

Further, the biotin-small molecule drug conjugate includes, but is not limited to, Biotin-PEG12-Dxd, Biotin-PEG10-Dxd, Biotin-PEG6-Dxd, Biotin-PEG6-Triazole-Dxd and Biotin-PEG10-Triazole-Dxd:

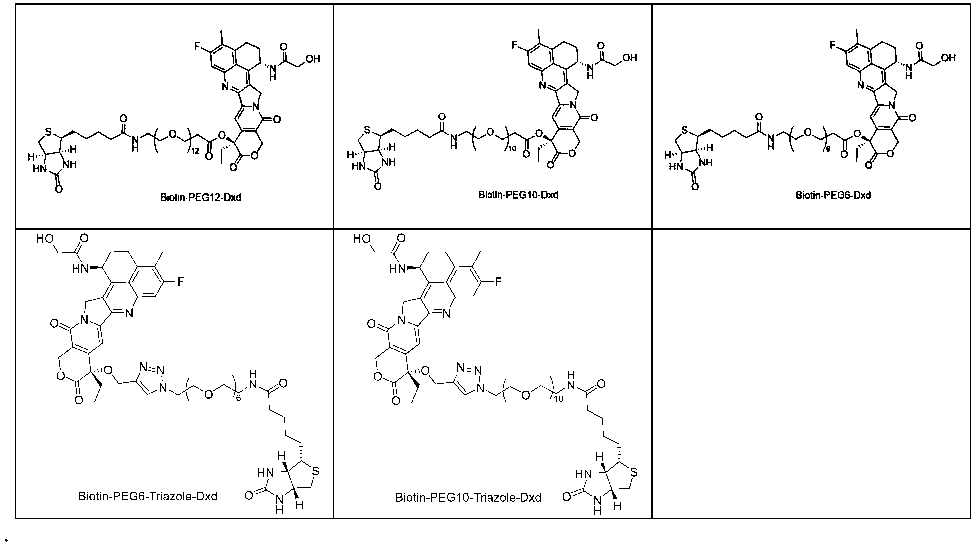

Further, a payload epitope masking unit capable of preventing the recovery unit from binding to the payload that remains conjugated to the ligand-drug conjugate is linked to the linker or the payload.

Further, the payload epitope masking unit does not interfere with the normal release of the payload after the ligand-drug conjugate enters a target cell.

The present disclosure also provides a method for producing the ligand-drug conjugate, comprising steps of:
1)screening to obtain an antibody, an antigen-binding fragment, a receptor, a fusion protein, a peptide, an oligopeptide, a peptidomimetic or an aptamer that specifically binds to the payload;
2)producing a ligand-drug conjugate using the screened antibody, antigen-binding fragment, receptor, fusion protein, peptide, oligopeptide, peptidomimetic or aptamer that specifically binds to the payload as a recovery unit;
3)detecting whether the screened antibody, antigen-binding fragment, receptor, fusion protein, peptide, oligopeptide, peptidomimetic or aptamer binds to the produced ligand-drug conjugate, and if not, a target ligand-drug conjugate is obtained; or detecting whether the antibody, antigen-binding fragment, receptor, fusion protein, peptide, oligopeptide, peptidomimetic, or aptamer binds to the produced drug-linker, and if not, a target ligand-drug conjugate is obtained;
4)if the screened antibody, antigen-binding fragment, receptor, fusion protein, peptide, oligopeptide, peptidomimetic or aptamer binds to the produced ligand-drug conjugate or the produced drug-linker, or molecular aggregation occurs between the ligand-drug conjugates or between the drug-linkers, then:
   A. further modifying or replacing the antibody, antigen-binding fragment, receptor, fusion protein, peptide, oligopeptide, peptidomimetic or aptamer in the recovery unit until ligand-drug conjugates or drug-linkers that do not aggregate and do not bind to the target ligand-drug conjugate or drug-linker are obtained; and/or
   B. screening a payload epitope masking unit capable of being linked to the linker or payload of the ligand-drug conjugate, wherein the linked payload epitope masking unit is capable of preventing the screened antibody, antigen-binding fragment, receptor, fusion protein, peptide, oligopeptide, peptidomimetic or aptamer from binding to the ligand-drug conjugate and does not affect the normal release of the payload after the ligand-drug conjugate enters the cell; and after a suitable payload epitope masking unit is screened, producing a target ligand-drug conjugate.

The present disclosure also provides a method for treating or preventing a disease, comprising administering a prophylactically or therapeutically effective amount of the ligand-drug conjugate, or a pharmaceutically acceptable salt, deuterated form, or solvate thereof; or the pharmaceutical composition, or a pharmaceutically acceptable salt, deuterated form, or solvate thereof; and a pharmaceutically acceptable excipient thereof to a subject in need thereof. The disease is preferably a tumor.

The present disclosure also provides use of the ligand-drug conjugate or a pharmaceutically acceptable salt, deuterated form, or solvate thereof, or the produced pharmaceutical composition in the manufacture of a medicament for treating or preventing a disease.

The present disclosure also provides an immunogen for screening a recovery unit.

Further, the immunogen includes, but is not limited to, MMAF, ML022-Dxd, ML022-D633-004, ML026-Dxd, ML031-D633-004, ML037-D633-004, ML042-Dxd, ML047-DX8951, MPA-Dxd, Biotin-PEG12-Dxd, Biotin-PEG10-Dxd, Biotin-PEG6-Dxd, Biotin-PEG6-Triazole-Dxd, or Biotin-PEG10-Triazole-Dxd, and a conjugate of a carrier protein with MMAF, ML022-Dxd, ML022-D633-004, ML026-Dxd, ML031-D633-004, ML037-D633-004, ML042-Dxd, ML047-DX8951, or MPA-Dxd.

Further, the carrier protein includes, but is not limited to, KLH, BSA, or OVA.

The present disclosure also provides a payload recovery unit that can specifically bind to a free payload of a ligand-drug conjugate, comprising:
(1) a VHH domain with CDR1-3 selected from amino acid sequences defined by:
   CDR1-3 as set forth in SEQ ID NOs: 103, 104, and 105;
   or (2) an amino acid sequence selected from the amino acid sequence as set forth in SEQ ID NO: 102;
   or (3) an amino acid sequence selected from combinations of VH and VL defined as follows:
      a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 7 respectively;
      a VH comprising HCDR1-3 with amino acid sequences as set forth in SEQ ID NOs: 9, 10, and 11 respectively, and a VL comprising LCDR1-3 with amino acid sequences as set forth in SEQ ID NO: 6, FAS, and SEQ ID NO: 13 respectively;
      a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 16 respectively;
      a VH comprising HCDR1-3 with amino acid sequences as set forth in SEQ ID NOs: 2, 3, and 20 respectively, and a VL comprising LCDR1-3 with amino acid sequences as set forth in SEQ ID NO: 6, YAS, and SEQ ID NO: 7 respectively;
      a VH comprising HCDR1-3 with amino acid sequences as set forth in SEQ ID NOs: 22, 23, and 24 respectively, and a VL comprising LCDR1-3 with amino acid sequences as set forth in SEQ ID NO: 26, WAS, and SEQ ID NO: 27 respectively;
      a VH comprising HCDR1-3 with amino acid sequences as set forth in SEQ ID NOs: 29, 30, and 31 respectively, and a VL comprising LCDR1-3 with amino acid sequences as set forth in SEQ ID NO: 33, KVS, and SEQ ID NO: 34 respectively;
      a VH comprising HCDR1-3 with amino acid sequences as set forth in SEQ ID NOs: 29, 36, and 37 respectively, and a VL comprising LCDR1-3 with amino acid sequences as set forth in SEQ ID NO: 33, KLS, and SEQ ID NO: 34 respectively;
      a VH comprising HCDR1-3 with amino acid sequences as set forth in SEQ ID NOs: 40, 41, and 42 respectively, and a VL comprising LCDR1-3 with amino acid sequences as set forth in SEQ ID NO: 44, KVS, and SEQ ID NO: 45 respectively;
      a VH comprising HCDR1-3 with amino acid sequences as set forth in SEQ ID NOs: 47, 48, and 42 respectively, and a VL comprising LCDR1-3 with amino acid sequences as set forth in SEQ ID NO: 50, KVS, and SEQ ID NO: 51 respectively;
      a VH comprising HCDR1-3 with amino acid sequences as set forth in SEQ ID NOs: 53, 54, and 55 respectively, and a VL comprising LCDR1-3 with amino acid sequences as set forth in SEQ ID NO: 57, DTS, and SEQ ID NO: 58 respectively;
      a VH comprising HCDR1-3 with amino acid sequences as set forth in SEQ ID NOs: 62, 63, and 64 respectively, and a VL comprising LCDR1-3 with amino acid sequences as set forth in SEQ ID NO: 66, SAS, and SEQ ID NO: 67 respectively;
      a VH comprising HCDR1-3 with amino acid sequences as set forth in SEQ ID NOs: 69, 70, and 71 respectively, and a VL comprising LCDR1-3 with amino acid sequences as set forth in SEQ ID NO: 73, WAS, and SEQ ID NO: 74 respectively;
      a VH comprising HCDR1-3 with amino acid sequences as set forth in SEQ ID NOs: 76, 77, and 78 respectively, and a VL comprising LCDR1-3 with amino acid sequences as set forth in SEQ ID NO: 80, GAS, and SEQ ID NO: 81 respectively;
      a VH comprising HCDR1-3 with amino acid sequences as set forth in SEQ ID NOs: 83, 84, and 85 respectively, and a VL comprising LCDR1-3 with amino acid sequences as set forth in SEQ ID NO: 87, SAS, and SEQ ID NO: 67 respectively;
      a VH comprising HCDR1-3 with amino acid sequences as set forth in SEQ ID NOs: 89, 90, and 91 respectively, and a VL comprising LCDR1-3 with amino acid sequences as set forth in SEQ ID NO: 93, HGT, and SEQ ID NO: 94 respectively;
      a VH comprising HCDR1-3 with amino acid sequences as set forth in SEQ ID NOs: 76, 77, and 78 respectively, and a VL comprising LCDR1-3 with amino acid sequences as set forth in SEQ ID NOs: 99, 77, and 100 respectively;
      a VH comprising an amino acid sequence as set forth in SEQ ID NO: 1, and a VL comprising an amino acid sequence as set forth in SEQ ID NO: 5;
      a VH comprising an amino acid sequence as set forth in SEQ ID NO: 8, and a VL comprising an amino acid sequence as set forth in SEQ ID NO: 12;
      a VH comprising an amino acid sequence as set forth in SEQ ID NO: 14, and a VL comprising an amino acid sequence as set forth in SEQ ID NO: 15;
      a VH comprising an amino acid sequence as set forth in SEQ ID NO: 17, and a VL comprising an amino acid sequence as set forth in SEQ ID NO: 18;
      a VH comprising an amino acid sequence as set forth in SEQ ID NO: 19, and a VL comprising an amino acid sequence as set forth in SEQ ID NO: 18;
      a VH comprising an amino acid sequence as set forth in SEQ ID NO: 21, and a VL comprising an amino acid sequence as set forth in SEQ ID NO: 25;
      a VH comprising an amino acid sequence as set forth in SEQ ID NO: 28, and a VL comprising an amino acid sequence as set forth in SEQ ID NO: 32;
      a VH comprising an amino acid sequence as set forth in SEQ ID NO: 35, and a VL comprising an amino acid sequence as set forth in SEQ ID NO: 38;
      a VH comprising an amino acid sequence as set forth in SEQ ID NO: 39, and a VL comprising an amino acid sequence as set forth in SEQ ID NO: 43;
      a VH comprising an amino acid sequence as set forth in SEQ ID NO: 46, and a VL comprising an amino acid sequence as set forth in SEQ ID NO: 49;
      a VH comprising an amino acid sequence as set forth in SEQ ID NO: 52, and a VL comprising an amino acid sequence as set forth in SEQ ID NO: 56;
      a VH comprising an amino acid sequence as set forth in SEQ ID NO: 59, and a VL comprising an amino acid sequence as set forth in SEQ ID NO: 60;
      a VH comprising an amino acid sequence as set forth in SEQ ID NO: 61, and a VL comprising an amino acid sequence as set forth in SEQ ID NO: 65;
      a VH comprising an amino acid sequence as set forth in SEQ ID NO: 68, and a VL comprising an amino acid sequence as set forth in SEQ ID NO: 72;
      a VH comprising an amino acid sequence as set forth in SEQ ID NO: 75, and a VL comprising an amino acid sequence as set forth in SEQ ID NO: 79;
      a VH comprising an amino acid sequence as set forth in SEQ ID NO: 82, and a VL comprising an amino acid sequence as set forth in SEQ ID NO: 86;
      a VH comprising an amino acid sequence as set forth in SEQ ID NO: 88, and a VL comprising an amino acid sequence as set forth in SEQ ID NO: 92;
      a VH comprising an amino acid sequence as set forth in SEQ ID NO: 95, and a VL comprising an amino acid sequence as set forth in SEQ ID NO: 96; and
      a VH comprising an amino acid sequence as set forth in SEQ ID NO: 97, and a VL comprising an amino acid sequence as set forth in SEQ ID NO: 98;
      wherein the CDR1, CDR2 and CDR3 sequences, the HCDR1, HCDR2 and HCDR3 sequences, and the LCDR1, LCDR2 and LCDR3 sequences are obtained based on an IMGT definition scheme;

or (4) a peptide comprising an amino acid sequence defined by SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, or SEQ ID NO: 129;
or (5) an aptamer comprising a nucleotide sequence defined by SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, or SEQ ID NO: 162.

The present disclosure also provides a method for reducing toxicity of a ligand-drug conjugate, wherein an effective amount of the corresponding payload recovery unit capable of binding free payloads of the ligand-drug conjugate is administered to a patient undergoing a treatment with the ligand-drug conjugate.

Further, the ligand-drug conjugate and the payload recovery unit may be administered simultaneously or sequentially.

The present disclosure also provides a nucleic acid comprising a sequence encoding the amino acid sequence in the payload recovery unit.

The present disclosure also provides a vector comprising the nucleic acid.

The present disclosure also provides a cell comprising the vector. Still further, the cell is selected from the group consisting of a CHO cell, a COS cell, a HEK-293 cell, anNSO cell, a PER.C6^{®} cell, and an Sp2.0 cell.

### BRIEF DESCRIPTION OF DRAWINGS

FIG. 1 is a schematic diagram of a ligand-drug conjugate (the ligand unit is in an IgG form, the recovery unit is a VHH specifically binding to a free payload, and a hexagram symbol represents a conjugated or free payload, similarly hereinafter).
FIG. 2 is a schematic diagram of a ligand-drug conjugate (the ligand unit is in an IgG form, and the recovery unit is an scFv specifically binding to a free payload).
FIG. 3 is a schematic diagram of a ligand-drug conjugate (the ligand unit is in an IgG form, and the recovery unit is a peptide specifically binding to a free payload).
FIG. 4 is a schematic diagram of a ligand-drug conjugate (the ligand unit is in an IgG form, and the recovery unit is an aptamer specifically binding to a free payload).
FIG. 5 is a schematic diagram of a ligand-drug conjugate (the ligand unit is in a VHH+Fc form, and the recovery unit is a VHH specifically binding to a free payload).
FIG. 6 is a schematic diagram of a ligand-drug conjugate (the ligand unit is in a VHH+Fc form, and the recovery unit is an scFv specifically binding to a free payload).
FIG. 7 is a schematic diagram of a ligand-drug conjugate (the ligand unit is in a VHH+Fc form, and the recovery unit is a peptide specifically binding to a free payload).
FIG. 8 is a schematic diagram of a ligand-drug conjugate (the ligand unit is in a VHH+Fc form, and the recovery unit is an aptamer specifically binding to a free payload).
FIG. 9 is a schematic diagram of a ligand-drug conjugate (the ligand unit is in a peptide form, and the recovery unit is a VHH specifically binding to a free payload; the schematic diagram does not represent that the payload is conjugated to the first amino acid site of the peptide, and the payload may be conjugated to any suitable site of the peptide, similarly hereinafter).
FIG. 10 is a schematic diagram of a ligand-drug conjugate (the ligand unit is in a peptide form, and the recovery unit is an scFv specifically binding to a free payload).
FIG. 11 is a schematic diagram of a ligand-drug conjugate (the ligand unit is in a peptide form, and the recovery unit is a peptide specifically binding to a free payload).
FIG. 12 is a schematic diagram of a ligand-drug conjugate (the ligand unit is in a peptide form, and the recovery unit is an aptamer specifically binding to a free payload).
FIG. 13 is a schematic diagram of a ligand-drug conjugate (the ligand unit is in a VHH form, and the recovery unit is an aptamer specifically binding to a free payload).
FIG. 14 is a schematic diagram of a ligand-drug conjugate (the ligand unit is in an scFv form, and the recovery unit is an aptamer specifically binding to a free payload).
FIG. 15 is a schematic diagram of a ligand-drug conjugate (the ligand unit is in an aptamer form, and the recovery unit is a peptide specifically binding to a free payload).
FIG. 16 is a schematic diagram of a ligand-drug conjugate (the ligand unit is in an aptamer form, and the recovery unit is an aptamer specifically binding to a free payload).
FIG. 17 is a schematic diagram of a ligand-drug conjugate (the ligand unit is in a bispecific antibody form, and the recovery unit is a VHH specifically binding to a free payload).
FIG. 18 is a schematic diagram of a ligand-drug conjugate (the ligand unit is in a bispecific antibody form, and the recovery unit is an scFv specifically binding to a free payload).
FIG. 19 is a schematic diagram of a ligand-drug conjugate (the ligand unit is in a bispecific antibody form, and the recovery unit is a peptide specifically binding to a free payload).
FIG. 20 is a schematic diagram of a ligand-drug conjugate (the ligand unit is in a bispecific antibody form, and the recovery unit is an aptamer specifically binding to a free payload).
FIG. 21 is a schematic diagram of specific binding of the recovery unit of a ligand-drug conjugate to a free payload (the ligand unit is in an IgG form, the recovery unit is a VHH specifically binding to the free payload, and the payload is conjugated to the VHH).
FIG. 22 is a schematic diagram of a ligand-drug conjugate (the ligand unit is in an IgG form, the recovery unit is a VHH specifically binding to a free payload, and the payload is conjugated to a linker unit between the IgG and the VHH).
FIG. 23 is a schematic diagram of a ligand-drug conjugate (the ligand unit is in an IgG form, the recovery unit is a VHH specifically binding to a free payload, and the IgG and the VHH are directly connected, i.e., there is no linker unit between the ligand unit and the recovery unit).
FIG. 24 shows cell protection experiment results of No. 62 and No. 64 chimeric antibodies.
FIG. 25 shows cell protection activity of PP-1M-#60 and PP-1M-#66 molecules.
FIG. 26 shows cell protection experiment results of No. J4 chimeric antibody.
FIG. 27 shows cell protection experiment results of DXD1-1, DXD1-2, DXD2-1, DXD2-2, DXD5, DXD8, and DXD10 antibodies.
FIG. 28 shows cell protection experiment results of JLD1-JLD27 antibodies.
FIG. 29 shows cell protection experiment results of D2 and other antibodies.
FIG. 30 shows cell protection activity of PP-1X-#9 and PP-1X-#17 molecules.
FIG. 31 shows experiment results of specific binding of No. 86 antibody to DXD.
FIG. 32 shows cell protection experiment results of No. 86 antibody.
FIG. 33 is a schematic diagram of a structure of an anti-MMAE peptide+Fc fusion molecule.
FIG. 34 shows experiment results of specific binding of various molecules to OVA-MMAF and OVA.
FIG. 35 shows identification results of competitive binding of free MMAE to a peptide molecule.
FIG. 36 is a schematic diagram of a structure of an anti-MMAE peptide+VHH+Fc fusion molecule.
FIG. 37 shows verification of specific binding of a PP-1A-#600 fusion molecule to MMAE.
FIG. 38 shows a cell protection experiment of a PP-1A-#600 fusion molecule.
FIG. 39 is a schematic diagram of a structure of an anti-DXD peptide+Fc fusion molecule.
FIG. 40 shows Dot Blot experiment results.
FIG. 41 shows a cell protection experiment of a PP-1A peptide.
FIG. 42 shows experiment results of specific binding of a fusion molecule to OVA-MMAF and BSA-MMAF.
FIG. 43 shows competitive ELISA experiment results of a PP-1B-#74 molecule with OVA-MMAF and BSA-MMAF.
FIG. 44 shows experiment results of specific binding of PP-1D molecules to BSA-MMAF and OVA-MMAF.
FIG. 45 is a schematic diagram of a structure of an antibody-aptamer conjugate (targeting MMAE).
FIG. 46 shows experiment results of specific binding of various molecules to OVA-DXD and OVA, or BSA-DXD and BSA.
FIG. 47 shows competitive ELISA binding experiment results of various molecules.
FIG. 48 is a schematic diagram of a structure of an antibody-aptamer conjugate (targeting DXD).
FIG. 49 shows ELISA binding experiment results.
FIG. 50 shows experiment results of cell protection effects of various molecules (inhibiting DXD toxicity).
FIG. 51 shows cytotoxicity test results of PR-ADC.
FIG. 52 shows results of a pharmacodynamic study of PR-ADC in a human bladder cancer cell HT-1376 subcutaneous xenograft model.
FIG. 53 shows a pharmacodynamic study of PR-ADC in a human colon cancer cell RKO subcutaneous xenograft model.
FIG. 54 is a schematic diagram of a ligand-drug conjugate (the ligand unit is in an scFv form, the recovery unit is an IgG specifically binding to a free payload, and the payload is conjugated to the scFv).
FIG. 55 is a schematic diagram of a structure of a bispecific antibody-linker-drug (MMAE).
FIG. 56 is a schematic diagram of a ligand-drug conjugate (the ligand unit is in an IgG form, and the recovery unit is an scFv specifically binding to a free payload).
FIG. 57 is a schematic diagram of a conventional antibody-drug conjugate.
FIG. 58 is a schematic diagram of a structure of a bispecific antibody-linker-drug (DXD).

### DETAILED DESCRIPTION

In the present disclosure, the term "ligand-drug conjugate" refers to a class of drugs that can be used for inhibiting proliferation of tumor cells or cancer cells and used for treating cancer or for treating a disease such as an autoimmune disease or inflammation in a patient. Ligand-drug conjugates are correspondingly used in a variety of settings to treat a variety of diseases such as cancer, an autoimmune disease, or inflammation. Ligand-drug conjugates are used to deliver a drug to tumor cells, cancer cells, or other cells. Without being bound by theory, in some embodiments, a ligand unit of the ligand-drug conjugate will bind or conjugate to an associated antigen of a tumor or non-tumor target, and the ligand-drug conjugate is taken up within the tumor cell, cancer cell, or other cell via receptor-mediated endocytosis or other internalization mechanisms. In some embodiments, the antigen is connected to the tumor cell, cancer cell, or other cell, or may be an extracellular matrix protein associated with the tumor cell, cancer cell, or other cell. Once entered into the cell, a payload is released intracellularly via activation of an activation unit.

In the present disclosure, the term "tumor" generally refers to all neoplastic cell growth and proliferation, whether malignant or benign, and all pre-cancerous and cancerous cells and tissues. In the examples of the present disclosure, the tumor may include a solid tumor and/or a hematological tumor. The terms "cancer", "cancerous", "cell proliferative disorder", "proliferative disorder", and "tumor" are not mutually exclusive when referred to herein. In some embodiments, a tumor may refer to a physical mass containing a majority of cancer cells, for example, cells displaying characteristics of any cancer described herein. Examples of a tumor can include a primary tumor of any of the above-mentioned types of cancer or a metastatic tumor at a second site derived from any of the above-mentioned types of cancer.

In the present disclosure, the term "tumor antigen" generally includes meanings known in the art, which includes any molecule expressed on a tumor cell (or associated with tumor cell development), known or believed to contribute to tumorigenic properties of the tumor cell. Many tumor antigens are known in the art. Whether a molecule is a tumor antigen can also be determined according to techniques and assays well known to those skilled in the art, such as a clonogenic assay, transformation assay, in vitro or in vivo tumor formation assay, gel migration assay, gene knockout analysis, and the like. The term "tumor antigen" can refer to a human transmembrane protein, i.e., a cell membrane protein anchored in a lipid bilayer of a cell.

In the present disclosure, the term "ligand unit" refers to a class of small molecule or macromolecule compounds capable of specifically binding to a biomolecule (such as a protein, a nucleic acid, a receptor, etc.). In the present disclosure, the ligand unit can be, but is not limited to, an antibody or an antigen-binding fragment, a receptor, a peptide, an oligopeptide, a peptidomimetic, a fusion protein, or an aptamer specifically binding to a tumor antigen.

In the present disclosure, the term "payload" refers to a drug molecule that may cause cytotoxicity; for example, any molecule used for treating a tumor or cancer can be used, including a compound, DNA, RNA, a peptide, an immunostimulant, and the like; it can also be a natural drug (e.g., a drug naturally containing one or more functional groups allowing covalent connection to a conjugate), or can be a drug chemically modified to incorporate a functional group allowing covalent connection to an adjacent group or moiety (e.g., a group selected from the group consisting of a hydroxyl group, a carboxyl group, an amino group, and a thiol group), provided that the modified drug has pharmacological activity.

In the present disclosure, the term "free payload" refers to a molecule portion containing a payload that is released from a ligand-drug conjugate after the ligand-drug conjugate enters a human body, especially a molecular structure that causes damage to normal cells of the human body.

In the present disclosure, the term "conjugation" refers to a process of connecting two or more molecules via a chemical bond to form a new complex molecule, such as a covalent bond.

In the present disclosure, the term "antibody" (also synonymously referred to as "immunoglobulin (Ig)") covers an antibody in a natural form and an antibody from artificial screening, configuration modification or amino acid mutation, including and not limited to a monoclonal antibody, a bispecific antibody, a multispecific antibody, a nanobody (heavy chain antibody, HcAb), and an immunoglobulin new antigen receptor (IgNAR). For example, it includes, in particular, a recombinant antibody, a monoclonal antibody, and a polyclonal antibody. The antibody can be an antibody of a different isotype, for example, an IgG (e.g., IgG1, IgG2, IgG3, or IgG4 subtype), IgA1, IgA2, IgD, IgE, or IgM antibody.

In the present disclosure, the term "antigen-binding fragment" refers to a fragment or a fragment combination of an antibody that functionally binds to an antigen, usually not containing an Fc region, such as scFv, Fab, Fab', F(ab')₂, Fd, Fv, dAb, Fd, sdAb, VHH, Diabody, and variable domain of immunoglobulin new antigen receptor (VNAR), which comprises at least a portion of an antibody sufficient to confer specific antigen-binding ability to the fragment. An antigen-binding fragment of an antibody (e.g., the above-mentioned antibody fragment) can be obtained from a given antibody using conventional techniques known to those skilled in the art (e.g., recombinant DNA technology or enzymatic or chemical cleavage methods), and the antigen-binding fragment of the antibody is screened for specificity in the same manner as for a complete antibody.

Preferably, the antibody is an IgG protein, more preferably an IgG1, IgG2, IgG3, or IgG4 protein. Most preferably, the antibody is an IgG1 protein. The antibody can be a human antibody or derived from other species. Preferably, the antibody is a human antibody. The antibody can be a monovalent antibody or a bivalent antibody or a multivalent antibody. A human IgG or IgD in a natural form is a Y-shaped tetrameric molecule (bivalent) composed of two heavy chains and two light chains, each heavy chain having a variable domain (VH) and three constant domains (CH1, CH2, CH3). Each light chain has a variable region (VL) and a constant region (CL).

In the present disclosure, the term "specific binding" refers to binding of an antibody or an antigen-binding fragment and the like to a corresponding epitope of a target antigen thereof in a highly selective manner without binding to a variety of other antigens. Typically, the antibody or the antigen-binding fragment and the like binds with an affinity of at least about 1×10⁻⁴ M, 10⁻⁵ M, 10⁻⁶ M, 10⁻⁷ M, 10⁻⁸ M, 10⁻⁹ M, 10⁻¹⁰ M, 10⁻¹¹ M, 10⁻¹² M or 10⁻¹³ M.

In the present disclosure, the term "peptide" can refer to a complete protein encoded by a complete amino acid sequence or a part thereof. The term "oligopeptide", also known as "small peptide" or "oligo", is a short-chain peptide formed by a few amino acids connected by peptide bonds.

In the present disclosure, the term "fusion protein" refers to a single protein molecule capable of being expressed intracellularly formed by connecting coding genes of two or more proteins via genetic engineering technology. It is typically used for enhancing stability of a protein, improving solubility thereof, increasing expression efficiency, or conferring a new function to a protein. The fusion protein in the present disclosure includes, but is not limited to, a tag fusion protein, a domain fusion protein, and a multifunctional fusion protein.

In the present disclosure, the term "peptidomimetic" refers to a class of compounds mimicking a peptide structure, which have chemical or biological properties similar to a peptide but are not a natural peptide. They can mimic a secondary structure of a peptide (such as an α-helix or β-sheet), or have an ability to interact with a specific biological target. A molecular structure of a peptidomimetic can comprise a non-natural amino acid, a connection manner other than a peptide bond, and various side chain modifications.

In the present disclosure, the term "aptamer" typically refers to a special oligonucleotide fragment capable of binding multiple target substances with high specificity and high selectivity, which can be a DNA, RNA, or XNA (nucleic acid analog) sequence, typically obtained from a nucleic acid molecule library through an in vitro screening technique-Systematic Evolution of Ligands by Exponential Enrichment (SELEX), and is widely applied in biosensors, drug delivery, disease treatment, and microbial detection. Compared with a traditional protein probe, the aptamer has advantages such as easy synthesis, good stability, smaller molecular weight and volume, and also has characteristics such as better thermal stability and ease of modification.

In the present disclosure, the term "bispecific antibody" is used in its broadest sense, covering an antibody having specificities for two epitopes, referring to an antibody capable of specifically binding at least two different antigenic determinants. Typically, a bispecific antibody comprises two antigen-binding sites, wherein each is specific for a different antigenic determinant. In certain embodiments, the bispecific antibody is capable of simultaneously binding two antigenic determinants, particularly two antigenic determinants expressed on two different cells. The entire contents of "Kontermann RE, Brinkmann U. Bispecific antibodies. Drug Discov Today. 2015 Jul;20(7):838-47. and Thakur A, Huang M, Lum LG. Bispecific antibody based therapeutics: Strengths and challenges. Blood Rev. 2018 Jul;32(4):339-347" are incorporated herein by reference.

In the present disclosure, the term "multispecific antibody" is used in its broadest sense, covering an antibody having specificities for two or more epitopes; these multispecific antibodies include, but are not limited to: an antibody comprising a heavy chain variable region (VH) and a light chain variable region (VL), wherein the VH-VL unit has specificities for two or more epitopes; an antibody having two or more VH and VL regions, each VH-VL unit binding to a different target or a different epitope of a same target; an antibody having two or more single variable regions (e.g., VHH), each single variable region binding to a different target or a different epitope of a same target.

In the present disclosure, the term "low affinity" refers to that an equilibrium dissociation constant K_{D}(M) of a recovery unit with a payload in a conjugated state in the ligand-drug conjugate is greater than 10⁻⁷.

In the present disclosure, the term "pharmaceutically acceptable" ingredient generally refers to a substance suitable for use in humans and/or animals without undue adverse side effects (such as toxicity, irritation, and allergic response), i.e., having a reasonable benefit/risk ratio.

In the present disclosure, the term "solvate" generally refers to an association or complex of one or more solvent molecules with the compound of the embodiment of the present disclosure. Non-limiting examples of solvents forming solvates include, but are not limited to, water, isopropanol, ethanol, methanol, DMSO, ethyl acetate, acetic acid, and ethanolamine.

In the present disclosure, the term "isotope" generally includes atoms having the same atomic number but different mass numbers. Examples of isotopes that can be incorporated into the compound and the pharmaceutically acceptable salt thereof described in the embodiments of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹⁵N, ¹⁷O, ¹⁸O, ³¹P, ³²P, ³⁵S, ¹⁸F, ³⁶Cl, ¹²³I, and ¹²⁵I.

In the present disclosure, the term "non-exposed epitope" is sometimes also referred to as "cryptic epitope" or "hidden epitope". A non-exposed epitope refers to an epitope derived from a cancer-specific antigen that is not necessarily processed/presented as an antigen and is "hidden" from immune recognition. Non-exposed epitopes typically appear on APCs at very low concentrations and the autoreactive T cells are not absent. A non-exposed epitope is not presented for recognition by T cells unless it is produced at an abnormally high concentration, or unless it escapes a configuration of its native antigen. A cryptic antigen epitope derived from a cancer-specific antigen can be used to break tolerance of T cells to a tumor and induce an effective immune response against the tumor. Such a principle has been described in Pardoll, et al., PNAS, Vol. 96, pp. 5340-5342 (1999), the entire contents of which are incorporated herein by reference.

In the present disclosure, the term "treatment" refers to clinical intervention designed to alter a natural course of an individual or a cell being treated during a clinicopathological process. Desired therapeutic effects include reducing a rate of disease progression, improving or alleviating a disease state, and alleviating or improving a prognosis. For example, one or more symptoms associated with a treated disease or condition (such as cancer, inflammation, or an autoimmune disease, etc.) are alleviated or eliminated.

In the present disclosure, the term "effective amount" refers to an amount sufficient to cure or at least partially arrest a disease and a complication thereof in a patient already suffering from the disease. Determination of such an effective amount is completely within the capability of those skilled in the art. For example, an amount effective for therapeutic use will depend on severity of the disease to be treated, an overall state of the patient's own immune system, general conditions of the patient such as age, weight, and gender, a mode of administration of the drug, and other treatments administered simultaneously, etc.

Hereinafter, embodiments of the present disclosure will be described in detail in conjunction with examples, but those skilled in the art will understand that the following examples are only used to illustrate the present disclosure and should not be considered as limiting the scope of the present disclosure.

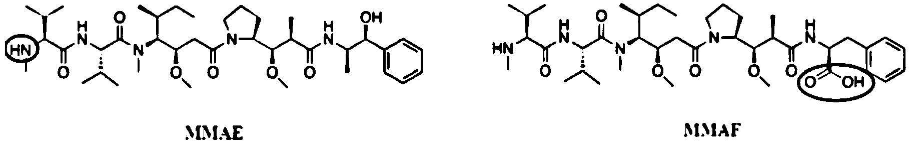

Structures of MMAE and MMAF are shown above. According to reports in the literature (Bordeau BM, Nguyen TD, Polli JR, Chen P, Balthasar JP. Payload-Binding Fab Fragments Increase the Therapeutic Index of MMAE Antibody-Drug Conjugates. Mol Cancer Ther. 2023 Apr 3;22(4):459-470., Nguyen TD, Bordeau BM, Balthasar JP. Use of Payload Binding Selectivity Enhancers to Improve Therapeutic Index of Maytansinoid-Antibody-Drug Conjugates. Mol Cancer Ther. 2023 Nov 1;22(11):1332-1342.), because it is desired that the target epitope of the screened anti-MMAE/MMAF antibody binds the secondary amine at the N-terminus on the left side, and because the carboxyl group on the MMAF structure is easier to derivatize and conjugate for generating an immunogen, and conjugation does not affect the secondary amine at the N-terminus, MMAF was adopted to replace MMAE to construct the immunogen in some examples in the text of the present patent application. Experimental results showed that the relevant molecules obtained by screening had strong binding activity to MMAF, which can also be understood as that the relevant molecules were also capable of specifically binding free MMAE.

### Specific Examples

### Example 1 Preparation of Immunogens

### Example 1-1 Synthesis of ML022-Dxd

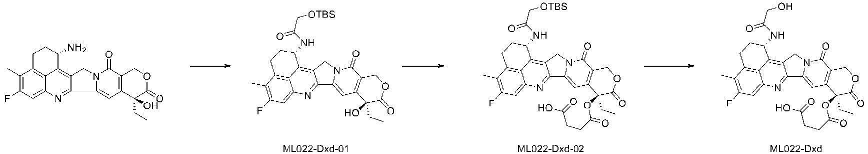

2-((Tert-butyldimethylsilyl)oxy)acetic acid (393.84 mg, 2.07 mmol) was weighed and placed in a 25 mL single-neck flask. 10 mL of N,N-dimethylformamide was added for complete dissolution. The mixture was stirred at room temperature. HATU (CAS#148893-10-1, 786.86 mg, 2.07 mmol), N,N-diisopropylethylamine (364.72 mg, 2.82 mmol), and exatecan mesylate (500 mg, 0.94 mmol) were weighed and added to the reaction system sequentially, and the reaction was performed at room temperature. Upon completion of the reaction as monitored by LC-MS, purification was performed by reversed-phase flash preparative chromatography. The target product ML022-Dxd-01 (430 mg) was obtained with a purity of 91.41% and a yield of 68.76%. ESI-MS (m/z): 608.75 [M+H]⁺.

ML022-Dxd-01 (300 mg, 0.49 mmol) was weighed and placed in a 25 mL single-neck flask. 10 mL of dichloromethane was added for complete dissolution. The mixture was stirred at 5°C. Succinic anhydride (74.1 mg, 0.74 mmol) and DBU (CAS #6674-22-2, 150.3 mg, 0.98 mmol) were weighed and added to the reaction system sequentially, and the reaction was performed at room temperature. Upon completion of the reaction as monitored by LC-MS, purification was performed by normal-phase column chromatography. The target product ML022-Dxd-02 (196 mg) was obtained with a purity of 88.1% and a yield of 49.36%. ESI-MS (m/z): 708.81 [M+H]⁺.

ML022-Dxd-02 (500 mg, 0.7 mmol) was weighed and placed in a 25 mL single-neck flask. 5 mL of tetrahydrofuran and 5 mL of water were added and dissolved by ultrasonication. The mixture was stirred at room temperature. 3 mL of formic acid was added dropwise to the system, and the reaction was performed at room temperature. Upon completion of the reaction as monitored by LC-MS, purification was performed by reversed-phase high-pressure preparative chromatography. The target product ML022-Dxd (256 mg) was obtained with a purity of 97.64% and a yield of 59.62%. ESI-MS (m/z): 594.51 [M+H]⁺.

### Example 1-2 Synthesis of ML022-D633-004

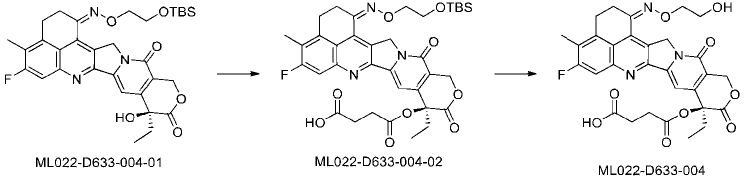

According to the synthesis procedure of ML022-Dxd, the raw material in the second step was changed to ML022-D633-004-01 to obtain the target product ML022-D633-004 (19.94 mg). Purity: 94.25%, yield: 44.82%. ESI-MS (m/z): 594.65 [M+H]⁺.

### Example 1-3 Synthesis of ML026-Dxd

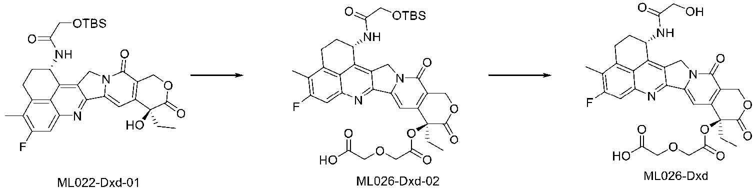

According to the synthesis procedure of ML022-Dxd, the raw material in the second step was changed to diglycolic anhydride. The target product ML026-Dxd (60 mg) was obtained with a purity of 95.89% and a yield of 32.01%. ESI-MS (m/z): 610.22 [M+H]⁺.

### Example 1-4 Synthesis of ML031-D633-004

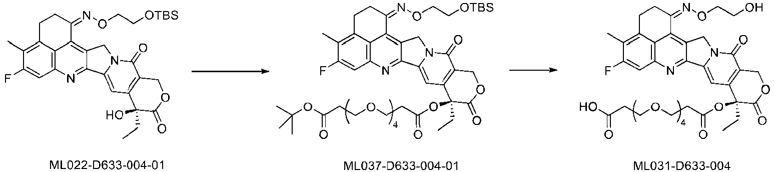

According to the synthesis procedure of ML022-D633-004, the raw material in the second step was changed to carboxylic acid-tetraethylene glycol-tert-butyl propionate, to obtain the target product ML031-D633-004 (16.06 mg) with a purity of 95.72% and a yield of 24.38%. ESI-MS (m/z): 769.97 [M+H]⁺.

### Example 1-5 Synthesis of ML037-D633-004

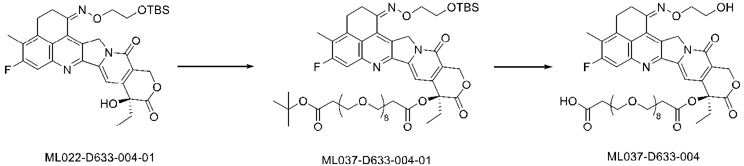

According to the synthesis procedure of ML022-D633-004, the raw material in the second step was changed to carboxyl-octaethylene glycol-tert-butyl propionate, to obtain the target product ML037-D633-004 (14.41 mg) with a purity of 94.52% and a yield of 10.72%. ESI-MS (m/z): 947.37 [M+H]⁺.

### Example 1-6 Synthesis of ML042-Dxd

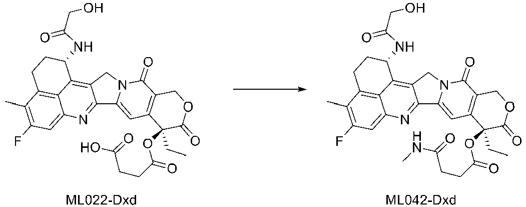

ML022-Dxd (30 mg, 0.54 mmol) was weighed and placed in a 25 mL single-neck flask. 10 mL of dichloromethane was added for complete dissolution. The mixture was stirred at room temperature. HATU (21.14 mg, 0.55 mmol), N,N-diisopropylethylamine (9.8 mg, 0.75 mmol), and methylamine hydrochloride (2.35 mg, 0.75 mmol) were weighed and added to the reaction system sequentially, and the reaction was performed at room temperature. The solvent was removed by rotary evaporation, and purification was performed by reversed-phase high-pressure preparative chromatography. The target product ML042-Dxd (14.19 mg) was obtained with a purity of 93.75% and a yield of 43.39%. ESI-MS (m/z): 607.67 [M+H]⁺.

### Example 1-7 Synthesis of ML047-DX8951

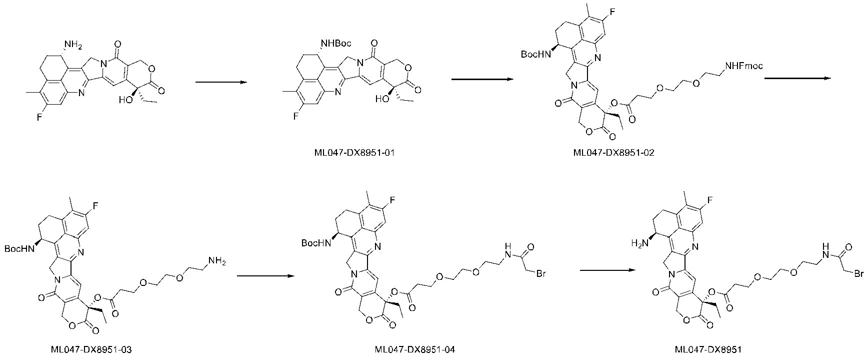

Exatecan mesylate (500 mg, 0.94 mmol), TEA (285.56 mg, 0.28 mmol), and Boc anhydride (410.59 mg, 1.88 mmol) were weighed and placed in a 100 mL single-neck flask sequentially. 50 mL of dichloromethane was added for complete dissolution, and the mixture was stirred at room temperature overnight. Upon completion of the reaction as monitored by LC-MS, the mixture was washed with water and saturated saline solution, and the organic phases were combined, dried over anhydrous sodium sulfate, and solvent was removed by rotatory evaporation. A crude product of ML047-DX8951-01 (630 mg) was obtained and directly used in the next step. ESI-MS (m/z): 535.95 [M+H]⁺.

1-(9H-Fluoren-9-yl)-3-oxo-2,7,10-trioxa-4-azatridecan-13-oic acid (820.41 mg, 2.05 mmol) was weighed and placed in a 100 mL single-neck flask. 50 mL of dichloromethane was added for complete dissolution. The mixture was stirred at room temperature. EDCI (CAS#25952-53-8, 1.07 g, 5.6 mmol), DMAP (CAS#1122-58-3, 684.3 mg, 5.8 mmol), and ML047-DX8951-01 (1.0 g, 1.87 mmol) were weighed and added to the reaction system sequentially, and the reaction was performed at room temperature. Upon completion of the reaction as monitored by LC-MS, the solvent was removed by rotary evaporation, and purification was performed by normal-phase silica gel column chromatography. The target product ML047-DX8951-02 (1.51 g) was obtained with a purity of 95.75% and a yield of 84.44%. ESI-MS (m/z): 918.47 [M+H]⁺.

ML047-DX8951-02 (200 mg, 0.21 mmol) was weighed and placed in a 25 mL single-neck flask. 10 mL of dichloromethane was added and dissolution was achieved by ultrasonication. The mixture was stirred at room temperature. 3 mL of diethylamine was added dropwise to the system, and the reaction was performed at room temperature. Upon completion of the reaction as monitored by LC-MS, the solvent was removed by rotary evaporation, the residue was dissolved in dichloromethane and solvent was removed by rotatory evaporation, which was repeated three times. A crude product of ML047-DX8951-03 (150 mg) was obtained and directly used in the next step. ESI-MS (m/z): 695.56 [M+H]⁺.

Crude ML047-DX8951-03 (30 mg, 0.043 mmol) was weighed and placed in a 25 mL single-neck flask. 10 mL of dichloromethane was added and dissolved by ultrasonication. The mixture was stirred at room temperature. 2,5-pyrrolidinedione, 1-(bromoacetyl)- (9CI) (28.5 mg, 0.12 mmol) was weighed and added to the above system. Upon completion of the reaction as monitored by LC-MS, the mixture was washed with water and saturated saline solution, and the organic phases were combined and solvent was removed by rotatory evaporation to obtain crude ML047-DX8951-04 (35 mg), which was directly used in the next step. ESI-MS (m/z): 815.96 [M+H]⁺.

Crude ML047-DX8951-04 (30 mg, 0.036 mmol) was weighed and placed in a 25 mL single-neck flask. 5 mL of dichloromethane was added and dissolution was achieved by ultrasonication. The mixture was stirred at room temperature. 2 mL of TFA (CAS#76-05-1) was added dropwise to the system, and the reaction was performed at room temperature. Upon completion of the reaction as monitored by LC-MS, purification was performed by reversed-phase high-pressure preparative chromatography. The target product ML047-DX8951 (3.13 mg) was obtained with a purity of 96.40% and a yield of 11.46%. ESI-MS (m/z): 715.75 [M+H]⁺.

### Example 1-8 Synthesis of MPA-Dxd

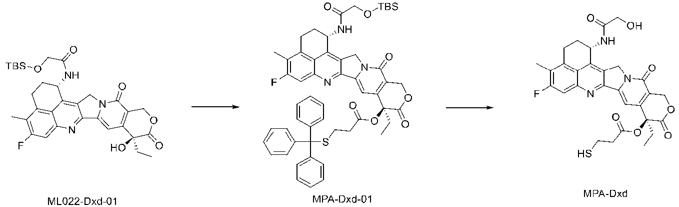

Compound ML022-Dxd-01 (270.12 mg, 0.34 mmol), 3-(tritylthio)propionic acid (324.54 mg, 0.68 mmol), DIC (162 µL, 0.68 mmol), and DMPA (27.41 mg, 0.068 mmol) were weighed and dissolved in 6 mL of dichloromethane. The reaction was performed at room temperature for 4 h. 10 mL of purified water was added to the reaction system for extraction. The solvent in the organic phase was removed by rotatory evaporation to obtain 240.14 mg of crude product MPA-Dxd-01 with a yield of 57.54%. ESI-MS (m/z): 938.36 [M+H]⁺.

The crude product MPA-Dxd-01 was reconstituted with 7 mL of dichloromethane, and 2 mL of TFA and 1 mL of triethylsilane were added. The reaction was performed at room temperature for 18 h. After purification by preparative liquid chromatography, the target product MPA-Dxd (62.14 mg) was obtained with a yield of 41.61%. ESI-MS (m/z): 582.16 [M+H]⁺.

### Example 1-9 Synthesis of Biotin-PEG12-Dxd

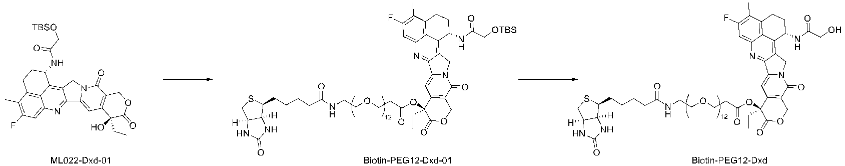

Biotin-PEG12-carboxyl (27.78 mg, 32.91 µmol), DCC (CAS#538-75-0, 13.58 mg, 65.82 µmol), and DMAP (2.01 mg, 16.45 µmol) were weighed and placed in a 10 mL single-neck flask sequentially. 3 mL of dichloromethane and 3 mL of tetrahydrofuran were added for dissolution, and the mixture was stirred at 0°C. ML022-Dxd-01 (20 mg, 32.91 µmol) was weighed and dissolved in 2 mL of dichloromethane, then added dropwise to the above system, and the reaction was performed at room temperature. Upon completion of the reaction as monitored by LC-MS, the reaction solution was evaporated to dryness under rotation, and the obtained crude product Biotin-PEG12-Dxd-01 (50 mg) was directly used in the next step. ESI-MS (m/z): 1434.34 [M+H]⁺.

Crude Biotin-PEG12-Dxd-01 (50 mg, 0.28 mmol) was weighed and placed in a 25 mL single-neck flask. 2 mL of tetrahydrofuran and 2 mL of water were added and dissolved by ultrasonication. While stirring at room temperature, 1 mL of formic acid was added dropwise to the system, and the reaction was performed at room temperature. Upon completion of the reaction as monitored by LC-MS, purification was performed by reversed-phase high-pressure preparative chromatography. The target product Biotin-PEG12-Dxd (7.89 mg) was obtained with a purity of 95.88% and a yield of 35.74%. ESI-MS (m/z): 1319.94 [M+H]⁺.

### Example 1-10 Synthesis of Biotin-PEG10-Dxd

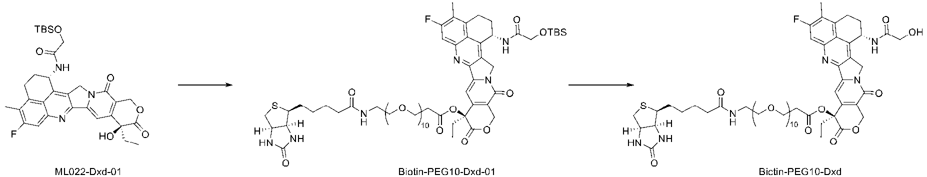

According to the synthesis route of Biotin-PEG12-Dxd, the raw material was changed to biotin-PEG10-carboxyl. The target product Biotin-PEG10-Dxd (16.6 mg) was obtained with a purity of 95.88% and a yield of 13.18%. ESI-MS (m/z): 1232.41 [M+H]⁺.

### Example 1-11 Synthesis of Biotin-PEG6-Dxd

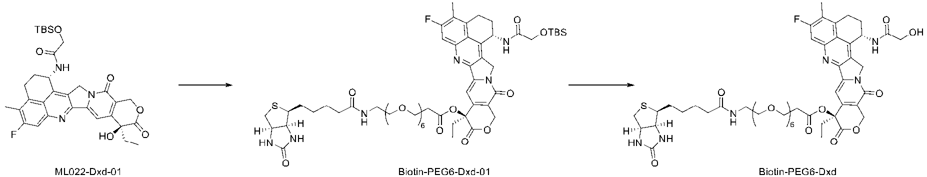

According to the synthesis route of Biotin-PEG12-Dxd, the raw material was changed to biotin-PEG6-carboxyl. The target product Biotin-PEG6-Dxd (22.14 mg) was obtained with a purity of 95.91% and a yield of 24.51%. ESI-MS (m/z): 1056.98 [M+H]⁺.

### Example 1-12 Synthesis of Biotin-PEG6-Triazole-Dxd

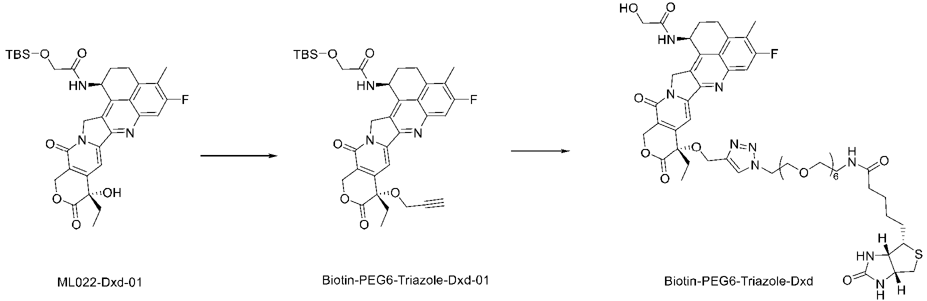

NaH (31 mg) was weighed and placed in a three-neck flask, and the system was purged and protected with argon, cooled to 0°C, and stirred. A solution of ML022-Dxd-01 (300 mg, 0.5 mmol) in N,N-dimethylformamide (20 mL) was slowly added dropwise, the mixture was stirred at low temperature for 30 minutes, and a solution of propargyl bromide (91 mg) in N,N-dimethylformamide (10 mL) was slowly added dropwise. The mixture was stirred at low temperature and gradually warmed to room temperature, then stirred overnight. The system was purified by reversed-phase high-pressure preparative chromatography. The target product Biotin-PEG6-Triazole-Dxd-01 (23 mg) was obtained as a crude product. ESI-MS (m/z): 646.21 [M+H]⁺.

Biotin-PEG6-Triazole-Dxd-01 (8 mg, 12 µmol) was dissolved in 2 mL of acetone and 1 mL of water. Compound N3-PEG6-Biotin (7.5 mg, 12 µmol) and copper sulfate pentahydrate (5 mg) were added at room temperature. The system was placed in a 10°C low-temperature bath and stirred. An aqueous solution (1 mL) containing sodium ascorbate (4 mg) was slowly added dropwise to the reaction. The mixture was stirred at 10°C for 1 h. The system was quenched with acetonitrile (1 mL) and purified by preparative liquid chromatography to obtain the target product Biotin-PEG6-Triazole-Dxd (3.85 mg) with a purity of 94.48% and a yield of 25%. ESI-MS (m/z): 1108.25 [M+H]⁺.

### Example 1-13 Synthesis of Biotin-PEG10-Triazole-Dxd

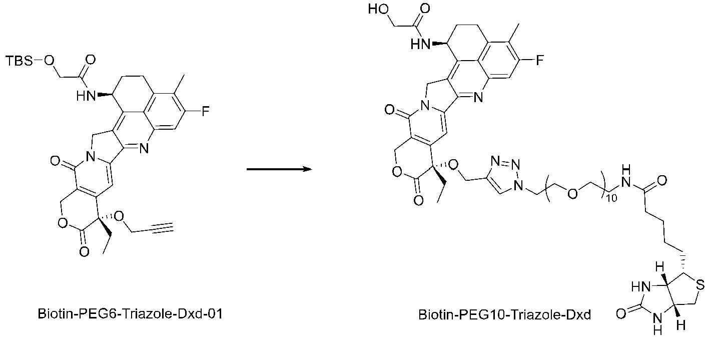

According to the synthesis route of Biotin-PEG6-Triazole-Dxd, the raw material in the second step was changed to N3-PEG10-Biotin. The target product Biotin-PEG10-Triazole-Dxd (2.77 mg) was obtained with a yield of 15%. ESI-MS (m/z): 1329.09 [M+H]⁺.

### Example 1-14 Synthesis of Immunoconjugates (Carrier Protein (BSA/OVA/KLH)-MMAF, Carrier Protein (BSA/OVA/KLH)-ML022-Dxd, Carrier Protein (BSA/OVA/KLH)-ML022-D633-004, Carrier Protein (BSA/OVA/KLH)-ML031-D633-004)

Drug Activation Process: The drug, EDC, and NHS were dissolved in DMF, respectively. The drug was activated using EDC and NHS, and reacted at room temperature with shaking at 600 rpm for 15 min.

Conjugation Process of Drug and Carrier Protein (BSA/OVA/KLH): The protein was dissolved in PBS. 10 to 30 eq of the activated drug was added to the carrier protein (BSA/OVA/KLH) system, and the concentration was adjusted to 5 mg/ml. The reaction was performed overnight at room temperature.

Buffer Exchange and Determination : After the reaction was completed, the reaction system was subjected to thorough buffer exchange with PBS buffer to remove excess small molecules, and the sample was subjected to ELISA for determination of conjugation status.

### Example 1-15 Synthesis of Immunoconjugates (Carrier Protein (BSA/OVA/KLH)-ML047-DX8951, Carrier Protein (BSA/OVA/KLH)-MPA-Dxd)

Reduction Process of Carrier Protein (BSA/OVA/KLH): The protein was dissolved in a system of 20 mM boric acid and 5 mM borax at pH 8.5. 10% DTPA and 5 to 20 eq TCEP were added to the reaction system. The reaction was performed at room temperature for 2 h.

Conjugation Process of Drug and Carrier Protein (BSA/OVA/KLH): 5 to 20 eq of the drug was added to the above reaction system. The reaction was performed at room temperature at 600 rpm for 3 h.

Buffer Exchange and Determination : After the reaction was completed, the buffer of the system was exchanged to PBS, and an appropriate amount of sample was taken for ELISA determination of conjugation status.

### Example 2 Screening of antibodies specifically binding to MMAE

**Table 1. Mouse immunization protocol - immunogen was BSA-MMAF (prepared in Examples 1-14)**

| Adjuvant type | Freund's adjuvant | Water-soluble adjuvant |
|---|---|---|
| Specific protocol | The immunogen (40 µg) emulsified with Freund's complete (first immunization) or incomplete adjuvant was used for single immunization. Female mice were subcutaneously injected once every 2 weeks. | A mixture of 40 µg of the immunogen and adjuvant at a ratio of 1:1 was used for single immunization. Female mice were subcutaneously injected once every 1 week, with an interval of 10 days between the first and second immunizations. |
| | The first titer determination was performed after two immunizations, the second titer determination was performed after the third immunization, and the third titer determination was performed after the fourth immunization. | The first titer determination was performed after two immunizations, the second titer determination was performed after the third and fourth immunizations, and the third titer determination was performed after the fifth immunization. |

**Table 2. Mouse immunization protocol - Immunogen was KLH-MMAF (prepared in Examples 1-14)**

| Adjuvant type | Freund's adjuvant | Water-soluble adjuvant |
|---|---|---|
| Specific protocol | The immunogen (40 µg) emulsified with Freund's complete (first immunization) or incomplete adjuvant was used for single immunization. Female mice were subcutaneously injected once every 2 weeks. | A mixture of 40 µg of the immunogen and adjuvant at a ratio of 1:1 was used for single immunization. Female mice were subcutaneously injected once every 1 week, with an interval of 10 days between the first and second immunizations. |
| | The first titer determination was performed after three immunizations, followed by the fourth immunization. | The first titer determination was performed after three immunizations, the second titer determination was performed after the fourth and fifth immunizations, followed by the sixth immunization. |

### Example 2-1 Screening of antibodies binding to MMAE (round 1)

### Generation of Hybridomas

Mice were immunized using BSA-MMAF. Each mouse (strain CD1) was injected with 40 µg of immunogen each time. Spleen cells from mice with excellent titer determination results were selected and fused with SP2/0 myeloma cells at a ratio of 1:1 by electrofusion. The cells were plated into 96-well cell culture plates using HAT medium containing 20% FBS, with 30 plates plated for each mouse. After 7 days of culture, the medium was completely replaced with HT medium containing 10% FBS. After 3 days of culture, the supernatant was subjected to ELISA determination.

### Primary Screening of Hybridomas

OVA-MMAF was coated onto ELISA plates at a concentration of 200 ng/mL and incubated at 37°C for 1 h. The plates were blocked with 5% skim milk and incubated at 37°C for 2 h. 100 µL of hybridoma supernatant was added to the plates and incubated at 37°C for 1 h. HRP-Goat Anti-mouse IgGΔIgM diluted at 1:5000 was added, incubated at 37°C for 1 h, and then subjected to TMB color development. The OD value was determined at 450 nm. Negative screening was performed on positive hybridoma supernatants to screen for hybridomas that specifically bound to MMAE but not to MMAE-Linker (structure: MC-Val-Cit-PAB-MMAE): OVA-MMAF was coated onto ELISA plates at a concentration of 200 ng/mL and incubated at 37°C for 1 h. The plates were blocked with 5% skim milk and incubated at 37°C for 2 h. 50 µL of hybridoma supernatant was collected, and PBS, free MMAE, and disitamab vedotin were added respectively. The mixtures were co-incubated at 37°C for 1 h. HRP-Goat Anti-mouse IgG△IgM diluted at 1:5000 was added, incubated at 37°C for 1 h, and then subjected to TMB color development. The OD value was determined at 450 nm. Based on the results, hybridomas with the same readings in the PBS added group and the disitamab vedotin added group, but lower values in the free MMAE added group, were selected, which were considered to specifically bind to free MMAE but not to MMAE-Linker. Subcloning was performed on these hybridomas by limiting dilution, with a total of more than 500 plates of subclones. Fusion screening was performed on a total of 24 mice. More than 46,000 hybridoma strains were determined in the primary screening, and more than 800 plates were subjected to subcloning.

### Monoclonal Screening

Monoclonal screening was performed according to the method for screening positive clones. The variable regions of the murine antibodies were grafted onto human constant regions, and more than 160 chimeric antibodies were finally obtained for subsequent analysis.

### Chimeric Antibody Analysis - Affinity Analysis

ELISA determination: OVA-MMAF was coated onto ELISA plates at a concentration of 200 ng/mL and incubated at 37°C for 1 h. The plates were blocked with 5% skim milk and incubated at 37°C for 2 h. The chimeric antibodies were subjected to gradient dilution at concentrations of 10000, 5000, 2500, 1250, 625, 125, 25, 5, 1, 0.1, 0.01, and 0 ng/mL. 100 µL of the diluted antibodies was added to the plates and incubated at 37°C for 1 h. Goat Anti-Human IgG H&L (HRP) (abcam, ab6858) diluted at 1:5000 was added, incubated at 37°C for 1 h, and then subjected to TMB color development. The OD value was determined at 450 nm.

The experimental results indicated (Table 3) that chimeric antibodies No. 62 and No. 64 had high affinity with OVA-MMAF, which was superior to ABC3320.

**Table 3. EC50 (ng/mL) values for affinity determination of some candidate molecules**

| Antibody No. | 2 | 3 | 4 | 8 | 9 | 14 | 62 | 64 | 68 | ABC3320 |
|---|---|---|---|---|---|---|---|---|---|---|
| EC50 | 178.8 | 407.4 | 314.5 | 282.1 | 365 | 6007 | 126.5 | 62.11 | 211.3 | 199.8 |

### Biacore determination (Binding Affinity with Free MMAE)

Antibody immobilization: 20 µg/mL of antibody was immobilized onto the chip surface using a ProA probe; Binding: Antigen MMAE concentrations were 50, 16.7, 5.5, 1.85, and 0 nM. The flow rate was set at 30 µL/min, and the contact time was 600 s; Dissociation: The chip was washed with HEPES buffer for 300 s. Regeneration: Regeneration was performed with 10 mM Glycine-HCl.

The experimental results indicated (Table 4) that the affinity of chimeric antibody No. 62 with free MMAE was high, comparable to that of ABC3320.

**Table 4. Affinity determination of some candidate molecules**

| Antibody No. | ka (1/Ms) | kd (1/s) | KD (M) |
|---|---|---|---|
| ABC3320 | 1.00E+05 | 3.18E-05 | 3.18E-10 |
| 62 | 1.05E+05 | 7.78E-05 | 7.41E-10 |
| 1 | 5.35E+04 | 9.52E-05 | 1.78E-09 |
| 44 | 3.26E+05 | 5.80E-04 | 1.78E-09 |
| 89 | 6.16E+04 | 1.30E-04 | 2.11E-09 |
| 74 | 8.44E+04 | 1.80E-04 | 2.14E-09 |
| 69 | 7.02E+04 | 1.50E-04 | 2.14E-09 |
| 92 | 6.73E+04 | 1.79E-04 | 2.66E-09 |
| 93 | 8.57E+04 | 2.41E-04 | 2.81E-09 |

Chimeric Antibody Analysis - Specificity Analysis: In order to verify whether the chimeric antibodies specifically bind to free MMAE but not to MMAE-Linker (i.e., screening for antibodies that also do not bind to the payload conjugated in the ADC), competition and blocking experiments of the chimeric antibodies were performed.

### Competition Experiment:

1) OVA-MMAF was coated onto ELISA plates at a concentration of 200 ng/mL and incubated at 37°C for 1 h. The plates were blocked with 5% skim milk and incubated at 37°C for 2 h;
2) The antibodies were diluted to final concentrations of 10000, 5000, 2500, 1250, 625, 125, 25, 5, 1, 0.1, 0.01, and 0 ng/mL. MMAE and MMAE-Linker were diluted to a final concentration of 5 µM;
3) 50 µL PBS + 50 µL chimeric antibody, 50 µL MMAE + 50 µL chimeric antibody, and 50 µL MMAE-Linker + 50 µL chimeric antibody were added respectively to the ELISA plates. The total volume per well was 100 µL. The plates were incubated at 37°C for 1 h. After incubation, the reaction solution was discarded, and the plates were washed 3 times with PBST;
4) Goat Anti-Human IgG H&L (HRP) (abcam, ab6858) diluted at 1:5000 was added, incubated at 37°C for 1 h, and then subjected to TMB color development. The OD value was determined at 450 nm.

The experimental results (Table 5) indicated that the competition EC50 values of chimeric antibodies No. 62 and No. 64 were 80.9 ng/mL and 84.52 ng/mL, respectively. Chimeric antibodies No. 62 and No. 64 were capable of specifically binding to free MMAE and did not bind to MMAE-Linker.

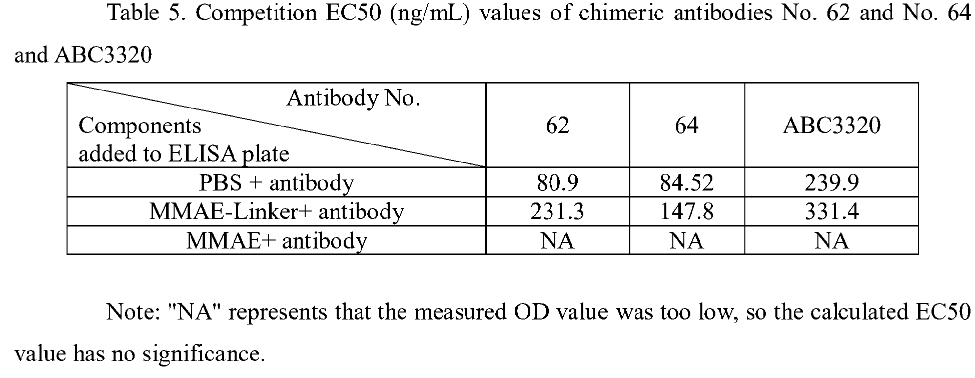

Blocking Experiment: OVA-MMAF was coated onto ELISA plates at a concentration of 200 ng/mL and incubated at 37°C for 1 h. The plates were blocked with 5% skim milk and incubated at 37°C for 2 h. The antibody was diluted to a final concentration of 100 ng/mL. MMAE and MMAE-Linker were diluted at concentrations of 600, 200, 66.67, 22.22, 7.41, 2.47, 0.82, 0.27, 0.09, 0.03, and 0.01 nM. 50 µL MMAE + 50 µL chimeric antibody and 50 µL MMAE-Linker + 50 µL chimeric antibody were added respectively to the ELISA plates. The total volume per well was 100 µL. The plates were incubated at 37°C for 1 h. Goat Anti-Human IgG H&L (HRP) (abcam, ab6858) diluted at 1:5000 was added, incubated at 37°C for 1 h, and then subjected to TMB color development. The OD value was determined at 450 nm. According to the experimental results (Table 6), the blocking IC50 values of chimeric antibodies No. 62 and No. 64 were 1.427 nM and 4.762 nM, respectively.

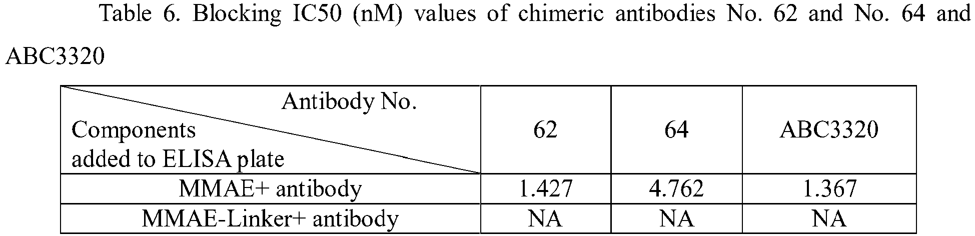

Chimeric Antibody Analysis - Cell Protection Effect Analysis: OVCAR3 cells were plated at 5000 cells/well. After 24 h, the supernatant was discarded, and the antibody and free MMAE were added. The final concentration of MMAE was 69.5 nM. The antibodies were at final concentrations of 60, 30, 15, 10, 7.5, 5, 3.75, and 2.5 µg/mL. 50 µL MMAE + 50 µL chimeric antibody were mixed evenly and added to OVCAR3 cells for culture for 72 h. CellTiter-Meiluncell Luminescent Cell Viability Assay Kit (Meilunbio, PWL111) was added for chemiluminescence reading.

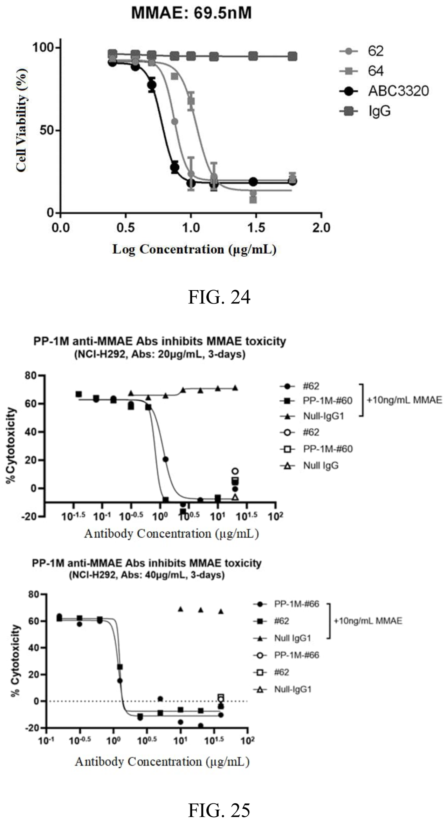

As shown in FIG. 24, the experimental results indicated that chimeric antibodies No. 62 and No. 64 had good protection effects against MMAE killing, which was comparable to ABC3320.

In summary, among the above candidate molecules, chimeric antibodies No. 62 and No. 64 possess superior performance.

Chimeric antibodies No. 62 and No. 64 were sequenced respectively, and the amino acid sequences of the heavy chain variable regions (62H, 64H) and light chain variable regions (62L, 64L) are as follows:
>62H (SEQ ID NO: 1; the bold and underlined regions are CDR1-3 (IMGT system), respectively): EVQLQESGPGLVKSFQTVSLTCTVTGYSITNANHWWNWFRQVSGSKLEWIGYISSSGTT DINPSLKSRFSITRDTSKNQLFLQLNAVTTEDIATYFCVRTYYDGTYEENWGPGTTLTVSS
>62H HCDR1 (SEQ ID NO:2)
   GYSITNANHW
>62H HCDR2 (SEQ ID NO:3)
   ISSSGTT
>62H HCDR3 (SEQ ID NO:4)
   VRTYYDGTYEEN
>62L (SEQ ID NO: 5; the bold and underlined regions are CDR1-3 (IMGT system), respectively): 
DIVMTQTPKFLPVSAGDRVTMTCKASQSVGNNVAWYQQKPGQSPKLLIYYASNRYTGVP
DRFTGSGSGTDFTFTISSVQVEDLAVYFCQQHYNSPWTFAGGTKLEIK
>62L LCDR1 (SEQ ID NO:6)
   QSVGNN
>62L LCDR2
   YAS
>62L LCDR3 (SEQ ID NO:7)
   QQHYNSPWTFAGG
>64H (SEQ ID NO: 8; the bold and underlined regions are CDR1-3 (IMGT system), respectively): 
EVQLQESGPGLVKPSQTVSLTCTVTGNSITNTNNWWNWIRQVSGSKLEWIGYISSSGST
DSNPSLKSRISITRDTSRNQLFLQLKSVTPEDVATYYCVRTYYDGSYEENWGQGTTLTVS
S
>64H HCDR1 (SEQ ID NO:9)
   GNSITNTNNW
>64H HCDR2 (SEQ ID NO:10)
   ISSSGST
>64H HCDR3 (SEQ ID NO:11)
   VRTYYDGSYEEN
>64L (SEQ ID NO: 12; the bold and underlined regions are CDR1-3 (IMGT system), respectively): 
DIVMTQTPKFLPVSARDRATMTCKASQSVGNNVAWYQQKPGQSPKLLIYFASKRYTGVP
DRFTGRGSGTDFTFTISSVQVEDLAVYFCQQHYISPWTFGGGTKLELK
>64L LCDR1 (SEQ ID NO:6)
   QSVGNN
>64L LCDR2
   FAS
>64L LCDR3 (SEQ ID NO: 13)
   QQHYISPWT

### Humanized Sequence Design of Antibody No. 62

Through sequence similarity alignment, the antibody germline with the highest similarity to murine antibody No. 62 was selected as a humanization template. The CDRs of the template were replaced with the CDRs of the heavy chain and light chain, respectively. Subsequently, key amino acids affecting antibody activity were back-mutated according to the three-dimensional structure predicted by artificial intelligence. The specific humanization process was as follows:
(1) IGHV4-30-4*01 and IGHJ6*01 were selected as the heavy chain humanization templates for murine antibody No. 62, and Kappa IGKV1-33*01 and IGKJ4*01 were selected as the light chain humanization templates for murine antibody No. 62. The CDRs of the humanization templates were replaced with the heavy chain or light chain CDRs of murine antibody No. 62, to obtain heavy chain variable region sequence 5759 (SEQ ID NO: 14) and light chain variable region sequence 5764 (SEQ ID NO: 15), respectively;
(2) The variable regions of murine antibody No. 62 were subjected to structural prediction using the ESMFold module in WeMol software to obtain a three-dimensional structural model;
(3) According to the variable region structure of murine antibody No. 62, key amino acids in the framework regions that affect the interaction between the heavy chain and the light chain as well as the interaction with CDRs were judged, and amino acid sites for back-mutation were determined. Heavy chain variable region sequence 5954 (SEQ ID NO: 17) and light chain variable region sequence 6052 (SEQ ID NO: 18) were obtained respectively, and the two formed the PP-1M-#60 molecule; meanwhile, the potential isomerization site DG in the heavy chain CDR3 was removed by post-translational modification to obtain heavy chain variable region sequence 6265 (SEQ ID NO: 19), which formed the PP-1M-#66 molecule with light chain variable region sequence 6052 (SEQ ID NO: 18).

>5759 (SEQ ID NO: 14; the regions in bold and underlined are CDR1-3 (IMGT system), respectively): 
QVQLQESGPGLVKPSQTLSLTCTVSGYSITNANHWWSWIRQPPGKGLEWIGYISSSGTT
YYNPSLKSRVTISVDTSKNQFSLKLSSVTAADTAVYYCVRTYYDGTYEENWGQGTTVTV
SS
>5759 HCDR1 (SEQ ID NO:2)
   GYSITNANHW
>5759 HCDR2 (SEQ ID NO:3)
   ISSSGTT
>5759 HCDR3 (SEQ ID NO:4)
   VRTYYDGTYEEN
>5764 (SEQ ID NO: 15; the regions in bold and underlined are CDR1-3 (IMGT system), respectively): 
DIQMTQSPSSLSASVGDRVTITCQASQSVGNNLNWYQQKPGKAPKLLIYYASNLETGVPS
RFSGSGSGTDFTFTISSLQPEDIATYYCQQHYNSPWTFGGGTKVEIK
>5764 LCDR1 (SEQ ID NO:6)
   QSVGNN
>5764 LCDR2
   YAS
>5764 LCDR3 (SEQ ID NO:16)
   QQHYNSPWT
>5954 (SEQ ID NO: 17; the regions in bold and underlined are CDR1-3 (IMGT system), respectively): 
QVQLQESGPGLVKPSQTLSLTCTVSGYSITNANHWWNWFRQPPGKGLEWIGYISSSGTT
DINPSLKSRVTISRDTSKNQLSLKLSSVTAADTAVYYCVRTYYDGTYEENWGQGTTVTV
SS
>5954 HCDR1 (SEQ ID NO:2)
   GYSITNANHW
>5954 HCDR2 (SEQ ID NO:3)
   ISSSGTT
>5954 HCDR3 (SEQ ID NO:4)
   VRTYYDGTYEEN
>6052 (SEQ ID NO: 18; the regions in bold and underlined are CDR1-3 (IMGT system), respectively): 
DIQMTQSPSSLSASVGDRVTITCQASQSVGNNVAWYQQKPGKAPKLLIYYASNLYTGVPS
RFSGSGSGTDFTFTISSLQPEDIATYFCQQHYNSPWTFAGGTKVEIK
>6052 LCDR1 (SEQ ID NO:6)
   QSVGNN
>6052 LCDR2
   YAS
>6052 LCDR3 (SEQ ID NO:7)
   QQHYNSPWTFAGG
>6265 (SEQ ID NO: 19; the regions in bold and underlined are CDR1-3 (IMGT system), respectively): 
QVQLQESGPGLVKPSQTLSLTCTVSGYSITNANHWWNWFRQPPGKGLEWIGYISSSGTT
DINPSLKSRVTISRDTSKNQLSLKLSSVTAADTAVYYCVRTYYDSTYEENWGQGTTVTVS
S
>6265 HCDR1 (SEQ ID NO:2)
   GYSITNANHW
>6265 HCDR2 (SEQ ID NO:3)
   ISSSGTT
>6265 HCDR3 (SEQ ID NO:20)
   VRTYYDSTYEEN

### Construction, Expression and Purification of Humanized Antibodies

Through primer design, the heavy chain sequence and light chain sequence of the antibody were constructed onto expression vectors respectively, and Expi293 cells were used for transient transfection and expression.

### Cell Protection Experiment of Humanized Anti-MMAE Antibody

NCI-H292 cells were digested, centrifuged at 1200 rpm for 5 minutes, and the supernatant was discarded. The cells were resuspended in 1640 + 10% FBS medium, counted, adjusted to the corresponding concentration, and added into Corning 3610 plates at 60 µL per well (5000 cells per well). The plates were placed in a 37°C incubator for later use;
The antibody concentration was diluted (40 µg/mL or 20 µg/mL, 2-fold serial dilution), and the concentration of free MMAE was fixed to 10 ng/mL. 30 µL of MMAE and 30 µL of antibody were added at a 1:1 ratio into the 3610 plates with cells. The total culture system was 120 µL. The cells were cultured in a 37°C incubator for 3 days;
The plates were taken out and equilibrated to room temperature. 100 µL of CellTiter-Glo^{®} Luminescent Cell was added to each well, mixed well, and left to stand at room temperature protected from light for 10 min;
Luminescence values were determined on a microplate reader, and the calculation formula was: %Cytotoxicity = (Blank - Sample) / Blank * 100%.

The experimental results are shown in FIG. 25. The results indicated that molecules PP-1M-#60 and PP-1M-#66 have good cell protection activity.

### Example 2-2 Screening of Antibodies Binding to MMAE (Round 2)

### Generation of Hybridomas

Mice were immunized using KLH-MMAF. Each mouse (strains were CD1 and Balb/c) was injected with 40 µg of immunogen each time. Spleen cells from mice with excellent titer determination results were selected and fused with SP2/0 myeloma cells at a ratio of 1:1 by electrofusion. The cells were plated into 96-well cell culture plates using HAT medium containing 20% FBS, with 30 plates plated for each mouse. After 7 days of culture, the medium was completely replaced with HT medium containing 10% FBS. After 3 days of culture, supernatant was subjected to ELISA determination.

### Primary Screening of Hybridomas

OVA-MMAF was coated onto ELISA plates at a concentration of 200 ng/mL and incubated at 37°C for 1 h. The plates were blocked with 3% BSA and incubated at 37°C for 2 h. 100 µL of hybridoma supernatant was taken into the plates and incubated at 37°C for 1 h. HRP-Goat Anti-mouse IgGΔIgM diluted at 1:5000 was added, incubated at 37°C for 1 h. TMB color development was performed, and OD values were determined at 450 nm. Negative screening was performed on positive hybridoma supernatants to screen for hybridomas that specifically bound to MMAE but not to MMAE-Linker: OVA-MMAF was coated onto ELISA plates at a concentration of 200 ng/mL and incubated at 37°C for 1 h. The plates were blocked with 3% BSA and incubated at 37°C for 2 h. 50 µL of hybridoma supernatant was taken, and 300 nM free MMAE and 300 nM MMAE-Linker were added respectively. The mixtures were co-incubated at 37°C for 1 h. HRP-Goat Anti-mouse IgGΔIgM diluted at 1:5000 was added, incubated at 37°C for 1 h. TMB color development was performed, and OD values were determined at 450 nm. Based on the results, hybridomas with high readings in both the PBS group and the MMAE-Linker group but lower values in the free MMAE group were selected, which were considered to specifically bind to free MMAE without binding to MMAE-Linker. Subcloning was performed on these hybridomas via limiting dilution. A total of 38 plates were subcloned, and more than 700 hybridoma strains were determined.

### Monoclonal Screening

Monoclonal screening was performed by repeating the above screening method. The variable regions of the murine antibodies were grafted onto human constant regions, and 6 chimeric antibodies were finally obtained for subsequent analysis.

### Chimeric Antibody Analysis - Specificity Analysis

Competition Experiment: The experimental steps were the same as the above competition experiment. The experimental results (Table 7) indicated that the competition EC50 value of chimeric antibody No. J4 was 27.27 ng/mL, which was significantly superior to ABC3320.

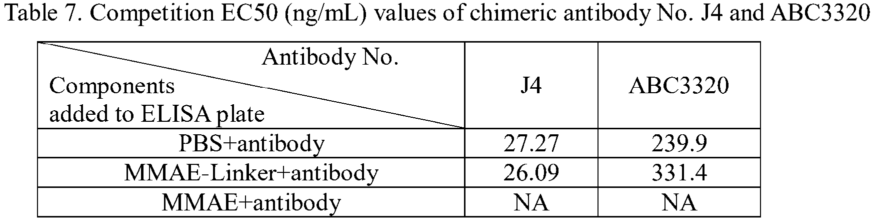

Blocking Experiment: The experimental steps were the same as the above blocking experiment. According to the experimental results (Table 8), the blocking IC50 value of chimeric antibody No. J4 was 1.89 nM.

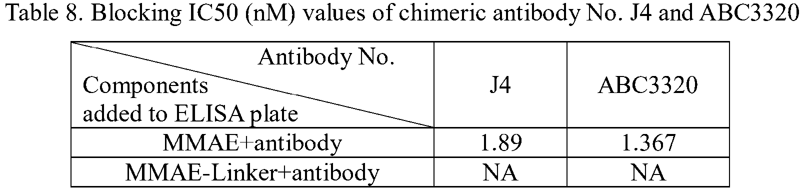

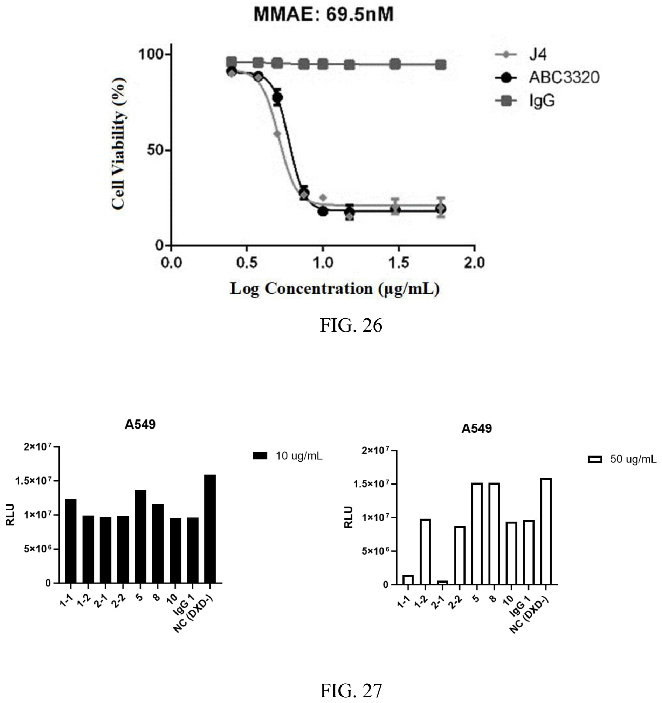

Chimeric Antibody Analysis - Cell Protection Effect Analysis: The experimental steps were the same as the above cell protection effect analysis experiment. The experimental results are shown in FIG. 26. The results indicated that the cell protection effect of chimeric antibody No. J4 was comparable to that of ABC3320.

In summary, among the above candidate molecules, chimeric antibody No. J4 possesses superior performance.

Chimeric antibody No. J4 was sequenced, and the amino acid sequences of the heavy chain variable region (J4H) and light chain variable region (J4L) are as follows:
> J4H (SEQ ID NO:21; the regions in bold and underlined are CDR1-3 (IMGT system), respectively): 
EVQMQQSGAELVKPGASVKLSCKTSGFTFSSSYISWLKQKPGQSLEWIAWIYAGTGNTN
YNQKFAGRAQLTVDTSSSTAYMQFSGLTTEDSAIYYCSRDGYDGRSYDLDYWGQGTSV
TVSS
>J4H HCDR1 (SEQ ID NO:22)
   GFTFSSSY
>J4H HCDR2 (SEQ ID NO:23)
   IYAGTGNT
>J4H HCDR3 (SEQ ID NO:24)
   SRDGYDGRSYDLDY
> J4L (SEQ ID NO:25; the regions in bold and underlined are CDR1-3 (IMGT system), respectively): 
DVVLTQSHKFMSTSVGDRVSITCKASQDVGTSVAWYQQKPGQSPKLLIYWASTRHTGVP
DRFTGSGSGTDFTLTIGNVQSEDLADYFCQQFSTYPLTFGGGTKLEIK
>J4L LCDR1 (SEQ ID NO:26)
   QDVGTS
>J4L LCDR2
   WAS
>J4L LCDR3 (SEQ ID NO:27)
   QQFSTYPLT

### Example 2-3 Screening of Antibodies Binding to MMAE (Round 3)

### Generation of Hybridomas

Mice were immunized using KLH-MMAF. Each mouse (strain was Balb/c) was injected with 40 µg of immunogen each time. Spleen cells from mice with excellent titer determination results were selected and fused with SP2/0 myeloma cells at a ratio of 1:1 by electrofusion. The cells were plated into 96-well cell culture plates using HAT medium containing 20% FBS, with 30 plates plated for each mouse. After 7 days of culture, the medium was completely replaced with HT medium containing 10% FBS. After 3 days of culture, supernatant was subjected to ELISA determination.

### Primary Screening of Hybridomas

OVA-MMAF was coated onto ELISA plates at a concentration of 200 ng/mL and incubated at 37°C for 1 h. The plates were blocked with 3% BSA and incubated at 37°C for 2 h. 100 µL of hybridoma supernatant was taken onto the plates and incubated at 37°C for 1 h. HRP-Goat Anti-mouse IgGΔIgM diluted at 1:5000 was added, incubated at 37°C for 1 h. TMB color development was performed, and OD values were determined at 450 nm. Negative screening was performed on positive hybridoma supernatants to screen for hybridomas that specifically bound to MMAE but not to MMAE-Linker: OVA-MMAF was coated onto ELISA plates at a concentration of 200 ng/mL and incubated at 37°C for 1 h. The plates were blocked with 3% BSA and incubated at 37°C for 2 h. 50 µL of hybridoma supernatant was taken, and 300 nM free MMAE and 300 nM MMAE-linker were added respectively. The mixtures were co-incubated at 37°C for 1 h. HRP-Goat Anti-mouse IgG△IgM diluted at 1:5000 was added, incubated at 37°C for 1 h. TMB color development was performed, and OD values were determined at 450 nm. Based on the results, hybridomas with the same readings in the PBS group and the disitamab vedotin added group but lower values in the free MMAE group were selected, which were considered to specifically bind to free MMAE without binding to MMAE-Linker. Subcloning was performed on these hybridomas via limiting dilution. A total of more than 20 plates were subcloned, and more than 400 hybridoma strains were determined.

### Monoclonal Screening

Monoclonal screening was performed by repeating the above screening method. The variable regions of the murine antibodies were grafted onto human constant regions, and 10 chimeric antibodies were finally obtained for subsequent analysis.

### Chimeric Antibody Analysis - Specificity Analysis

Competition Experiment: The experimental steps were the same as the above competition experiment. The experimental results (Table 9) indicated that the competition EC50 values of chimeric antibodies No. 92 and No. 93 were 30.63 ng/mL and 37.22 ng/mL, respectively, which were significantly better than ABC3320.

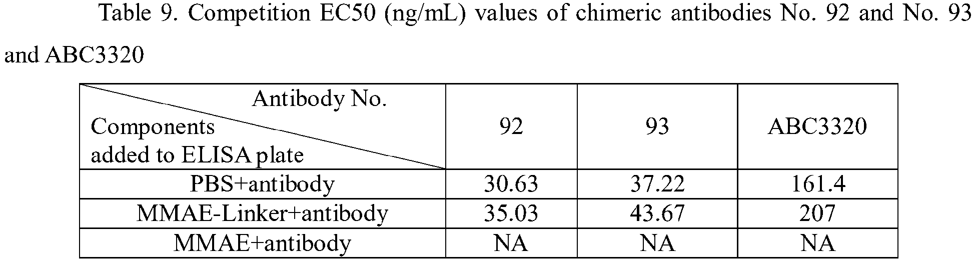

Blocking Experiment: The experimental steps were the same as the above blocking experiment. According to the experimental results (Table 10), the blocking IC50 values of chimeric antibodies No. 92 and No. 93 were 83.75 nM and 54.69 nM, respectively.

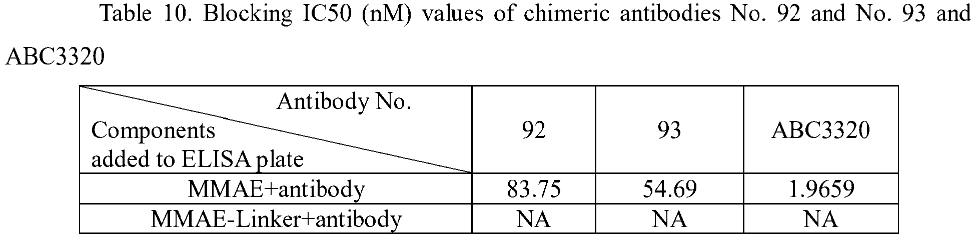

In summary, among the above candidate molecules, chimeric antibodies No. 92 and No. 93 possess superior performance.

Chimeric antibodies No. 92 and No. 93 were sequenced respectively, and the amino acid sequences of the heavy chain variable regions (92H, 93H) and light chain variable regions (92L, 93L) are as follows:
>92H (SEQ ID NO: 28; the regions in bold and underlined are CDR1-3 (IMGT system), respectively): 
EVQLQQSGPELVKPGASVKMSCKASGYTFTSYVMHWVKQKPGQGLEWIGYIIHYNDV
TKYNENFRGKATLTSDKSSSTAYMEVSSLTSEDSAVYYCTRSPYYYDGSFDYWGQGTTL
TVSS
>92H HCDR1 (SEQ ID NO:29)
   GYTFTSYV
>92H HCDR2 (SEQ ID NO:30)
   IIHYNDVT
>92H HCDR3 (SEQ ID NO:31)
   TRSPYYYDGSFDY
>92L (SEQ ID NO:32; the regions in bold and underlined are CDR1-3 (IMGT system), respectively): 
DVVMTQTPLSLPVSLGDQASISCRSSQSIVHSNGNTYLEWFLQKPGQSPKLLIYKVSNRF
SGVPDRFSGSGSGTDFTLKISRVEAEDLGVYYCFQGSHAPYTFGGGTKLEIK
>92L LCDR1 (SEQ ID NO:33)
   QSIVHSNGNTY
>92L LCDR2
   KVS
>92L LCDR3 (SEQ ID NO:34)
   FQGSHAPYT
>93H (SEQ ID NO:35; the regions in bold and underlined are CDR1-3 (IMGT system), respectively): 
QVQLQQSGPELVKPGASVKMSCKASGYTFTSYVMHWVKQKPGQGLEWIGYIIHYNDG
TKYNEKFRGKATLTSDKSSSTAYMELSSLTSEDSAVYYCARSPYYSDGSFDYWGQGTTLT
VSS
>93H HCDR1 (SEQ ID NO:29)
   GYTFTSYV
>93H HCDR2 (SEQ ID NO:36)
   IIHYNDGT
>93H HCDR3 (SEQ ID NO:37)
   ARSPYYSDGSFDY
>93L (SEQ ID NO:38; the regions in bold and underlined are CDR1-3 (IMGT system), respectively): 
DILMTQTPLSLPVSLGDQASISCRSSQSIVHSNGNTYLEWFLQKPGQSPKLLIYKLSNRFS
GVPDRFSGSGSGTDFTLKISRVEAEDLGVYYCFQGSHAPYTFGGGTKLEIK
>93L LCDR1 (SEQ ID NO:33)
   QSIVHSNGNTY
>93L LCDR2
   KLS
>93L LCDR3 (SEQ ID NO:34)
   FQGSHAPYT

### Example 2-4 Screening of MMAE-binding antibodies (4th round)

### Generation of Hybridomas

Mice were immunized using BSA-MMAF. Each mouse (strains were CD1 and Balb/c) was injected with 40 µg of immunogen each time. Spleen cells from mice with excellent titer determination results were selected and fused with SP2/0 myeloma cells at a ratio of 1:1 by electrofusion. The cells were plated into 96-well cell culture plates using HAT medium containing 20% FBS, with 30 plates plated for each mouse. After 7 days of culture, the medium was completely replaced with HT medium containing 10% FBS. After 3 days of culture, supernatant was subjected to ELISA determination.

### Primary Screening of Hybridomas

OVA-MMAF was coated onto ELISA plates at a concentration of 200 ng/mL and incubated at 37°C for 1 h. The plates were blocked with 3% BSA and incubated at 37°C for 2 h. 100 µL of hybridoma supernatant was taken onto the plates and incubated at 37°C for 1 h. HRP-Goat Anti-mouse IgGΔIgM diluted at 1:5000 was added, incubated at 37°C for 1 h. TMB color development was performed, and OD values were determined at 450 nm. Negative screening was performed on positive hybridoma supernatants to screen for hybridomas that specifically bound to MMAE but not to MMAE-Linker. OVA-MMAF was coated onto ELISA plates at a concentration of 200 ng/mL and incubated at 37°C for 1 h. The plates were blocked with 3% BSA and incubated at 37°C for 2 h. 50 µL of hybridoma supernatant was taken, and 300 nM free MMAE and 300 nM MMAE-linker were added respectively. The mixtures were co-incubated at 37°C for 1 h. HRP-Goat Anti-mouse IgGΔIgM diluted at 1:5000 was added, incubated at 37°C for 1 h. TMB color development was performed, and OD values were determined at 450 nm. Based on the results, hybridomas with high readings in both the PBS group and the disitamab vedotin group but lower values in the free MMAE group were selected, which were considered to specifically bind to free MMAE without binding to MMAE-Linker. Subcloning was performed on these hybridomas via limiting dilution. A total of more than 50 plates were subcloned, and more than 1000 hybridoma strains were determined.

### Monoclonal Screening

Monoclonal screening was performed by repeating the above screening method. The variable regions of the murine antibodies were grafted onto human constant regions, and 23 chimeric antibodies were finally obtained for subsequent analysis.

### Chimeric Antibody Analysis - Specificity Analysis

Competition Experiment: The experimental steps were the same as the above competition experiment. The experimental results (Table 11) indicated that the competition EC50 values of chimeric antibodies No. 132 and No. 133 were 70.61 ng/mL and 34.84 ng/mL, respectively, which were significantly superior to ABC3320.

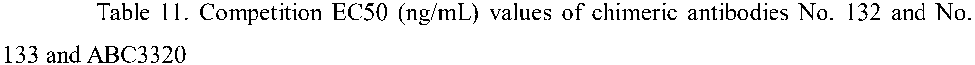

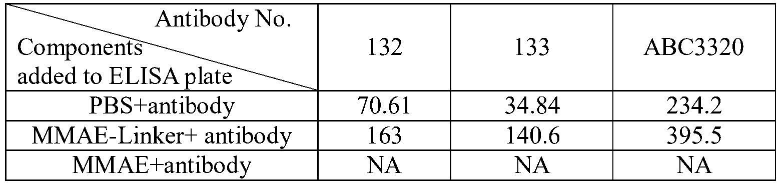

Blocking Experiment: The experimental steps were the same as the above blocking experiment. According to the experimental results (Table 12), the blocking IC50 values of chimeric antibodies No. 132 and No. 133 were 1.318 nM and 1.593 nM, respectively.

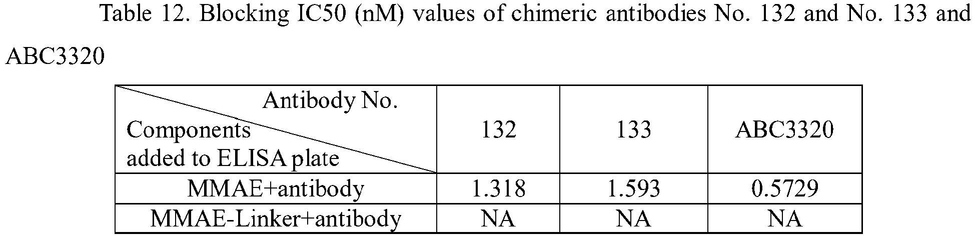

In summary, among the above candidate molecules, chimeric antibodies No. 132 and No. 133 possess superior performance.

Chimeric antibodies No. 132 and No. 133 were sequenced respectively, and the amino acid sequences of the heavy chain variable regions (132H, 133H) and light chain variable regions (132L, 133L) are as follows:
>132H (SEQ ID NO:39; the regions in bold and underlined are CDR1-3 (IMGT system), respectively): 
EVQLVESGGGLVKPGGSLKLSCAASGFTFSAYYMHWIRQTPEERLEWVATISDGGSNTY
YPDSVKGRFIISRDNAKNILYLQMSSLKSEDTAMYYCARDGGWLLYYFDYWGQGTTLT
VSS
>132H HCDR1 (SEQ ID NO:40)
   GFTFSAYY
>132H HCDR2 (SEQ ID NO:41)
   ISDGGSNT
>132H HCDR3 (SEQ ID NO:42)
   ARDGGWLLYYFDY
>132L (SEQ ID NO:43; the regions in bold and underlined are CDR1-3 (IMGT system), respectively): 
DVVMTQTPLSLPVSLGDQASISCRSSQAIVQSNGETYLEWYLQKSGQSPKLLIYKVSNRF
TGVPDRFSGSGSGTDFTLKISRVEAEDLGVYYCFQGSHVPWTFGGGTKLEIK
>132L LCDR1 (SEQ ID NO:44)
   QAIVQSNGETY
>132L LCDR2
   KVS
>132L LCDR3 (SEQ ID NO:45)
   FQGSHVPWT
>133H (SEQ ID NO:46; the regions in bold and underlined are CDR1-3 (IMGT system), respectively): 
EVQLVESGGGLVKPGGSLKLSCAASGFIFSDHYMYWVRQTPEKRLEWVAVISDGGTYT
NYPDSVKGRFTISRDNAKNTLYLHIHSLKSEDTAMYYCARDGGWLLYYFDYWGQGTTL
TVSS
>133H HCDR1 (SEQ ID NO:47)
   GFIFSDHY
>133H HCDR2 (SEQ ID NO:48)
   ISDGGTYT
>133H HCDR3 (SEQ ID NO:42)
   ARDGGWLLYYFDY
>133L (SEQ ID NO:49; the regions in bold and underlined are CDR1-3 (IMGT system), respectively): 
DVVLTQTPLSLPVSLGDQASISCRSSQNIVQSDGNTYLEWYLQKPGQSPKLLIYKVSNRF
SGVPDRFSGSGSGTDFTLKISRVEAEDLGVYYCFQGSHLPWTFGGGTKLEIK
>133L LCDR1 (SEQ ID NO:50)
   QNIVQSDGNTY
>133L LCDR2
   KVS
>133L LCDR3 (SEQ ID NO:51)
   FQGSHLPWT

### Example 3 Screening of Antibodies Specifically Binding to DXD

### Example 3-1 Screening of Antibodies Binding to DXD (Round 1)

### Generation and Primary Screening of Hybridomas

1) Mice were immunized using KLH-DXD. 40 µg of immunogen was injected each time. Spleen cells from 2 mice (strains Balb/c and CD1, respectively) with excellent titer determination results were selected and fused with SP2/0 myeloma cells by electrofusion. The hybridoma cells were plated on 70 96-well plates and cultured in HAT medium. After one week, the medium was replaced with HT medium. After the fused cells grew to a certain extent and secreted antibodies, positive hybridoma cells were screened.
2) Cell wells with OD450 values greater than 0.2 were selected for negative screening with ADC (structure shown in FIG. 58, Ab-MC-GGFG-DXD) and free DXD (fermentation supernatant was co-incubated with quantitative free DXD or ADC; cell wells were selected where ADC incubation did not affect the OD450 reading while DXD incubation reduced the OD450 reading). 20 positive hybridoma strains were obtained for subcloning. Through monoclonal screening by ELISA, hybridomas from 7 pools were finally obtained for sequence retrieval. The obtained chimeric antibodies were numbered as DXD1-1, DXD1-2, DXD2-1, DXD2-2, DXD5, DXD8, and DXD10, respectively.

### Characterization of Chimeric Antibodies

Primary screening of chimeric antibodies: The above chimeric antibodies were expressed using 293F cells. After 7 days, the fermentation supernatant was collected and quantified using OCTET.

A549 cells were plated at 5000 cells per well. After 24 h, the fermentation supernatant (diluted to 10 µg/mL and 50 µg/mL, respectively) and DXD (100 nM) were added for co-incubation. Cell viability was determined after 72 h. Fermentation broths with DXD protection effects were preliminarily screened for purification and affinity verification.

The experimental results are shown in FIG. 27. The results indicated that the antibodies with cell protection effects preliminarily screened were DXD5 and DXD8.

The chimeric antibodies with protective effects preliminarily screened were purified, and the binding activity of the antibodies with OVA-DXD was determined by ELISA: 500 ng/mL of OVA-DXD was coated onto 96-well plates at 37°C for 2 h. After washing twice with PBST, blocking was performed with 5% milk powder at 37°C for 2 h. After the plates were washed twice with PBST, the antibodies were diluted to concentrations of 20000, 10000, 5000, 2500, 1250, 625, 125, 25, 5, 0.5, and 0.05 ng/mL. The diluted antibodies were added to the 96-well plates and incubated at 37°C for 1 h. After the plates were washed twice with PBST, Anti-mouse IgG HRP was added and incubated at 37°C for 1 h. After the plates were washed three times with PBST, TMB chromogenic solution was added. When the color developed to a certain extent, 2 M H₂SO₄ was added, and reading was performed using a microplate reader. The EC50 values are shown in Table 13. Chimeric antibodies with better binding activity to OVA-DXD were DXD1-1, DXD5, and DXD8.

**Table 13. EC50 (µg/mL) values of chimeric antibody binding to OVA-DXD**

| Antibody No. | 1-1 | 1-2 | 2-1 | 2-2 | 5 | 8 | 10 |
|---|---|---|---|---|---|---|---|
| EC50 | 0.05194 | 10.25 | 5.797 | 28.75 | 0.05238 | 0.06915 | 9.379 |

### Example 3-2 Screening of Antibodies Binding to DXD (Round 2)

### Generation and Primary Screening of Hybridomas

1) Mice were immunized using KLH-DXD. 40 µg of immunogen was injected each time. Spleen cells from a mouse (strain Balb/c) with excellent titer determination results were selected and fused with SP2/0 myeloma cells by electrofusion. The hybridoma cells were plated on 30 96-well plates and cultured in HAT medium. After one week, the medium was replaced with HT medium. After the fused cells grew to a certain extent and secreted antibodies, positive hybridoma cells were screened.
2) Cell wells with OD450 values greater than 1.8 were selected for negative screening with ADC and free DXD (fermentation supernatant was co-incubated with quantitative free DXD or ADC; cell wells were selected where ADC incubation did not affect the OD450 reading while DXD incubation reduced the OD450 reading). 52 positive hybridoma strains were obtained for subcloning. Through monoclonal screening by ELISA, hybridomas from 27 pools were finally obtained for sequence retrieval. The obtained chimeric antibodies were numbered JLD1 to JLD27, respectively.

### Characterization of Chimeric Antibodies

Primary screening of chimeric antibodies: The above chimeric antibodies were expressed using 293F cells. After 7 days, the fermentation supernatant was collected and quantified using OCTET.

A549 cells were plated at 5000 cells per well. After 24 h, the fermentation supernatant (diluted to 10 µg/mL and 50 µg/mL, respectively) and DXD (100 nM) were added for co-incubation. Cell viability was determined after 72 h. Fermentation broths with DXD protection effects were preliminarily screened for purification and affinity verification.

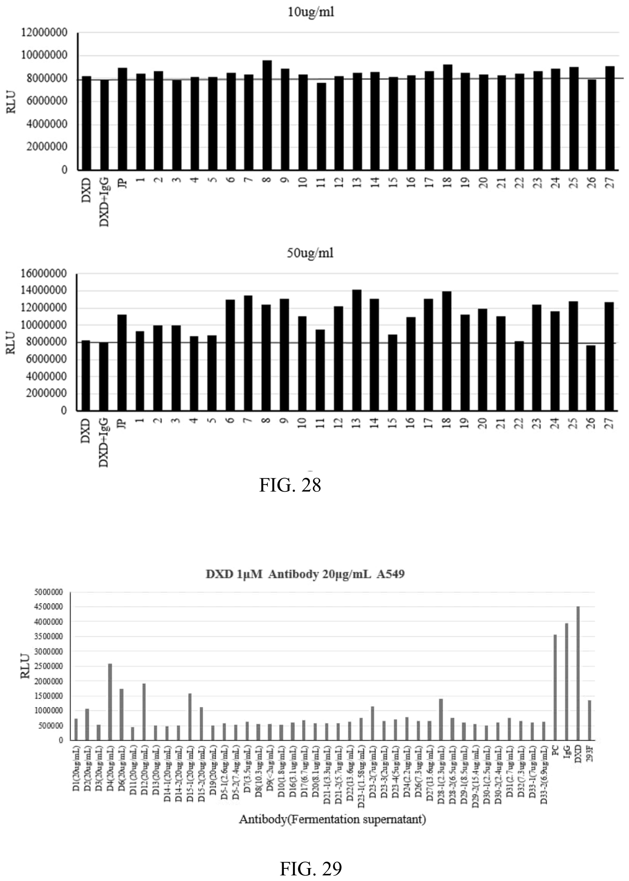

The experimental results are shown in FIG. 28. The results indicated that the antibodies with cell protection effects preliminarily screened were JLD6, JLD7, JLD8, JLD9, JLD10, JLD12, JLD13, JLD14, JLD16, JLD17, JLD18, JLD19, JLD20, JLD21, JLD23, JLD24, JLD25, and JLD27.

The chimeric antibodies with protective effects preliminarily screened were purified, and the binding activity of the antibodies with OVA-DXD was determined by ELISA: 500 ng/mL of OVA-DXD was coated onto 96-well plates at 37°C for 2 h. After the plates were washed twice with PBST, blocking was performed with 5% milk powder at 37°C for 2 h. After the plates were washed twice with PBST, the antibodies were diluted to concentrations of 20000, 10000, 5000, 2500, 1250, 625, 125, 25, 5, 0.5, and 0.05 ng/mL. The diluted antibodies were added to the 96-well plates and incubated at 37°C for 1 h. After the plates were washed twice with PBST, Anti-mouse IgG HRP was added and incubated at 37°C for 1 h. After the plates were washed three times with PBST, TMB chromogenic solution was added. When the color developed to a certain extent, 2 M H₂SO₄ was added, and reading was performed using a microplate reader. The EC50 values are shown in Table 14. Chimeric antibodies with better binding activity to OVA-DXD were JLD6, JLD7, JLD17, and JLD20.

**Table 14. EC50 (ng/mL) values of chimeric antibody binding to OVA-DXD**

| Antibody No. | JLD6 | JLD7 | JLD 17 | JLD18 | JLD20 | JLD24 | IgG1 |
|---|---|---|---|---|---|---|---|
| EC50 | 36.06 | 32.19 | 86.89 | 188.6 | 42.59 | 38270 | NA |

### Example 3-3 Screening of Antibodies Binding to DXD (Round 3)

### Generation and Primary Screening of Hybridomas

1) Mice were immunized using KLH-DXD. 40 µg of immunogen was injected each time. Spleen cells from 2 mice (strains Balb/c and CD1, respectively) with excellent titer determination results were selected and fused with SP2/0 myeloma cells by electrofusion. Hybridoma cells from the Balb/c mouse were plated on 30 96-well plates, and hybridoma cells from the CD1 mouse were plated on 30 96-well plates. They were cultured in HAT medium. After one week, the medium was replaced with HT medium. After the fused cells grew to a certain extent and secreted antibodies, positive hybridoma cells were screened.
2) Cell wells with OD450 values greater than 2 were selected for negative screening with ADC and free DXD (fermentation supernatant was co-incubated with quantitative free DXD or ADC; cell wells were selected where ADC incubation did not affect the OD450 reading while DXD incubation reduced the OD450 reading). 212 positive hybridoma strains were obtained for subcloning. Through monoclonal screening by ELISA, hybridomas from 65 pools were finally obtained for sequence retrieval. The obtained chimeric antibodies were numbered D1 to D65, respectively.

### Characterization of Chimeric Antibodies

Primary screening of chimeric antibodies: The above chimeric antibodies were expressed using 293F cells. After 7 days, the fermentation supernatant was collected and quantified using OCTET.

A549 cells were plated at 5000 cells per well. After 24 h, the fermentation supernatant (diluted to 20 µg/mL) and DXD (1 µM) were added for co-incubation. Cell viability was determined after 72 h. Fermentation broths with DXD protection effects were preliminarily screened for purification and affinity verification.

The experimental results are shown in FIG. 29. The results indicated that the antibodies with cell protection effects preliminarily screened were D2, D4, D6, D12, D15-1, D15-2, D23-1, D23-2, D24, D28-2, D31, D34, D41, D42, D47, D43-1, D43-3, D49, D50, D52, D56-2, D61, D62, D63, D64, and D65.

The chimeric antibodies with protective effects preliminarily screened were purified, and the binding activity of the antibodies with OVA-DXD was determined by ELISA: 500 ng/mL of OVA-DXD was coated onto 96-well plates at 37°C for 2 h. After the plates were washed twice with PBST, blocking was performed with 5% milk powder at 37°C for 2 h. After the plates were washed twice with PBST, the antibodies were diluted to concentrations of 20000, 10000, 5000, 2500, 1250, 625, 125, 25, 5, 0.5, and 0.05 ng/mL. The diluted antibodies were added to the 96-well plates and incubated at 37°C for 1 h. After the plates were washed twice with PBST, Anti-mouse IgG HRP was added and incubated at 37°C for 1 h. After the plates were washed three times with PBST, TMB chromogenic solution was added. When the color developed to a certain extent, 2 M H₂SO₄ was added, and reading was performed using a microplate reader. The EC50 values are shown in Table 15. Chimeric antibodies with better binding activity to OVA-DXD were D2, D4, D6, D52, D61, D62, D63, and D64.

**Table 15. EC50 (ng/mL) values of chimeric antibody binding to OVA-DXD**

| Antibody No. | D64 | D4 | D2 | D6 | D63 | D62 | D61 | D52 | D15-2 |
|---|---|---|---|---|---|---|---|---|---|
| EC50 | 29.83 | 49.17 | 52.23 | 54.74 | 58.31 | 67.83 | 68.17 | 68.85 | 94.19 |

| Antibody No. | D12 | D47 | D24 | D49 | D15-1 | D43-1 | D28-2 | D41 | D28-1 |
|---|---|---|---|---|---|---|---|---|---|
| EC50 | 94.52 | 97.92 | 98.93 | 104 | 125.3 | 144.1 | 169.4 | 204.2 | 361.1 |

| Antibody No. | D65 | D31 | D43-3 | D50 | D23-1 | D34 | D23-2 | D42 | D56-2 |
|---|---|---|---|---|---|---|---|---|---|
| EC50 | 546.6 | 10700 | 12314 | 18281 | 18446 | 32099 | 38302 | NA | NA |

### Example 3-4 Cell Protection Experiment of Chimeric Antibodies

### Determination of Binding Specificity of Chimeric Antibodies

The binding activity of the antibody to DXD in the presence of ADC (structure shown in FIG. 58, Ab-MC-GGFG-DXD) or free DXD was determined by ELISA. DXD-OVA was coated at 200 ng/mL and incubated at 37°C for 2 h. Blocking was performed with 5% milk powder at 37°C for 2 h. Gradient diluted antibodies were co-incubated with PBS, ADC (500 nM), and free DXD (40 µM), respectively, at 37°C for 1 h. Anti-human HRP was added and incubated at 37°C for 1 h. After the plates were washed three times with PBST, TMB chromogenic solution was added. When the color developed to a certain extent, 2 M H₂SO₄ was added, and reading was performed using a microplate reader. The IC50 (µg/mL) values are shown in Table 16. Except for D28-2, other chimeric antibodies had good binding specificity.

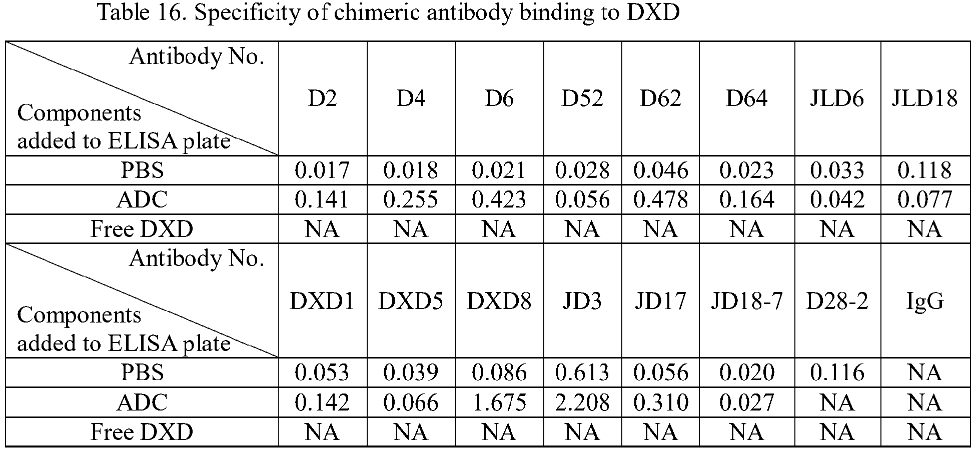

### Cell Protection Experiment of Chimeric Antibodies Against DXD Killing

NCI-H292 was used as a DXD-sensitive cell line. The cells were plated into white 96-well plates at 5000 cells/well and cultured in an incubator for 24 h. A mixture of gradient diluted antibody and small molecule DXD was added for cell killing and antibody protection experiments. After 72 h, the cell protection efficiency of the antibody was determined by measuring the amount of ATP in viable cells in each well using a chemiluminescence determination kit. The IC50 values are shown in Table 17. Chimeric antibodies with better protection effects against DXD killing were DXD8, JLD6, JLD18, D2, D4, D28-2, D52, and D6.

**Table 17. Inhibitory IC50 (µg/mL) values of chimeric antibodies on DXD killing cells**

| Antibody No. | DXD8 | JLD6 | JLD18 | D2 | D4 | D28-2 | D52 |
|---|---|---|---|---|---|---|---|
| IC50 | 1.669 | 1.739 | 2.59 | 2.702 | 3.404 | 3.529 | 3.733 |

| Antibody No. | D6 | DXD5 | D64 | D62 | DXD-3 | IgG | |
|---|---|---|---|---|---|---|---|
| IC50 | 3.822 | 5.741 | 5.793 | 6.609 | ~25.61 | NA | |

Based on the above experimental results, among the above candidate molecules, chimeric antibodies D4, D6, D28-2, D52, JLD6, JLD18, and DXD8 possess superior performance and can effectively bind to the free DXD toxin to reduce cell killing.

The above chimeric antibodies were sequenced respectively, and the amino acid sequences of the heavy chain and light chain variable regions are as follows:
>D4H (SEQ ID NO:52; the regions in bold and underlined are CDR1-3 (IMGT system), respectively): 
EVQLVESGGGLVKPGGSLKLSCAASGFTFSDNYMYWVRQTPEKRLEWVATISAGGSYT
YYPDSVKGRFTISRDNAKNNLYLQMSSLKSEDTAMYYCAGTAMDYWGQGTSVTVSS
>D4H HCDR1 (SEQ ID NO:53)
   GFTFSDNY
>D4H HCDR2 (SEQ ID NO:54)
   ISAGGSYT
>D4H HCDR3 (SEQ ID NO:55)
   AGTAMDY
>D4L (SEQ ID NO:56; the regions in bold and underlined are CDR1-3 (IMGT system), respectively): 
DIVLTQSPAIMSASPGEKVTMTCSASSSVSYMHWYQQKSGTSPKRWIYDTSKLASGVPAR
FSGSGSGTSYSLTISSLEAEDAATYYCLQWKSYPPTFGAGTKLEIK
>D4L LCDR1 (SEQ ID NO:57)
   SSVSY
>D4L LCDR2
   DTS
>D4L LCDR3 (SEQ ID NO:58)
   LQWKSYPPT
>D6H (SEQ ID NO:59; the regions in bold and underlined are CDR1-3 (IMGT system), respectively): 
EVQLVESGGGLVKPGGSLKLSCAASGFTFSDNYMYWVRQTPEKRLEWVATISAGGSYT
YYPDSVKGRFTISRDNAMNNLYLQMSSLKSEDTAMYYCAGTAMDYWGQGTSVTVSS
>D6H HCDR1 (SEQ ID NO:53)
   GFTFSDNY
>D6H HCDR2 (SEQ ID NO:54)
   ISAGGSYT
>D6H HCDR3 (SEQ ID NO:55)
   AGTAMDY
>D6L (SEQ ID NO:60; the regions in bold and underlined are CDR1-3 (IMGT system), respectively): 
DIVLTQSPAIMSASPGEKVTMTCSASSSVSYMHWYQQKSGTSPKRWIYDTSKLASGVPAR
FSGSGSGTSYSLTISSLEAEDAATYYCLQWKSYPPTFGAGTKLEIK
>D6L LCDR1 (SEQ ID NO:57)
   SSVSY
>D6L LCDR2
   DTS
>D6L LCDR3 (SEQ ID NO:58)
   LQWKSYPPT
>D28H (SEQ ID NO:61; the regions in bold and underlined are CDR1-3 (IMGT system), respectively): 
EVQLQESGGGLVQPGGSMKLSCVASGFTFINYWMNWVRQSPERGLEWVAQISLISDDY
ATHYAESVKGRFTISRDDSKSSLYLQMNNLRAEDTGIYYCTEAGYFFDYWGQGTTLTVS
S
>D28H HCDR1 (SEQ ID NO:62)
   GFTFINYW
>D28H HCDR2 (SEQ ID NO:63)
   ISLISDDYAT
>D28H HCDR3 (SEQ ID NO:64)
   TEAGYFFDY
>D28L (SEQ ID NO:65; the regions in bold and underlined are CDR1-3 (IMGT system), respectively): 
DVVMTQSQQFMSTSVGDRVSVTCKASQNVGTNVAWYQQRPGQSPKALIYSASYRYSGV
PDRFTGSGSGTDFTLTISNVQSEDLAEYFCQQYNSYPLTFGAGTKLELK
>D28L LCDR1 (SEQ ID NO:66)
   QNVGTN
>D28L LCDR2
   SAS
>D28L LCDR3 (SEQ ID NO:67)
   QQYNSYPLT
>D52H (SEQ ID NO:68; the regions in bold and underlined are CDR1-3 (IMGT system), respectively): 
EVQLQESGGGLVQPGGSMKLSCVASGFTLSNYWMNWVRQSPEKGLEWVAQIRPKSYN
YATHYAESVKGRFTISRDGSKSSVYLEMSNLRAEDTGIYYCTGFPFDYWGQGTTLTVSS
>D52H HCDR1 (SEQ ID NO:69)
   GFTLSNYW
>D52H HCDR2 (SEQ ID NO:70)
   IRPKSYNYAT
>D52H HCDR3 (SEQ ID NO:71)
   TGFPFDY
>D52L (SEQ ID NO:72; the regions in bold and underlined are CDR1-3 (IMGT system), respectively): 
DIVMTQSHKFMSTSVGDRVSITCKASQDVGTAVAWYQQKPGQSPKLLIYWASTRHTGVP
DRFTGSGSWTDFTLTINNVQSEDLADYFCQQYSSYPLTFGAGTKLEIK
>D52L LCDR1 (SEQ ID NO:73)
   QDVGTA
>D52L LCDR2
   WAS
>D52L LCDR3 (SEQ ID NO:74)
   QQYSSYPLT
>JLD6H (SEQ ID NO:75; the regions in bold and underlined are CDR1-3 (IMGT system), respectively): 
DVQLQESGPGLVKPSQTVSLTCTVTGISITNGNHWWNWIRQLSGNKLEWIGYIRPSGST
DSNPSLKSRISITRDTSKNQVFLQLNSVTPEDVATYYCARGGLEDGKYYYAMDYWGQG
TSL
>JLD6H HCDR1 (SEQ ID NO:76)
   GISITNGNHW
>JLD6H HCDR2 (SEQ ID NO:77)
   IRPSGST
>JLD6H HCDR3 (SEQ ID NO:78)
   ARGGLEDGKYYYAMDY
>JLD6L (SEQ ID NO:79; the regions in bold and underlined are CDR1-3 (IMGT system), respectively): 
DIVLTQSPPSLAVSLGQSVTISCRASENVEYFGTSLMQWYQQKPGQPPKLLIYGASSVES
GVPARFSGSGSGTDFSLKIHPVEEDDIAMYFCQQSRKVPSTFGSGTKLEIK
>JLD6L LCDR1 (SEQ ID NO:80)
   ENVEYFGTSL
>JLD6L LCDR2
   GAS
>JLD6L LCDR3 (SEQ ID NO:81)
   QQSRKVPST
>JLD18H (SEQ ID NO:82; the regions in bold and underlined are CDR1-3 (IMGT system), respectively): DVQLQESGGGLVQPGGSMKLSCIASGFTFTNYWMNWVRQSPEKGLEWVAQIRLMSNN 
CATHYAESVKGRFTISRDASKSFFYLQMNNLRAEDTGIYYCTYYDYDYYYSMDYWGQ
GTSVTVSS
>JLD18H HCDR1 (SEQ ID NO:83)
   GFTFTNYW
>JLD18H HCDR2 (SEQ ID NO:84)
   IRLMSNNCAT
>JLD18H HCDR3 (SEQ ID NO:85)
   TYYDYDYYYSMDY
>JLD18L (SEQ ID NO:86; the regions in bold and underlined are CDR1-3 (IMGT system), respectively): 
DVVMTQSQKFMSTSVGDRVSVTCKASQNVATNVAWYQQKPGQSPKALFYSASYRYSGV
PDRFTGSGSGTDFTLTISNVQSEDLAEYFCQQYNSYPLTFGAGTKLEIK
>JLD18L LCDR1 (SEQ ID NO:87)
   QNVATN
>JLD18L LCDR2
   SAS
>JLD18L LCDR3 (SEQ ID NO:67)
   QQYNSYPLT
>DXD8H (SEQ ID NO:88; the regions in bold and underlined are CDR1-3 (IMGT system), respectively): 
EVQLQQSGPELVKPGASVKMSCKASGHTFTSYVMNWVKQKPGLGLDWIGYINPYNGG
IKYNEKFKGKATLTSDKSSRTAYMELSSLTSEDSAVYYCYYYGNSYGYWGQGTTLTVSS
>DXD8H HCDR1 (SEQ ID NO:89)
   GHTFTSYV
>DXD8H HCDR2 (SEQ ID NO:90)
   INPYNGGI
>DXD8H HCDR3 (SEQ ID NO:91)
   YYYGNSYGY
>DXD8L (SEQ ID NO:92; the regions in bold and underlined are CDR1-3 (IMGT system), respectively): 
DILMTQSPSSMSVSLGDTVSITCHASQGINSNIGWLQQKPGKSFKGLIYHGTNLEDGVPS
RFSGGGSGADYSLTISSLESEDFADYYCIQYAQFPFTFGSGTKLEIK
>DXD8L LCDR1 (SEQ ID NO:93)
   QGINSN
>DXD8L LCDR2
   HGT
>DXD8L LCDR3 (SEQ ID NO:94)
   IQYAQFPFT

### Humanized Sequence Design of Antibody JLD6

Through sequence similarity alignment, the antibody germline with the highest similarity to the murine antibody JLD6 was selected as the humanization template. The CDRs of the template were replaced with the CDRs of the heavy chain and light chain, respectively, and then key amino acids affecting antibody activity were back-mutated according to the three-dimensional structure predicted by artificial intelligence. The specific humanization process was as follows:
(1) IGHV4-30-4*01 and IGHJ6*01 were selected as the heavy chain humanization templates for the murine antibody JLD6, and Kappa IGKV4-1*01 and IGKJ2*01 were selected as the light chain humanization templates for the murine antibody JLD6. The CDRs of the humanization templates were replaced with the CDRs of the heavy chain or light chain of the murine antibody JLD6, to obtain heavy chain variable region sequence 6231 (SEQ ID NO: 95) and light chain variable region sequence 6235 (SEQ ID NO: 96), respectively;
(2) The variable regions of the murine antibody JLD6 were subjected to structural prediction using the ESMFold module in WeMol software to obtain a three-dimensional structural model;
(3) According to the variable region structure of the murine antibody JLD6, key amino acids in the framework regions that affect the interaction between the heavy chain and the light chain as well as the interaction with CDRs were determined, and the amino acid sites for back-mutation were identified. Heavy chain variable region sequence 6233 (SEQ ID NO: 97) was obtained, which formed the PP-1X-#9 molecule with light chain variable region sequence 6235. Meanwhile, the potential isomerization site DG in the heavy chain CDR3 was removed by modification by post-translational modification, obtaining heavy chain variable region sequence 6496 (SEQ ID NO: 98), which formed the PP-1X-#17 molecule with light chain variable region sequence 6235.

>6231 (SEQ ID NO:95; the regions in bold and underlined are CDR1-3 (IMGT system), respectively): 
QVQLQESGPGLVKPSQTLSLTCTVSGISITNGNHWWSWIRQPPGKGLEWIGYIRPSGSTY
YNPSLKSRVTISVDTSKNQFSLKLSSVTAADTAVYYCARGGLEDGKYYYAMDYWGQGT
TVTVSS
>6231 HCDR1 (SEQ ID NO:76)
   GISITNGNHW
>6231 HCDR2 (SEQ ID NO:77)
   IRPSGST
>6231 HCDR3 (SEQ ID NO:78)
   ARGGLEDGKYYYAMDY
>6235 (SEQ ID NO:96; the regions in bold and underlined are CDR1-3 (IMGT system), respectively): 
DIVMTQSPDSLAVSLGERATINCKSSENVEYFGTSLLAWYQQKPGQPPKLLIYGASTRES
GVPDRFSGSGSGTDFTLTISSLQAEDVAVYYCQQSRKVPSTFGQGTKLEIK
>6235 LCDR1 (SEQ ID NO:80)
   ENVEYFGTSL
>6235 LCDR2
   GAS
>6235 LCDR3 (SEQ ID NO:81)
   QQSRKVPST
>6233 (SEQ ID NO:97; the regions in bold and underlined are CDR1-3 (IMGT system), respectively): 
QVQLQESGPGLVKPSQTLSLTCTVSGISITNGNHWWNWIRQPPGKGLEWIGYIRPSGST
YSNPSLKSRVTISRDTSKNQVSLKLSSVTAADTAVYYCARGGLEDGKYYYAMDYWGQG
TTVTVSS
>6233 HCDR1 (SEQ ID NO:76)
   GISITNGNHW
>6233 HCDR2 (SEQ ID NO:77)
   IRPSGST
>6233 HCDR3 (SEQ ID NO:78)
   ARGGLEDGKYYYAMDY
>6496 (SEQ ID NO:98; the regions in bold and underlined are CDR1-3 (IMGT system), respectively): 
QVQLQESGPGLVKPSQTLSLTCTVSGISITNANHWWNWIRQPPGKGLEWIGYIRPSGST
YSNPSLKSRVTISRDTSKNQVSLKLSSVTAADTAVYYCARGGLEEGKYYYAMDYWGQG
TTVTVSS
>6496 HCDR1 (SEQ ID NO:99)
   GISITNANHW
>6496 HCDR2 (SEQ ID NO:77)
   IRPSGST
>6496 HCDR3 (SEQ ID NO:100)
   ARGGLEEGKYYYAMDY

### Construction and Expression and Purification of Humanized Antibodies

Through primer design, the heavy chain sequence and light chain sequence of the antibody were constructed onto the expression vector pcDNA3.4, respectively, to obtain the corresponding plasmids. Expi293 cells were used for transient transfection. The specific transfection and purification processes were as follows:
HEK293SQ cells were cultured in Expi293 expression medium. Using the ExpiFectamine 293 transfection kit, transient transfection was performed according to the manufacturer's instructions. A transfection enhancer was added 20 h after transfection, and the supernatant was collected 120 h later for subsequent protein purification.

Magnetic beads were activated with 0.1 M NaOH and PBST, respectively. The supernatant was added and incubated at room temperature for 1.5 h. Impurities were washed away with PBST and water, respectively. The target protein was eluted with 1000 µL of elution buffer, and the purity of the sample was determined by 4% to 12% SDS-PAGE.

### Cell Protection Experiment of Humanized Anti-Dxd Antibody

NCI-H292 cells were digested, centrifuged at 1200 rpm for 5 minutes, and the supernatant was discarded. The cells were resuspended in 1640 + 10% FBS medium, counted, adjusted to the corresponding concentration, and added into Corning 3610 plates at 60 µL per well (5000 cells per well). The plates were placed in a 37°C incubator for later use;
The antibody concentration was diluted (40 µg/mL or 20 µg/mL, 2-fold serial dilution), and the concentration of free Dxd was fixed to 10 ng/mL. 30 µL of Dxd and 30 µL of antibody were added at a 1:1 ratio into the 3610 plates with cells. The total culture system was 120 µL. The cells were cultured in a 37°C incubator for 3 days;
The plates were taken out and equilibrated to room temperature. 100 µL of CellTiter-Glo^{®} Luminescent Cell was added to each well, mixed well, and left to stand at room temperature protected from light for 10 min;
Luminescence values were determined on a microplate reader, and the calculation formula was: %Cytotoxicity = (Blank - Sample) / Blank * 100%.

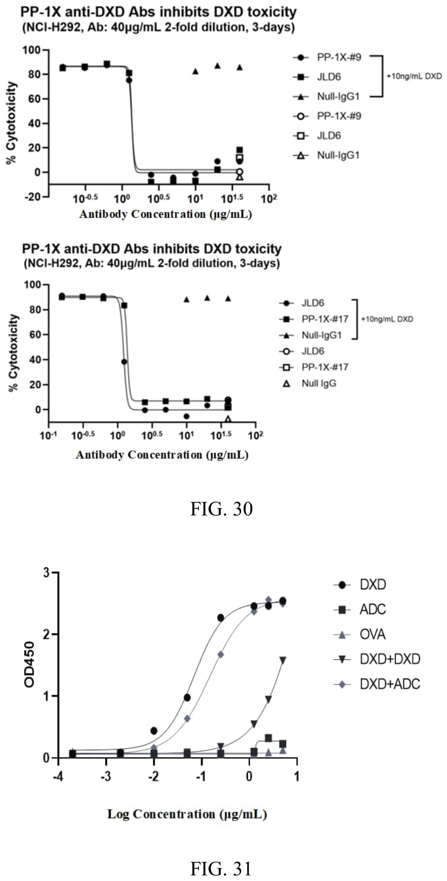

The experimental results are shown in FIG. 30. The results indicated that molecules PP-1X-#9 and PP-1X-#17 possess good cell protection activity.

### Example 4. Screening of Nanobodies Specifically Binding to DXD

### Example 4-1 Alpaca Immunization Protocol and Screening Method

**Table 18. Alpaca immunization scheme**

| Group | Alpaca 2-1 | Alpaca 2-2 | Alpaca 2-3 |
|---|---|---|---|
| Adjuvant | Freund's Adjuvant | Water-based Adjuvant | Freund's Adjuvant |
| Immunogen | KLH-DXD | KLH-DXD | OVA-DXD |
| Immunization Frequency | 4 times | 4 times | 3 times |
| Interval | 2 weeks | 2 weeks | 2 weeks |

### Alpaca 2-1/2-3 Group:

Primary Immunization: An emulsion prepared with Freund's Complete Adjuvant (FCA) and the antigen at a 1:1 volume ratio was used. The immunization dose was 500 µg antigen per animal.

Booster Immunization: An emulsion was prepared using Freund's Incomplete Adjuvant (FIA) every 14 days, with the dose adjusted to 250 µg per animal.

### Alpaca 2-2 Group:

Primary Immunization: Dose of 500 µg antigen per animal.

Booster Immunization: Dose of 250 µg antigen per animal, with the immunization interval cycle the same as Group 2-1.

### Screening Method After Immunization:

For Alpaca 2-3, venous blood was collected 14 days after the third immunization. For Alpacas 2-1/2-2, venous blood was collected on the 7th day after the fourth immunization. Peripheral blood mononuclear cells (PBMCs) were isolated, and RNA extraction was performed. After reverse transcribing RNA into cDNA, a nanobody phage library was constructed via PCR, restriction enzyme digestion, and ligation reactions.

After two rounds of panning, unique sequences were selected by ELISA determination. A total of 1000 antibody strains were screened in this batch. Subsequently, antibodies in a VHH+Fc form were constructed for antibody ELISA determination and cell protection experiments.

### Example 4-2 Antibody ELISA determination

1) Unique sequences were constructed into expression vectors and transiently transfected into HEK293F suspension cells.
2) Protein Purification: Protein A magnetic bead affinity chromatography was performed. The purified proteins were subjected to ELISA determination. OVA-DXD/ADC (structure shown in FIG. 58, Ab-MC-GGFG-DXD)/OVA were coated at 100 ng/well, respectively, and incubated overnight at 4°C.
3) The next day, the liquid in the plates was discarded, and the plates were placed in a plate washer and washed 3 times. Blocking: The blocking solution was 2%-3% BSA. 200 µL of blocking solution was added to each well and incubated at 37°C for 1 h.
4) Washing: The liquid in the plates was discarded, and the plates were placed in a plate washer and washed 3 times.
5) Antibody Gradient Dilution: Each antibody corresponded to five experimental groups. Group 1 was OVA-DXD plate + antibody + PBS; Group 2 was OVA-DXD plate + antibody + DXD (40 µM); Group 3 was OVA-DXD plate + antibody + ADC (500 nM); Group 4 was OVA plate + antibody + PBS; and Group 5 was ADC plate + antibody + PBS.
6) 30 µL each of antibody, PBS, DXD, and ADC were added and incubated at 37°C for 1 h.
7) The liquid in the plates was discarded, and the plates were placed in a plate washer and washed 3 times.
8) Secondary Antibody: Anti ALFA tag Mab HRP diluted at 1:5000 was added and incubated at 37°C for 1 h. Washing: The liquid in the plates was discarded, and the plates were placed in a plate washer and washed 3 times.
9) Chromogenic solution (100 µL TMB) was added, and color development was performed protected from light. 50 µL of stop solution was added, and the OD450nm value was measured using a microplate reader.

The experimental results are shown in FIG. 31. The results indicated that antibody No. 86 performed better in terms of specificity.

### Example 4-3 Cell protection experiment

NCI-H292 cells were resuscitated and cultured in 1640 medium containing 10% FBS until 80% confluence. The cells were digested with trypsin, centrifuged, resuspended, and the cell density was adjusted to 3 × 10⁴ cells/mL. The cell suspension was inoculated into 96-well plates at 100 µL per well (approximately 3000-5000 cells/well). Edge wells were filled with PBS to reduce evaporation interference. The cells were incubated in a 37°C, 5% CO₂ incubator for 24 h to allow cell attachment. The following groups were set up: Antibody Protection Group: cells + antibody pretreatment + DXD toxin + medium; control antibody protection group: cells + control antibody IgG1 pretreatment + DXD toxin + medium. The original medium was aspirated and discarded. 100 µL of antibody at a concentration of 200 µg/mL prepared in fresh medium was added to each well. Medium containing DXD toxin (10 nM) was added, and the cells were cultured at 37°C with 5% CO₂ for 48 h. 50 µL of cell proliferation/cytotoxicity determination reagent was added to each well, shaken gently to mix evenly, and incubated at 37°C protected from light for 5 to 30 min. Chemiluminescence values were measured using a microplate reader.

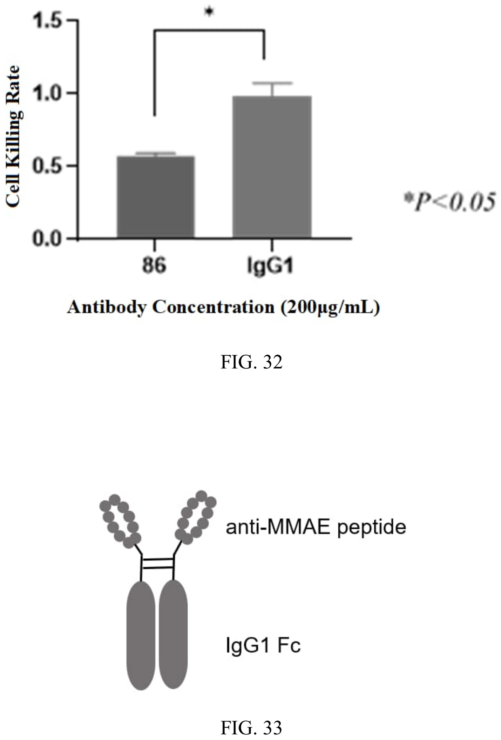

The experimental results are shown in FIG. 32. The results indicated that antibody No. 86 was able to produce cell protection effects compared to the control group.

Antibody No. 86 was sequenced, and the amino acid sequence is as follows:
>86 (SEQ ID NO:101; the regions in bold and underlined are CDR1-3 (IMGT system), respectively): 
QVQLQESGGGLVQPGGSLRLSCAASGFTFSIYYMGWVRQAPGKGPEWVSAINTGGGST
WYADSVKGRFTISRDNAKNTLYLQMNSLKPEDTALYYCARVLDPSTWEYDTWGQGTQ
VTVSSEPKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKAL
PAPIEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPG
>86 VHH (SEQ ID NO:102; the regions in bold and underlined are CDR1-3 (IMGT system), respectively): 
QVQLQESGGGLVQPGGSLRLSCAASGFTFSIYYMGWVRQAPGKGPEWVSAIN
TGGGSTWYADSVKGRFTISRDNAKNTLYLQMNSLKPEDTALYYCARVLDPSTWEYDT
WGQGTQVTVSS
>86 CDR1 (SEQ ID NO:103)
   GFTFSIYY
>86 CDR2 (SEQ ID NO:104)
   INTGGGST
>86 CDR3 (SEQ ID NO:105)
   ARVLDPSTWEYDT

### Example 5 Screening of Peptides Specifically Binding to MMAE

### Example 5-1 Construction of phage display library of octa/dodeca-cyclic peptide

Synthesis of random octa-/dodeca-cyclic peptide oligonucleotide primers.

The design strategy for the oligonucleotide fragments encoding the random octa-/dodeca-cyclic peptides adopted the NNK codon coding method, wherein N is any nucleotide (A/T/C/G), and K is G/T. The synthesized random nucleotide sequences are shown in Table 19.

**Table 19. Nucleotide sequences used for peptide library construction**

| Name | Sequence |
|---|---|
| Nucleotide sequence of octa-cyclic peptide | CTGGCTGGTTTCGCTACCGTGGCCCAGG^CGG CCGCTTGT(NNK)8TGCGGTGGAGGTGGCCAG GC^CGGCCAGCACCATCACCATCACCAT |
| Nucleotide sequence of dodeca-cyclic peptide | CTGGCTGGTTTCGCTACCGTGGCCCAGG^CGG CCGCTTGT(NNK)12TGCGGTGGAGGTGGCCAG GC^CGGCCAGCACCATCACCATCACCAT |
| Exten-R extension primer | ATGGTGATGGTGATGGTGCT |
| Exten-R corresponding peptide chain | Ala Cys (X)_{8 or12} Cys Gly Gly Gly |

### PCR amplification of double-stranded DNA fragments

A palindromic sequence was designed at the 3' end of the long-chain DNA primer to serve as a primer for DNA polymerase, guiding DNA synthesis from the 3' end to the 5' end to obtain a complete double-stranded DNA sequence. The synthesized oligonucleotide template encoding the octa-cyclic peptide library was added to ddH₂O and dissolved into a 100 µM solution. 5 µg was taken and mixed with an equimolar amount of universal extension primer in 50 µL of ddH₂O, thermally denatured in a 95°C water bath for 5 min, and naturally cooled in the water bath to below 37°C to form an annealing mixture. An equal volume of PrimeSTAR Max Premix (TAKARA, CAT#: R045B) was added to the annealed reactant to prepare a PCR solution, which was mixed and momentarily centrifuged. The annealed product was extended at 37°C for 10 min and at 65°C for 15 min to synthesize double-stranded DNA fragments encoding the octa-/dodeca-cyclic peptide library.

### Digestion, ligation, and transformation of pComb3XSS phage vector and DNA Fragments

The prepared DNA fragments and pComb3XSS were digested using restriction endonuclease SfiI (NEB, CAT#: R0123L) and incubated at 50°C for 18 h. The pComb3XSS digested overnight was separated by 1% agarose gel electrophoresis, and purified and recovered according to the instructions of the gel extraction kit (MN, CAT#740609.250). Then, the linearized pComb3XSS plasmid and the digested DNA fragments were ligated using T4 ligase. The molar ratio of vector to DNA fragment was 1:10, and the mixture was incubated at a constant temperature of 16°C for 19 h. The ligation product was purified and recovered using a purification kit, eluted with ultrapure water (30 µL/tube), and the concentration was measured using Nanodrop. 350 µL of freshly prepared TG1 competent cells were added to each electroporation cuvette (2 mm, pre-cooled in an ice-water bath), followed immediately by the addition of 500 ng of the ligation product. The bottom of the cuvette was flicked to mix evenly, and incubated in an ice bath for 10 min. Water on the walls and bottom of the electroporation cuvette was thoroughly wiped dry, the voltage was set to 2.5 KV, and electroporation was performed.

### Identification of peptide library

12 clones were randomly picked and sequenced, and sequence alignment was performed to analyze the quality of the peptide library.

### Example 5-2 Enrichment screening for MMAE-binding peptides from phage-displayed cyclic peptide library

### Peptide phage display library panning

Prior to panning, immunotubes were coated. The immunotubes were washed 3 times with PBS, and the residual liquid was aspirated dry. According to the immunization strategy, immunotubes were coated with a negative screening antigen and a positive screening antigen, respectively, which were diluted to the final coating concentration using 500 µL of coating buffer. After mixing at room temperature and 150 rpm for 2 h, the tubes were placed at 4°C overnight for about 16 h. 1.35E+13 cfu of the P12P phage display library was taken as the input for the first round of panning; for the second to fourth rounds of panning, about 1.00E+13 cfu of the phage display library enriched in the previous round was taken as the input. The input was blocked using a Casein solution (Thermo Scientific, Cat. 37528) with a concentration of 50%, and the volume was made up to 500 µL with 1× PBS. Overnight activated E. coli TG1 was inoculated into 120 mL of 2YT liquid medium and cultured at 37°C and 250 rpm for 2 to 3 h until an OD of 0.6 to 0.8 was reached for later use. After the immunotube coated with the positive screening antigen was washed with 1× PBS, 1 mL of Casein solution was added for blocking for 1 h. The blocked phage input was added to the immunotube coated with the negative screening protein and incubated at room temperature and 150 rpm for 1 h to remove the phages with the toxin-conjugated protein. After the immunotube coated with the positive screening antigen was blocked, the immunotube was washed, and the phages that had undergone negative screening were transferred into the immunotube and allowed to bind at room temperature and 150 rpm for 1 to 2 h. The immunotube was washed 3 times with 1× PBST, placed on a shaker at 150 rpm for 1 to 2 min during washing, then 5 nmol of Truncated MMAE solution was added, and competitive elution was performed at room temperature and 150 rpm for 1 h. The eluate was transferred to an immunotube coated with the protein of the second negative screening and incubated at room temperature and 150 rpm for 1 h to further negatively screen and remove the phages with the toxin-conjugated protein. The eluted phages were then added to 40 mL of TG1 bacterial culture at OD 0.6-0.8, and a glucose solution was added to a final concentration of 2%. Infection was performed at 37°C for 45-60 min, with the bacterial culture gently shaken every 15 min. After infection, 100 µL of bacterial culture was taken and diluted into four gradients of 10¹, 10², 10³, and 10⁴, and 100 µL of each was plated on small plates (10 cm*10 cm 2YT-Amp-Glu). The number of single colonies was counted the next day to monitor the enrichment of phages. In addition, 1 mL of bacterial culture was taken, added with glycerol, and stored at -80°C. 1/1000 Amp (100 mg/mL) and helper phage M13KO7 (Mol 20:1) (NEB, Cat. N0315S) were added to the remaining bacterial culture, and stationary infection was performed at 37°C for 1 h. The bacterial culture was collected by centrifugation, the supernatant was discarded, and 40 mL of 2YT-Amp liquid medium containing 1/1000 Kan (50 mg/mL) and IPTG (50 mg/mL) was added, followed by overnight culture at 30°C and 220 rpm. The overnight cultured bacterial liquid was centrifuged at 10,000 rpm for 10 min; the supernatant was collected, filtered through a 0.45 µm filter, and precipitated with 1/5 volume of PEG-NaCl. It was placed on ice for 1 h, centrifuged at 10,000 rpm at 4°C for 30 min, the supernatant was discarded, and the precipitate was resuspended in PBS for panning. The OD260 value of the phage was determined by Nanodrop. The amount of phage input used for panning was determined according to the OD260 value, where 1 OD corresponded to approximately 3.0E+12.

### Monoclonal screening of peptide phage display library

600 µL of Amp+ 2YT medium was added to each well of a 96-well plate (sterile, round bottom). Single colonies were picked from P12P P1 Round 2 to 4 plates for culture at 37°C and 220 rpm for 3 to 6 h until an OD of 0.6 was reached. The produced phages were used for phage ELISA. 150 µL of bacterial culture was transferred to a new 96-well plate, glycerol was added, and the strains were stored at -80°C. Helper phage M13KO7 (Mol 20:1) was added to the 96-well plate, followed by stationary infection at 37°C for 1 h. 1/1000 Kan (50 mg/mL) and IPTG (50 mg/mL) were added, followed by overnight culture at 30°C and 220 rpm. The phages cultured overnight in the 96-well plate were centrifuged at 4000 rpm for 30 min; the supernatant (400 µL) was transferred to a new 96-well plate for Phage ELISA.

### Phage ELISA experimental method

Phage ELISA blocking: The 96-well plate coated with antigen was washed once with PBST using a plate washer, and blocked by adding Casein solution at 200 µL/well at 37°C for 1 h. Phage ELISA primary antibody incubation: The plate was washed 3 times with PBST, and 100 µL of phage supernatant cultured overnight was added to each well. Two negative control wells were set: Blank and M13KO7 (1:100). The plate was incubated at 37°C for 1 to 2 h. Phage ELISA secondary antibody incubation: The plate was washed 6 times with PBST, and secondary antibody anti-M13KO7-HRP (1:10000) (Sino. Biological, Cat. 11973-MM05T-H) diluted with 3% Milk-PBS was added and incubated at 37°C for 1 h. Color development: The plate was washed 6 times with PBST, 100 µL of TMB color development solution was added to each well, and color was developed for 2 to 10 min protected from light. Termination: 100 µL of 2 M H₂SO₄ was added to each well to terminate the reaction. The OD450 value was read using a microplate reader, recorded, and saved. Data were analyzed, and specific positive clones were selected and sent for sequencing. The sequences were obtained through analysis using CLC software, followed by the next step of construction, expression, and purification. The format of the constructed molecule is shown in FIG. 33.

### Example 5-3 Verification of Specific Binding Between Peptide Molecules and MMAE

### ELISA Determination of Binding Specificity Between Fc Fusion Peptide and MMAE

The constructed Fc fusion peptide molecules PP-1A-#84 to #95 were used for identification of binding specificity to OVA-MMAF and OVA, to determine molecules with better specific binding activity to MMAE. After the first round of primary screening, it was further verified that #85/#86/#89/#93 specifically bound to OVA-MMAF but did not bind to the negative protein OVA; and repeated verification was performed on #84/#87/#88/#90/#91/#92/#94 to see whether they specifically bind to MMAE.

**Table 20. Amino acid sequence listing of PP-1A-#85 (peptide) to PP-1A-#94 (peptide) and PP-1A-#85 (Fc fusion peptide)**

| Sequence No. | Code Name | Sequence |
|---|---|---|
| SEQ ID NO:106 | PP-1A-#85 (peptide) | ACDESHGVPHTSGMC |
| SEQ ID NO:107 | PP-1A-#86 (peptide) | ACLPNSLGPSAMSSC |
| SEQ ID NO:108 | PP-1A-#87 (peptide) | ACLVLKLSTGIKSEC |
| SEQ ID NO:109 | PP-1A-#88 (peptide) | ACRLTGPDWLHTQRC |
| SEQ ID NO:110 | PP-1A-#89 (peptide) | ACTPALRSLAC |
| SEQ ID NO:111 | PP-1A-#90 (peptide) | ACLRNSPRKQC |
| SEQ ID NO:112 | PP-1A-#91 (peptide) | ACHWNAKADYGSFVC |
| SEQ ID NO:113 | PP-1A-#92 (peptide) | ACTGCSRTHSPRSRC |
| SEQ ID NO:114 | PP-1A-#93 (peptide) | ACVLSPHPQAC |
| SEQ ID NO:115 | PP-1A-#94 (peptide) | ACDSLGGSPVC |
| SEQ ID NO:116 | PP-1A-#85 (Fc fusion peptide) | ACDESHGVPHTSGMCGGGSDKT HTCPPCPAPELLGGPSVFLFPPKP KDTLMISRTPEVTCVVVDVSHE DPEVKFNWYVDGVEVHNAKTK PREEQYNSTYRVVSVLTVLHQD WLNGKEYKCKVSNKALPAPIEK TISKAKGQPREPQVYTLPPSREE MTKNQVSLTCLVKGFYPSDIAVE WESNGQPENNYKTTPPVLDSDG SFFLYSKLTVDKSRWQQGNVFSC SVMHEALHNHYTQKSLSLSPG |

### Peptide ELISA binding experiment method

(1) Antigen coating: MMAF conjugated protein and negative protein were coated onto a microplate using coating buffer at a concentration of 1 µg/mL, and the plate was placed at 4°C overnight for about 16 h. (2) Blocking: The plate coated with antigen was washed once with PBST using a plate washer, and blocked by adding Casein solution at 200 µL/well at room temperature for 1 h. (3) Primary antibody incubation: The plate was washed 3 times with PBST, and peptide samples with an initial concentration of 2000 nM diluted in a 5-fold gradient using Casein solution were added. The negative control was the Fc.uIgG1 molecule. Incubation was performed at room temperature for 2 h. (4) Secondary antibody incubation: The plate was washed 3 times with PBST, and secondary antibody Goat pAb to Hu IgG(HRP) (1:10000) (Abcam, Cat. Ab97225) diluted with Casein solution was added and incubated at room temperature for 1 h. (5) Color development: The plate was washed 6 times with PBST, 100 µL of TMB color development solution was added to each well, and color was developed for 2 to 10 min protected from light. (6) Termination: 100 µL of 2 M H₂SO₄ was added to each well to terminate the reaction. The OD450 value was read using a microplate reader, recorded, and saved. Finally, the data were analyzed using GraphPad software.

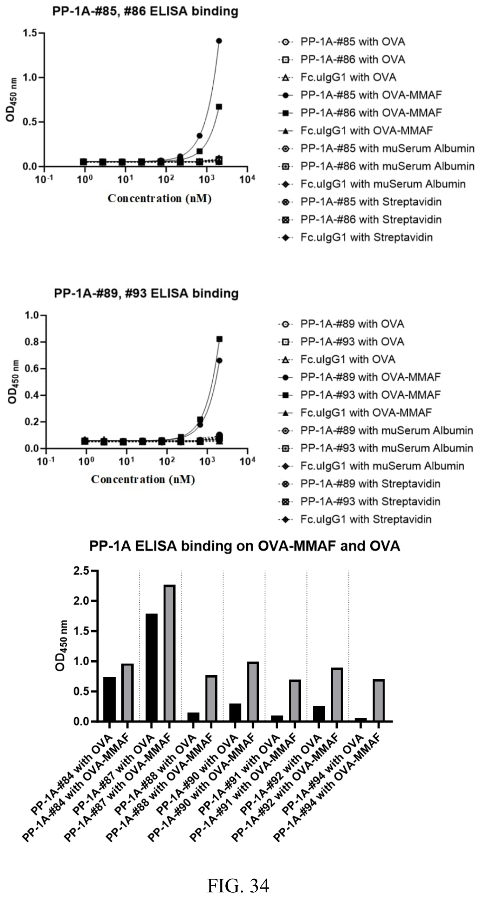

The ELISA binding results are as shown in FIG. 34. #85/#86/#89/#93 had good binding activity to OVA-MMAF and did not bind to OVA; molecules #88/#90/#91/#92 had specific binding to OVA-MMAF, but the binding activity was relatively weak.

### Example 5-4 Identification of Binding Activity of Peptide Molecules with Free MMAE by Competitive ELISA

### Identification of Competitive Binding of Free MMAE to Peptide Molecules

Competitive experiments were adopted to further analyze whether PP-1A-#85/#93 bound to free MMAE and did not bind to MMAE-conjugated antibody.

### Peptide Competitive ELISA Experimental Method

(1) Antigen coating: BSA-MMAF was coated onto a microplate using coating buffer at a concentration of 1 µL/mL, and placed at 4°C overnight for about 16 h. (2) Blocking: The microplate coated with antigen was washed once with PBST using a plate washer, and blocked by adding Casein solution at 200 µL/well at room temperature for 1 h. (3) Primary antibody incubation: The plate was washed 3 times with PBST, and a premixture of peptide sample diluted with Casein solution and truncated free MMAE/null-ADC was added. The final concentration of the peptide sample was 2000 nM. The initial concentration of truncated free MMAE and null-ADC (an IgG1 antibody-conjugated MMAE that does not bind to any target) was 1000 nM, diluted in a 10-fold gradient. The positive control was molecule ABC3320 with a final concentration of 1.28 nM. Incubation was performed at room temperature for 2 h. (4) Secondary antibody incubation: The plate was washed 3 times with PBST, and secondary antibody Goat pAb to Hu IgG(HRP) (1:10000) diluted with Casein solution was added and incubated at room temperature for 1 h. (5) Color development: The plate was washed 6 times with PBST, 100 µL of TMB color development solution was added to each well, and color was developed for 2 to 10 min protected from light. (6) Termination: 100 µL of 2 M H₂SO₄ was added to each well to terminate the reaction. The OD450 value was read using a microplate reader, recorded, and saved. Finally, the data were analyzed using GraphPad software.

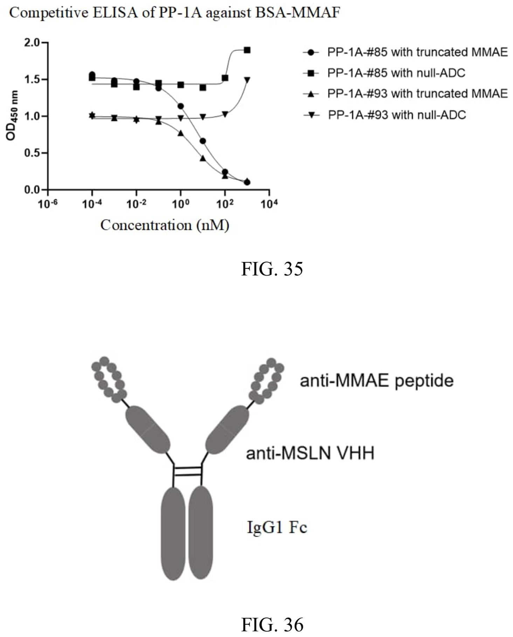

The competitive ELISA results are as shown in FIG. 35. The binding of PP-1A-#85/#93 to BSA-MMAF was blocked by truncated free MMAE, but not blocked by null-ADC, indicating that the two peptide molecules #85/#93 specifically bound to MMAE and did not bind to null-ADC.

### Example 5-5 Prokaryotic expression of peptide molecules

### Identification of Specific Binding Between Antibody Fusion Peptide and MMAE

Based on the binding activity and competitive identification, molecule PP-1A-#85 was selected to construct an Anti-MSLN-MMAE antibody fusion peptide, and the format of the constructed molecule is as shown in FIG. 36. After the vector was constructed, chemical transformation of the BL21 competent strain was performed, and single colonies were picked for activation culture. After the activated bacterial culture was sequenced to confirm that the sequence was correct, it was inoculated at a ratio of 1:100 into 2 × YT medium (1/1000 Kan (50 mg/mL)) and cultured at 37°C and 220 rpm for 1 to 2 h until the OD value of the bacterial culture was 0.6 to 0.8. The shaker temperature was adjusted to 16°C. After the temperature was reached, IPTG solution (0.3 mM) (Solarbio, Cat. 12.02.11020) was added to the cultured bacterial liquid, and induction culture was performed at 16°C and 220 rpm for 24 h. The bacteria were collected by centrifugation and thoroughly resuspended using pre-cooled PBS solution. The resuspended bacterial liquid was subjected to ultrasonic disruption at a power of 120 W for a disruption time of about 1.25 h. A low-temperature state of the bacterial liquid needed to be maintained during the ultrasonic disruption process. The bacterial liquid after ultrasonic disruption was centrifuged multiple times to collect the supernatant, obtaining a relatively clear solution for the next step of protein purification.

**Table 21. Amino acid sequence listing of PP-1A-#600**

| Sequence No. | Code Name | Sequence |
|---|---|---|
| SEQ ID NO: 117 | PP-1A-#600 (PP-1A-#85 peptide + MSLN VHH+ Fc) | ACDESHGVPHTSGMCGGGSEVQLVESGG GLVQPGGSLRLSCAVSGFPLSSYGMSWAR QAPGEERELVAHIGTTGGSTNYADSVKGR FTISRDNAKNSVYLQMNSLRAEDTAVYYC RARRQMPSNEYWGQGTTVTVSSDKTHTC PPCPAPELLGGPSVFLFPPKPKDTLMISRTP EVTCVVVDVSHEDPEVKFNWYVDGVEV HNAKTKPREEQYNSTYRVVSVLTVLHQD WLNGKEYKCKVSNKALPAPIEKTISKAKG QPREPQVYTLPPSREEMTKNQVSLTCLVK GFYPSDIAVEWESNGQPENNYKTTPPVLD SDGSFFLYSKLTVDKSRWQQGNVFSCSVM HEALHNHYTQKSLSLSPG |

### Example 5-6 Verification of Specific Binding Between Antibody Fusion Peptide and MMAE

The binding activity of antibody fusion peptide molecule PP-1A-#600 (amino acid sequence as shown in Table 21) with OVA-MMAF and OVA was determined using ELISA to verify its specific binding to MMAE.

### Peptide ELISA Binding Experimental Method

(1) Antigen coating: OVA-MMAF and OVA proteins were coated onto a microplate using coating buffer at a concentration of 1 µg/mL, and placed at 4°C overnight for about 16 h. (2) Blocking: The microplate coated with antigen was washed once with PBST using a plate washer, and blocked by adding Casein solution at 200 µL/well at room temperature for 1 h. (3) Primary antibody incubation: The plate was washed 3 times with PBST, and peptide samples with an initial concentration of 2000 nM diluted in a 5-fold gradient using Casein solution were added. The positive control was the ABC3320 molecule, and the negative control was the Fc.uIgG1 molecule, at a concentration of 100 nM. Incubation was performed at room temperature for 2 h. (4) Secondary antibody incubation: The plate was washed 3 times with PBST, and secondary antibody Goat pAb to Hu IgG(HRP) (1:10000) diluted with Casein solution was added and incubated at room temperature for 1 h. (5) Color development: The plate was washed 6 times with PBST, 100 µL of TMB color development solution was added to each well, and color was developed for 2 to 10 min protected from light. (6) Termination: 100 µL of 2 M H₂SO₄ was added to each well to terminate the reaction. The OD450 value was read using a microplate reader, recorded, and saved. Finally, data were analyzed using GraphPad software.

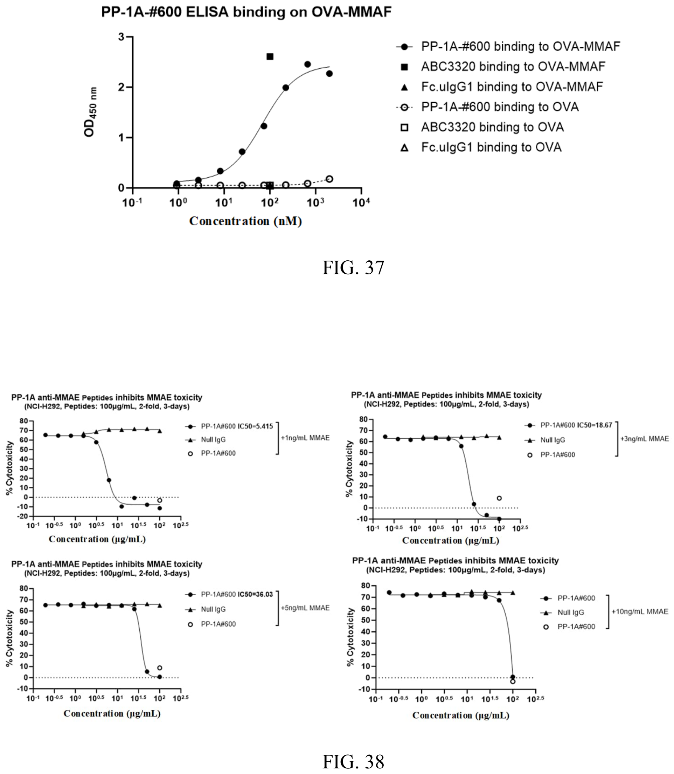

The ELISA binding results are as shown in FIG. 37. PP-1A-#600 had strong binding activity with OVA-MMAF, but did not bind to OVA, demonstrating good binding specificity.

### Example 5-7 Cell experiment for detecting the inhibitory effect of MMAE-binding peptides on toxin cytotoxicity

NCI-H292 tumor cell line was used to detect the inhibitory effect of PP-1A-#600 on MMAE toxicity.
(1) NCI-H292 cells were digested, counted, and plated at 5E3 cells/well, with 60 µL per well, and allowed to adhere in a 37°C incubator. (2) Peptide PP-1A-#600, control antibody BMK219 (ABC3320-Fc), and nonsense control antibody Null IgG were 2-fold diluted for 10 gradients starting from a concentration of 100 µg/mL. The MMAE concentrations were fixed at 1 ng/mL, 3 ng/mL, 5 ng/mL, and 10 ng/mL, respectively. The test substances were incubated with MMAE at room temperature for 2 h. (3) The incubated samples were added into the 96-well plate at 60 µL/well; and cultured in a 37°C incubator for 3 days. (4) 100 µL of Cell Counting-Lite 2.0 Reagent was added to each well, and the plate was shaken on a shaker for 10 min. (5) The fluorescence signal was read using a microplate reader.

The experimental results are as shown in FIG. 38. The results showed that when the MMAE concentration was 1, 3, 5, and 10 ng/mL, the PP-1A-#600 fusion molecule was able to exhibit a good cytoprotective effect.

### Example 6 Screening of Peptides Specifically Binding to DXD

### Example 6-1 Construction of Phage Display Octa-/Pentadeca-Linear Peptide Library

### Synthesis of random octa-/pentadeca-linear peptide oligonucleotide primers

The design strategy for the oligonucleotide fragments encoding random linear octapeptides and pentadecapeptides adopted the NNK or NHC codon coding method, wherein N is any nucleotide (A/T/C/G), K is G/T, and H is A/C/T. The synthesized random nucleotide sequences are shown in Table 22.

**Table 22. Nucleotide sequences used for peptide library construction**

| Name | Sequence |
|---|---|
| Octapeptide nucleotide sequence | CTGGCTGGTTTCGCTACCGTGGCCCAGG^CG GCCNHC(NNK)8NHCGGTGGAGGTAGCGGCC AGGC^CGGCCAGCACCATCACCATCACCAT CTGGCTGGTTTCGCTACCGTGGCCCAGG^CG GCC(NHC)15GGTGGAGGTAGCGGCCAGGC^C GGCCAGCACCATCACCATCACCAT |
| Pentadecapeptide nucleotide sequence | CTGGCTGGTTTCGCTACCGTGGCCCAGG^CG GCC(NHC)15GGTGGAGGTAGCGGCCAGGC^C GGCCAGCACCATCACCATCACCAT |
| Exten-R extension primer | ATGGTGATGGTGATGGTGCT |
| Exten-R corresponding peptide chain | (X)_{8 or 15} Cys Gly Gly Gly Ser-gIII |

The PCR amplification of double-stranded DNA fragments, and the digestion, ligation, and transformation of the pComb3XSS phage vector and DNA fragments were performed with reference to the relevant steps in Example 5-1.

### Identification of peptide library

20-30 clones were randomly picked and sequenced, and sequence alignment was performed to analyze the quality of the peptide library.

### Example 6-2 Enrichment and Screening of Phage Display Peptide Library for DXD-Binding Peptides

### Panning of peptide phage display library

A cross strategy of liquid phase and solid phase panning was performed. Liquid phase panning experimental method: Streptavidin magnetic beads (Magnosphere, Cat. MS300/Streptavidin) were used. Before use, the magnetic beads and the preservation solution were mixed, and the magnetic beads of the required concentration were taken and thoroughly washed 3 times with PBS. Negative screening magnetic beads were used to remove non-specific binding between phages and magnetic beads; they were blocked by incubating with 3% BSA (Bovogen, Cat. BSAS 0.1) solution at room temperature for 30 min. To the positive screening magnetic beads, 1 mL of positive screening antigen biotin-DXD diluted in PBS was added, and incubated at room temperature for 1 h for binding. 1.00E+13 cfu of the phage display library was taken respectively as the input for the first round of panning; for the second round of panning, about 1.00E+13 cfu of the phage display library enriched in the previous round was taken as the input. The phage input was added to the blocked negative screening magnetic beads; the blocking solution was a 3% BSA solution, and the volume was made up with 1× PBS. At the same time, the positive screening beads, after binding to the antigen, were washed with 1× PBS and then blocked by adding 3% BSA solution and incubating at room temperature for 1 h. The blocked positive screening beads were washed three times with PBS, and the phages from which non-specific binding had been removed were added and allowed to bind at room temperature for 1 h. The magnetic beads were washed with PBST/PBS, with the washing intensity increased appropriately with the number of panning rounds. After washing, 1 mL of 0.1M Gly-HCl (pH 2.2) was added for rotary elution at room temperature for 10 min. The eluate was collected, and 100 µL of 1 M Tris-HCl (pH 8.0) was added to the acid-washed eluate for neutralization.

Solid phase panning experimental method: Prior to panning, immunotubes were coated. The immunotubes were washed 3 times with PBS, and the residual liquid was aspirated dry. According to the immunization strategy, immunotubes were coated with a negative screening antigen and a positive screening antigen, respectively, which were diluted to the final coating concentration using 500 µL of coating buffer. After mixing at room temperature and 150 rpm for 2 h, the tubes were placed at 4°C overnight for about 16 h. About 1.00E+13 cfu of the phage display library enriched in the previous round was taken as the input for the third round of panning. The input was blocked using a Casein solution (Thermo Scientific, Cat. 37528) with a concentration of 50%, and the volume was made up to 500 µL with 1× PBS. After the immunotube coated with the positive screening antigen was washed with 1× PBS, 1 mL of Casein solution was added for blocking for 1 h. The blocked phage input was added to the immunotube coated with the negative screening protein and incubated at room temperature and 150 rpm for 1 h to remove the binding between the phages and the toxin-conjugated protein. After the immunotube coated with the positive screening antigen was blocked, the immunotube was washed, and the phages that had undergone negative screening were transferred into the immunotube and allowed to bind at room temperature and 150 rpm for 1 to 2 h. The immunotube was washed 3 times with 1 × PBST, placed on a shaker at 150 rpm for 1 to 2 min during washing, then 5 nmol of DXD solution was added, and competitive elution was performed at room temperature and 150 rpm for 1 h. The eluted phages were added to 40 mL of TG1 bacterial culture at OD 0.6-0.8, and a glucose solution was added to a final concentration of 2%. Infection was performed at 37°C for 45-60 min, with the bacterial culture gently shaken every 15 min. After infection, 100 µL of bacterial culture was taken and diluted in three gradients of 10¹, 10² and 10³, and 100 µL of each was plated on small plates (10 cm*10 cm 2YT-Amp-Glu). The number of single colonies was counted the next day to monitor the enrichment of phages. 1 mL of bacterial culture was taken, added with glycerol, and stored at -80°C. 1/1000 Amp (100 mg/mL) and helper phage M13KO7 (Mol 20:1) (NEB, Cat. N0315S) were added to the remaining bacterial culture, and stationary infection was performed at 37°C for 1 h. The bacterial culture was collected by centrifugation, the supernatant was discarded, and 40 mL of 2YT-Amp liquid medium containing 1/1000 Kan (50 mg/mL) and IPTG (50 mg/mL) was added, followed by overnight culture at 30°C and 220 rpm. The overnight cultured bacterial liquid was centrifuged at 10,000 rpm for 10 min; the supernatant was collected, filtered through a 0.45 µm filter, and precipitated with 1/5 volume of PEG-NaCl. It was placed on ice for 1 h, centrifuged at 10,000 rpm at 4°C for 30 min, the supernatant was discarded, and the precipitate was resuspended in PBS for panning. The OD260 value of the phage was determined by Nanodrop. The amount of phage input used for panning was determined according to the OD260 value, where 1 OD was approximately 3.0E+12.

### Monoclonal screening of peptide phage display library

600 µL of Amp+2YT medium was added to each well of a 96-well plate (sterile, round bottom). Single colonies were picked from P8P/P12P P5 Round 2/3 plates for culture. They were cultured at 37°C and 220 rpm for 3 to 6 h until an OD of 0.6 was reached. The produced phages were used for phage ELISA. 150 µL of bacterial culture was transferred to a new 96-well plate, glycerol was added, and the strains were stored at -80°C. Helper phage M13KO7 (Mol 20:1) was added to the 96-well plate, followed by stationary infection at 37°C for 1 h. 1/1000 Kan (50 mg/mL) and IPTG (50 mg/mL) were added, followed by overnight culture at 30°C and 220 rpm. The phages cultured overnight in the 96-well plate were centrifuged at 4000 rpm for 30 min; the supernatant (400 µL) was transferred to a new 96-well plate for phage ELISA.

### Phage ELISA experimental method

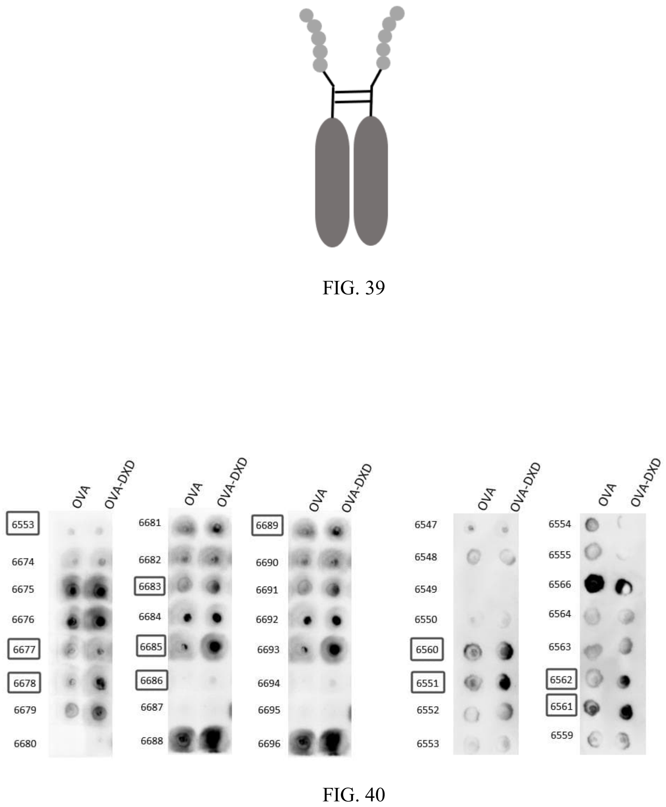

The method was performed with reference to the relevant steps in Example 5-1. The format of the constructed molecule is shown in FIG. 39.

### Example 6-3 Dot Blot Screening for DXD Specific Binding Peptides

Sample preparation: 50 mL of bacterial liquid induced with IPTG overnight (16°C, 16 h) was collected by centrifugation at 5000 rpm for 5 min, and the supernatant was discarded. 7 mL of PBS was added to resuspend the bacterial pellet, and it was disrupted at 200 W for 5 min. It was centrifuged at 12,000 rpm for 20 min, and the supernatant was collected for later use. Spotting: A nylon membrane (Beyotime, CAT#FFN10) was cut to an appropriate size and marked with lines. 3 µL of antigen sample (OVA or OVA-DXD, 20 µg/mL) was spotted onto the membrane using a pipette. The consistency of the spotted volume and the spacing between spots were carefully dealt with to avoid mutual interference between samples. After spotting, the membrane was allowed to dry naturally at room temperature. Blocking: The dried membrane was placed in a blocking solution (5% skim milk) and blocked on a shaker at room temperature for 1 to 2 h. The purpose of blocking was to prevent non-specific binding and reduce background signals. Washing: After incubation of the primary antibody, the membrane was thoroughly washed with washing buffer (PBST) 3 times for 5 min each time to remove unbound primary antibody. Secondary antibody incubation: The membrane was placed in a solution containing appropriately diluted secondary antibody and incubated at room temperature for 1 to 2 h. The secondary antibody was an antibody against the primary antibody, usually carrying a detectable label such as horseradish peroxidase (HRP) or alkaline phosphatase (AP). Washing: The membrane was washed again with washing buffer to remove unbound secondary antibody, 3 times for 5 min each time. Color development: An appropriate amount of ECL color development substrate solution was applied to the membrane, and the reaction was performed at room temperature protected from light. The color development was observed using a gel imager, and the membrane was photographed and recorded. Through observing the presence or absence and the color depth of the spots, the target protein in the sample can be qualitatively or semi-quantitatively analyzed.

The dot blot results are as shown in FIG. 40. The experimental results indicated that molecules 6553, 6677, 6678, 6683, 6685, 6686, 6689, 6560, 6551, 6561, and 6562 were peptides capable of specifically binding to DXD.

**Table 23. Amino acid sequence listing of the above molecules**

| Sequence No. | Molecule No. | Code Name | Sequence |
|---|---|---|---|
| SEQ ID NO:118 | 6551 | PP-1A-#330 (peptide) | VALTWYYPIYLFATR |
| SEQ ID NO:119 | 6553 | PP-1A-#340 (peptide) | ILTTVNVVPNTIHVR |
| SEQ ID NO:120 | 6677 | PP-1A-#414 (peptide) | ACLACWTSRSC |
| SEQ ID NO:121 | 6678 | PP-1A-#415 (peptide) | ACLSPWLFFWC |
| SEQ ID NO:122 | 6683 | PP-1A-#426 (peptide) | ACYPFGNPRGVRPSC |
| SEQ ID NO:123 | 6685 | PP-1A-#428 (peptide) | ACWTLWLPLLPLGSC |
| SEQ ID NO:124 | 6686 | PP-1A-#417 (peptide) | ACGSLSWRNCGSYRC |
| SEQ ID NO:125 | 6689 | PP-1A-#431 (peptide) | ACSQCVLLPPPQSVC |
| SEQ ID NO:126 | 6560 | PP-1A-#358 (peptide) | AHNVEILPPFDYTLS |
| SEQ ID NO:127 | 6561 | PP-1A-#362 (peptide) | LTVAMITTSRITTSN |
| SEQ ID NO:128 | 6562 | PP-1A-#367 (peptide) | APPTFPVSTSVVISR |

### Example 6-4 Cell Experiment for Detecting Inhibitory Effect of DXD-Binding Peptides on Toxin Cytotoxicity

NCI-H292 tumor cells were used to detect the inhibitory effect of DXD-binding peptides on DXD cytotoxicity.

NCI-H292 cells were digested, counted, and plated at 5E3 cells/well, with 60 µL per well, and allowed to adhere in a 37°C incubator. Peptide molecules and nonsense control antibody Null IgG were 2-fold diluted for 7 gradients starting at a concentration of 100 µg/mL The DXD concentration was fixed to 1 ng/mL. The test substances were incubated with DXD at room temperature for 2 h. The incubated samples were added into the 96-well plate at 60 µL/well and cultured in a 37°C incubator for 3 days. 100 µL of Cell Counting-Lite 2.0 Reagent was added to each well, and the plate was shaken on a shaker for 10 min. The fluorescence signal was read using a microplate reader.

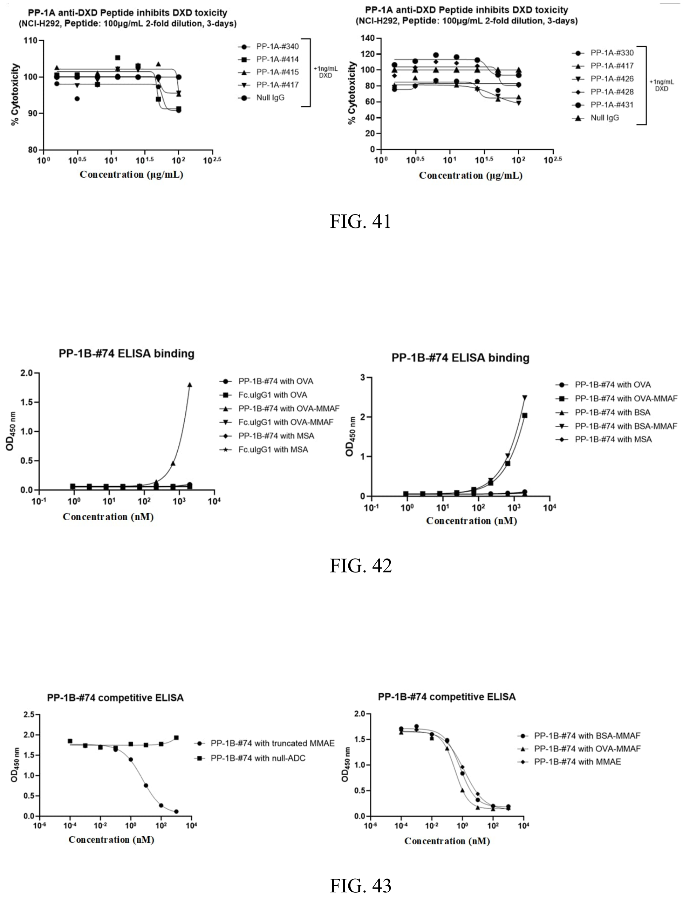

The experimental results are as shown in FIG. 41. The results showed that peptides PP-1A#414, PP-1A#340, PP-1A#415, and PP-1A#417 exhibited a cytoprotective effect, wherein PP-1A#414 had the best activity.

### Example 7: Screening of Peptides Specifically Binding to MMAE Based on Virtual Technology

### Example 7-1 Generation and Screening of Virtual Peptide Library

### Generation of Virtual Peptide Library

In the preliminary establishment, a natural tripeptide library containing a total of 8,000 tripeptides was generated by permuting and combining 20 natural amino acids. After batch structure prediction was performed on the tripeptide library using structure prediction software, batch molecular docking was performed with the small molecule compound MMAE, respectively. Based on affinity results, the top 50 tripeptides were screened, and 20 different natural amino acids were inserted at each possible position to generate new tetrapeptides after excluding repetitive sequences. By analogy, the same method was subsequently applied to generate pentapeptide, hexapeptide, heptapeptide, and octapeptide libraries, etc.

### Screening of Virtual Peptide Library

Molecular docking: Molecular docking was performed between predicted peptides and small molecules using AutoDock Vina docking software. The N-terminus of MMAE used for ADC coupling was set as the binding pocket, the num_modes parameter was set to 10, the energy_range parameter was set to 5, and the exhaustiveness parameter was set to 16. A flexible docking mode was adopted, with the N-terminus of MMAE as the binding center, allowing for flexible binding of the peptides. After docking was completed, all docking conformations were ranked. The lower the binding free energy, the more likely it was to be a potential optimal binding mode, thereby virtually screening for high-affinity peptides.

**Table 24. Affinity data**

| Heptapeptide | | |
|---|---|---|
| No. | Code Name | Affinity |
| ligand1765 | PP-1B-#56 | -5.3 |
| ligand6244 | PP-1B-#57 | -5.1 |
| ligand124 | PP-1B-#58 | -4.9 |
| ligand4139 | PP-1B-#59 | -4.9 |
| ligand1514 | PP-1B-#60 | -4.8 |
| ligand5681 | PP-1B-#61 | -4.8 |
| ligand2675 | PP-1B-#62 | -4.7 |
| ligand27 | PP-1B-#63 | -4.7 |
| ligand574 | PP-1B-#64 | -4.7 |
| ligand6481 | PP-1B-#65 | -4.7 |
| ligand11 | PP-1B-#66 | -4.6 |
| ligand4685 | PP-1B-#67 | -4.6 |
| ligand4766 | PP-1B-#68 | -4.6 |
| ligand488 | PP-1B-#69 | -4.6 |
| ligand283 | PP-1B-#70 | -4.5 |
| ligand3337 | PP-1B-#71 | -4.5 |
| ligand3948 | PP-1B-#72 | -4.5 |
| ligand6128 | PP-1B-#73 | -4.5 |
| ligand2770 | PP-1B-#74 | -4.4 |

Therefore, among the above candidate molecules, peptide PP-1B-#74 exhibited relatively high affinity, and its sequence is: YYVAWRW (SEQ ID NO: 129).

### Example 7-2 Construction, Expression, and Purification of Peptide Molecules

### Construction of Peptide Molecules

Through primer design, the peptide fragment PP-1B-#74 was ligated to the C-terminus of Fc to construct a plasmid producing the Fc fusion peptide. The amino acid sequence information is shown in SEQ ID NO: 130.

SEQ ID NO: 130:
DKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPPVLD
SDGSFFLYSKLTVDKSRWQQGNVFSCSVMHEALHNHYTQKSLSLSPGGSGGGSYYVAWR
W

### Expression and Purification of Peptide Molecules

HEK293SQ cells were cultured in Expi293 expression medium. Transient transfection was performed using the ExpiFectamine 293 transfection kit according to the manufacturer's instructions. A transfection enhancer was added 20 h after transfection, and the supernatant was collected 120 h later for subsequent protein purification. Magnetic beads were activated with 0.1M NaOH and PBST, respectively. After the supernatant was added and incubated at room temperature for 1.5 h, impurities were washed away with PBST and water, respectively. The target protein was eluted with 1000 µL of elution buffer, and the purity of the sample was determined by 4% to 12% SDS-PAGE.

### Example 7-3 Identification of Specific Binding Between Fc Fusion Peptide and MMAE

The binding of the constructed Fc fusion peptide molecules PP-1B-#70 to #74 with OVA-MMAF and OVA was identified respectively to screen for molecules with better specific binding specificity to MMAE. After the first round of primary screening was completed, the binding specificity of #74 with OVA-MMAF and BSA-MMAF was further verified.

### Peptide ELISA Binding Experimental Method

(1) Antigen coating: MMAF conjugated protein and negative protein were coated onto a microplate using coating buffer at a concentration of 1 µg/mL, and the plate was placed at 4°C overnight for about 16 h. (2) Blocking: The microplate coated with antigen was washed once with PBST using a plate washer, and blocked by adding Casein solution at 200 µL/well at room temperature for 1 h. (3) Primary antibody incubation: The plate was washed 3 times with PBST, and peptide samples with an initial concentration of 2000 nM diluted in a 5-fold gradient using Casein solution were added and the plate incubated at room temperature for 2 h. (4) Secondary antibody incubation: The plate was washed 3 times with PBST, and secondary antibody Goat pAb to Hu IgG(HRP) (1:10000) (Abcam, Cat. Ab97225) diluted with Casein solution was added and the plate was incubated at room temperature for 1 h. (5) Color development: The plate was washed 6 times with PBST, 100 µL of TMB color development solution was added to each well, and color was developed for 2 to 10 min protected from light. (6) Termination: 100 µL of 2 M H₂SO₄ was added to each well to terminate the reaction. The OD450 value was read using a microplate reader, recorded, and saved. Finally, the data were analyzed using GraphPad software.

The experimental results are as shown in FIG. 42. The results indicated that molecule PP-1B-#74 was able to specifically bind to OVA-MMAF and BSA-MMAF.

### Example 7-4 Identification of Competitive Binding of Free MMAE to Peptide Molecules

Based on the peptide molecule binding activity results, molecule PP-1B-#74 was further verified for its specific binding to MMAE through competitive experiments.

### Peptide Competitive ELISA Experimental Method

(1) Antigen coating: BSA-MMAF was coated onto a microplate using coating buffer at a concentration of 1 µg/mL, and the plate was placed at 4°C overnight for about 16 h. (2) Blocking: The microplate coated with antigen was washed once with PBST using a plate washer, and blocked by adding Casein solution at 200 µL/well at room temperature for 1 h. (3) Primary antibody incubation: The plate was washed 3 times with PBST, and a premixture of peptide sample diluted with Casein solution and truncated free MMAE/null-ADC was added. The final concentration of the peptide sample was 2000 nM. The initial concentration of truncated free MMAE and null-ADC was 1000 nM, diluted in a 10-fold gradient, and incubation was performed at room temperature for 2 h. (4) Secondary antibody incubation: The plate was washed 3 times with PBST, and secondary antibody Goat pAb to Hu IgG(HRP) (1:10000) diluted with Casein solution was added and the plate was incubated at room temperature for 1 h. (5) Color development: The plate was washed 6 times with PBST, 100 µL of TMB color development solution was added to each well, and color was developed for 2 to 10 min protected from light. Termination: 100 µL of 2 M H₂SO₄ was added to each well to terminate the reaction, and the OD450 value was read using a microplate reader, recorded, and saved. (6) Finally, the data were analyzed using GraphPad software.

The experimental results are as shown in FIG. 43. The results indicated that molecule PP-1B-#74 was able to specifically bind to MMAE.

### Example 8 Screening of Aptamers Specifically Binding to MMAE

### Example 8-1 First Round Synthesis and Screening of Aptamers

### Synthesis of single-stranded DNA library and primers shown in the following sequences

**Table 25. Aptamer Library and Primers**

| Name | Sequence |
|---|---|
| Pool-A-30N-N | CAGCACCGTCAACTGAATNNNNNNNNNNNNNNN NNNNNNNNNNNNNNGTGATGCGATGGAGATGT |
| 1214-F | CAGCACCGTCAACTGAAT |
| 1214-R, 1214-R-5B | ACATCTCCATCGCATCAC |

Wherein: The library consisted of single-stranded DNA (ssDNA) with a length of 66 bases, containing a random sequence (N) of 30 bases in the middle and fixed primer sequences at both ends. 5B indicates 5'-end Biotin modification. 1214-F and 1214-R-5B were used for symmetric PCR in each round, and 1214-F and 1214-R were used for amplifying the library to be sequenced. Both the single-stranded DNA library and primers were synthesized by Sangon Biotech (Shanghai) Co., Ltd.

### Aptamer Screening

The initial ssDNA library was centrifuged at 12,000 rpm for 1 min and dissolved in 100 µL of Binding buffer (D-PBS with a final concentration of 5 mM MgCl₂, i.e., DPBS containing 5 mM Mg²⁺), with a total DNA amount of 1 to 5 nmol. It was placed in a 95°C metal bath for 5 min and immediately placed on ice to cool for 5 min. This process was used for denaturation and renaturation of the library to refold the aptamer conformation. After renaturation, 100 µL of Binding buffer was added, and the mixture was added to a well plate for use. An empty microplate coated with 3% BSA was used for negative screening, and an immunotube coated with BSA-MMAF was used for positive screening. The renatured aptamer library was added to the positive screening immunotube and incubated at room temperature for 120 min to allow the conformationally folded aptamers to fully bind to the target MMAE. After incubation, the supernatant was discarded, and the tube was washed with a solution containing DPBS or DPBST. Then, 200 µL of elution buffer (20 mmol/L Tris-HCl, 4 mol/L Guanidine Isothiocyanate, 1 mmol/L dithiothreitol (pH 8.3)) was added for elution, and incubation was performed at 80°C for 15 min to wash off the ssDNA bound to the monoclonal antibody, and the eluate was collected. The eluate was used as a template for the first round of PCR amplification. The system was: 200 µL template, 400 µL PrimeSTAR Max, 32 µL 1214-F, 32 µL 1214-R, and 332 µL ddH₂O, with a total system volume of 800 µL, dispensed into 80 µL per PCR tube. Reaction conditions: pre-denaturation at 95°C for 2 min; denaturation at 95°C for 30 s, annealing at 60°C for 5 s, extension at 72°C for 5 s, amplified for 6 cycles; and finally extension at 72°C for 2 min.

To determine the optimal number of PCR cycles, 100 µL of the first round PCR product was taken as a template and prepared according to the above-mentioned reaction system. 80 µL was taken therefrom and added to 4 PCR tubes, respectively, and amplified for 2, 4, 6, and 8 cycles, followed immediately by 3% agarose gel electrophoresis. The number of rounds with clear bands and no non-specific amplification was used for the third PCR. The reaction system of the third PCR was consistent with the cycle number optimization system, and the remaining mixture was amplified according to the optimized number of cycles.

A certain amount of streptavidin magnetic beads was added to the biotinylated PCR product, and incubated on a shaker at room temperature for 15 min. After washing twice with 1× PBS, 200 mM NaOH was added and incubated for 5 min to unwind the DNA double strands into single strands in an alkaline environment. The tube was placed on a magnetic rack so that the biotin-containing ssDNA remained on the streptavidin magnetic beads and adsorbed to the tube wall, while the other non-biotinylated ssDNA existed in the supernatant. The obtained ssDNA secondary library was collected and purified by ethanol precipitation (mixed with 1/10 volume of 3 M NaAC and 2.5 volumes of absolute ethanol, mixed evenly by inversion, and placed in a -80°C refrigerator to precipitate overnight. The next day, it was taken out directly and centrifuged at 13,000 rpm at 4°C for 10 min. After pouring off the supernatant, 1 mL of 75% ethanol was added for washing, followed by centrifugation at 13,000 rpm at 4°C for 10 min. The supernatant was discarded, and after air-drying, the precipitate was dissolved in 50 µL of Binding buffer, and the concentration was determined using Nanodrop. 1 µg of DNA was taken as the secondary library for the next round of screening, and the excess was stored in a -20°C refrigerator).

In each round, the screening pressure was altered by adjusting the target usage amount, incubation time, and washing conditions. The positive screening antigen was reduced round by round from 100 µg to 30 µg, and the positive screening time was reduced round by round from 120 min to 30 min. The negative screening antigen was 10 µg to remove sequences with non-specific binding, and the incubation time was increased round by round from 20 min to 60 min.

A total of 6 aptamers, P-#1 to P-#6, were obtained in the first round of screening.

**Table 26. Aptamer sequences**

| Sequence No. | Code Name | Sequence |
|---|---|---|
| SEQ ID NO:131 | P-#1 | CAGCACCGTCAACTGAATGAAATGCGTGAAAGT TCGAAAATAGTTCTCGTGATGCGATGGAGATGT |
| SEQ ID NO:132 | P-#2 | CAGCACCGTCAACTGAATCGGGGCACGCAATTC GTAGTGTTAAGTTGTGTGATGCGATGGAGATGT |
| SEQ ID NO:133 | P-#3 | CAGCACCGTCAACTGAATCTACGCGTGATGCATA TTGGTCGAGCGGGGGTGATGCGATGGAGATGT |
| SEQ ID NO:134 | P-#4 | CAGCACCGTCAACTGAATGAAAAGCGCAAATCT GGCTAACATAGGCTCGTGATGCGATGGAGATGT |
| SEQ ID NO:135 | P-#5 | CAGCACCGTCAACTGAATGATCCAGAAAACGGC GACCGTCTCAGCGTAGTGATGCGATGGAGATGT |
| SEQ ID NO:136 | P-#6 | CAGCACCGTCAACTGAATAGATTCAAGGTCTTG CCGGGATATCGCCATGTGATGCGATGGAGATGT |

### Example 8-2 Second Round Synthesis and Screening of Aptamers

### Synthesis of single-stranded DNA library and primers shown in the following sequences

**Table 27. Aptamer library and primers**

| Name | Sequence |
|---|---|
| N10+N40-Cap-libr | CCTGCCACGCTCCGCAAGCTTNNNNNNNNNNCTGC AGCGATTCTTGATCGNNNNNNNNNNNNNNNNNNNN NNNNNNNNNNNNNNNNNNNNTAAGCTTGCACCCGC ATCGT |
| Capture oligo(10+40) | biotin-TACCGCAAAAAAAAACAAGAATCGCTGCAG |
| P1(10+40) | CCTGCCACGCTCCGCAAG |
| P2(10+40), P2-B(10+40) | ACGATGCGGGTGCCAAGCTTA |

Wherein: The library consisted of single-stranded DNA with a length of 110 bases.

### Aptamer Screening

The initial ssDNA library was centrifuged at 12,000 rpm for 1 min and dissolved in 1 mL of ddH₂O, with a total DNA amount of 1 to 5 nmol. Capture oligo (10+40) was then added and incubated with rotary mixing at room temperature for 60 min. The molar mass ratio of Capture oligo (10+40) to ssDNA input was 1.2:1. Streptavidin magnetic beads were added to the sample after incubation (based on the biotin carrying capacity of the magnetic beads, the ratio of bead carrying capacity to Biotin was 1.5:1). After incubating for 30 min, the EP tube was placed on a magnetic rack and left to stand for 30 s. The supernatant was discarded, and the beads were washed three times with 1× PBS. 15 nmol of truncated MMAE was added and incubated with rotation at room temperature for 120 min. Aptamers targeting MMAE would undergo conformational switching, detach from the Capture oligo, and convert into a conformation binding to MMAE. After incubation, the supernatant was collected. Using the collected supernatant as a template, the first round of PCR amplification was performed. The system was: all template, 400 µL PrimeSTAR Max, 32 µL P1(10+40), 32 µL P2(10+40), and 332 µL ddH₂O, with a total system volume of 800 µL, dispensed into 80 µL per PCR tube. Reaction conditions: pre-denaturation at 95°C for 2 min; denaturation at 95°C for 30 s, annealing at 60°C for 5 s, extension at 72°C for 5 s, amplified for 6 cycles; and finally extension at 72°C for 2 min.

To determine the optimal number of PCR cycles, 100 µL of the first round PCR product was taken as a template and prepared according to the reaction system in the table below. 80 µL was taken therefrom and added to 4 PCR tubes, respectively, and amplified for 2, 4, 6, and 8 cycles, followed immediately by 3% agarose gel electrophoresis. The number of rounds with clear bands and no non-specific amplification was selected for the third PCR. The reaction system of the third PCR was consistent with the cycle number optimization system, and the remaining mixture was amplified according to the optimized number of cycles.

A certain amount of streptavidin magnetic beads was added to the biotinylated PCR product, and incubated on a shaker at room temperature for 15 min. After washing twice with 1× PBS, 200 mM NaOH was added and incubated for 5 min to unwind the DNA double strands into single strands in an alkaline environment. The tube was placed on a magnetic rack so that the biotin-containing ssDNA remained on the streptavidin magnetic beads and adsorbed to the tube wall, while the other non-biotinylated ssDNA existed in the supernatant. The obtained ssDNA secondary library was collected and purified by ethanol precipitation (mixed with 1/10 3 M NaAC and 2.5 volumes of absolute ethanol, mixed evenly by inversion, and placed in a -80°C refrigerator to precipitate overnight. The next day, it was taken out directly and centrifuged at 13,000 rpm at 4°C for 10 min. After pouring off the supernatant, 1 mL of 75% ethanol was added for washing, followed by centrifugation at 13,000 rpm at 4°C for 10 min. The supernatant was discarded, and the precipitate was air-dried and dissolved in 50 µL of Binding buffer, and the concentration was determined using Nanodrop. 1 µg of DNA was taken as the secondary library for the next round of screening, and the excess was stored in a -20°C refrigerator).

MMAE coupled with vc was used as the negative screening antigen, and MMAF coupled with biotin was used as the positive screening antigen. In each round, the screening pressure was altered by adjusting the target usage amount, incubation time, and washing conditions. The positive screening antigen was reduced round by round from 15 nmol to 0.12 nmol, and the positive screening time was reduced round by round from 120 min to 60 min. From the third round onwards, a negative screening step was added to remove sequences non-specifically binding to streptavidin magnetic beads, with the incubation time increased round by round from 30 min to 60 min. After each round of incubation, the supernatant was collected, and the ssDNA binding to MMAE in the supernatant served as the template for subsequent PCR and single-strand generation steps.

A total of 6 aptamers, PP-1-#148 to PP-1-#153, were obtained in the second round of screening.

**Table 28. Aptamer sequences**

| Sequence No. | Code Name | Sequence |
|---|---|---|
| SEQ ID NO:137 | PP-1-#148 | CCTGCCACGCTCCGCAAGCTTAGTCATA GTACTGCAGCGATTCTTGATCGTAATACT AGCTAACTCTTGGCACTAGAAGAGATTG AGTCCTAAGCTTGCACCCGCATCGT |
| SEQ ID NO:138 | PP-1-#149 | CCTGCCACGCTCCGCAAGCTTTGAACGG ATTCTGCAGCGATTCTTGATCGTTACTTG GGCATTATCGTCATCAGCTTTCATGAGTA AGGGTAAGCTTGCACCCGCATCGT |
| SEQ ID NO:139 | PP-1-#150 | CCTGCCACGCTCCGCAAGCTTCTATTGA CTTCTGCAGGATTCTTGATCGCAAACATT GTCAGATGCGAGGTTGACAGCTGTAGTT ACACTAAGCTTGCACCCGCATCGT |
| SEQ ID NO:140 | PP-1-#151 | CCTGCCACGCTCCGCAAGCTTCCTATCAT GCCTGCAGCGATTCTTGATCGGATTAGT GTTTGCTACCTGTTTCTAGCAGTCCGTAG CTCATAAGCTTGCACCCGCATCGT |
| SEQ ID NO:141 | PP-1-#152 | CCTGCCACGCTCCGCAAGCTTGTTATAC GCCCTGCAGCGATTCTTGATCGTTCGTCT CCGATCCCAGCCTTACTACCGCTTTTTAG ATGCTAAGCTTGCACCCGCATCGT |
| SEQ ID NO:142 | PP-1-#153 | CCTGCCACGCTCCGCAAGCTTCCTTCTG GTCCTGCAGCGATTCTTGATCGGCTATCT CGCCTATAGTCTCGACGTAGTATGCAGGC ATGCTAAGCTTGCACCCGCATCGT |

### Example 8-3 Antigen Binding Experiment

### Experimental Steps

Coating: A 96-well microplate was coated with 1 µg/mL of BSA-MMAF and BSA, and OVA-MMAF and OVA, respectively, and incubated at 4°C overnight. Blocking: The antigen-coated 96-well plate was washed once with PBST using a plate washer, and blocked by adding 2% casein at 200 µL/well at 37°C for 1 h. Primary antibody: After washing 3 times with PBST, aptamers diluted with binding buffer were added to each well. The initial concentration was 3 µM, diluted in 3-fold gradients for 8 gradients. The mixture was carefully and thoroughly mixed by pipetting and incubated at 37°C for 1 to 2 h. Secondary antibody: After washing 3 times with PBST, secondary antibody Streptavidin-HRP (1:10000) diluted with binding buffer was added and incubated at 37°C for 1 h. Color development: After washing 6 times with DPBST (containing 5 mM Mg²⁺), 100 µL of TMB color development solution was added to each well, and color was developed for 2 to 10 min protected from light. Termination: 100 µL of ELISA stop solution was added to each well, the OD450 value was read using a microplate reader, recorded, and saved.

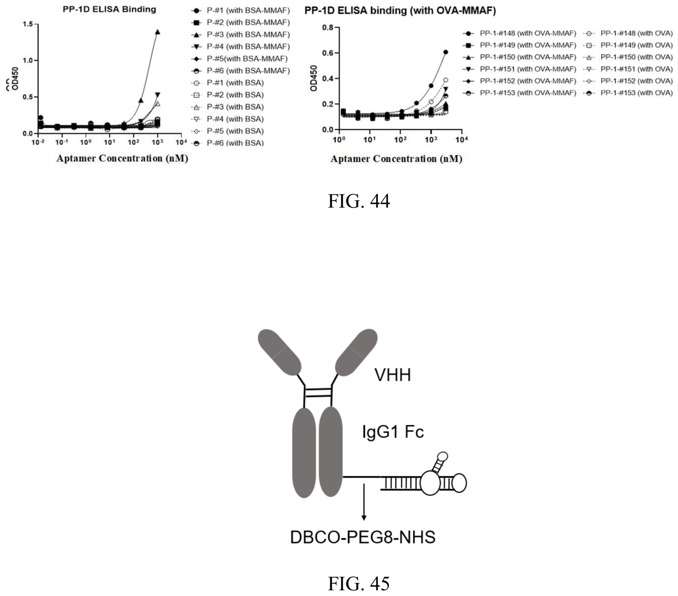

The experimental results are as shown in FIG. 44. The results indicated that P-#3, P-#4, PP-1-#148, and PP-1-#151 could specifically bind to MMAF.

### Example 8-4 Preparation of Antibody-Aptamer Conjugates

The NHS active ester group in the DBCO-PEG8-NHS linker was utilized to react with the primary amine group on the antibody to introduce a DBCO group, and the DBCO group covalently bound with the azide group in the aptamer through strain-promoted azide-alkyne cycloaddition (SPAAC) to obtain the antibody-aptamer conjugate.

### Experimental Steps

1 mg of antibody was taken, and 12 molar equivalents of DBCO-PEG8-NHS (MCE, 2553412-88-5) were added according to the molar ratio. Reaction was performed at 25°C for 3 h. After the reaction was completed, the sample was exchanged into PBS using a centrifugal desalting column (Zeba^{™}, 7K 0.5 mL). In the desalted sample above, 4 molar equivalents of azide-modified aptamer were added, and reaction was performed at 4°C overnight. After the reaction was completed, an ultrafiltration concentrator (ThermoFisher, 100K MW, 88503) was used to exchange the above sample into PBS to obtain the antibody-aptamer conjugate. The schematic diagram of the constructed antibody-aptamer conjugate is shown in FIG. 45.

### Example 9 Screening of Aptamers Specifically Binding to DXD

### Example 9-1 Synthesis and Screening of Aptamers

Synthesis of single-stranded DNA library and primers shown in the following sequences.

**Table 29. Aptamer library and primers**

| Name | Sequence |
|---|---|
| Pool-A-30N-N | CAGCACCGTCAACTGAATNNNNNNNNNNNNNNNN NNNNNNNNNNNNNNGTGATGCGATGGAGATGT |
| 1214-F | CAGCACCGTCAACTGAAT |
| 1214-R, 1214-R-5B | ACATCTCCATCGCATCAC |

Wherein: The library consisted of single-stranded DNA with a length of 66 bases, containing a random sequence (N) of 30 bases in the middle and fixed primer sequences at both ends. 5B indicates 5'-end Biotin modification. 1214-F and 1214-R-5B were used for symmetric PCR in each round, and 1214-F and 1214-R were used to amplify the library to be sequenced. Both the single-stranded DNA library and primers were synthesized by Sangon Biotech (Shanghai) Co., Ltd.

### Aptamer Screening

The initial single-stranded DNA library was centrifuged at 12,000 rpm for 1 min and dissolved in 100 µL of Binding buffer (D-PBS with a final concentration of 5 mM MgCl₂, i.e., DPBS containing 5 mM Mg²⁺), with a total DNA amount of 1 to 5 nmol. It was placed in a 95°C metal bath for 5 min and immediately placed on ice to cool for 5 min. This process was used for denaturation and renaturation of the library to refold the aptamer conformation. After renaturation, 900 µL of Binding buffer was added. 0.5 µg of DXD-Biotin was added and incubated with rotation at room temperature for 120 min to allow the folded aptamers to fully bind to the target DXD. Streptavidin magnetic beads were added to the sample after incubation (based on the biotin carrying capacity of the magnetic beads, the ratio of bead carrying capacity to Biotin was 1.5:1). After incubating for 15 min, the EP tube was placed on a magnetic rack and left to stand for 30 s. The supernatant was discarded, and the beads were washed three times with 1× PBS. The first round of PCR amplification was performed using the washed magnetic beads as the template. The system was: all template, 400 µL PrimeSTAR Max, 32 µL 1214-F, 32 µL 1214-R, and 332 µL ddH₂O, with a total system volume of 800 µL, dispensed into 80 µL per PCR tube. Reaction conditions: pre-denaturation at 95°C for 2 min; denaturation at 95°C for 30 s, annealing at 60°C for 5 s, extension at 72°C for 5 s, amplified for 6 cycles; and finally extension at 72°C for 2 min.

To determine the optimal number of PCR cycles, 100 µL of the first round PCR product was taken as a template, and reaction was performed along with 250 µL PrimeSTAR Max, 20 µL 1214-F, 20 µL 1214-R-5B, and 110 µL ddH₂O. 80 µL was taken therefrom and added to 4 PCR tubes, respectively, and amplified for 2, 4, 6, and 8 cycles, followed immediately by 3% agarose gel electrophoresis. The number of rounds with clear bands and no non-specific amplification was selected for the third PCR. The reaction system of the third PCR was consistent with the cycle number optimization system, and the remaining mixture was amplified according to the optimized number of cycles.

A certain amount of streptavidin magnetic beads was added to the biotinylated PCR product, and incubated on a shaker at room temperature for 15 min. After washing twice with 1× PBS, 200 mM NaOH was added and incubated for 5 min to unwind the DNA double strands into single strands in an alkaline environment. The tube was placed on a magnetic rack so that the biotin-containing ssDNA remained on the streptavidin magnetic beads and adsorbed to the tube wall, while the other non-biotinylated ssDNA existed in the supernatant. The obtained ssDNA secondary library was collected and purified by ethanol precipitation (mixed with 1/10 3 M NaAC and 2.5 volumes of absolute ethanol, mixed evenly by inversion, and placed in a -80°C refrigerator to precipitate overnight. The next day, it was taken out directly and centrifuged at 13,000 rpm at 4°C for 10 min. After pouring off the supernatant, 1 mL of 75% ethanol was added for washing, followed by centrifugation at 13,000 rpm at 4°C for 10 min. The supernatant was discarded, and the precipitate was air-dried and dissolved in 50 µL of Binding buffer, and the concentration was determined using Nanodrop. 1 µg of DNA was taken as the secondary library for the next round of screening, and the excess was stored in a -20°C refrigerator).

Streptavidin magnetic beads were used as the negative screening antigen, and biotin-coupled DXD was used as the positive screening antigen. In each round, the screening pressure was altered by adjusting the target usage amount, incubation time, and washing conditions. A total of six rounds of screening were performed. The positive screening antigen was reduced round by round from 0.5 µg to 0.2 µg, and the positive screening time was reduced round by round from 120 min to 60 min. From the third round onwards, a negative screening step was added to remove sequences non-specifically binding to streptavidin magnetic beads, with the incubation time increased round by round from 30 min to 60 min. After each round of incubation, the supernatant was discarded, and the beads were washed with a PBST solution containing 0.01% to 0.05% Tween 20, and then washed with 1× PBS. The supernatant was discarded, and the ssDNA bound to the magnetic beads served as the template for subsequent PCR and single-strand generation steps.

A total of 14 aptamers, D-#3 to D-#18, were obtained from the screening.

**Table 30. Aptamer sequences**

| Sequence No. | Code Name | Sequence |
|---|---|---|
| SEQ ID NO:143 | D-#3 | CAGCACCGTCAACTGAATTCCGTGGAG CCGTGGGTTATCCGTGCGACCGTGATG CGATGGAGATGT |
| SEQ ID NO:144 | D-#4 | CAGCACCGTCAACTGAATCCGTGTGGA TCCGTGCCTGCCGTGTCCACCGTGATG CGATGGAGATGT |
| SEQ ID NO:145 | D-#5 | CAGCACCGTCAACTGAATCCGCCGCG ACCGTGTGTTAGCCGTGGAGCCGTGAT GCGATGGAGATGT |
| SEQ ID NO:146 | D-#6 | CAGCACCGTCAACTGAATCCGCGGGA ACCGTGTCTGACCGTGTGACCCGTGAT GCGATGGAGATGT |
| SEQ ID NO:147 | D-#7 | CAGCACCGTCAACTGAATCGTGCAAA GCCGTATGATGTGCGTAGCCGTGTGAT GCGATGGAGATGT |
| SEQ ID NO:148 | D-#8 | CAGCACCGTCAACTGAATCCGTGGGC GCGCGCCTTTGCTGCTGCAACCCGTGA TGCGATGGAGATGT |
| SEQ ID NO:149 | D-#9 | CAGCACCGTCAACTGAATCCGTGTAGA GCCGTGTATCCGTGCGGCACCGTGATG CGATGGAGATGT |
| SEQ ID NO:150 | D-#10 | CAGCACCGTCAACTGAATCGTGGGGG AGGGTGGGGTGCGAAATGTGATGCGAT GGAGATGT |
| SEQ ID NO:151 | D-#13 | CAGCACCGTCAACTGAATCGTGCAAA GCCGTGTCTCCGTGCGTGCCGTGTGAT GCGATGGAGATGT |
| SEQ ID NO:152 | D-#14 | CAGCACCGTCAACTGAATCGTGTGACC CGTGTATACCGTGGGAGCCGTGTGATG CGATGGAGATGT |
| SEQ ID NO:153 | D-#15 | CAGCACCGTCAACTGAATCCGTGGGA GCCGCGAACCACCGTGCGACCCGTGA TGCGATGGAGATGT |
| SEQ ID NO:154 | D-#16 | CAGCACCGTCAACTGAATCCGGGCGG CGGCGGGCCCCTGCCGTGTGATGCGAT GGAGATGT |
| SEQ ID NO:155 | D-#17 | CAGCACCGTCAACTGAATTCTGGGAAC CGTGTGAAACCGTGTGACCGTGTGATG CGATGGAGATGT |
| SEQ ID NO:156 | D-#18 | CAGCACCGTCAACTGAATGAGGCTCTG AATCCGTGTGAAACCGTGTGAGTGATG CGATGGAGATGT |

To improve aptamer affinity, a complementary sequence was added to both ends of the aptamers for modification:

**Table 31. Modified aptamer sequences**

| Sequence No. | Code Name | Sequence |
|---|---|---|
| SEQ ID NO:157 | Capture D-#3 | GGAGGCTCTCGGGACGACCAGCACCGT CAACTGAATTCCGTGGAGCCGTGGGTTA TCCGTGCGACCGTGATGCGATGGAGATG TGTCGTCCCGATCACTTGAATGGTCT |
| SEQ ID NO:158 | Capture D-#5 | GGAGGCTCTCGGGACGACCAGCACCGT CAACTGAATCCGCCGCGACCGTGTGTTA GCCGTGGAGCCGTGATGCGATGGAGATG TGTCGTCCCGATCACTTGAATGGTCT |
| SEQ ID NO:159 | Capture D-#6 | GGAGGCTCTCGGGACGACCAGCACCGT CAACTGAATCCGCGGGAACCGTGTCTGA CCGTGTGACCCGTGATGCGATGGAGATG TGTCGTCCCGATCACTTGAATGGTCT |
| SEQ ID NO:160 | Capture D-#7 | GGAGGCTCTCGGGACGACCAGCACCGT CAACTGAATCGTGCAAAGCCGTATGATG TGCGTAGCCGTGTGATGCGATGGAGATG TGTCGTCCCGATCACTTGAATGGTCT |
| SEQ ID NO:161 | Capture D-#14 | GGAGGCTCTCGGGACGACCAGCACCGT CAACTGAATCGTGTGACCCGTGTATACC GTGGGAGCCGTGTGATGCGATGGAGATG TGTCGTCCCGATCACTTGAATGGTCT |
| SEQ ID NO:162 | Capture D-#17 | GGAGGCTCTCGGGACGACCAGCACCGT CAACTGAATTCTGGGAACCGTGTGAAAC CGTGTGACCGTGTGATGCGATGGAGATG TGTCGTCCCGATCACTTGAATGGTCT |

### Example 9-2 Antigen Binding Experiment

### Experimental Steps

Coating: A 96-well microplate was coated with 1 µg/mL of OVA-DXD and OVA, respectively, and incubated at 4°C overnight. Blocking: The antigen-coated 96-well plate was washed once with PBST using a plate washer, and blocked by adding 2% casein at 200 µL/well at 37°C for 1 h. Primary antibody: After washing 3 times with PBST, aptamers diluted with binding buffer were added to each well. The initial concentration was 3 µM, diluted in 3-fold gradients for 8 gradients. The mixture was carefully and thoroughly mixed by pipetting and incubated at 37°C for 1 to 2 h. Secondary antibody: After washing 3 times with PBST, secondary antibody Streptavidin-HRP (1:10000) diluted with binding buffer was added and incubated at 37°C for 1 h. Color development: After washing 6 times with DPBST (containing 5 mM Mg²⁺), 100 µL of TMB color development solution was added to each well, and color was developed for 2 to 10 min protected from light. Termination: 100 µL of ELISA stop solution was added to each well, the OD450 value was read using a microplate reader, recorded, and saved.

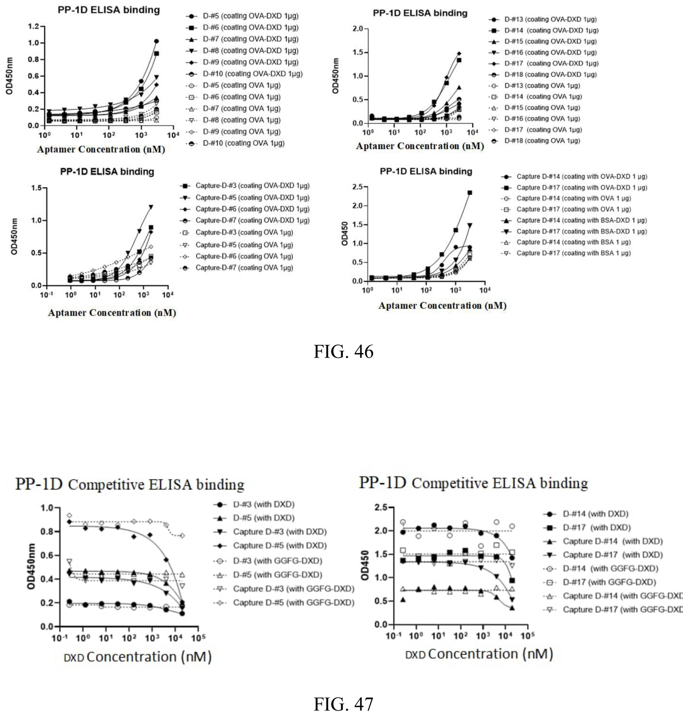

The experimental results are as shown in FIG. 46. The results indicated that D-#5, D-#14, and D-#17 could specifically bind to DXD, and after modification, the binding affinity of Capture D-#5, Capture D-#14, and Capture D-#17 with DXD was significantly improved.

### Example 9-3 Free DXD Competitive Binding Experiment

### Experimental Steps

Coating: A 96-well microplate was coated with 1 µg/mL of OVA-DXD and incubated at 4°C overnight. Blocking: The antigen-coated 96-well plate was washed once with PBST using a plate washer, and blocked by adding 2% casein at 200 µL/well at 37°C for 1 h. Primary antibody: After washing 3 times with PBST, aptamers diluted with binding buffer were added to each well at a concentration of 2000 nM. The initial concentration of DXD and GGFG-DXD was 20 µM, diluted in 3-fold gradients for 8 gradients. The diluted DXD and the aptamer were mixed at a volume ratio of 1:1, carefully and thoroughly mixed by pipetting, and incubated at 37°C for 1 to 2 h. Secondary antibody: After washing 3 times with PBST, secondary antibody Streptavidin-HRP (1:10000) diluted with binding buffer was added and incubated at 37°C for 1 h. Color development: After washing 6 times with DPBST, 100 µL of TMB color development solution was added to each well, and color was developed for 2 to 10 min protected from light. Termination: 100 µL of ELISA stop solution was added to each well, the OD450 value was read using a microplate reader, recorded, and saved.

The experimental results are as shown in FIG. 47. The results indicated that the parent molecules D-#5, D-#14, and D-#17 and the modified molecules Capture D-#5, Capture D-#14, and Capture D-#17 were all able to competitively bind to free DXD.

### Example 9-4 Preparation of Antibody-Aptamer Conjugates

The NHS active ester group in the DBCO-NHS or DBCO-PEG8-NHS linker was utilized to react with the primary amine group on the antibody to introduce a DBCO group, and the DBCO group covalently bound with the azide group in the aptamer through strain-promoted azide-alkyne cycloaddition (SPAAC) to obtain the antibody-aptamer conjugate. The specific experimental steps were as follows:

### Experimental Steps

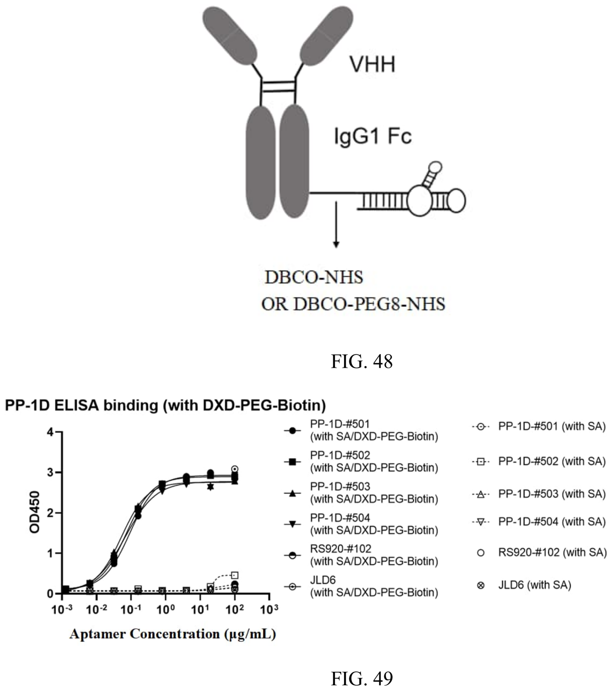

1 mg of antibody was taken, and 12 molar equivalents of DBCO-NHS (MCE, 1384870-47-6) or DBCO-PEG8-NHS (MCE, 2553412-88-5) were added according to the molar ratio. Reaction was performed at 25°C for 3 h. After the reaction was completed, the sample was exchanged into PBS using a centrifugal desalting column (Zeba^{™}, 7K 0.5 mL). In the desalted sample above, 4 molar equivalents of azide-modified aptamer were added, and reaction was performed at 4°C overnight. After the reaction was completed, an ultrafiltration concentrator (ThermoFisher, 100K MW, 88503) was used to exchange the above sample into PBS to obtain the antibody-aptamer conjugate. The schematic diagram of the constructed antibody-aptamer conjugate is shown in FIG. 48.

### Example 9-5 Antigen Binding Experiment of Antibody-Aptamer Conjugates

The experimental results are as shown in FIG. 49. The results indicated that the constructed antibody-aptamer conjugates PP-1D#501, PP-1D#502, PP-1D#503, and PP-1D#504 could all specifically bind to DXD.

### Example 9-6 Cytoprotection Experiment

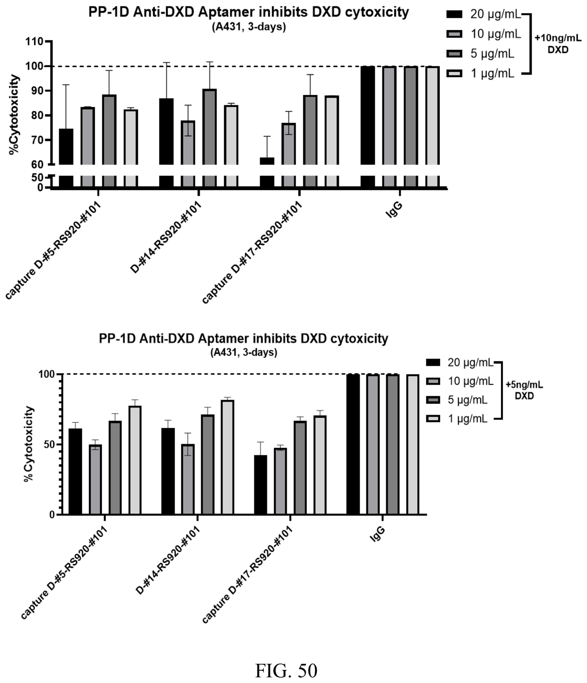

The experimental results are as shown in FIG. 50. The results indicated that capture D-#5-RS920-#101, D-#14-RS920-#101, and capture D-#17-RS920-#101 could all effectively inhibit DXD toxicity and exhibited varying degrees of protective effects on cells.

### Example 10 Preparation Methods of PR-ADC (Payload Recycling-Antibody Drug Conjugates) and Conventional ADC

### Example 10-1 Preparation of PR-ADC (CDCP1A9-DL01-MMAE, CDCP1A12-DL01-MMAE, Nectin4A14-DL01-MMAE, CDCP1A10-DL01-MMAE)

The configuration schematic of CDCP1A9-DL01-MMAE is shown in FIG. 2, wherein the ligand unit is an IgG antibody targeting CDCP1 (CDCP1A9), and the recovery unit is an anti-MMAE scFv; the two constitute an anti-CDCP1-IgG + anti-MMAE-scFv bispecific antibody. The structural schematic of the bispecific antibody-linker-drug is shown in FIG. 55.

The configuration schematic of CDCP1A12-DL01-MMAE is shown in FIG. 54, wherein the ligand unit is an scFv targeting CDCP1 (CDCP1A12), and the recovery unit is an anti-MMAE IgG antibody; the two constitute an anti-CDCP1-scFv + anti-MMAE-IgG bispecific antibody. The structural schematic of the bispecific antibody-linker-drug is shown in FIG. 55.

The configuration schematic of Nectin4A14-DL01-MMAE is shown in FIG. 56, wherein the recovery unit is an anti-MMAE scFv, and the ligand unit is an IgG antibody targeting Nectin-4 (Nectin4A14); the two constitute an anti-MMAE-scFv + anti-Nectin-4-IgG bispecific antibody. The structural schematic of the bispecific antibody-linker-drug is shown in FIG. 55.

The configuration schematic of CDCP1A10-DL01-MMAE is shown in FIG. 56, wherein the recovery unit is an anti-MMAE scFv, and the ligand unit is an IgG antibody targeting CDCP1 (CDCP1A10); the two constitute an anti-MMAE-scFv + anti-Nectin-4-IgG bispecific antibody. The structural schematic of the bispecific antibody-linker-drug is shown in FIG. 55.

### Preparation Method

A reducing agent and a metal chelator were prepared with purified water respectively as follows: 5 mM TCEP (Tris-2-carboxyethyl-phosphine) and 10 mM DTPA (Diethylene triamine pentaacetate acid) stock solutions. To 10 mg/mL of the corresponding bispecific antibody described above, DTPA was added at a volume ratio of 10%. TCEP was added at a final molar ratio of TCEP to antibody of 2:1 to 3:1 (the TCEP ratio was adjusted slightly according to the antibody), and the reaction was stirred at 25°C for 1 h.

The antibody reduced by TCEP could be directly subjected to conjugation. A certain concentration (5 mM) of linker-toxin (DL01-MMAE) dissolved in DMSO (dimethyl sulfoxide) was prepared. The drug was added slowly at a molar ratio of drug to thiol of 1.5 to 2:1, and the reaction was stirred at 25°C for 2 h. After the reaction was completed, centrifugal ultrafiltration was performed using ADC buffer to purify the product and remove residual unreacted drug and free small molecules such as DMSO. The conjugation status was determined using hydrophobic interaction chromatography-high performance liquid chromatography (HIC-HPLC) and liquid chromatography-mass spectrometry (LC-MS), and the purity of the sample after conjugation was determined by size exclusion chromatography (SEC).

In the prepared CDCP1A9-DL01-MMAE (PR-ADC), the drug-to-antibody ratio (DAR) was 3.86, and the SEC purity was 94.38%; in the prepared CDCP1A12-DL01-MMAE (PR-ADC), the DAR was 3.92, and the SEC purity was 94.38%; in the prepared Nectin4A14-DL01-MMAE (PR-ADC), the DAR was 3.89, and the SEC purity was 94.94%; and in the prepared CDCP1A10-DL01-MMAE (PR-ADC), the DAR was 4.10, and the SEC purity was 92.7%.

### Example 10-2 Preparation of Conventional ADC (CDCP1A6-DL01-MMAE, Enfortumab vedotin (in-house) - abbreviated as EV)

The configuration schematics of CDCP1A6-DL01-MMAE and EV are shown in FIG. 57, wherein the antibody parts are antibodies targeting CDCP1 and Nectin-4, respectively. The structural schematic of the antibody-linker-drug is shown in FIG. 55.

CDCP1A6-DL01-MMAE (conventional ADC) and EV (conventional ADC) were obtained with reference to the preparation method of Example 10-1.

In the prepared CDCP1A6-DL01-MMAE (conventional ADC), the DAR was 4.08, and the SEC purity was 99.37%; and in the prepared EV (conventional ADC), the DAR was 3.85, and the SEC purity was 98.81%.

### Example 11 Cell experiment

### Example 11-1 PR-ADC Cytotoxicity Test

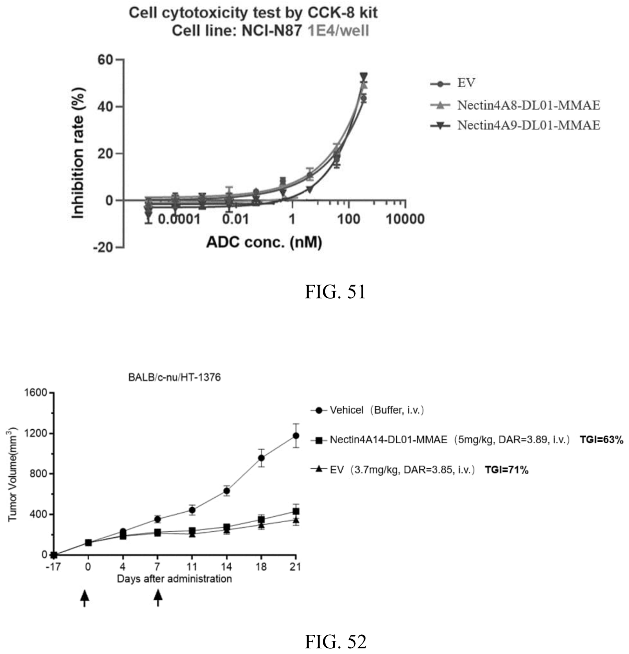

The experimental results (FIG. 51) indicated that the PR-ADCs (Nectin4A8-DL01-MMAE, Nectin4A9-DL01-MMAE) maintained cell killing activity similar to Enfortumab vedotin (prepared in-house, an antibody-drug conjugate targeting Nectin-4, abbreviated as EV).

### Example 11-2 Comparison of Binding Activity of Naked Antibody Molecules, Conventional ADC, and PR-ADC with Nectin-4

**Table 32. Comparison of binding activity of various molecules with Nectin-4**

| No. | Code Name | Top OD450 | EC50 |
|---|---|---|---|
| 1 | Enfortumab | 1.24 | 0.31 |
| 2 | Nectin4A8 | 1.12 | 0.66 |
| 3 | Nectin4A9 | 1.30 | 0.53 |
| 4 | EV | 1.23 | 0.35 |
| 5 | Nectin4A8-DL01-MMAE | 1.04 | 0.65 |
| 6 | Nectin4A9-DL01-MMAE | 1.30 | 0.81 |
| 7 | hIgG1 | 0.00 | NA |

The experimental results (Table 32) indicated that the binding capabilities of naked antibody molecules Nectin4A7 and Nectin4A8 and their corresponding conventional ADC (EV) or PR-ADC (Nectin4A8-DL01-MMAE) with Nectin-4 were comparable. Moreover, the binding capabilities were at the same level as Enfortumab and EV's binding capabilities with Nectin-4.

### Example 11-3 Cell Killing Activity

**Table 33. Experimental results of cell killing activity**

| No. | Code Name | HCC1954 | | OVCAR3 | | CHOK1 | |
|---|---|---|---|---|---|---|---|
| | | IC50 (nM) | MIR (%) | IC50 (nM) | MIR (%) | IC50 (nM) | MIR (%) |
| 1 | EV | 147.70 | 70.90 | 33.29 | 60.27 | NA | 17.40 |
| 2 | Nectin4A8-DL 01-MMAE | 153.30 | 72.32 | 35.60 | 61.79 | NA | 19.87 |
| 3 | MMAE | 0.31 | 60.95 | 0.31 | 3.69 | 34.88 | 83.94 |

The experimental results (Table 33) indicated that the PR-ADC (Nectin4A8-DL01-MMAE) and the conventional ADC (EV) had similar cell killing activity.

### Example 12 Safety Studies of PR-ADC (MMAE Payload) in Animal Models

### Example 12-1 Comparative Study of Toxicity in BALB/c Mice

### 1. Comparison Between Ligand Unit Targeting CDCP1 PR-ADC and Conventional ADC (MMAE Payload)

Test 1: 18 BALB/c mice, female, with body weights of 16 to 19 g at the time of grouping, were divided into a PR-ADC group (CDCP1A9-DL01-MMAE, 140 mg/kg) and a conventional ADC group (CDCP1A6-DL01-MMAE, 100 mg/kg), both at equimolar doses, with 9 mice in each group (5 for toxicology + 4 for toxicokinetic (TK)), for a total of 2 groups. A single dose was administered, with a dosing volume of 10 mL/kg. The drug-related toxic effects in BALB/c mice for each group were investigated and compared through aspects such as death, clinical observation, body weight, and toxicokinetic detection. The experiment lasted for 14 days.

Free MMAE was determined using a Hybrid LBA LC-MS/MS method. Protein A/G-Beads were used to separate the ADC/PR-ADC, MMAE-bound PR-ADC, and MMAE-bound anti-MMAE antibodies from the serum. The remaining samples were subjected to protein precipitation and then tested for free MMAE using the LC-MS/MS method.

**Table 34. Toxicity test results of animals in each group**

| Group | Dose (mg/kg) | Death | Max Body Weight Change Rate | Clinical Observation |
|---|---|---|---|---|
| PR-ADC | 140 | 0/9 | -16.4% (D7) | Piloerection (5/5), Hunched posture (5/5) |
| Conventional ADC | 100 | 1/9 | -24.1% (D7) | Piloerection (5/5), Hunched posture (5/5), Squinting eyes (2/5) |

According to the experimental results, the tolerance of BALB/c mice to the PR-ADC group (CDCP1A9-DL01-MMAE) was greater than 140 mg/kg, and the tolerance to the conventional ADC group (CDCP1A6-DL01-MMAE) was less than 100 mg/kg. The toxicity comparison result at equimolar doses was: Conventional ADC group (CDCP1A6-DL01-MMAE, 100 mg/kg) > PR-ADC group (CDCP1A9-DL01-MMAE, 140 mg/kg).

The results of this experiment showed that compared to the conventional ADC structure, the addition of the recovery unit in the PR-ADC structure effectively reduced toxicity to animals.

**Table 35. Toxicokinetic parameters of free MMAE in serum of animals in each group**

| Group | Dose (mg/kg) | Free MMAE | | |
|---|---|---|---|---|
| | | Cₘₐₓ (nM) | AUC (h*nM) | Tₘₐₓ (h) |
| PR-ADC | 140 | 6.2±0.6 | 726.68±101.74 | 24 |
| Conventional ADC | 100 | 75.8±16.03 | 8003.69±1615.32 | 24 |

The toxicokinetic results showed that in BALB/c mouse serum, the exposure amount (Cₘₐₓ and AUC) of free MMAE in the PR-ADC group (CDCP1A9-DL01-MMAE) was significantly lower than that in the conventional ADC group (CDCP1A6-DL01-MMAE). The exposure level of free MMAE was positively correlated with toxicity, indicating that PR-ADC could significantly improve the in vivo toxicity of conventional ADC.

Test 2: 16 BALB/c mice, female, with body weights of 16 to 20 g at the time of grouping, were divided into PR-ADC group (CDCP1A12-DL01-MMAE, 134 mg/kg) and conventional ADC group (CDCP1A6-DL01-MMAE, 100 mg/kg) dose group, both at equimolar doses, with 8 mice in each group (4 for toxicology + 4 for TK), for a total of 2 groups. A single dose was administered, with a dosing volume of 10 mL/kg. The drug-related toxic effects in BALB/c mice for each group were investigated and compared through aspects such as death, clinical observation, body weight, and toxicokinetic detection. The experiment lasted for 14 days.

Free MMAE was also determined using the Hybrid LBALC-MS/MS method.

**Table 36. Toxicity test results of animals in each group**

| Group | Dose (mg/kg) | Death | Max Body Weight Change Rate | Clinical Observation |
|---|---|---|---|---|
| PR-ADC | 134 | 0/8 | -16.43% (D4) | Piloerection (4/4), Loose stool (4/4) |
| Conventional ADC | 100 | 7/8 | -23.44% (D7) | Piloerection (4/4), Loose stool (1/4), Emaciation (2/4), Soft stool (1/4), Eves unable to open (3/4) |

According to the experimental results, the tolerance of BALB/c mice to the PR-ADC group (CDCP1A12-DL01-MMAE) was greater than 134 mg/kg, and the tolerance to the conventional ADC group (CDCP1A6-DL01-MMAE) was less than 100 mg/kg. The toxicity comparison result at equimolar doses was: Conventional ADC group (CDCP1A6-DL01-MMAE, 100 mg/kg) > PR-ADC group (CDCP1A12-DL01-MMAE, 134 mg/kg).

The results of this experiment also showed that compared to the conventional ADC structure, the addition of the recovery unit in the PR-ADC structure effectively reduced in vivo toxicity to animals.

**Table 37. Toxicokinetic parameters of free MMAE in serum of animals in each group**

| Group | Dose (mg/kg) | Free MMAE | | |
|---|---|---|---|---|
| | | Cₘₐₓ (nM) | AUC (h*nM) | Tₘₐₓ (h) |
| PR-ADC | 134 | 6.06±4.27 | 619.43±363.31 | [24,72] |
| Conventional ADC | 100 | 22.95±4.44 | 2165.03±1003.1 2 | 24 |

The toxicokinetic results showed that in BALB/c mouse serum, the exposure (Cₘₐₓ and AUC) of free MMAE in the mouse serum of the PR-ADC group (CDCP1A12-DL01-MMAE) was significantly lower than that in the conventional ADC group (CDCP1A6-DL01-MMAE). The exposure level of free MMAE was essentially positively correlated with toxicity, indicating that PR-ADC could significantly improve the in vivo toxicity of conventional ADC.

### 2. Comparison Between Ligand Unit Targeting Nectin-4 PR-ADC and Conventional ADC (MMAE Payload)

18 BALB/c mice, female, with body weights of 16 to 19 g at the time of grouping, were divided into a PR-ADC group (Nectin4A14-DL01-MMAE, 136 mg/kg) and a conventional ADC group (EV, 100 mg/kg), both at equimolar doses, with 9 mice in each group (5 for toxicology + 4 for TK), for a total of 2 groups.

Free MMAE was also determined using the Hybrid LBA LC-MS/MS method.

**Table 38. Toxicity test results of animals in each group**

| Group | Dose (mg/kg) | Death | Max Body Weight Change Rate | Clinical Observation |
|---|---|---|---|---|
| PR-ADC | 136 | 0/9 | -8.7% (D4) | Piloerection (4/4) |
| Conventional ADC | 100 | 2/9 | -24.4% (D7) | Piloerection (4/4), Hunched posture (5/5), Squinting eyes (5/5), Lethargy (1/5) |

According to the experimental results, the tolerance of BALB/c mice to the PR-ADC group (Nectin4A14-DL01-MMAE) was greater than 136 mg/kg, and the tolerance to the conventional ADC group (EV) was less than 100 mg/kg. The toxicity comparison result at equimolar doses was: Conventional ADC group (EV, 100 mg/kg) > PR-ADC group (Nectin4A14-DL01-MMAE, 136 mg/kg).

The results of this experiment also showed that compared to the conventional ADC structure, the addition of the recovery unit in the PR-ADC structure effectively reduced in vivo toxicity to animals.

**Table 39. Toxicokinetic parameters of free MMAE in serum of animals in each group**

| Group | Dose (mg/kg) | Free MMAE | | |
|---|---|---|---|---|
| | | Cₘₐₓ (nM) | AUC (h*nM) | Tₘₐₓ (h) |
| PR-ADC | 136 | 10.05±1 | 1410.51±71.39 | 24 |
| Convention al ADC | 100 | 76.08±4.88 | 9090.97±655.17 | 24 |

The toxicokinetic results showed that in BALB/c mouse serum, the exposure (Cₘₐₓ and AUC) of free MMAE in the mouse serum of the PR-ADC group (Nectin4A14-DL01-MMAE) was significantly lower than that in the conventional ADC group (EV). The exposure level of free MMAE was essentially positively correlated with toxicity, indicating that PR-ADC could significantly improve the in vivo toxicity of conventional ADC.

### Example 12-2 Comparative Toxicity Study in SD Rats

### 1. Comparison Between Ligand Unit Targeting CDCP1 PR-ADC and Conventional ADC (MMAE Payload)

16 SD rats, female, with body weights of 220 to 270 g at the time of grouping, were divided into a PR-ADC group (CDCP1A9-DL01-MMAE, 42 mg/kg) and a conventional ADC group (CDCP1A6-DL01-MMAE, 30 mg/kg), both at equimolar doses, with 8 rats in each group (4 for toxicology + 4 for TK), for a total of 2 groups. The experiment involved single intravenous administration, with a dosing volume of 10 mL/kg. The drug-related toxic effects in SD rats for each group were investigated and compared through aspects such as death, clinical observation, body weight, and toxicokinetic detection. The experiment lasted for 14 days.

Free MMAE was also determined using the Hybrid LBA LC-MS/MS method.

**Table 40. Toxicity test results of animals in each group**

| Group | Dose (mg/kg) | Death | Max Body Weight Change Rate | Clinical Observation |
|---|---|---|---|---|
| PR-ADC | 42 | 1/8 | -5.2% (D4) | / |
| Conventional ADC | 30 | 3/8 | -5.4% (D4) | Hunched posture (2/4), Scabbing (3/4), Yellow urine |

According to the experimental results, the tolerance of SD rats to the PR-ADC group (CDCP1A9-DL01-MMAE) was less than 42 mg/kg, and the tolerance to the conventional ADC group (CDCP1A6-DL01-MMAE) was less than 30 mg/kg. The toxicity comparison result was: Conventional ADC group (CDCP1A6-DL01-MMAE, 30 mg/kg) > PR-ADC group (CDCP1A9-DL01-MMAE, 42 mg/kg).

The SD rat toxicity test also showed that compared with the conventional ADC structure, the addition of the recovery unit in the PR-ADC structure effectively reduced in vivo toxicity to animals.

**Table 41. Toxicokinetic parameters of free MMAE in serum of animals in each group**

| Group | Dose (mg/kg) | Free MMAE | | |
|---|---|---|---|---|
| | | Cₘₐₓ (nM) | AUC (h*nM) | Tₘₐₓ (h) |
| PR-ADC | 42 | 9.03±1.85 | 780.36±117.77 | [48,72] |
| Conventional ADC | 30 | 9.58±2.51 | 994.81±335.93 | [72,120] |

The toxicokinetic results showed that under equimolar dose conditions, in SD rat serum, the exposure (Cₘₐₓ and AUC) of free MMAE in the PR-ADC group (CDCP1A9-DL01-MMAE) was lower than that in the conventional ADC group (CDCP1A6-DL01-MMAE). The exposure level of free MMAE was positively correlated with toxicity. This also indicates that PR-ADC could significantly improve the toxicity of the conventional ADC group.

### Example 13 Efficacy Studies of PR-ADC in Animal Models

### Example 13-1 Comparative Study of Efficacies of PR-ADC with Ligand Unit Targeting Nectin-4 and Conventional ADC in Human Bladder Cancer Cell HT-1376 Subcutaneous Xenograft Model

### Research Methods

Human bladder cancer cells HT-1376 were expanded and cultured, and then subcutaneously inoculated into the right forelimb axilla near the back of BALB/c-nu mice (GemPharmatech, female). Tumor growth was observed. When the tumor volume reached about 90 to 200 mm³, the mice were randomized into groups based on tumor volume and administered with drugs.

The day of administration was designated as D0. During the experiment, tumor long diameter, short diameter, and body weight were measured twice a week, accompanied by observation of clinical symptoms. At the endpoint of the experiment, the experimental mice were euthanized.

The experimental results (FIG. 52 and Table 42) indicated that in the HT-1376 subcutaneous xenograft model, both PR-ADC and conventional ADC molecules exhibited significant anti-tumor effects (P≤0.05). Under equimolar doses, the anti-tumor effect of the PR-ADC group (Nectin4A14-DL01-MMAE) was comparable to that of the conventional ADC group (EV).

**Table 42. TGI results of each group**

| **Group** | **Molecule** | **TGI** |
|---|---|---|
| PR-ADC | Nectin4A14-DL01-MMAE | 63% |
| Conventional ADC | EV | 71% |
| Control | Vehicle | 0 |

### Example 13-2 Comparative Study of Efficacies of PR-ADC with Ligand Unit Targeting CDCP1 and Conventional ADC in Human Colon Cancer Cell RKO Subcutaneous Xenograft Model

### Research Methods

Human colon cancer cells RKO were expanded and cultured, and then subcutaneously inoculated into the right forelimb axilla near the back of BALB/c-nu mice (GemPharmatech, female). Tumor growth was observed. When the tumor volume reached about 100 to 200 mm³, the mice were randomized into groups based on tumor volume and administered with drugs.

The day of administration was designated as D0. During the experiment, tumor long diameter, short diameter, and body weight were measured twice a week, accompanied by observation of clinical symptoms. At the endpoint of the experiment, the experimental mice were euthanized.

The experimental results (FIG. 53 and Table 43) indicated that in the RKO subcutaneous xenograft model, all molecules exhibited significant anti-tumor effects (P≤0.01). Under equimolar doses, the anti-tumor effects of the PR-ADC groups (CDCP1A10-DL01-MMAE, CDCP1A12-DL01-MMAE) were comparable to that of the conventional ADC group (CDCP1A6-DL01-MMAE).

**Table 43. TGI results of each group**

| **Group** | **Molecule** | **TGI** |
|---|---|---|
| PR-ADC | CDCP1A10-DL01-MMAE | 96% |
| PR-ADC | CDCP1A12-DL01-MMAE | 95% |
| Conventional ADC | CDCP1A6-DL01-MMAE | 99% |
| Control | Vehicle | 0 |

The above disclosed are merely some examples of the present disclosure and is not intended to limit the present disclosure in other forms. A person of ordinary skill in the art can understand that the present disclosure is not limited to the specific embodiments. For a person of ordinary skill in the art, several improvements and modifications can be made to the present disclosure without departing from the principles of the present disclosure, and these improvements and modifications also fall within the protection scope of the claims of the present disclosure.

## Claims

1. A ligand-drug conjugate, comprising a ligand unit targeting a tumor antigen or a non-tumor antigen, a conjugated payload unit, and a recovery unit capable of binding to a free payload released from the ligand-drug conjugate, wherein the payload unit comprises a payload and a linker.

2. The ligand-drug conjugate according to claim 1, wherein the ligand unit and the recovery unit are directly connected or not directly connected to each other.

3. The ligand-drug conjugate according to claim 1 or 2, wherein a linker unit is contained between the ligand unit and the recovery unit.

4. The ligand-drug conjugate according to any one of claims 1 to 3, further comprising an additional functional unit.

5. The ligand-drug conjugate according to any one of claims 1 to 4, wherein the payload is conjugated to the ligand unit, and/or the recovery unit, and/or the linker unit, and/or the additional functional unit via the linker.

6. The ligand-drug conjugate according to any one of claims 1 to 5, wherein the ligand unit comprises an antibody or an antigen-binding fragment, a receptor, a peptide, an oligopeptide, a peptidomimetic, a fusion protein, or an aptamer that specifically binds to the tumor antigen or the non-tumor antigen.

7. The ligand-drug conjugate according to claim 6, wherein the antibody includes, but is not limited to, a monoclonal antibody, a bispecific antibody, a multispecific antibody, a nanobody (heavy chain antibody, HcAb), and an immunoglobulin new antigen receptor (IgNAR); the antigen-binding fragment includes, but is not limited to, an scFv, an Fab, an Fab', an F(ab')₂, an Fd, an Fv, a dAb, an Fd, an sdAb, a VHH, and a Diabody; and the receptor includes, but is not limited to, a T cell receptor.

8. The ligand-drug conjugate according to claim 6 or 7, wherein the antibody is selected from a murine antibody, a chimeric antibody, a humanized antibody, a fully human antibody, and an artificially engineered antibody.

9. The ligand-drug conjugate according to claim 8, wherein the antibody further comprises a constant region of an immunoglobulin.

10. The ligand-drug conjugate according to claim 9, wherein the immunoglobulin is selected from the group consisting of human IgG1, IgG2, IgG3 and IgG4.

11. The ligand-drug conjugate according to any one of claims 1 to 10, wherein the tumor antigen or non-tumor antigen includes, but is not limited to, CD9, CD19, CD20, CD22, CD24, CD25, CD29, CD30, CD33, CD37, CD44, CD45, CD46, CD47, CD49b, CD51, CD52, CD56, CD73(NT5E), CD79b, CD123, CD133, CD138, CD157, CD166, DLL-3, CDH6, Tissue factor, EpCAM, PSMA, MUC1, MUC16, FOLR1, GPC3, ROR1, ROR2, PD-L1, ENPP3, TDGF1, MSLN, TIM-1, LRRC15, LIV-1(ZIP6), Claudin6, Claudin9, Claudin 18.2, Mesothelin, HER2(ErbB2), HER3(ErbB3), EGFR, c-MET, SLITRK6, KIT(CD117), STEAP1, NaPi2B(SLC34A2), SLC44A4, GPNMB, AXL, CD166, B7-H3(CD276), B7-H4(VTCN1), PTK7(CCK4), EFNA4, 5T4, NOTCH3, Nectin-4, TROP-2, GPR20, EphA2, LYPD3, FGFR2, FGFR3, FRα, CEACAMs, CAIX, P-cadherin(CDH3), CDH17, GD3, Cadherin 6, LAMP1, FLT3, BCMA, SLTRK6, Lewis Y, ASCT2, CA-IX, Cripto, DPEP3, Globo H, Ly6E, RNF43, DR5, CDCP1, CEACAM6, FUT3, GD2, CD26E, CD70, CD74, CD163, PRLR, TNFα, CXCR4, LXR, IL-6, CEACAM5, GPRC5D, HLA-DR, CTLA4(CD152), FAPα and IL2R.

12. The ligand-drug conjugate according to any one of claims 1 to 11, wherein the antibody that specifically binds to the tumor antigen or the non-tumor antigen includes, but is not limited to, Alemtuzumab, Altumomab pentetate, Amivantamab, Atezolizumab, Inetetamab, Anetumab, Avelumab, Bectumomab, Bermekimab, Bevacizumab, Blinatumomab, Brentuximab vedotin, Catumaxomab, Cemiplimab, Cetuximab, Clivatuzumab, Daclizumab, Daratumumab, Denosumab, Dinutuximab beta, Dostarlimab, Durvalumab, Edrecolomab, Enfortumab vedotin, Epcoritamab, Epratuzumab, Etaracizumab, Genmab, Glofitamab, Girentuximab, Ibritumomab, Inebilizumab, Inotuzumab ozogamicin, Ipilimumab, Isatuximab, Labetuzumab, Loncastuximab, Mogamulizumab, Loncastuximab tesirine, Mosunetuzumab, Nimotuzumab, Naratuximab, Naxitamab, Nivolumab, Ocrelizumab, Ofatumumab, Olaratumab, Oregovomab, Panitumumab, Pembrolizumab, Pertuzumab, Polatuzumab vedotin, Racotumomab, Ramucirumab, Ramucirumab, Rituximab, Sacituzumab govitecan, Teprotumumab, Tocilizumab, Tositumomab, Trastuzumab, Votumumab, Zalutumumab, Tocilizumab, Zanolimumab, and antigen-binding fragments and derivatives thereof; the peptide, oligopeptide, fusion protein that specifically binds to a specific antigen includes, but is not limited to, a bicyclic peptide (Bicycle), angiopep-2, a linear peptide, a pH-sensitive peptide, SOR-C27, A6 peptide, SOR13, and variants and derivatives thereof; the aptamer that specifically binds to a specific antigen includes AS1411, sgc8, sgc4f, sgd5a, TC01, TD05, A9, A10, APT, Min.2, AIR-3A, E3, E07, Waz, P19, Zy1, and variants and derivatives thereof.

13. The ligand-drug conjugate according to any one of claims 1 to 12, wherein the ligand-drug conjugate is capable of specifically binding to the free payload, and molecular aggregation does not occur between the ligand-drug conjugates.

14. The ligand-drug conjugate according to claim 13, wherein the recovery unit specifically binds to the free payload.

15. The ligand-drug conjugate according to claim 14, wherein the recovery unit comprises an antibody, an antigen-binding fragment, a receptor, a fusion protein, a peptide, an oligopeptide, a peptidomimetic, or an aptamer that specifically binds to the free payload.

16. The ligand-drug conjugate according to claim 15, wherein the antibody that specifically binds to the free payload includes, but is not limited to, a monoclonal antibody, a bispecific antibody, a multispecific antibody, a nanobody (heavy chain antibody, HcAb), an immunoglobulin new antigen receptor (IgNAR); the antigen-binding fragment includes, but is not limited to, an scFv, an Fab, an Fab', an F(ab')₂, an Fd, an Fv, a dAb, an Fd, an sdAb, a VHH, and a Diabody; and the receptor includes, but is not limited to, a T cell receptor.

17. The ligand-drug conjugate according to claim 16, wherein the antibody that specifically binds to the free payload is selected from the group consisting of a murine antibody, a chimeric antibody, a humanized antibody, a fully human antibody, and an artificially engineered antibody.

18. The ligand-drug conjugate according to any one of claims 13 to 17, wherein the recovery unit is capable of specifically binding to the free payload means that the equilibrium dissociation constant K_{D}(M) of the recovery unit to the free payload is less than 10⁻⁴, or further less than 10⁻⁵, or further less than 10⁻⁶, or further less than 10⁻⁷, or further less than 10⁻⁸, or further less than 10⁻⁹.

19. The ligand-drug conjugate according to claim 18, wherein the recovery unit is capable of specifically binding to the free payload means that the equilibrium dissociation constant K_{D}(M) of the recovery unit to the free payload is less than 10⁻⁷, or further less than 10⁻⁸, or further less than 10⁻⁹.

20. The ligand-drug conjugate according to claim 19, wherein the recovery unit is capable of specifically binding to the free payload means that the equilibrium dissociation constant K_{D}(M) of the recovery unit to the free payload is less than 10⁻⁹.

21. The ligand-drug conjugate according to claim 18, wherein the recovery unit does not bind to, or has a low affinity to, a payload in a conjugated state in the ligand-drug conjugate, and the low affinity also prevents the ligand-drug conjugate molecules from aggregating.

22. The ligand-drug conjugate according to claim 21, wherein the recovery unit specifically targets a non-exposed epitope on the conjugated payload unit.

23. The ligand-drug conjugate according to any one of claims 13 to 22, wherein a payload epitope masking unit capable of preventing the recovery unit from binding to the payload that remains conjugated to the ligand-drug conjugate is linked to the linker or the payload.

24. The ligand-drug conjugate according to claim 23, wherein the payload epitope masking unit does not interfere with the normal release of the payload after the ligand-drug conjugate enters a target cell.

25. The ligand-drug conjugate according to any one of claims 1 to 24, wherein the ligand-drug conjugate comprises one, two, or more recovery units.

26. The ligand-drug conjugate according to claim 15, wherein the recovery unit comprises:
(1) a VHH domain with CDR1-3 selected from amino acid sequences defined by:
CDR1-3 as set forth in SEQ ID NOs: 103, 104, and 105;
or (2) an amino acid sequence selected from the amino acid sequence as set forth in SEQ ID NO: 102;
or (3) an amino acid sequence selected from combinations of VH and VL defined as follows:
a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 7 respectively;
a VH comprising HCDR1-3 with amino acid sequences as set forth in SEQ ID NOs: 9, 10, and 11 respectively, and a VL comprising LCDR1-3 with amino acid sequences as set forth in SEQ ID NO: 6, FAS, and SEQ ID NO: 13 respectively;
a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 16 respectively;
a VH comprising HCDR1-3 with amino acid sequences as set forth in SEQ ID NOs: 2, 3, and 20 respectively, and a VL comprising LCDR1-3 with amino acid sequences as set forth in SEQ ID NO: 6, YAS, and SEQ ID NO: 7 respectively;
a VH comprising HCDR1-3 with amino acid sequences as set forth in SEQ ID NOs: 22, 23, and 24 respectively, and a VL comprising LCDR1-3 with amino acid sequences as set forth in SEQ ID NO: 26, WAS, and SEQ ID NO: 27 respectively;
a VH comprising HCDR1-3 with amino acid sequences as set forth in SEQ ID NOs: 29, 30, and 31 respectively, and a VL comprising LCDR1-3 with amino acid sequences as set forth in SEQ ID NO: 33, KVS, and SEQ ID NO: 34 respectively;
a VH comprising HCDR1-3 with amino acid sequences as set forth in SEQ ID NOs: 29, 36, and 37 respectively, and a VL comprising LCDR1-3 with amino acid sequences as set forth in SEQ ID NO: 33, KLS, and SEQ ID NO: 34 respectively;
a VH comprising HCDR1-3 with amino acid sequences as set forth in SEQ ID NOs: 40, 41, and 42 respectively, and a VL comprising LCDR1-3 with amino acid sequences as set forth in SEQ ID NO: 44, KVS, and SEQ ID NO: 45 respectively;
a VH comprising HCDR1-3 with amino acid sequences as set forth in SEQ ID NOs: 47, 48, and 42 respectively, and a VL comprising LCDR1-3 with amino acid sequences as set forth in SEQ ID NO: 50, KVS, and SEQ ID NO: 51 respectively;
a VH comprising HCDR1-3 with amino acid sequences as set forth in SEQ ID NOs: 53, 54, and 55 respectively, and a VL comprising LCDR1-3 with amino acid sequences as set forth in SEQ ID NO: 57, DTS, and SEQ ID NO: 58 respectively;
a VH comprising HCDR1-3 with amino acid sequences as set forth in SEQ ID NOs: 62, 63, and 64 respectively, and a VL comprising LCDR1-3 with amino acid sequences as set forth in SEQ ID NO: 66, SAS, and SEQ ID NO: 67 respectively;
a VH comprising HCDR1-3 with amino acid sequences as set forth in SEQ ID NOs: 69, 70, and 71 respectively, and a VL comprising LCDR1-3 with amino acid sequences as set forth in SEQ ID NO: 73, WAS, and SEQ ID NO: 74 respectively;
a VH comprising HCDR1-3 with amino acid sequences as set forth in SEQ ID NOs: 76, 77, and 78 respectively, and a VL comprising LCDR1-3 with amino acid sequences as set forth in SEQ ID NO: 80, GAS, and SEQ ID NO: 81 respectively;
a VH comprising HCDR1-3 with amino acid sequences as set forth in SEQ ID NOs: 83, 84, and 85 respectively, and a VL comprising LCDR1-3 with amino acid sequences as set forth in SEQ ID NO: 87, SAS, and SEQ ID NO: 67 respectively;
a VH comprising HCDR1-3 with amino acid sequences as set forth in SEQ ID NOs: 89, 90, and 91 respectively, and a VL comprising LCDR1-3 with amino acid sequences as set forth in SEQ ID NO: 93, HGT, and SEQ ID NO: 94 respectively;
a VH comprising HCDR1-3 with amino acid sequences as set forth in SEQ ID NOs: 76, 77, and 78 respectively, and a VL comprising LCDR1-3 with amino acid sequences as set forth in SEQ ID NOs: 99, 77, and 100 respectively;
a VH comprising an amino acid sequence as set forth in SEQ ID NO: 1, and a VL comprising an amino acid sequence as set forth in SEQ ID NO: 5;
a VH comprising an amino acid sequence as set forth in SEQ ID NO: 8, and a VL comprising an amino acid sequence as set forth in SEQ ID NO: 12;
a VH comprising an amino acid sequence as set forth in SEQ ID NO: 14, and a VL comprising an amino acid sequence as set forth in SEQ ID NO: 15;
a VH comprising an amino acid sequence as set forth in SEQ ID NO: 17, and a VL comprising an amino acid sequence as set forth in SEQ ID NO: 18;
a VH comprising an amino acid sequence as set forth in SEQ ID NO: 19, and a VL comprising an amino acid sequence as set forth in SEQ ID NO: 18;
a VH comprising an amino acid sequence as set forth in SEQ ID NO: 21, and a VL comprising an amino acid sequence as set forth in SEQ ID NO: 25;
a VH comprising an amino acid sequence as set forth in SEQ ID NO: 28, and a VL comprising an amino acid sequence as set forth in SEQ ID NO: 32;
a VH comprising an amino acid sequence as set forth in SEQ ID NO: 35, and a VL comprising an amino acid sequence as set forth in SEQ ID NO: 38;
a VH comprising an amino acid sequence as set forth in SEQ ID NO: 39, and a VL comprising an amino acid sequence as set forth in SEQ ID NO: 43;
a VH comprising an amino acid sequence as set forth in SEQ ID NO: 46, and a VL comprising an amino acid sequence as set forth in SEQ ID NO: 49;
a VH comprising an amino acid sequence as set forth in SEQ ID NO: 52, and a VL comprising an amino acid sequence as set forth in SEQ ID NO: 56;
a VH comprising an amino acid sequence as set forth in SEQ ID NO: 59, and a VL comprising an amino acid sequence as set forth in SEQ ID NO: 60;
a VH comprising an amino acid sequence as set forth in SEQ ID NO: 61, and a VL comprising an amino acid sequence as set forth in SEQ ID NO: 65;
a VH comprising an amino acid sequence as set forth in SEQ ID NO: 68, and a VL comprising an amino acid sequence as set forth in SEQ ID NO: 72;
a VH comprising an amino acid sequence as set forth in SEQ ID NO: 75, and a VL comprising an amino acid sequence as set forth in SEQ ID NO: 79;
a VH comprising an amino acid sequence as set forth in SEQ ID NO: 82, and a VL comprising an amino acid sequence as set forth in SEQ ID NO: 86;
a VH comprising an amino acid sequence as set forth in SEQ ID NO: 88, and a VL comprising an amino acid sequence as set forth in SEQ ID NO: 92;
a VH comprising an amino acid sequence as set forth in SEQ ID NO: 95, and a VL comprising an amino acid sequence as set forth in SEQ ID NO: 96; and
a VH comprising an amino acid sequence as set forth in SEQ ID NO: 97, and a VL comprising an amino acid sequence as set forth in SEQ ID NO: 98;
wherein the CDR1, CDR2 and CDR3 sequences, the HCDR1, HCDR2 and HCDR3 sequences, and the LCDR1, LCDR2 and LCDR3 sequences are obtained based on an IMGT definition scheme;
or (4) a peptide comprising an amino acid sequence defined by SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, or SEQ ID NO: 129;
or (5) an aptamer comprising a nucleotide sequence defined by SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, or SEQ ID NO: 162.

27. The ligand-drug conjugate according to any one of claims 1 to 26, wherein the recovery unit is obtained by immunizing a non-human animal with a conjugate of a small molecule drug and a carrier protein or a biotin-small molecule drug conjugate, or obtained by screening with phage display technology, or obtained by screening with virtual technology, or obtained by screening with SELEX technology, or obtained by screening with hybridoma technology, or obtained by screening with nanobody technology, or obtained by yeast display technology, or obtained by single B cell cloning technology, or obtained by microfluidic technology, or obtained by surface plasmon resonance assisted screening technology.

28. The ligand-drug conjugate according to claim 27, wherein the small molecule drug includes, but is not limited to, MMAE, MMAD, DM1, DM2, DM4, eribulin, Exatecan, SN38, DXD, calicheamicin, pyrrolobenzodiazepine (PBD), indolinobenzodiazepine (IBD), duocarmycin, amanitin, Maaa-1181a, taxol, daunomycin, vinblastine, Doxorubicin, methotrexate, MMAF, ML022-Dxd, ML022-D633-004, ML026-Dxd, ML031-D633-004, ML037-D633-004, ML042-Dxd, ML047-DX8951, and MPA-Dxd; 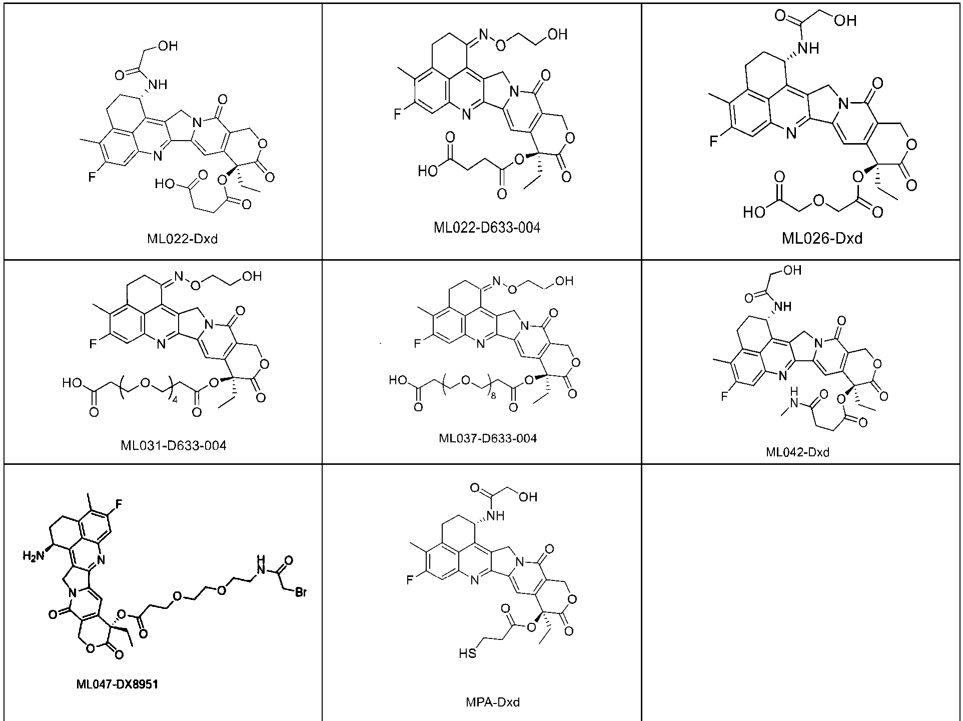 preferably, the carrier protein includes, but is not limited to, KLH, BSA and OVA.

29. The ligand-drug conjugate according to claim 28, wherein the biotin-small molecule drug conjugate includes, but is not limited to, Biotin-PEG12-Dxd, Biotin-PEG10-Dxd, Biotin-PEG6-Dxd, Biotin-PEG6-Triazole-Dxd and Biotin-PEG10-Triazole-Dxd:

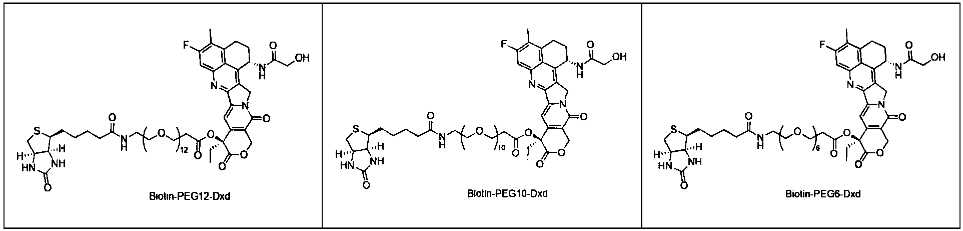

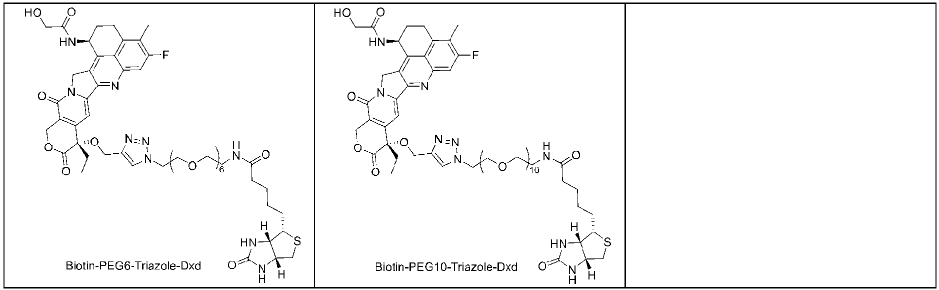

30. The ligand-drug conjugate according to any one of claims 1 to 29, wherein the conjugated payload includes a cytotoxic molecule, a nucleic acid, an immunostimulant, and a modulator.

31. The ligand-drug conjugate according to claim 30, wherein the ligand-drug conjugate comprises one, two or more payloads.

32. The ligand-drug conjugate according to claim 31, wherein the number of payload is 1, 2, 3, 4, 5, 6, 7, 8 or more.

33. The ligand-drug conjugate according to any one of claims 30 to 32, wherein when two or more payloads are conjugated, the payloads may be the same or different.

34. The ligand-drug conjugate according to any one of claims 30 to 33, wherein the payload is conjugated to the ligand unit and/or the recovery unit and/or the linker unit and/or the additional functional unit via site-specific or random conjugation.

35. The ligand-drug conjugate according to any one of claims 30 to 34, wherein the cytotoxic molecule includes, but is not limited to, a tubulin inhibitor, a DNA inhibitor, or other toxin molecules; the DNA inhibitor further includes a DNA damaging agent or a topoisomerase I inhibitor.

36. The ligand-drug conjugate according to claim 35, wherein the tubulin inhibitor further includes, but is not limited to, auristatins, maytansinoid derivatives, tubulysin, halichondrin, cryptophycins or EG5 inhibitors.

37. The ligand-drug conjugate according to claim 35, wherein the auristatins further include, but are not limited to, auristatin F (AF), monomethyl auristatin D (MMAD), monomethyl auristatin E (MMAE), and monomethyl auristatin F (MMAF); the maytansinoid derivatives further include, but are not limited to, DM1, DM2, and DM4; the halichondrin further includes, but is not limited to, eribulin.

38. The ligand-drug conjugate according to claim 35, wherein the DNA damaging agent further includes, but is not limited to, calicheamicins, pyrrolobenzodiazepines (PBDs), indolinobenzodiazepines (IBDs), and duocarmycins; and the topoisomerase I inhibitor further includes, but is not limited to, camptothecin derivatives.

39. The ligand-drug conjugate according to claim 35, wherein the camptothecin derivatives further include, but are not limited to, Exatecan, SN38, and DXD.

40. The ligand-drug conjugate according to claim 35, wherein the other toxin molecules include, but are not limited to, amanitin, Maaa-1181a, taxol, daunomycin, vinblastine, Doxorubicin, methotrexate, and a radioisotope and/or a pharmaceutically acceptable salt thereof.

41. The ligand-drug conjugate according to claim 30, wherein the nucleic acid used as the payload includes, but is not limited to, an antisense oligonucleotide (ASO), a small interfering RNA (siRNA), a microRNA (miRNA), a small activating RNA (saRNA), a messenger RNA (mRNA), and an oligonucleotide (AOC).

42. The ligand-drug conjugate according to claim 30, wherein the nucleic acid used as the payload includes but is not limited to DMPK siRNA, Exon-44-skipping PMO, DUX4 siRNA, Exon-51-skipping PMO, DMPK ASO, DUX4 ASO, and CpG.

43. The ligand-drug conjugate according to claim 30, wherein the immunostimulant used as the payload includes but is not limited to a TLR7 agonist, a TLR8 agonist, a TLR9 agonist, a TGF-β inhibitor, a TNIK inhibitor, and a STING agonist.

44. The ligand-drug conjugate according to claim 30, wherein the modulator used as the payload includes but is not limited to a glucocorticoid receptor modulator, an antibiotic, a kinase inhibitor, a liver X receptor (LXR) agonist, a phosphodiesterase (PDE4) inhibitor, and a bisphosphonate.

45. The ligand-drug conjugate according to claim 30, wherein the free payload has a structure selected from the group consisting of the following structure, and an isomer or a deuterated form thereof:

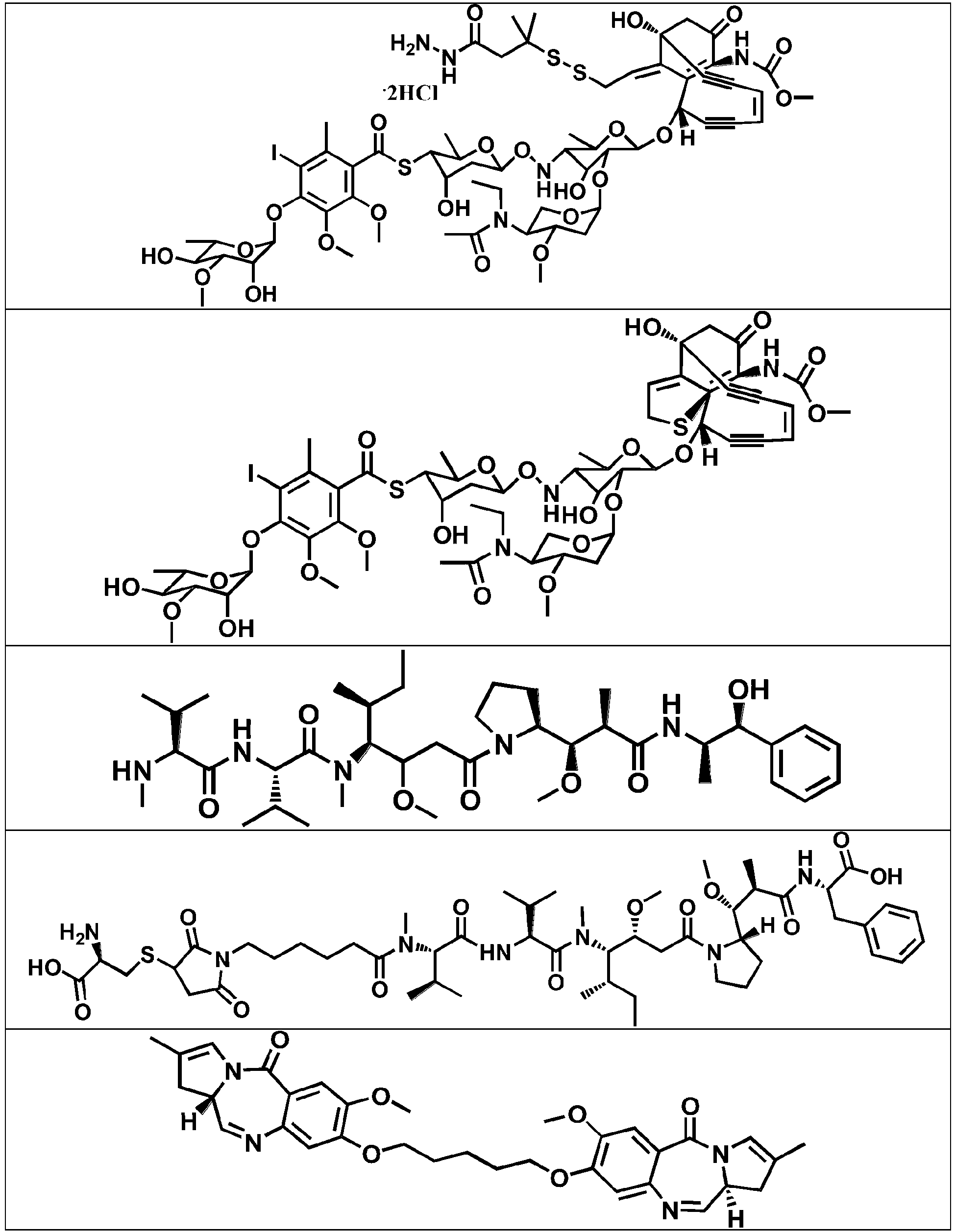

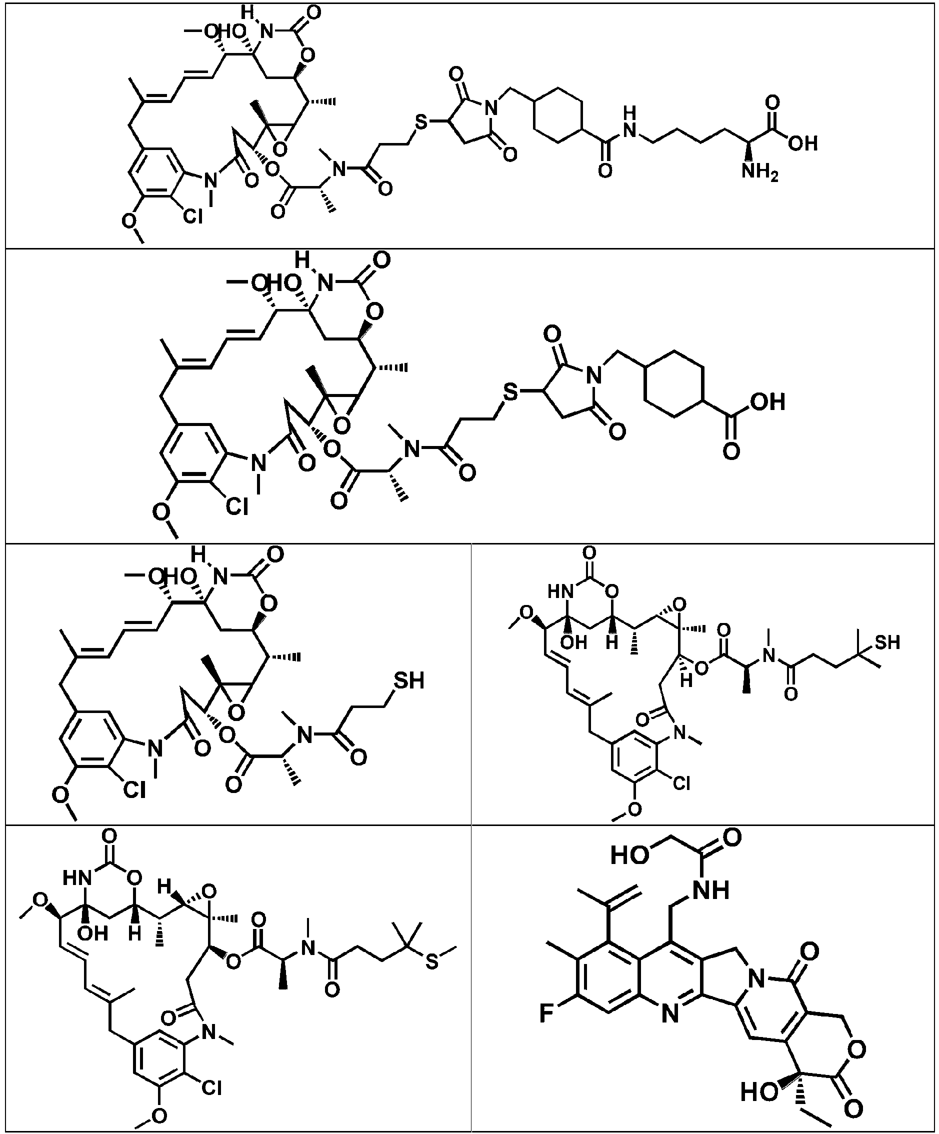

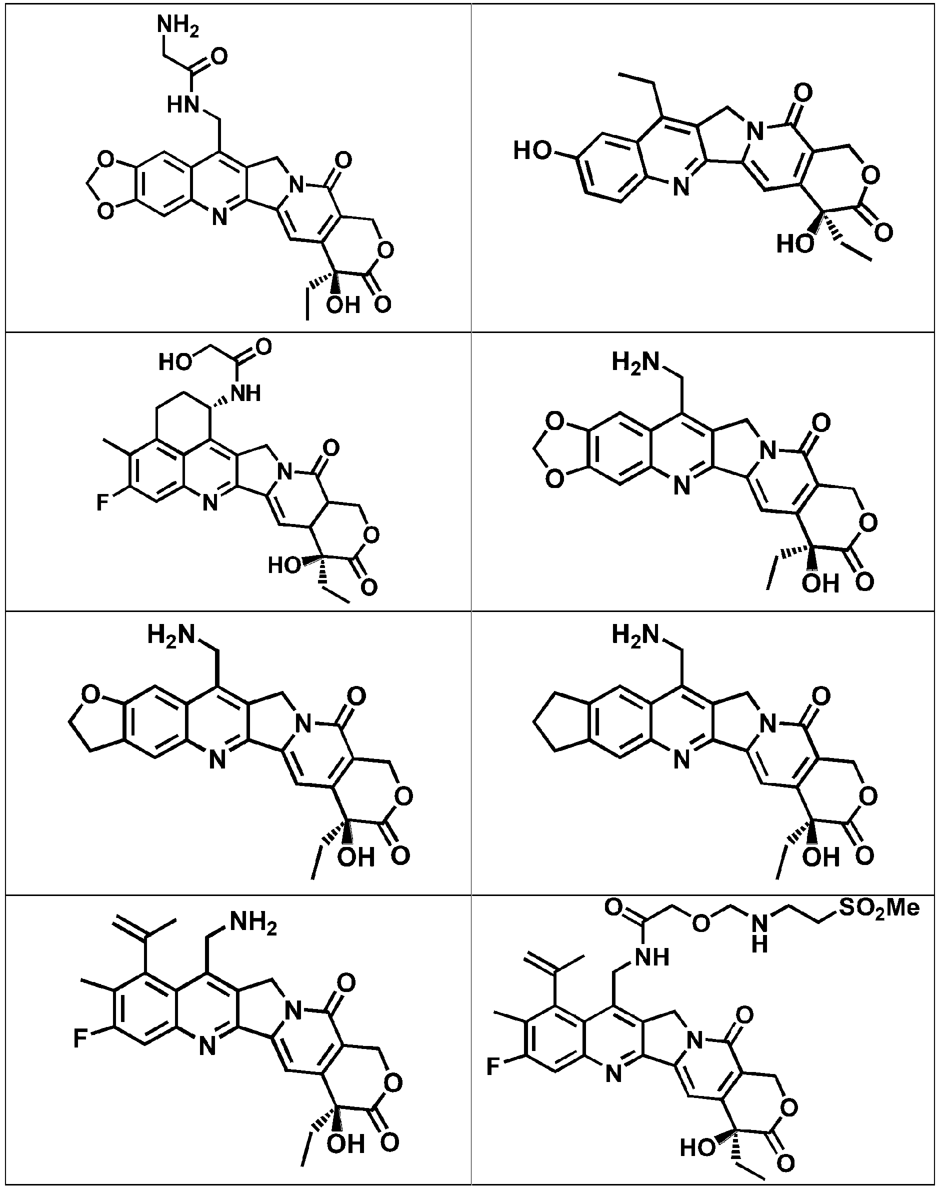

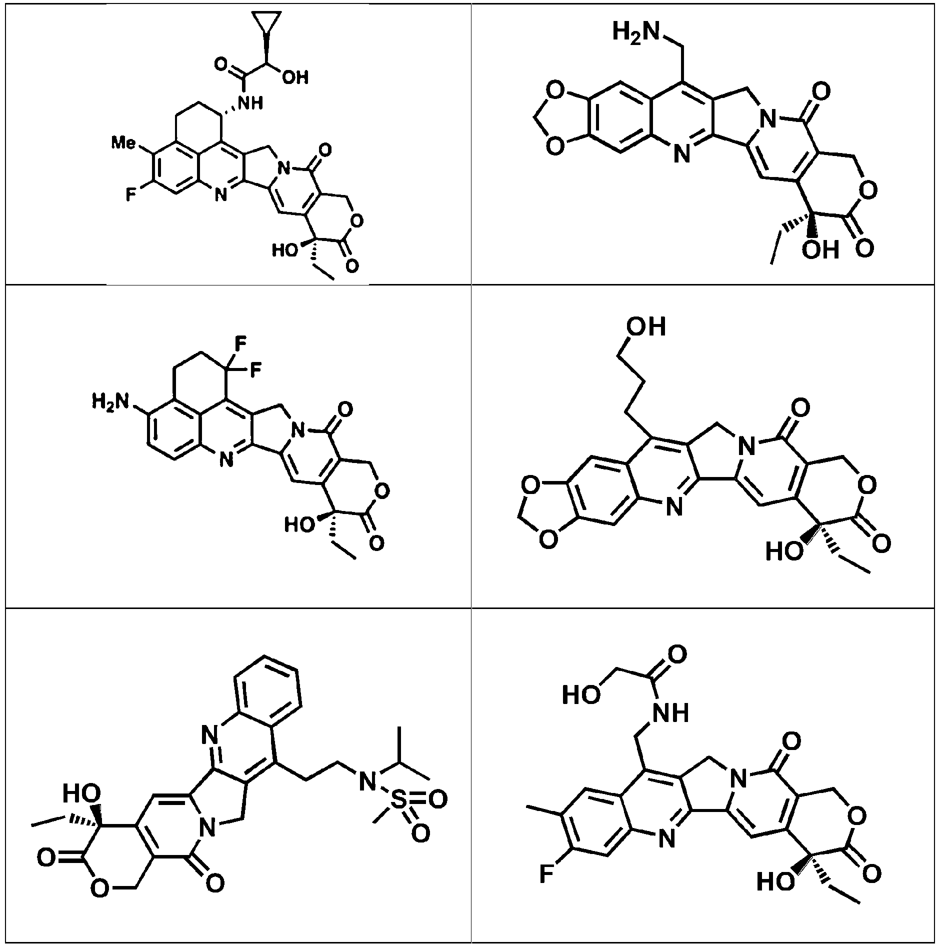

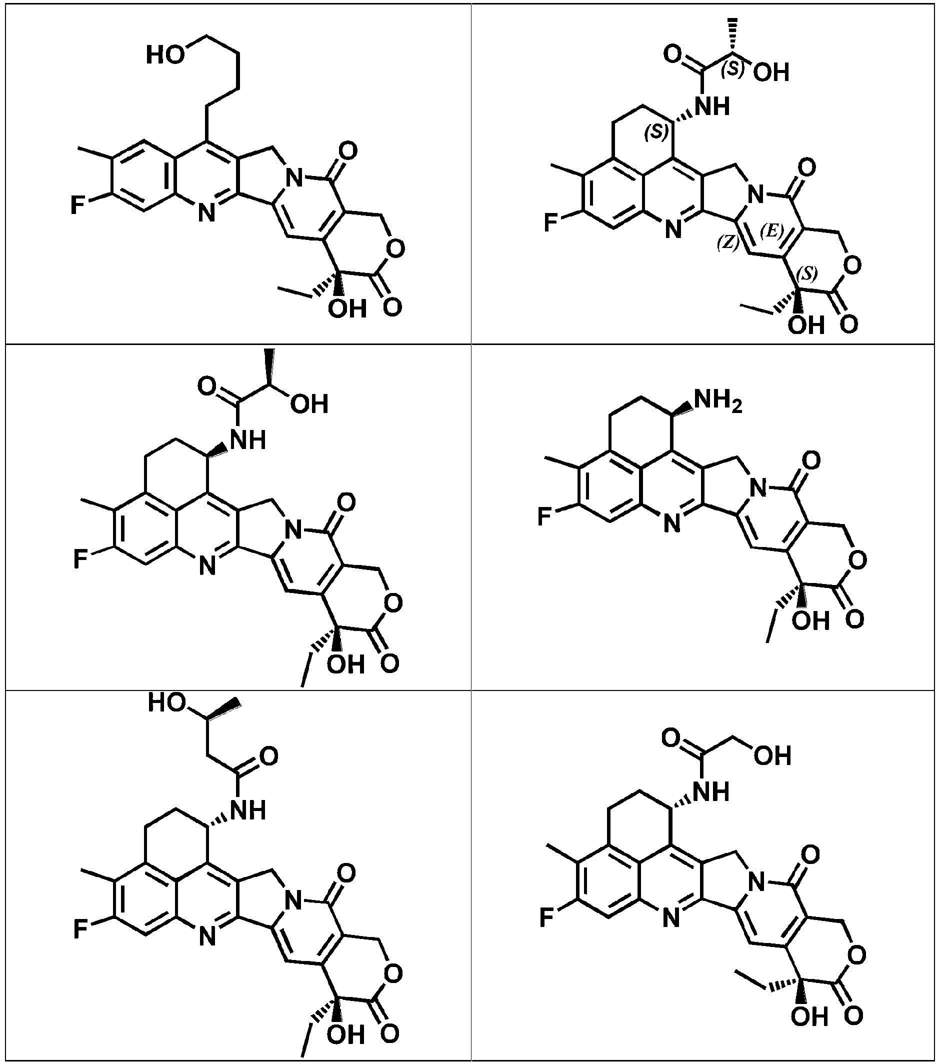

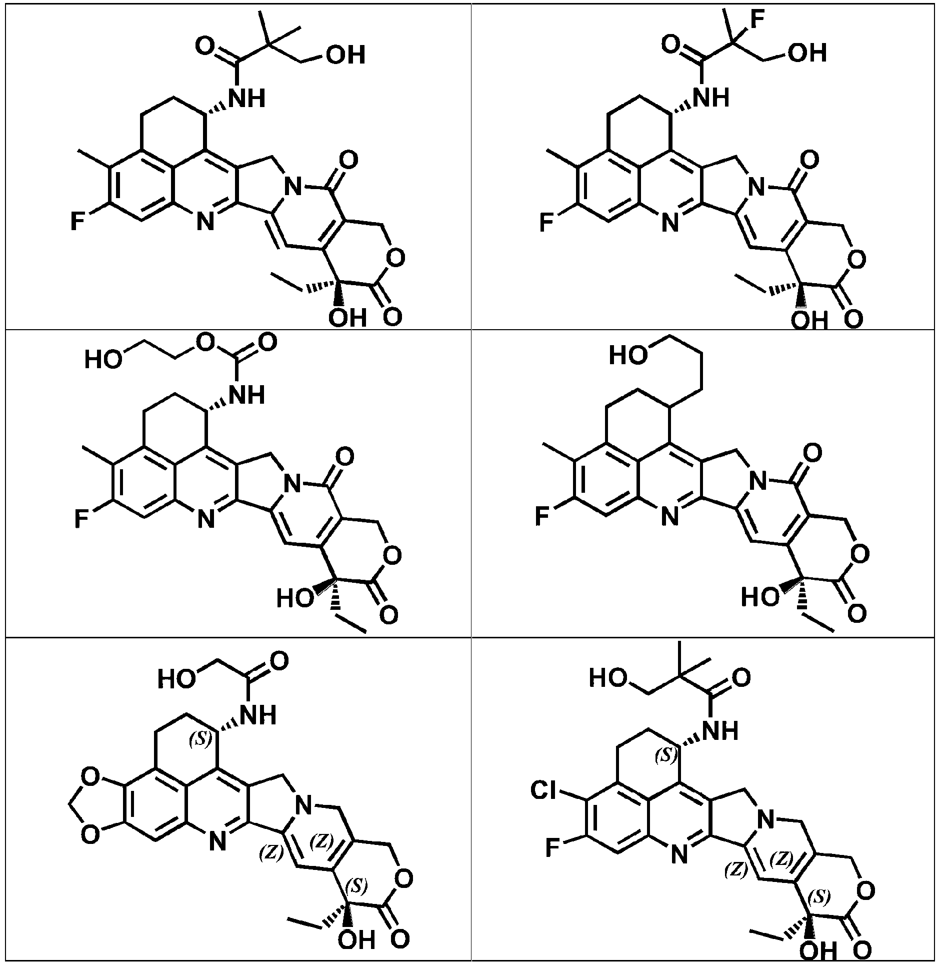

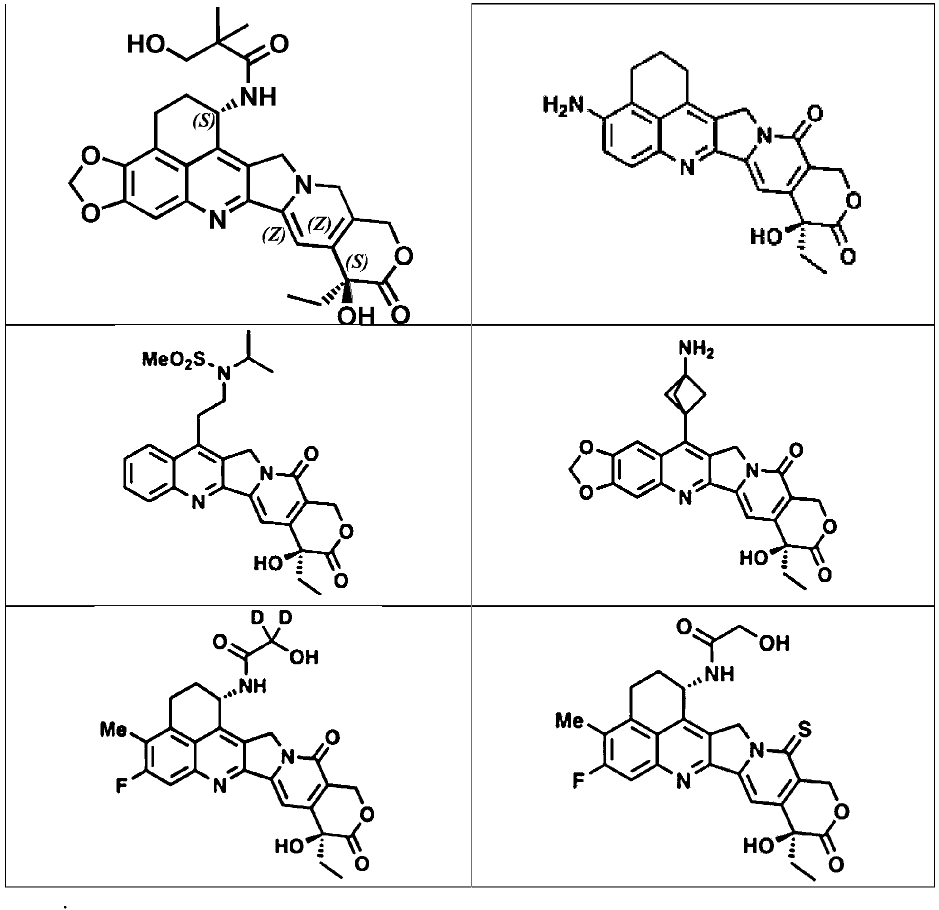

46. The ligand-drug conjugate according to any one of claims 1 to 45, wherein the linker unit includes, but is not limited to, a peptide linker, an Fc domain or a fragment thereof; preferably, the peptide linker is a flexible peptide linker; further preferably, the peptide linker comprises one or more amino acids; further preferably, the peptide linker comprises at least 5 amino acids; or the amino acid sequence of the peptide linker comprises (GGGGS)n, (VPGXG)n, (SG)n, (EAAAK)n, (G)n, (R)n, A(EAAAK)n ALEA(EAAAK)nA, A(EAAAK)nA, (AP)n, VSQTSKLTRAETVFPDV, PLGLWA, RVLAEA, EDVVCCSMSY, GGIEGRGS, TRHRQPRGWE, AGNRVRRSVG, GFLG, or LE, wherein n is equal to 1, 2, 3 or 4.

47. A pharmaceutically acceptable salt, a deuterated form, or a solvate of the ligand-drug conjugate according to claims 1 to 46.

48. A pharmaceutical composition comprising the ligand-drug conjugate according to any one of claims 1 to 46 or the salt, deuterated form, or solvate according to claim 47, and a pharmaceutically acceptable excipient.

49. Use of a recovery unit capable of binding to a free payload released from a ligand-drug conjugate in the preparation of a ligand-drug conjugate comprising a free payload recovery unit.

50. The use according to claim 49, wherein the recovery unit comprises an antibody or an antigen-binding fragment, a receptor, a fusion protein, a peptide, an oligopeptide, a peptidomimetic, or an aptamer that specifically binds to the free payload.

51. The use according to claim 50, wherein the antibody that specifically binds to the free payload includes, but is not limited to, a monoclonal antibody, a bispecific antibody, a multispecific antibody, a nanobody (heavy chain antibody, HcAb), and an immunoglobulin new antigen receptor (IgNAR); the antigen-binding fragment includes, but is not limited to, an scFv, an Fab, an Fab', an F(ab')₂, an Fd, an Fv, a dAb, an Fd, an sdAb, a VHH, and a Diabody; and the receptor includes, but is not limited to, a T cell receptor.

52. The use according to claim 51, wherein the antibody that specifically binds to the free payload is selected from the group consisting of a murine antibody, a chimeric antibody, a humanized antibody, a fully human antibody, and an artificially engineered antibody.

53. The use according to any one of claims 49 to 52, wherein the recovery unit is capable of specifically binding to the free payload means that the equilibrium dissociation constant K_{D}(M) of the recovery unit to the free payload is less than 10⁻⁴, or further less than 10⁻⁵, or further less than 10⁻⁶, or further less than 10⁻⁷, or further less than 10⁻⁸, or further less than 10⁻⁹.

54. The use according to any one of claims 49 to 53, wherein the recovery unit is capable of specifically binding to the free payload means that the equilibrium dissociation constant K_{D}(M) of the recovery unit to the free payload is less than 10⁻⁷, or further less than 10⁻⁸, or further less than 10⁻⁹.

55. The use according to any one of claims 49 to 54, wherein the recovery unit is capable of specifically binding to the free payload means that the equilibrium dissociation constant K_{D}(M) of the recovery unit to the free payload is less than 10⁻⁹.

56. The use according to any one of claims 49 to 55, wherein the recovery unit does not bind to, or has a low affinity to, a payload in a conjugated state in the ligand-drug conjugate, and the low affinity also prevents the ligand-drug conjugate molecules from aggregating.

57. The use according to claim 49, wherein the recovery unit comprises:
(1) a VHH domain with CDR1-3 selected from amino acid sequences defined by:
CDR1-3 as set forth in SEQ ID NOs: 103, 104, and 105;
or (2) an amino acid sequence selected from the amino acid sequence as set forth in SEQ ID NO: 102;
or (3) an amino acid sequence selected from combinations of VH and VL defined as follows:
a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 7 respectively;
a VH comprising HCDR1-3 with amino acid sequences as set forth in SEQ ID NOs: 9, 10, and 11 respectively, and a VL comprising LCDR1-3 with amino acid sequences as set forth in SEQ ID NO: 6, FAS, and SEQ ID NO: 13 respectively;
a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 16 respectively;
a VH comprising HCDR1-3 with amino acid sequences as set forth in SEQ ID NOs: 2, 3, and 20 respectively, and a VL comprising LCDR1-3 with amino acid sequences as set forth in SEQ ID NO: 6, YAS, and SEQ ID NO: 7 respectively;
a VH comprising HCDR1-3 with amino acid sequences as set forth in SEQ ID NOs: 22, 23, and 24 respectively, and a VL comprising LCDR1-3 with amino acid sequences as set forth in SEQ ID NO: 26, WAS, and SEQ ID NO: 27 respectively;
a VH comprising HCDR1-3 with amino acid sequences as set forth in SEQ ID NOs: 29, 30, and 31 respectively, and a VL comprising LCDR1-3 with amino acid sequences as set forth in SEQ ID NO: 33, KVS, and SEQ ID NO: 34 respectively;
a VH comprising HCDR1-3 with amino acid sequences as set forth in SEQ ID NOs: 29, 36, and 37 respectively, and a VL comprising LCDR1-3 with amino acid sequences as set forth in SEQ ID NO: 33, KLS, and SEQ ID NO: 34 respectively;
a VH comprising HCDR1-3 with amino acid sequences as set forth in SEQ ID NOs: 40, 41, and 42 respectively, and a VL comprising LCDR1-3 with amino acid sequences as set forth in SEQ ID NO: 44, KVS, and SEQ ID NO: 45 respectively;
a VH comprising HCDR1-3 with amino acid sequences as set forth in SEQ ID NOs: 47, 48, and 42 respectively, and a VL comprising LCDR1-3 with amino acid sequences as set forth in SEQ ID NO: 50, KVS, and SEQ ID NO: 51 respectively;
a VH comprising HCDR1-3 with amino acid sequences as set forth in SEQ ID NOs: 53, 54, and 55 respectively, and a VL comprising LCDR1-3 with amino acid sequences as set forth in SEQ ID NO: 57, DTS, and SEQ ID NO: 58 respectively;
a VH comprising HCDR1-3 with amino acid sequences as set forth in SEQ ID NOs: 62, 63, and 64 respectively, and a VL comprising LCDR1-3 with amino acid sequences as set forth in SEQ ID NO: 66, SAS, and SEQ ID NO: 67 respectively;
a VH comprising HCDR1-3 with amino acid sequences as set forth in SEQ ID NOs: 69, 70, and 71 respectively, and a VL comprising LCDR1-3 with amino acid sequences as set forth in SEQ ID NO: 73, WAS, and SEQ ID NO: 74 respectively;
a VH comprising HCDR1-3 with amino acid sequences as set forth in SEQ ID NOs: 76, 77, and 78 respectively, and a VL comprising LCDR1-3 with amino acid sequences as set forth in SEQ ID NO: 80, GAS, and SEQ ID NO: 81 respectively;
a VH comprising HCDR1-3 with amino acid sequences as set forth in SEQ ID NOs: 83, 84, and 85 respectively, and a VL comprising LCDR1-3 with amino acid sequences as set forth in SEQ ID NO: 87, SAS, and SEQ ID NO: 67 respectively;
a VH comprising HCDR1-3 with amino acid sequences as set forth in SEQ ID NOs: 89, 90, and 91 respectively, and a VL comprising LCDR1-3 with amino acid sequences as set forth in SEQ ID NO: 93, HGT, and SEQ ID NO: 94 respectively;
a VH comprising HCDR1-3 with amino acid sequences as set forth in SEQ ID NOs: 76, 77, and 78 respectively, and a VL comprising LCDR1-3 with amino acid sequences as set forth in SEQ ID NOs: 99, 77, and 100 respectively;
a VH comprising an amino acid sequence as set forth in SEQ ID NO: 1, and a VL comprising an amino acid sequence as set forth in SEQ ID NO: 5;
a VH comprising an amino acid sequence as set forth in SEQ ID NO: 8, and a VL comprising an amino acid sequence as set forth in SEQ ID NO: 12;
a VH comprising an amino acid sequence as set forth in SEQ ID NO: 14, and a VL comprising an amino acid sequence as set forth in SEQ ID NO: 15;
a VH comprising an amino acid sequence as set forth in SEQ ID NO: 17, and a VL comprising an amino acid sequence as set forth in SEQ ID NO: 18;
a VH comprising an amino acid sequence as set forth in SEQ ID NO: 19, and a VL comprising an amino acid sequence as set forth in SEQ ID NO: 18;
a VH comprising an amino acid sequence as set forth in SEQ ID NO: 21, and a VL comprising an amino acid sequence as set forth in SEQ ID NO: 25;
a VH comprising an amino acid sequence as set forth in SEQ ID NO: 28, and a VL comprising an amino acid sequence as set forth in SEQ ID NO: 32;
a VH comprising an amino acid sequence as set forth in SEQ ID NO: 35, and a VL comprising an amino acid sequence as set forth in SEQ ID NO: 38;
a VH comprising an amino acid sequence as set forth in SEQ ID NO: 39, and a VL comprising an amino acid sequence as set forth in SEQ ID NO: 43;
a VH comprising an amino acid sequence as set forth in SEQ ID NO: 46, and a VL comprising an amino acid sequence as set forth in SEQ ID NO: 49;
a VH comprising an amino acid sequence as set forth in SEQ ID NO: 52, and a VL comprising an amino acid sequence as set forth in SEQ ID NO: 56;
a VH comprising an amino acid sequence as set forth in SEQ ID NO: 59, and a VL comprising an amino acid sequence as set forth in SEQ ID NO: 60;
a VH comprising an amino acid sequence as set forth in SEQ ID NO: 61, and a VL comprising an amino acid sequence as set forth in SEQ ID NO: 65;
a VH comprising an amino acid sequence as set forth in SEQ ID NO: 68, and a VL comprising an amino acid sequence as set forth in SEQ ID NO: 72;
a VH comprising an amino acid sequence as set forth in SEQ ID NO: 75, and a VL comprising an amino acid sequence as set forth in SEQ ID NO: 79;
a VH comprising an amino acid sequence as set forth in SEQ ID NO: 82, and a VL comprising an amino acid sequence as set forth in SEQ ID NO: 86;
a VH comprising an amino acid sequence as set forth in SEQ ID NO: 88, and a VL comprising an amino acid sequence as set forth in SEQ ID NO: 92;
a VH comprising an amino acid sequence as set forth in SEQ ID NO: 95, and a VL comprising an amino acid sequence as set forth in SEQ ID NO: 96; and
a VH comprising an amino acid sequence as set forth in SEQ ID NO: 97, and a VL comprising an amino acid sequence as set forth in SEQ ID NO: 98;
wherein the CDR1, CDR2 and CDR3 sequences, the HCDR1, HCDR2 and HCDR3 sequences, and the LCDR1, LCDR2 and LCDR3 sequences are obtained based on an IMGT definition scheme;
or (4) a peptide comprising an amino acid sequence defined by SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, or SEQ ID NO: 129;
or (5) an aptamer comprising a nucleotide sequence defined by SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, or SEQ ID NO: 162.

58. The use according to any one of claims 49 to 57, wherein the recovery unit is obtained by immunizing a non-human animal with a conjugate of a small molecule drug and a carrier protein or a biotin-small molecule drug conjugate, or obtained by screening with phage display technology, or obtained by screening with virtual technology, or obtained by screening with SELEX technology, or obtained by screening with hybridoma technology, or obtained by screening with nanobody technology, or obtained by yeast display technology, or obtained by single B cell cloning technology, or obtained by microfluidic technology, or obtained by surface plasmon resonance assisted screening technology.

59. The use according to claim 58, wherein the small molecule drug includes, but is not limited to, MMAE, MMAD, DM1, DM2, DM4, eribulin, Exatecan, SN38, DXD, calicheamicin, pyrrolobenzodiazepine (PBD), indolinobenzodiazepine (IBD), duocarmycin, amanitin, Maaa-1181a, taxol, daunomycin, vinblastine, Doxorubicin, methotrexate, MMAF, ML022-Dxd, ML022-D633-004, ML026-Dxd, ML031-D633-004, ML037-D633-004, ML042-Dxd, ML047-DX8951, MPA-Dxd, and PEG12-Dxd; 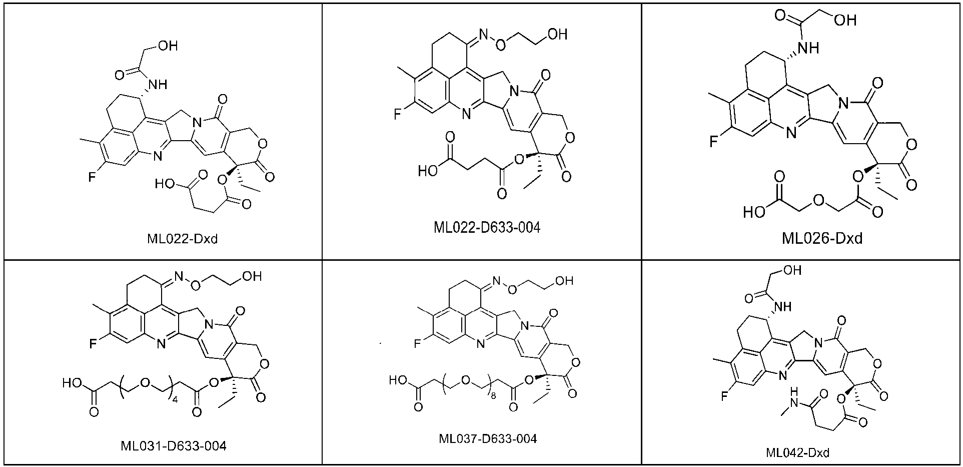 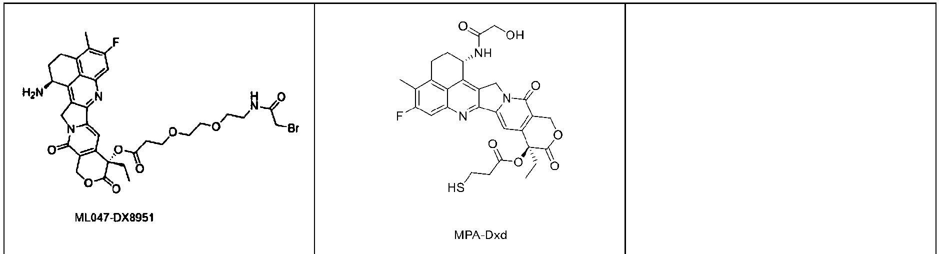 preferably, the carrier protein includes, but is not limited to, KLH, BSA and OVA.

60. The use according to claim 59, wherein the biotin-small molecule drug conjugate includes, but is not limited to, Biotin-PEG12-Dxd, Biotin-PEG10-Dxd, Biotin-PEG6-Dxd, Biotin-PEG6-Triazole-Dxd and Biotin-PEG10-Triazole-Dxd:

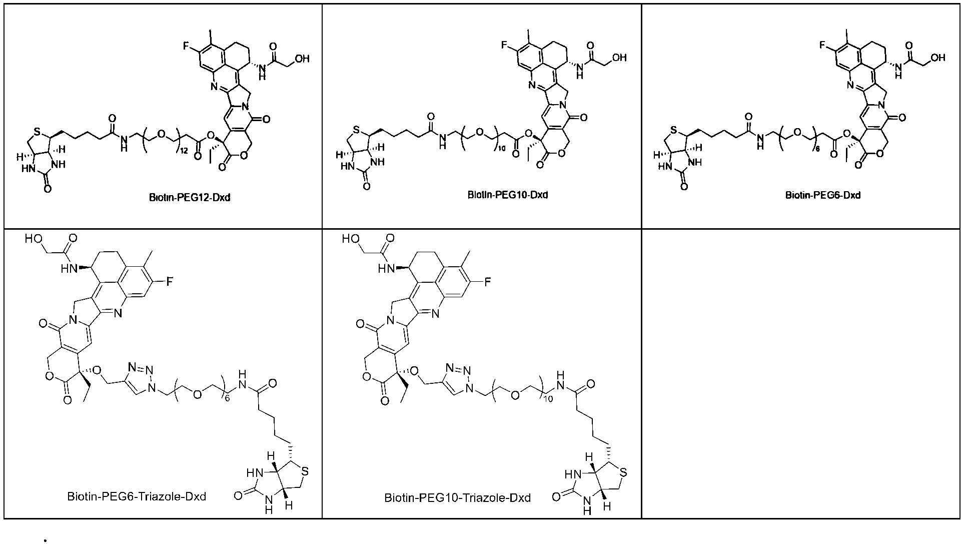

61. The use according to claim 49 or 50, wherein a payload epitope masking unit capable of preventing the recovery unit from binding to the payload that remains conjugated to the ligand-drug conjugate is linked to the linker or the payload.

62. The use according to claim 61, wherein the payload epitope masking unit does not interfere with the normal release of the payload after the ligand-drug conjugate enters a target cell.

63. A method for producing the ligand-drug conjugate according to any one of claims 1 to 46, comprising steps of:
1)screening to obtain an antibody, an antigen-binding fragment, a receptor, a fusion protein, a peptide, an oligopeptide, a peptidomimetic or an aptamer that specifically binds to the payload;
2)producing a ligand-drug conjugate using the screened antibody, antigen-binding fragment, receptor, fusion protein, peptide, oligopeptide, peptidomimetic or aptamer that specifically binds to the payload as a recovery unit;
3)detecting whether the screened antibody, antigen-binding fragment, receptor, fusion protein, peptide, oligopeptide, peptidomimetic or aptamer binds to the produced ligand-drug conjugate, and if not, a target ligand-drug conjugate is obtained; or detecting whether the antibody, antigen-binding fragment, receptor, fusion protein, peptide, oligopeptide, peptidomimetic, or aptamer binds to the produced drug-linker, and if not, a target ligand-drug conjugate is obtained;
4)if the screened antibody, antigen-binding fragment, receptor, fusion protein, peptide, oligopeptide, peptidomimetic or aptamer binds to the produced ligand-drug conjugate or the drug-linker, or molecular aggregation occurs between the produced ligand-drug conjugates or between the produced drug-linkers, then:
A. further modifying or replacing the antibody, antigen-binding fragment, receptor, fusion protein, peptide, oligopeptide, peptidomimetic or aptamer in the recovery unit until the obtained ligand-drug conjugates or drug-linkers do not aggregate and do not bind to the target ligand-drug conjugate or drug-linker; and/or
B. screening a payload epitope masking unit capable of being linked to the linker or payload of the ligand-drug conjugate, wherein the linked payload epitope masking unit is capable of preventing the screened antibody, antigen-binding fragment, receptor, fusion protein, peptide, oligopeptide, peptidomimetic or aptamer from binding to the antibody-drug conjugate and does not affect the normal release of the payload after the ligand-drug conjugate enters a cell; and after a suitable payload epitope masking unit is screened, producing a target ligand-drug conjugate.

64. A method for treating or preventing a disease, comprising administering a prophylactically or therapeutically effective amount of the ligand-drug conjugate according to any one of claims 1 to 46, or a pharmaceutically acceptable salt, deuterated form, or solvate thereof; or the pharmaceutical composition according to claim 48, or a pharmaceutically acceptable salt, deuterated form, or solvate thereof; and a pharmaceutically acceptable excipient thereof to a subject in need thereof.

65. The method according to claim 64, wherein the disease is a tumor.

66. Use of the ligand-drug conjugate according to any one of claims 1 to 46, or a pharmaceutically acceptable salt, deuterated form, or solvate thereof; or the pharmaceutical composition according to claim 43, in the manufacture of a medicament for treating or preventing a disease.

67. An immunogen for screening the recovery unit of claim 1.

68. The immunogen according to claim 67, wherein the immunogen includes, but is not limited to, MMAF, ML022-Dxd, ML022-D633-004, ML026-Dxd, ML031-D633-004, ML037-D633-004, ML042-Dxd, ML047-DX8951, MPA-Dxd, Biotin-PEG12-Dxd, Biotin-PEG10-Dxd, Biotin-PEG6-Dxd, Biotin-PEG6-Triazole-Dxd, or Biotin-PEG10-Triazole-Dxd, and a conjugate of a carrier protein with MMAF, ML022-Dxd, ML022-D633-004, ML026-Dxd, ML031-D633-004, ML037-D633-004, ML042-Dxd, ML047-DX8951, or MPA-Dxd.

69. The immunogen according to claim 67 or 68, wherein the carrier protein includes, but is not limited to, KLH, BSA, and OVA.

70. A payload recovery unit that can specifically bind to a free payload of a ligand-drug conjugate, comprising:
(1) a VHH domain with CDR1-3 selected from amino acid sequences defined by:
CDR1-3 as set forth in SEQ ID NOs: 103, 104, and 105;
or (2) an amino acid sequence selected from the amino acid sequence as set forth in SEQ ID NO: 102;
or (3) an amino acid sequence selected from combinations of VH and VL defined as follows:
a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 7 respectively;
a VH comprising HCDR1-3 with amino acid sequences as set forth in SEQ ID NOs: 9, 10, and 11 respectively, and a VL comprising LCDR1-3 with amino acid sequences as set forth in SEQ ID NO: 6, FAS, and SEQ ID NO: 13 respectively;
a VH comprising HCDR1-3 with amino acid sequences as set forth in SEQ ID NOs: 2, 3, and 4 respectively, and a VL comprising LCDR1-3 with amino acid sequences as set forth in SEQ ID NO: 6, YAS, and SEQ ID NO: 16 respectively;
a VH comprising HCDR1-3 with amino acid sequences as set forth in SEQ ID NOs: 2, 3, and 20 respectively, and a VL comprising LCDR1-3 with amino acid sequences as set forth in SEQ ID NO: 6, YAS, and SEQ ID NO: 7 respectively;
a VH comprising HCDR1-3 with amino acid sequences as set forth in SEQ ID NOs: 22, 23, and 24 respectively, and a VL comprising LCDR1-3 with amino acid sequences as set forth in SEQ ID NO: 26, WAS, and SEQ ID NO: 27 respectively;
a VH comprising HCDR1-3 with amino acid sequences as set forth in SEQ ID NOs: 29, 30, and 31 respectively, and a VL comprising LCDR1-3 with amino acid sequences as set forth in SEQ ID NO: 33, KVS, and SEQ ID NO: 34 respectively;
a VH comprising HCDR1-3 with amino acid sequences as set forth in SEQ ID NOs: 29, 36, and 37 respectively, and a VL comprising LCDR1-3 with amino acid sequences as set forth in SEQ ID NO: 33, KLS, and SEQ ID NO: 34 respectively;
a VH comprising HCDR1-3 with amino acid sequences as set forth in SEQ ID NOs: 40, 41, and 42 respectively, and a VL comprising LCDR1-3 with amino acid sequences as set forth in SEQ ID NO: 44, KVS, and SEQ ID NO: 45 respectively;
a VH comprising HCDR1-3 with amino acid sequences as set forth in SEQ ID NOs: 47, 48, and 42 respectively, and a VL comprising LCDR1-3 with amino acid sequences as set forth in SEQ ID NO: 50, KVS, and SEQ ID NO: 51 respectively;
a VH comprising HCDR1-3 with amino acid sequences as set forth in SEQ ID NOs: 53, 54, and 55 respectively, and a VL comprising LCDR1-3 with amino acid sequences as set forth in SEQ ID NO: 57, DTS, and SEQ ID NO: 58 respectively;
a VH comprising HCDR1-3 with amino acid sequences as set forth in SEQ ID NOs: 62, 63, and 64 respectively, and a VL comprising LCDR1-3 with amino acid sequences as set forth in SEQ ID NO: 66, SAS, and SEQ ID NO: 67 respectively;
a VH comprising HCDR1-3 with amino acid sequences as set forth in SEQ ID NOs: 69, 70, and 71 respectively, and a VL comprising LCDR1-3 with amino acid sequences as set forth in SEQ ID NO: 73, WAS, and SEQ ID NO: 74 respectively;
a VH comprising HCDR1-3 with amino acid sequences as set forth in SEQ ID NOs: 76, 77, and 78 respectively, and a VL comprising LCDR1-3 with amino acid sequences as set forth in SEQ ID NO: 80, GAS, and SEQ ID NO: 81 respectively;
a VH comprising HCDR1-3 with amino acid sequences as set forth in SEQ ID NOs: 83, 84, and 85 respectively, and a VL comprising LCDR1-3 with amino acid sequences as set forth in SEQ ID NO: 87, SAS, and SEQ ID NO: 67 respectively;
a VH comprising HCDR1-3 with amino acid sequences as set forth in SEQ ID NOs: 89, 90, and 91 respectively, and a VL comprising LCDR1-3 with amino acid sequences as set forth in SEQ ID NO: 93, HGT, and SEQ ID NO: 94 respectively;
a VH comprising HCDR1-3 with amino acid sequences as set forth in SEQ ID NOs: 76, 77, and 78 respectively, and a VL comprising LCDR1-3 with amino acid sequences as set forth in SEQ ID NOs: 99, 77, and 100 respectively;
a VH comprising an amino acid sequence as set forth in SEQ ID NO: 1, and a VL comprising an amino acid sequence as set forth in SEQ ID NO: 5;
a VH comprising an amino acid sequence as set forth in SEQ ID NO: 8, and a VL comprising an amino acid sequence as set forth in SEQ ID NO: 12;
a VH comprising an amino acid sequence as set forth in SEQ ID NO: 14, and a VL comprising an amino acid sequence as set forth in SEQ ID NO: 15;
a VH comprising an amino acid sequence as set forth in SEQ ID NO: 17, and a VL comprising an amino acid sequence as set forth in SEQ ID NO: 18;
a VH comprising an amino acid sequence as set forth in SEQ ID NO: 19, and a VL comprising an amino acid sequence as set forth in SEQ ID NO: 18;
a VH comprising an amino acid sequence as set forth in SEQ ID NO: 21, and a VL comprising an amino acid sequence as set forth in SEQ ID NO: 25;
a VH comprising an amino acid sequence as set forth in SEQ ID NO: 28, and a VL comprising an amino acid sequence as set forth in SEQ ID NO: 32;
a VH comprising an amino acid sequence as set forth in SEQ ID NO: 35, and a VL comprising an amino acid sequence as set forth in SEQ ID NO: 38;
a VH comprising an amino acid sequence as set forth in SEQ ID NO: 39, and a VL comprising an amino acid sequence as set forth in SEQ ID NO: 43;
a VH comprising an amino acid sequence as set forth in SEQ ID NO: 46, and a VL comprising an amino acid sequence as set forth in SEQ ID NO: 49;
a VH comprising an amino acid sequence as set forth in SEQ ID NO: 52, and a VL comprising an amino acid sequence as set forth in SEQ ID NO: 56;
a VH comprising an amino acid sequence as set forth in SEQ ID NO: 59, and a VL comprising an amino acid sequence as set forth in SEQ ID NO: 60;
a VH comprising an amino acid sequence as set forth in SEQ ID NO: 61, and a VL comprising an amino acid sequence as set forth in SEQ ID NO: 65;
a VH comprising an amino acid sequence as set forth in SEQ ID NO: 68, and a VL comprising an amino acid sequence as set forth in SEQ ID NO: 72;
a VH comprising an amino acid sequence as set forth in SEQ ID NO: 75, and a VL comprising an amino acid sequence as set forth in SEQ ID NO: 79;
a VH comprising an amino acid sequence as set forth in SEQ ID NO: 82, and a VL comprising an amino acid sequence as set forth in SEQ ID NO: 86;
a VH comprising an amino acid sequence as set forth in SEQ ID NO: 88, and a VL comprising an amino acid sequence as set forth in SEQ ID NO: 92;
a VH comprising an amino acid sequence as set forth in SEQ ID NO: 95, and a VL comprising an amino acid sequence as set forth in SEQ ID NO: 96; and
a VH comprising an amino acid sequence as set forth in SEQ ID NO: 97, and a VL comprising an amino acid sequence as set forth in SEQ ID NO: 98;
wherein the CDR1, CDR2 and CDR3 sequences, the HCDR1, HCDR2 and HCDR3 sequences, and the LCDR1, LCDR2 and LCDR3 sequences are obtained based on an IMGT definition scheme;
or (4) a peptide comprising an amino acid sequence defined by SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, or SEQ ID NO: 129;
or (5) an aptamer comprising a nucleotide sequence defined by SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, or SEQ ID NO: 162.

71. A method for reducing toxicity of a ligand-drug conjugate, wherein an effective amount of the corresponding payload recovery unit according to claim 70 capable of binding free payloads of the ligand-drug conjugate is administered to a patient undergoing a treatment with the ligand-drug conjugate.

72. The method according to claim 71, wherein the ligand-drug conjugate and the payload recovery unit can be administered simultaneously or sequentially.

73. A nucleic acid comprising a sequence encoding the amino acid sequence of the payload recovery unit according to claim 70.

74. A vector comprising the nucleic acid according to claim 73.

75. The vector according to claim 74, wherein the vector is an expression vector.

76. A cell comprising the vector according to claim 74 or claim 75.

77. The cell according to claim 76, wherein the cell is selected from the group consisting of a CHO cell, a COS cell, an HEK-293 cell, an NSO cell, a PER.C6^{®} cell, and an Sp2.0 cell.
